(19) Europäisches Patentamt
European Patent Office
Office européen des brevets

(11) Publication number: 0 271 829 B1

(12) # EUROPEAN PATENT SPECIFICATION

(45) Date of publication of patent specification: 25.08.93

(51) Int. Cl.5: C07C 257/00, C07C 271/06, C07C 233/45, C07C 243/26, C07D 295/12, C07D 295/18, C07F 7/18, A61K 31/155, A61K 31/27, A61K 31/16, A61K 31/195

(21) Application number: 87118314.1

(22) Date of filing: 10.12.87

(54) **Delta-hydroxy-beta-lysine derivatives and their production.**

(30) Priority: 10.12.86 JP 294432/86
02.12.87 JP 306382/87

(43) Date of publication of application:
22.06.88 Bulletin 88/25

(45) Publication of the grant of the patent:
25.08.93 Bulletin 93/34

(84) Designated Contracting States:
AT BE CH DE ES FR GB GR IT LI LU NL SE

(56) References cited:
EP-A- 0 153 720
EP-A- 0 229 313
CH-A- 653 015
FR-A- 2 408 579
GB-A- 1 553 416

(73) Proprietor: Takeda Chemical Industries, Ltd.
1-1, Doshomachi 4-chome
Chuo-ku, OSAKA(JP)

(72) Inventor: Masuya, Hiromoto 1-19 Matsuodai
4-chome
Inagawa-cho Kawabe-gun Hyogo
666-02(JP)
Inventor: Harada, Setsuo 3-31 Seiwadainishi
2-chome
Kawanishi
Hyogo 666-01(JP)
Inventor: Natsugari, Hideaki 11-601
1 Midori-cho
Ashiya, Hyogo 659(JP)

(74) Representative: von Kreisler, Alek, Dipl.-Chem. et al
Patentanwälte Von Kreisler-Selting-Werner,
Postfach 10 22 41, Bahnhofsvorplatz 1
D-50462 Köln (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

TETRAHEDRON, vol. 36, no. 7, 1980, Pergamon Press ,Oxford, New York, Toronto, Paris, Frankfurt, Sydney - André PIERDET et al.:"Synthese total de la (+/-)Negamycine", pages 1764-1766, 1768

JOURNAL OF THE AMERICAN CHEMICAL SOCIETY, vol. 93, no. 22, November 3, 1971 - Shinichi KONDO et al.:"Negamycin, a Novel Hydrazide Antibiotic", pages 6305-6306

## Description

This invention relates to a novel δ-hydroxy-β-lysine derivatives which is useful as a therapeutic agent for diseases caused by bacterial infection.

As antibiotics-having δ-hydroxy-β-lysine as a constituent amino acid have heretofore been known negamycin[2-(3R,5R)-3,6-diamino-5-hydroxyhexanoyl-1-methylhydrazinoacetic acid] [cf. H. Umezawa et al., The Journal of the American Chemical Society, 93, 6305 (1971)] (±)-negamycin (A. Pierdet et al., Tetrahedron, 36, 1763 (1980)], its 6-N-leucyl derivatives [cf. H. Umezawa et al., The Journal of Antibiotics, 24, 732 (1971)], and its carboxamide derivatives [cf. W. Streicher et al., The Journal of Antibiotics, 31, 725 (1978)], but no improvement is recognized in the antibiotics activity in these derivatives as compared with negamycin.

EP-A-0 229 313 discloses compounds related to antibiotic TAN-749 and the production thereof.

GB-A-1 553 416 discloses negamycin derivatives and the synthesis thereof.

Due to the development of the therapeutics using antibiotics, diseases caused by bacteria have been overcome to a considerable extent. However, increase of diseases due to changes in the flora of disease-causative bacteria (replacement of bacteria) or the advent of drug-resistant bacteria (acquisition of drug-resistance) resulting from long-term or high-dose medication with conventional antibiotics is still a serious problem in the field of medication of infectious diseases. For overcoming this problem, antibiotics having a novel structure and thus novel biological activities are always sought for in this field.

The present inventors, noting that compounds having δ-hydroxy-β-lysine as the constituent amino acid have antibiotic activities, attempted chemical modification in a broader extent and found that some of the derivatives showed excellent antibiotic activities, thus accomplishing the present invention.

More specifically, the present invention relates to

(1) A compound of the formula (I)

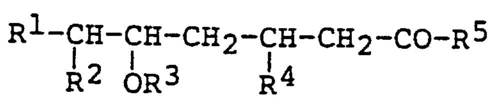

(I)

wherein

| | |
|---|---|
| $R^1$ | is 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino, |
| $R^2$ and $R^3$ | are hydrogen, |
| $R^4$ | is amino, methylamino, ethylamino, glycylamino, serylamino, alanylamino or 2-amino-O-methylpimelylamino, |
| $R^5$ | is 2-amidinoethylamino, 2-amidinopropylamino or 2-(N-methyl)amidinoethylamino or a salt thereof, |

(2) a method of preparing a compound of the formula (III)

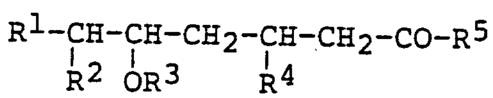

(III)

wherein

| | |
|---|---|
| $R^1$ | is 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino, |
| $R^2$ and $R^3$ | are hydrogen, |
| $R^4$ | is amino, methylamino, ethylamino, glycylamino, serylamino, alanylamino or 2-amino-O-methylpimelylamino, |
| $R^5$ | is 2-amidinoethylamino, 2-amidinopropylamino or 2-(N-methyl)amidinoethylamino, or a salt thereof, |

which comprises allowing a compound of the formula (II)

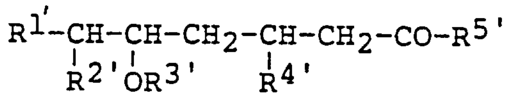

(II)

wherein $R^{2'}$ and $R^{3'}$ are hydrogen, $R^{1'}$ is amino or of the same meaning as $R^1$, and $R^{4'}$ is of the same meaning as $R^4$,

(a) to react with a compound capable of introducing

for $R^1$ 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,

for $R^4$ amino, methylamino, ethylamino, glycylamino, serylamino, alanylamino or 2-amino-O-methylpimelylamino

into a compound wherein at least one of $R^{1'}$ and $R^{4'}$ is amino or

(b) to react with a compound capable of introducing

for $R^5$ 2-amidinoethylamino, 2-amidinopropylamino or 2-(N-methyl)amidinoethylamino

into a compound wherein $R^{5'}$ is hydroxyl or a reactive derivative thereof at the carboxyl group.

A compound representable by the above general formulae, in case of its containing a carboxylic group, may form a salt with an inorganic base such as sodium, potassium, lithium, calcium, magnesium and ammonium, or an organic base such as pyridine, collidine, triethylamine and triethanolamine. Further, a compound representable by the above general formulae, in case of its containing a basic group such as amino group, may form a salt with an organic acid such as acetic acid, tartaric acid or methanesulfonic acid, with an inorganic acid such as hydrochloric acid, hydrobromic acid, sulfuric acid or phosphoric acid, or with an acidic amino acid such as aspartic acid or glutamic acid.

Next, the production method of the compound of this invention is described below.

The compound (III) can be obtanied by allowing the compound (II-1), which is a compound (II) wherein $R^{1'}$ is an amino group, to react with a compound capable of forming 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino, bonded through nitrogen by acylation.

A description of said reaction is given below.

The acylation of amino group can be achieved by allowing the starting compound to react with an acylating agent containing an acyl group in the group $R^{1'}$, for example a reactive derivative of carboxylic acid, in a solvent. As the reactive derivatives of carboxylic acid, use is made of, for example, acid halides, acid anhydrides, amido compounds, active esters and active thioesters. These reactive derivatives are specifically described in the following.

1) Acid halides:

Acid halides usable herein include, for example, acid chlorides and acid bromides.

2) Acid anhydrides :

As acid anhydrides, use is made of, for example, monoalkyl carbonic acid mixed anhydrides, mixed acid anhydrides composed of aliphatic carboxylic acid(e.g. acetic acid, pivalic acid, valeric acid, isovaleric acid or trichloroacetic acid), mixed acid anhydrides composed of aromatic carboxylic acid(e.g. benzoic acid) and symmetric-type acid anhydrides.

3) Amido compounds :

As amido compounds, use is made of, for example, compounds having an acyl group bonded to the nitrogen in the ring, such as pyrazole, imidazole, 4-substituted imidazole, dimethylpyrazole and benzotriazole.

4) Active esters :

As active esters, use is made of, for example, esters such as methyl esters, ethyl esters, methoxymethyl esters, propargyl esters, 4-nitrophenyl esters, 2,4-dinitrophenyl esters, trichlorophenyl esters,

pentachlorophenyl esters or mesylphenyl esters, as well as esters formed with 1-hydroxy-1H-2-pyridone, N-hydroxysuccinimide, N-hydroxyphthalimide or N-hydroxybenzotriazole.

5) Active thio esters :

As active thio esters, use is made of, for example, thioesters formed with a heterocyclic thiol such as 2-pyridylthiol and 2-benzthiazolylthiol.

Various reactive derivatives as described above can be properly selected depending on the type of the carboxylic acid.

This reaction may be, in some cases, carried out in the presence of a base. Bases which can be used for this purpose include tertiary amines such as aliphatic tertiary amines (e.g. trimethylamine, triethylamine, tripropylamine, tri-n-butylamine), N-methylpiperidine, N-methylpyrrolidine, cyclohexyldimethylamine and N-methylmorpholine), dialkylamine such as di-n-butylamine, diisobutylamine and dicyclohexylamine, aromatic amines such as pyridine, lutidine and $\gamma$-collidine, and hydroxides or carbonates of an alkali metal (e.g. lithium, sodium and potassium), or of an alkaline earth metal (e.g. calcium and magnesium).

In this method, the reactive derivative of carboxylic acid is employed usually in equivalent relative to the compound (II-1), but it may also be used in excess, as long as it does not interfere with the reaction. When a base is employed, the amount of the base is usually in an amount of 1 to 30 equivalents, preferably 1 to 10 equivalents relative to the compound (II-1), though it varies with the types of the starting compound (II-1), the kinds of the reactive derivatives of carboxylic acid and other reaction conditions then employed. This reaction is usually carried out in a solvent. As the solvent, use is made of, for example, ethers such as dioxane, tetrahydrofuran, diethylether, diisopropylether, propylene oxide and butylene oxide, esters such as ethyl acetate and ethyl formate, halogenated hydrocarbons such as chloroform, dichloromethane, 1,2-dichloroethane and 1,1,1-trichloroethane, hydrocarbons such as benzene, toluene and n-hexane, amides such as N,N-dimethylformamide and N,N-dimethylacetamide, or nitriles such as acetonitrile, and these organic solvents are used singly or in combination.

Of the above-mentioned bases, liquid ones can also be used for the dual purposes of base and solvent. Reaction temperature is not specifically limited, as long as the reaction proceeds, but the reaction is carried out usually within the range of from -50 °C to 150 °C, preferably from -30 °C to 80 °C. The reaction usually completes within several ten minutes to several ten hours, though the reaciton time varies with the types of the stating compound, the base, the reaction temperature and the solvent then employed, but, in some cases, it requires several ten days.

Compound (II-2), which is Compound (II) wherein $R^{1'}$ is of the same meaning as $R^1$ and $R^{4'}$ is amino group, can be converted to Compound (III) wherein $R^4$ is amino, methylamino, ethylamino, glycylamino, serylamino, alanylamino or 2-amino-O-methylpimelylamino, bonded through nitrogen, by subjecting the former to acylation or alkylation. The acylation can be carried out in the same manner as that for converting Compound (II-1) to Compound (III).

The alkylation of Compound (II-2) can be conducted by allowing Compound (II-2) to react with an alkylating agent containing a group bonded to the nitrogen of the group $R^{4'}$ through carbon. Examples of the alkylating agent include the halogenated alkyl compounds benzyl chloride, benzyl bromide, methyl iodide and ethyl iodide, the dialkylsulfates dimethyl sulfate and diethyl sulfate and the substituted sulfonic acid esters such as methyl mesylate, ethyl mesylate, methyl tosylate and ethyl tosylate. This reaction is usually conducted in a solvent such as water, methanol, ethanol, benzyl alcohol, benzene, N,N-dimethylformamide, tetrahydrofuran or acetonitrile. The reaction temperature ranges from 20 °C to 200 °C. and the reaction time ranges from 30 minutes to 50 hours. This reaction, by changing reaction conditions such as the molar ratio of the alkylating agent to Compound (II-2), permits the selective production of a secondary amine compound, a tertiary amine compound or a quaternary amine compound. Introduction of a different substituent into the relevant nitrogen is also possible by conducting the reaction stepwise. The reaction of introducing a group bonded through carbon, other than alkyl, can also be carried out in the same manner as described above.

The said alkylation can also be achieved by allowing Compound (II-2) to be bonded to a carbonyl compound in the presence of a reducing agent. As the reducing agent, use is made of lithium aluminium hydride, sodium cyanoborohydride, sodium borohydride, sodium, sodium amalgam and combinations of zinc and acid. This reaction can also be achieved by catalytic reduction using, for example, palladium, platinum, rhodium or the like as the catalyst.

Conversion of Compound (II-3), which is a compound (II) wherein $R^{1'}$ is of the same meaning as $R^1$ and $R^{4'}$ is amino, methylamino, ethylamino, glycylamino, serylamino, alanylamino or 2-amino-O-methylpimelylamino, bonded through nitrogen and $R^{5'}$ is hydroxyl group, to Compound (III) is achieved by

allowing the former to react with 2-amidinoethylamine, 2-amidinopropylamine or 2-(N-methyl)-amidinoethylamine. This reaction is carried out by allowing (II-3) or its reactive derivative at its carboxyl group [acid anhydride of the starting compound is synthesized from, for example, acid chloride (such as ethyl chlorocarbonate and benzyl chlorocarbonate), or acid anhydride (acetic anhydride and anhydrous trifluoroacetic acid)] to react with the above-mentioned amines. Alternatively, the above conversion can be achieved by allowing (II-3) to react with the above-mentioned amines in the presence of a dehydrative condensing agent such as dicyclohexylcarbodiimide, N-3-dimethylaminopropyl-N-ethylcarbodiimide or N-ethoxycarbonyl-2-ethoxy-1,2-dihydroquinoline. The above-mentioned reaction is conducted by allowing the reaction to proceed in a solvent(e.g. dichloromethane, tetrahydrofuran or N,N-dimethylformamide) at temperatures ranging from 0 °C to the refluxing temperature for 15 minutes to 16 hours, and the amount of amines is usually in excess relative to (II-3), i.e. 1 to 5 equivalents. Additionally, Compound (III) can be obtained by converting (II-3) to Compound (VII) represented by the formula

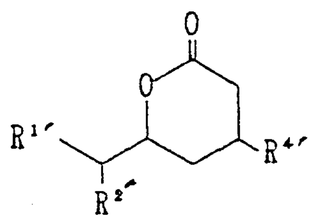

(VII)

wherein $R^{1'}$, $R^{2'}$, $R^{4'}$ are of the same meaning as defined above followed by allowing (VII) to react with amines. The conversion of (II-3) to (VII) can be conducted by allowing (II-3) to react with acid chloride, acid anhdyride or a dehydrative condensing agent as mentioned above.

Thus-obtained Compounds (III) may have protecting groups, those protecting groups can be removed upon necessity. The deprotection reaction can be carried out according to the conventional manner, and it can be achieved using a suitable method selected from routine methods such as those using an acid, those using a base, those using hydrazine and those by reduction, according to the type of the protective group or with the other conditions. In the case of the method using acid, examples of the acid include inorganic acids such as hydrochloric acid, sulfuric acid and phosphoric acid, organic acids such as formic acid, acetic acid, trifluoroacetic acid and propionic acid and, besides, acidic ion-exchange resin, while their selection varies with the type of the protective group and with the other conditions. In the case of the methods using a base, bases which can be used include inorganic bases such as hydroxides and carbonates of alkali metals(e.g. sodium potassium) or alkaline earth metals(e.g. calcium and magnesium) and organic bases such as metal alkoxides, organic amines and quaternary ammonium salts and, besides, basic ion-exchange resin, while their selection varies with the type of the protective group and the other factors. When a solvent is used in the case of the above-mentioned methods using an acid or a base, hydrophlic organic solvents, water or mixed solvents are frequently employed.

In the case of the methods by reduction, procedures which can be employed include the procedure using either a metal such as tin or zinc or a metal compound such as chrominum dichloride or chromium acetate in combination with an organic or inorganic acid such as acetic acid, propionic acid or hydrochloric acid and the procedure of reduction in the presence of a metal catalyst for catalytic reduction, while their selection varies with the type of the protective group and the other factors. Examples of the catalyst for catalytic reduction include platinum catalysts such as platinum wire, platinum sponge, platinum black, platinum oxide and colloidal platinum, palladium catalysts such as palladium sponge, palladium black, palladium oxide, palladium barium sulfate, palladium barium carbonate, palladium carbon, palladium silica gel and colloidal palladium, and reduced nickel such as nickel oxide, Raney nickel and Urushibara nickel. In the case of the reduction methods using a metal and an acid in combination, metals which can be used include iron and chromium, and acids which can be used include inorganic acids such as hydrochloric acid organic acids such as formic acid, acetic acid and propionic acid. The method by reduction is usually carried out in a solvent, for example, in catalytic reduction, ethyl acetate and alcohols such as methanol, ethanol, propyl alcohol and isopropyl alcohol are frequently employed. In the procedure of reduction using a metal and an acid in combination, water and acetone are frequently used, and when the acid is in a liquid state, the acid itself can be used as a solvent.

Reaction temperatures in the respective methods of using an acid or a base or by reduction range usually from those under cooling to those under warming.

Elimination of the protective group from each group of the compound obtained can be conducted in the same manner as described above.

In this invention, among the starting compound (II), some compounds are novel and they are included in the desired compound (I).

As the compound (II), for example, Antibiotic TAN-749 A, B, C and D (Compound I to IV in Table 1), their derivatives (Compound V to XXVI in Table 2) or the compounds which are chemically synthesized from the amino acids shown below are used.

Specific examples of the starting compound (II) in the present invention include antibiotics TAN-749-(A,B,C and D)(No.I to IV) (Table 1) and compounds derivable therefrom(No.V to XXVI) (Table 2), which can be produced by methods disclosed in Reference Examples described later.

TAN-749 is a novel antibiotic isolated from the culture broth of a strain belonging to the genus Pseudomonas, showing antibiotic actions against gram-positive and gram-nagative beacteria. The TAN-749 producing strain, Pseudomonas fluorescens YK-437 has been deposited at the Institute for Fermentation, Osaka (IFO) under the accession number of IFO 14446 since June 7, 1985. This microbe was deposited on June 15, 1985 also at Fermentation Research Institute, Agency of Industrial Science and Technology, Ministry of International Trade and Industry, Japan (FRI) under the accession number of FERM P-8312, the deposit being converted to a deposit under the Budapest Treaty, and has been stored at FRI under the accession number of FERM BP-1005.

Table 1

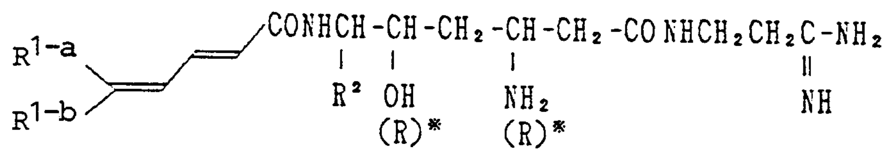

| Compd.No. | TAN-749 | $R^{1-a}$ | $R^{1-b}$ | $R^2$ |
|---|---|---|---|---|
| I | A | $-CH_3$ | -H | -H |
| II | B | $-CH_3$ | -H | $-CH_3$ |
| III | C | -H | $-CH_3$ | -H |
| IV | D | -H | $-CH_3$ | $-CH_3$ |

*: (R) shows steric configuration by R-S method.

Table 2

$$R^{1}-\underset{\displaystyle R^{2}}{\underset{|}{CH}}-\underset{\displaystyle \underset{(R)}{OH}}{\underset{|}{CH}}-CH_2-\underset{\displaystyle \underset{(R)}{R^{4}}}{\underset{|}{CH}}-CH_2-COR^{5}$$

| Compound No. | $R^1$ | $R^2$ | $R^4$ | $R^5$ |
|---|---|---|---|---|
| V | $CH_3CH=CH-CH=CH-CONH-$ | H- | $C_6H_5CONH-$ | $-NHCH_2CH_2C(=NH)NH_2$ |
| VI | 〃 | 〃 | $CH_3C(CH_3)_2OCONH-$ | 〃 |
| VII | 〃 | 〃 | $C_6H_5CH_2OCONH-$ | 〃 |
| VIII | $CH_3(CH_2)_4CONH-$ | H- | $-NH_2$ | 〃 |
| IX | 〃 | $CH_3-$ | 〃 | 〃 |
| X | 〃 | H- | $CH_3C(CH_3)_2OCONH-$ | 〃 |
| XI | 〃 | $CH_3-$ | 〃 | 〃 |
| XII | $NH_2-$ | H- | $-NH_2$ | 〃 |
| XIII | 〃 | 〃 | $CH_3C(CH_3)_2OCONH-$ | 〃 |
| XIV | 〃 | $CH_3-$ | 〃 | 〃 |
| XV | $CH_3CH=CH-CH=CH-CONH-$ | H- | $-NH_2$ | -OH |
| XVI | 〃 | $CH_3-$ | 〃 | 〃 |
| XVII | 〃 | H- | $CH_3C(CH_3)_2OCONH-$ | 〃 |
| XVIII | $CH_3(CH_2)_4CONH-$ | H- | $-NH_2$ | 〃 |
| XIX | 〃 | $CH_3-$ | 〃 | 〃 |
| XX | 〃 | H- | $CH_3C(CH_3)_2OCONH-$ | 〃 |
| XXI | 〃 | $CH_3-$ | 〃 | 〃 |
| XXII | 〃 | H- | $C_6H_5CH_2OCONH-$ | 〃 |
| XXIII | $NH_2-$ | H- | $-NH_2$ | -OH |
| XXIV | 〃 | $CH_3-$ | 〃 | 〃 |
| XXV | 〃 | H- | $CH_3C(CH_3)_2OCONH-$ | 〃 |
| XXVI | 〃 | $CH_3-$ | 〃 | 〃 |

In this invention, the starting compound (II) and (VII) may also be produced by subjecting the compound represented by the formula (IV)

$$R^{1'}-\underset{\displaystyle R^{2'}}{\underset{|}{CH}}-\underset{\displaystyle OR^{3'}}{\underset{|}{CH}}-CH_2-\underset{\displaystyle O}{\underset{\|}{C}}-CH_2CO-R^{5'} \qquad (IV)$$

wherein $R^{1'}$, $R^{2'}$, $R^{3'}$ and $R^{5'}$ are of the same meaning as defined above, or its lactone represented by the formula (IV-I)

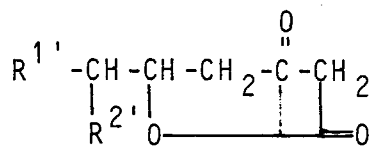

(IV-I)

wherein $R^{1'}$ and $R^{2'}$ are of the same meaning as defined above and a compound represented by the formula (V)

$H_2N$-A (V)

wherein A is hydrogen, hydroxyl, methyl or ethyl, to reduction reaction.

The reduction reaction proceeds by way of the compound represented by the formula (VIII) which is the dehydrated-condensate of (IV) and (V).

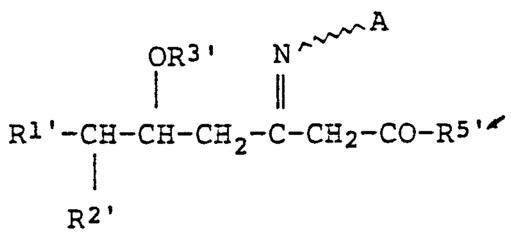

(VIII)

wherein $R^{1'}$, $R^{2'}$, $R^{3'}$, $R^{5'}$ and A are of the same meaning as defined above, $R^{3'}$ may form together with $R^{5'}$ a lactone compound of the formula (VIII-I)

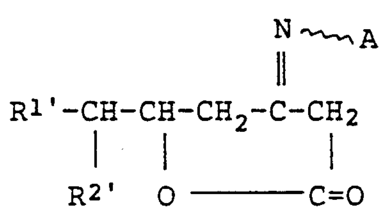

(VIII-1)

or the formula (VIII-2)

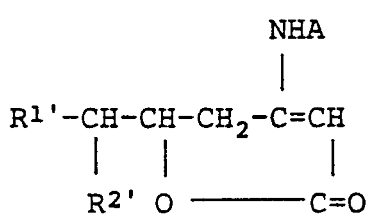

(VIII-2)

wherein $R^{1'}$, $R^{2'}$ and A are of the same meaning as defined above. Therefore, in some cases, it is advantageous to conduct the reduction reaction after isolating the dehydrated-condensate. The condensation reaction is usually conducted using the organic solvents such as methanol, ethanol, ethylether, tetrahydrofuran, dioxane, methylenechloride, chloroform, benzene, toluene, acetic acid, dimethylformamide or dimethylacetamide. The reaction temperature is preferably -20 °C to 100 °C.

The compound (V) may be used in the free form and also in the form of salt of acid such as acetic acid, phosphoric acid, hydrochloric acid or sulfuric acid in the reaction.

The compound (V) is usually used in the amount of 1 to 50 mol. relative to one mol of the compound (IV).

In some cases, the reaction advantageously proceeds in the presence of a dehydrating agent such as molecular sieves and phosphorus pentoxide, or a base such as pyridine, triethylamine, diisopropylethylamine, sodium hydrogencarbonate, potassium hydrogencarbonate, sodium carbonate and

potassium carbonate.

As reaction conditions for the reduction of the compounds (VIII), (VIII-1) or (VIII-2), those of the catalytic reductions using metals such as platinum, palladium, rhodium and Raney nickel, or mixtures of the above-mentioned metals and optional carriers such as carbon, barium sulfate, calcium sulfate, barium carbonate and calcium carbonate, those of the reductions using metal hydrides such as lithium alminum hydride, lithium borohydride, lithium cyanoborohydride, sodium borohydride and sodium cyanoborohydride, those of the reductions using metallic sodium and metallic magnesium in combination with alcohols, those of the reductions using the metals such as iron and zinc in combination with the acid such as hydrochloric acid and acetic acid, those of the electrolytic reductions or those of the reductions using a reduction-enzyme are mentioned.

The reduction reaction is usually conducted in the solvent used in the condensation reaction of the above-mentioned (IV) and (V). In some cases, the addition of the acid advantageously makes the reaction to advance. The acids are exemplified by formic acid, acetic acid, propionic acid, trichloro-acetic acid, trifluoro-acetic acid, oxalic acid, methane-sulfonic acid, p-tolune-sulfonic acid, hydrogen chloride, sulfuric acid, phosphoric acid, boron trifluoride ethylether and alminum chloride. In some cases, in the presence of said acids, the compound (VIII), (VIII-1) or (VIII-2) is hydrolyzed to the compound (IV) or (IV-1) by the concomitant or by-produced water. In some cases, the dehydrating agents such as molecular-sieves and phosphorus pentoxide are used in the reaction for preventing the cases. Though the reaction temperature varies with the types of the reduction, it is preferably -80 °C to 100 °C. Though the reaction proceeds sufficiently at atmospheric pressure, it may be conducted under pressure or reduced pressure.

In the reduction reaction, when the compound represented by the formula (V) and of which A is the hydroxyl group which may be substituted, is used, it is advantageous to subject the dehydrated-condensate (VIII), (VIII-1) or (VIII-2) prepared beforehand to the reduction reaction. In some cases, in the reduction, the compound representable by (VIII), (VIII-1) or (VIII-2) and of which A is hydrogen, is formed and then the reduction reaction proceed according to the types of the reducing agent.

The compounds (II) and (VII) obtained by the reduction has a free-amino group as $R^4$. The compound may be isolated as it is, and also it may be isolated after protecting the formed amino group. Examples of the protecting group include aromatic acyl groups including phthaloyl, 4-nitrobenzoyl, 4-tert-butylbenzoyl, 4-tert-butylbenzenesulfonyl, benzenesulfonyl and toluenesulfonyl, aliphatic acyl groups including formyl, acetyl, propionyl, monochloroacetyl, dichloroacetyl, trichloroacetyl, methanesulfonyl, ethanesulfonyl, trifluoroacetyl, malonyl and succinyl, esterified carboxyl groups including methoxycarbonyl, ethoxycarbonyl, t-butoxycarbonyl, isopropoxycarbonyl, 2-cyanoethoxycarbonyl, 2,2,2-trichloroethoxycarbonyl, benzyloxycarbonyl, 4-nitrobenzyloxycarbonyl, 4-methoxybenzyloxycarbonyl, diphenylmethyloxycarbonyl, methoxymethyloxycarbonyl, acetylmethyloxycarbonyl, isobornyloxycarbonyl and phenyloxycarbonyl, methylene group such as (hexahydro-1H-azepin-1-yl)methylene, sulfonyl group such as 2-amino-2-carboxyethylsulfonyl, and further, amino-protecting groups, other than acyl group, such as trityl, 2-nitrophenylthio, benzylidene, 4-nitrobenzylidene, di- or tri-alkylsilyl, benzyl and 4-nitrobenzyl. Selection of the above-mentioned protecting groups is not specifically limitative in the present invention, and specifically preferable ones being monochloroacetyl, benzyloxycarbonyl, 4-methoxybenzyloxycarbonyl and 4-nitrobenzyloxycarbonyl.

For the compounds (II) and (VII), stereoisomers based on the asymmetric center which newly formed at the substituted position of the formed amino group, exist.

The formation ratio varies with the substituents of the compound (IV) used as starting material or the conditions of the reduction reaction.

The isomers are isolated as the mixture or the single isomer separated from the mixture by the known method such as chromatography, crystallization and recrystallization, followed to be used in the production of the compound (I).

The compound (IV) or (IV-1) which is used as the starting material in the above method, is novel and can be produced by the reaction process shown below.

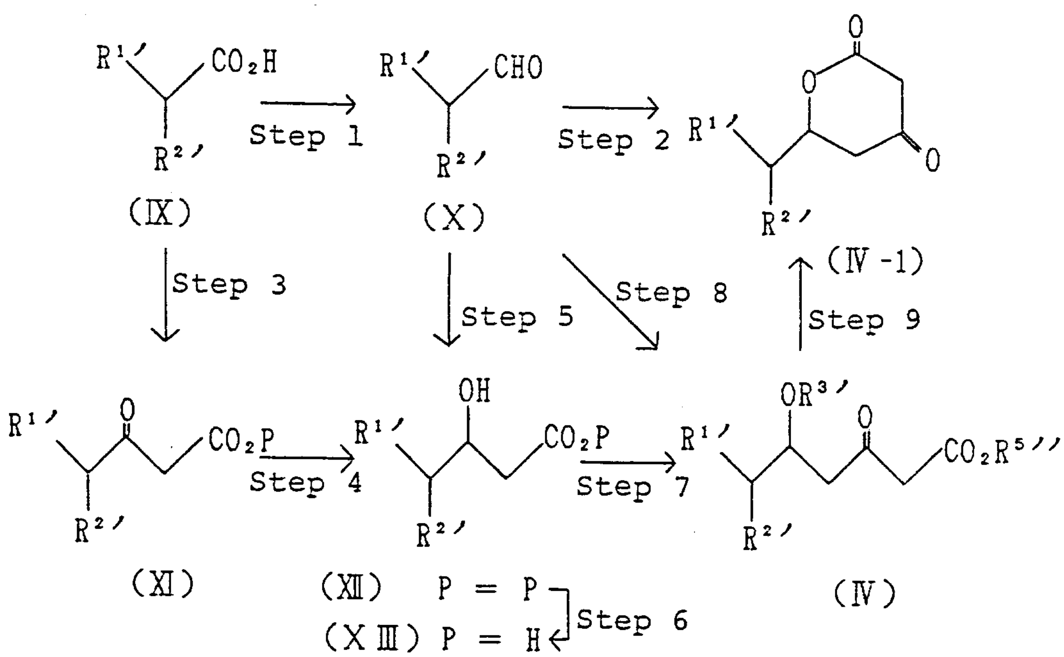


In the above chart, $R^{1'}$, $R^{2'}$, $R^{3'}$ and $R^{5'}$ are of the same meaning as defined above, and P is a protecting group of the carboxyl, which is exemplified by methyl, ethyl, n-propyl, isopropyl, tert-butyl, tert-amyl, benzyl, p-nitrobenzyl, p-methoxybenzyl, benzhydryl, phenacyl, phenyl, p-nitrophenyl, methoxymethyl, ethoxymethyl, benzyloxymethyl, acetoxymethyl, pivaloyloxymethyl, β-methylsulfonylethyl, β-trimethylsilylethyl, methylthiomethyl, trityl, β,β,β -trichloroethyl, β-iodoethyl, trimethylsilyl, dimethylsilyl, acetylmethyl, p-nitrobenzoylmethyl, p-mesylbenzoylmethyl, phthalimidomethyl, propionyloxymethyl, 1,1-dimethylpropyl, 3-methyl-3-butenyl, succinimidomethyl, 3,5-di tert-butyl-4-hydroxybenzyl, mesylmethyl, benzenesulfonylmethyl, phenylthiomethyl, dimethylaminoethyl, pyridine-1-oxide-2-methyl, methylsulfinylmethyl, bis(p-methoxyphenyl)methyl, 2-cyano-1,1-dimethylethyl or silyl groups.

As the starting material, the compound (IX) is used, which is derived from the known d-, ℓ- or dℓ-amino acids and of which each functional group other than the carboxyl group of the α-position is optionally protected.

Said amino acid is exemplified by glycine, alanine, 2-amino-butyric acid, valine, leucine, isoleucine, phenylalanine, tyrosine, serine, cysteine, threonine, methionine, aspartic acid, asparagine, glutamic acid, glutamine, arginine, lysine, histidine, proline and pyroglutamic acid.

In the step 1, the carboxyl group is converted to the aldehyde group, and the aldehyde compound (X) is synthesized by the known method [for example, Y. Hamada and T. Shioiri, Chemical Pharmaceutical Bulletin, 30, 1921 (1982)] or an analogous one thereto.

In the step 2, the compound (X) is subjected to a condensed-ring-formation reaction with the equivalent compound for the acetoacetic acid ester to obtain the compound (IV-1). The examples of the equivalent compound for the acetoacetic acid ester include the known diene such as 1,3-bis(trimethylsilyloxy)-1-methoxybuta-1,3-diene [P. Brownbridge et al., Canadian Journal of Chemistry, 61, 688 (1982)], 1,1-dimethoxy-3-trimethylsilyloxybuta-1,3-diene [J. Branville et al., Journal of Chemical Society, Perkin I, 1976, 1852] and 1,3-dimethoxy-1-trimethylsilyloxybuta-1,3-diene [J. Savard et al., Tetrahedron Letters, 1979, 4911]. Among them, 1,3-bis(trimethylsilyloxy)-1-methoxybuta-1,3-diene is preferably used.

In this reaction, the equivalent compound for the acetoacetic acid ester is used in an amount of 1 to 30 mol, preferably 1 to 10 mol relative to one mol of the compound (X).

This reaction is usually subjectjed in a solvent. The solvent is exemplified by ethers such as dioxane, tetrahydrofuran, diethylether and diisopropylether, esters such as ethyl acetate and ethyl formate, halogenated hydrocarbons such as chloroform, dichloromethane and 1,2-dichloroethane, hydrocarbons such as benzene, toluene and n-hexane, amides such as N,N-dimethylformamide and N,N-dimethylacetamide, nitriles such as acetonitrile.

The above-enumerated organic solvent is used simply or in combination, usually under anhydrous condition. In the ring-formation reaction, Lewis acid is preferably used as a catalyst. The examples of the Lewis acid include salts of tin such as stannous chloride, stannic chloride, stannous fluoride, stannous iodide, stannous oxalate and stannous trifluoromethanesulfonate , salts of zinc such as zinc chloride, zinc

bromide, zinc iodide and zinc acetate, salts of magnesium such as magnesium chloride, magnesium bromide, magnesium iodide and magnesium fluoride, salts of titanium such as titanium tetrachloride, titanium tetrabromide, salts of aluminum such as alaminum chloride, lanthanide compounds such as tris-(6,6,7,7,8,8,8-heptafluoro-2,2-dimethyl-3,5-octanedionate) europium [Eu(fod)$_3$], tris-[3-(heptafluoropropylhydroxymethylene)-d-campholate]europium [Eu(hfc)$_3$] and tris-(6,6,7,7,8,8,8-heptafluoro-2,2-dimethyl-3,5-octanedionate)ytterbium [Yb(fod)$_3$], and boron compound such as boron trifluoride ethylacetate.

The amount of the used catalyst is usually 0.00001 mol to 10 mol, preferably 0.0001 mol to 2 mol relative to one mol of the compound (X).

The reaction temperature is not limited so far as the reaction proceeds, however, it is usually -50 °C to 100 °C, preferably -20 °C to 35 °C. The reaction period varies with the types of the used starting materials and solvents, and the reaction period, however, the reaction usually proceeds within several 10 minutes to several 10 hours.

In the step 3, the carboxyl group of the compound (IX) is activated and then two carbon atoms are prolonged to obtain β-ketoester compound (XI) by the known production method, for example that described in T. Honori et al., European Journal of Medicinal Chemistry - Chimica Therapeutica, 13, 429 (1978); R. Steulmann et al., Liebigs Annalen der Chemie, 1975, 2245; B. D. Harris et al., Tetrahedron Letters, 28, 2837 (1987).

In the step 4, the carbonyl group of the compound (XI) is reduced to obtain β-hydroxyester compound (XII). As the reaction conditions, the same ones mentioned in the production of the compound (VI) from the compound (VIII), are mentioned. And in this reaction, the known method which is mentioned in the above-mentioned references in the step 3 is employed.

In this reduction reaction, in some cases, the reduction proceeds with high stereoselectivity (enantio and/or diastereo) by using a microorganisms or reduction enzyme thereof. As the microorganism or reduction enzyme thereof used for the reaction, any one which are able to reduce the compound (XI) to obtain the compound (XII) are used; however, Baker's yeast is preferably used.

As the reaction solution used for the reduction, the aqueous solution (pH 5 to 7) which may be buffer, containing 1 to 30 g/ℓ of the compound (XI) is preferable.

Said aqueous solution may contain 0 to 100 mℓ/ℓ of organic solvent such as alcohol, for example, methanol or ethanol.

When the reduction enzyme being used, the reaction is carried out by using an excess of the reduction enzyme. In the case of using the Baker's yeast, 1 to 500 weight parts, preferably 1 to 50 weight parts of that is used relative to one weight part of the compound (XI). The reaction temperature is 15 to 40 °C, preferably 20 to 35 °C and the reaction period is one hour to two weeks. When the microorganisms other than the Baker's yeast being used, the reaction may be carried out analogously to the case using the Baker's yeast. In the asymmetric reduction using the microorganism, it is preferable to add sugar such as sucrose or glucose into the reaction solution as carbon sources.

And also, if necessary, bactotrypton, yeast extract, ammonium sulfate and so on, may be added into the reaction solution. After the reaction, the compound (XII) can be extracted with the solvent such as ethyl acetate, ether or alcohol after destroying the cell bodies using surface-active agents, bacteriolytic enzymes or glass beads or without destroying the cell bodies. The extraction of the compound (XII) from the reaction solution of the asymmetric reduction using the reduction enzyme, can be similarly carried out.

In the above-mentioned reduction using the microorganism or the enzyme, in the formula (XI), it is preferable that $R^{2'}$ is hydrogen or lower alkyl such as methyl and ethyl, $R^{1'}$ is phenyl $C_{1-4}$ alkoxycarbonylamino such as benzyloxycarbonyl, or $C_{1-4}$ alkoxycarbonylamino such as t-butyloxycarbonylamino and P is $C_{1-6}$ alkyl such as methyl and ethyl. Said reduction reaction offers a novel production method of (R)-4-amino-3-hydroxybutyric acid or (3R,4S)- or (3S,4R)-4-amino-3-hydroxypentanoic acid and derivatives thereof, having a useful biological activity.

In the step 5, the acetic acid unit is added to the aldehyde group of the compound (X) to obtain the compound (XII) by the known method such as W.-S. Liu and G. I. Glover, Journal of Organic Chemistry, 43, 754 (1978); D. H. Lich, E. T. Sun, and A. S. Boparai, Journal of Organic Chemistry, 43, 3624 (1978); D. H. Lich, E. T. Sun, and E. Ulm, Journal of Medicinal Chemistry, 23, 27 (1980); K. E. Rittle, C. F. Hommick, G. S. Ponticello, and B. E. Evans, Journal of Organic Chemistry, 47, 3016 (1982); J. Boger, L. S. Payne, D. S. Perlow, N. S. Lohr, M. Poe, E. H. Blaine, E. H. Ulm, T. W. Schorn, B. I. LaMont, T.-Y. Lim, M. Kawai, D. H. Rich, and D. F. Veber, Journal of Medical Chemistry, 28, 1779 (1985); G. J. Hanson, J. S. Baran, and T. Lindberg, Tetrahedron Letters, 27, 3577 (1986); H. L. Sham, C. A. Rempel, H. Stein, adn J. Cohen, Journal of Chemical Society, Chemical Communication, 1987, 683; F. G. Salituro, N. Agarwal, T. Hofmann, and D. H. Rich, Journal of Medicinal Chemistry, 30, 286 (1987).

In the step 6, the protecting group P of the carboxyl group of the compound (XII) is eliminated to obtain the compound (XIII).

In the deprotection reaction, according to the types of the protecting group, the one using a base or acid or the one by reduction is selected, which are mentioned as the deprotection method of the above protecting groups.

In the step 7, the carboxyl group of the compound (XIII) is activated and then two carbon atoms are prolonged to obtain β-ketoester compound (IV) by the method similar with that in the above-mentioned step 3. In the step, the compound (XIII) (1 mol) is reacted with 1 to 2 mol of the reagent such as 1,1'-carbonyldiimidazole or 1,1'-carbonylbis(2-methylimidazole) in the solvent such as tetrahydrofuran, dimethoxyethane at 0°C to 50°C to a imidazolide compound. Then, without isolating the imidazolide compound, the resultant is reacted with one to 3 mol of the magnesium salt of the malonic acid derivative of the formula (XIV)

$(R^{5\prime}OCOCH_2COO)_2Mg$    (XIV)

wherein $R^{5\prime}$ is of the same meaning as defiend above at 0°C to 50° C for one to 48 hours.

In the step, the compound of which the hydroxyl group of the compound (XIII) is protected, may be used instead of the compound (XIII). Said compound is prepared by protecting the hydroxyl group of the compound (XII) obtained in the step 4 or 5 and then deprotecting the carboxyl group.

In the step 8, the acetoacetic unit is added to the aldehyde group of the compound (X) to obtain the compound (IV). As the source of acetoacetic unit, acetoacetic acid ester, diketene, etc. are used. As the reaction condition, when using acetoacetic ester, the similar one used in the step 5 is adapted, and when using diketene, the similar one used in the step 2 is adopted.

In the step 9, the protecting group $R^{5\prime}$ of the carboxyl group of the compound (IV), is eliminated to obtain the lactone compound (IV-1). The lactone-formation in the step readily proceeds and the dehydrative ring-formation usually occurs immediately after $R^{5\prime}$ is eliminated. Therefore, the same reaction condition in the step 6 is adopted.

For the compounds (IV), (IV-1), (XII) and (XIII) obtained, in the above method, stereoisomers (optical isomer, diastereomer) referring to the carbon atom substituted by $R^{1\prime}$ and the oxygen atom, exist. The formation ratio varies with the types of the used starting compound, the reaction conditon, etc. The isomer may be isolated by the known means such as pH-change, phase transfer, solvent extraction, chromatography, crystallization and recrystallization, and may be used as the mixture.

The object compound (I) of this invention may form a salt with an acid or a base. When (I) is obtained in a free form, it may be derived into the salt by a convenrional manner, and, when (I) is obtained in a form of salt, it may be converted into the free form by a conventional means.

Compound (I), in some cases, forms an internal salt, which is included in the present invention as well.

Stereoisomers of Compound (I), either singly or in an optional mixture thereof, can be used as medicines.

The object compound (I) thus obtained can be isolated and purified by per se known means such as concentration, liquid property conversion, phase transfer, solvent extraction, lyophilization, crystallization, recrystallization, fractional distillation and chromatography.

The object compound (I) has, in its basic skeleton, two asymmetric carbons, thus existing theoretically four types of stereoisomers, and each of such isomers as well as a mixture thereof are included in the present invention. In case where the substituents of Compound (I) have asymmetric carbons, steric isomers occur likewise, and each isomer as well as a mixture thereof are included in the present invention. When these isomers are mixedly produced by the above-mentioned reaction, the respective isomers can be isolated by conventional means such as various types of chromatography, recrystallization, etc. if necessary.

Thus obtained Compound (I) is useful as medicines, for example, antibiotics against certain types of gram-positive and gram-negative beacteria. The antibacterial activities against gram-positive bacteria(S. aureus 308A-1) are as shown in Table 3.

Table 3

| Compound | (No.) | MIC($\mu g/m\ell$)* | $ED_{50}$(mg/kg)** |
|---|---|---|---|
| 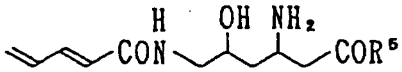 | (38b) | 100 | 6.25 |
| 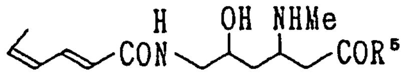 | (80) | >100 | 9.64 |
| 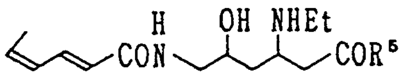 | (82) | >100 | 25 |
| 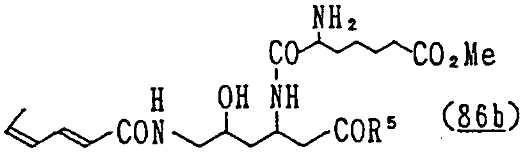 | (86b) | >100 | 25 |
| 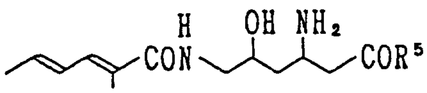 | (44b) | 100 | 21.5 |
| 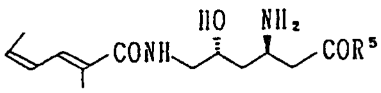 | (101 b) | >100 | 7.02 |
| 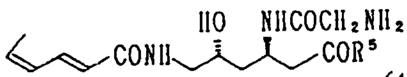 | (133 b) | >100 | 8.41 |
| 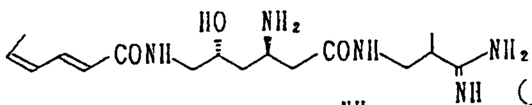 | (124 b) | >100 | 7.20 |
| 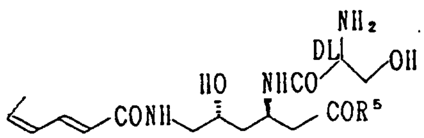 | (134 b) | >100 | 7.79 |
| 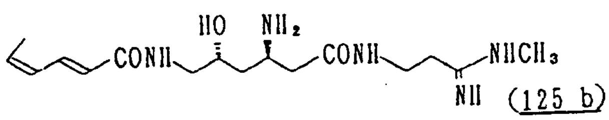 | (125 b) | >100 | 19.2 |

Table 3 (continued)

| Compound | (No.) | MIC(μg/mℓ)* | ED₅₀(mg/kg)** |
|---|---|---|---|
| 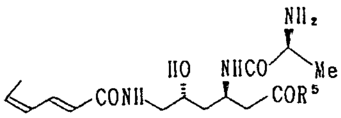 | (136 b) | >100 | 8.16 |
| 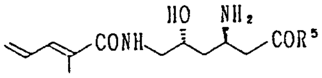 | (112 b) | >100 | 6.25 |
| 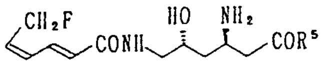 | (119 b) | >100 | 4.42 |
| 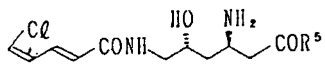 | (120 b) | >100 | 8.61 |
| Negamycin | | >100 | 38.6 |

*. Medium: Tripticase Soy Agar
Inoculum size : $10^{8}$ cfu/mℓ

**. mice, s.c.,

***$R^5$ = 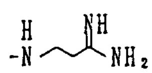

As is clear from the above data, Compound (I) or its salts of this invention have antibacterial activity and their toxicities are low, thus can be used as therapeutic agents or antibacterial agents for bacterial infections (e.g. respiratory infections, urinary tract infections, suppurative diseases, bile duct infections, intraintestinal infections, gynecological infections and surgical infections) in mammals (e.g. mice, rats, dogs, bovines, pigs and man).

The daily dosage of Compound (I) or a salt thereof is 1 to 100 mg/kg, more preferably 5 to 50 mg/kg, calculated on the basis of Compound (I).

Compound (I) or a pharmacologically acceptable salt thereof can be orally administered in combination with a suitable pharmacological allowable carrier, excipient and diluent in the dose form of, for example, tablet, granule, capsule or drop. They can also be parenterally administered in the dose form of an injection prepared by a conventional method with the use of a sterile carrier prepared by a conventional means.

For producing the above-mentioned oral pharmaceutical preparations, for example, tablets, there may be incorporated properly a binder (e.g. hydroxypropyl cellulose, hydroxypropylmethyl cellulose or macrogol), a disintegrating agent (e.g. starch or carboxymethylcellulose calcium), an excipient (e.g. lactose or starch) and a lubricant (e.g. magnesium stearate or talc).

For producing the above-mentioned parenteral pharmaceutical preparations, for example, injections, there may be properly incorporated an isotonicity (e.g. glucose, D-sorbitol, D-mannitol or sodium chloride), a preservative (e.g. benzyl alcohol, chlorobutanol, methyl para-hydroxybenzoate or propyl para-hydroxybenzoate) and a buffering agent ( e.g. phosphate buffer solution or sodium acetate buffer solution).

Examples

The present invention is hereinafter described in more detail with the following examples and reference examples, but these examples are nothing more than mere practical examples and not to be construed as limitations of the present invention, and may be varied as long as they do not deviate from the scope of the present invention.

Unless specifically stated, elution in column chromatography in examples and reference examples was carried out while observing by means of TLC (Thin Layer Chromatography). In TLC observation, Merck $60F_{254}$ was used as the TLC plate, the solvent used as the elution solvent for column chromatography was used as the developing solvent, and a UV detector was used for detection of the desired products. The detection method based on the phenomenon that after a series of treatments of 48% HBr spraying, hydrolysis while heating, ninhydrin reagent spraying and re-heating, the color of TLC plate spots corresponding to eluted fractions containing the desired products changes to red to red-purple, was used in combination with the UV detection method to identify the eluted fractions containing the desired products, which were then collected. When a mixture solvent was employed as the developing solvent, the mixing ratio was shown by volume.

Percentages used in the description of culture media show weight/volume %, unless otherwise specified.

In high performance liquid chromatography (hereinafter abbreviated as HPLC in some cases), YMC Packs S-30 and A312 (manufactured by Yamamura Chemical Laboratories) were used as the preparative carrier and the analytical carrier, respectively.

It should be noted that "room temperature" means usually 0 °C to 40 °C.

The symbols used in examples and reference examples have the meanings shown below.

| | |
|---|---|
| TFA | : trifluoroacetic acid |
| Boc | : t-butoxycarbonyl |
| Cbz | : benzyloxycarbonyl |
| MEM | : methoxyethoxymethyl |
| MOM | : methoxymethyl |
| THF | : tetrahydrofuran |
| DMSO | : dimethylsulfoxide |
| DMF | : N,N-dimethylformamide |
| EEDQ | : 1-ethoxycarbonyl-2-ethoxy-1,2-dihydroquinoline |
| DCC | : 1,3-dicyclohexylcarbodiimide |
| WSC | : 1-ethyl-3-(3-dimethylaminopropyl)carbodiimide |
| BOCON | : 2-t-butoxycarbonyloxyimino-2-phenylacetonitrile |
| HOBT | : 1-hydroxybenzotriazole |
| Hz | : Herz |
| J | : coupling constant |
| m | : multiplet |
| d | : doublet |
| s | : singlet |
| br | : broad |
| sh | : shoulder |

Reference example 1

Pseudomonas fluorescens YK-437 (IFO 14446, FERM BP-1005) grown on an enriched agar slant medium was inoculated into a 2ℓ Sakaguchi flask containing 500mℓ of a medium prepared by adding 0.5% precipitating calcium carbonate to an aqueous solution (pH 7.0) containing 2% glucose, 3% soluble starch, 1% unprocessed soybean flour, 0.3% corn steep liquor, 0.5% Polypepton (Daigo Nutritive Chemical, Japan) and 0.3% sodium chloride, after which it was subjected to reciprocal shaking culture at 24 °C for 48 hours. The entire quantity of the resulting culture liquid was then inoculated into a 50ℓ fermentor containing 30ℓ of a medium prepared by adding 0.05% Actcol (Takeda Chemical Industries, Japan), an antifoaming agent, to said medium, and cultured at 24 °C, with a 30ℓ/min. aeration rate and at 200 rpm for 48 hours. Six liters of the culture liquid was then inoculated into a 200ℓ fermentor containing 120ℓ of a medium containing 3% glycerol, 0.1% glucose, 0.5% Polypepton (Daigo Nutritive Chemicals, Japan), 0.5% meat extract (Wako Pure Chemical Industries, Japan), 0.5% sodium chloride, 0.05% sodium thiosulfate, 2 ppm cobalt chloride and 0.05% Actcol, after which it was incubated at 24 °C, with a 120ℓ/min. aeration rate and at 170 rpm for

66 hours.

The culture liquid (105ℓ), after being adjusted to pH 6.5 with 2N hydrochloric acid, was added to a Hyflo super Cel (Jones Manville Product, USA) and subjected to filtration and water washing, yielding a filtrate (102ℓ). The filtrate, after adjustment to pH 6.5, was passed through a column packed with IRC-50 ($Na^+$ type, 2ℓ). The column, after washing with water, was subjected to elution with a 2M saline solution (500ℓ). The resulting eluate was passed through a column packed with activated charcoal (2ℓ), washed with water, and then subjected to elution using an 8% isobutanol water solution (15ℓ) as an eluent. The eluate, after adjusting to pH 6.2, was concentrated to 2ℓ and then passed through a column packed with CM-Sephadex C-25 ($Na^+$ type, 0.5ℓ). Active fractions were then eluted using 0.1M brine (20ℓ) as an eluent.

A TAN-749B fraction appeared in the first half of the chromatogram and a TAN-749A fraction appeared in the last half of the chromatogram.

Each resulting fraction was subjected to chromatography using activated charcoal (1.0ℓ or 4.0ℓ) as the packing and then desalted, after which it was concentrated and lyophilized, yielding a crude TAN-749B product (4.0g) or a crude TAN-749A product (8.9g).

Crude product B (4.0g) was then subjected to reversed-phase high performance liquid chromatography for separation [Mobile phase: 8% methanol/0.02M phosphate solution (pH 3)], yielding an active fraction. The active fraction was subjected to column chromatography using CM-Sephadex C-25 ($Na^+$ type, 0.25ℓ) and then subjected to column chromatography using activated charcoal (0.3ℓ), yielding a purified fraction. The fraction was then concentrated and lyophilized, yielding TAN-749B dihydrochloride (0.66g) in the form of a white powder. Crude product A (8.9g) was treated with the same processes, yielding TAN-749A dihydrochloride in the form of a white powder (4.7g).

Reference example 2

Pseudomonas fluorescens YK-437 (IFO 14446, FERM BP-1005) grown on an enriched agar slant meidum was inoculated into a 2ℓ Sakaguchi flask containing 500mℓ of a medium prepared by adding 0.5% precipitating calcium carbonate to an aqueous solution containing 2% glucose, 3% soluble starch, 1% unprocessed soybean powder, 0.3% corn steep liquor, 0.5% Polypepton, and 0.3% sodium chloride, after which it was subjected to reciprocal shaking culture at 24 °C for 48 hours. The entire quantity of the culture liquid was inoculated into a 200ℓ fermentor containing 120ℓ of a medium prepared by adding 0.05% Actcol, an antiforming agent, to the said medium, and then incubated at 24 °C, with a 120ℓ/min. aeration rate and at 180 rpm for 48 hours. Fifty liters of the resulting culture liquid was inoculated into a 2,000ℓ fermentor containing 1,200ℓ of a medium containing 3% glycerol, 0.1% glucose, 0.5% Polypepton, 0.5% meat extract, 0.5% sodium chloride, 0.05% sodium thiosulfate, 2 ppm cobalt chloride and 0.05% Actcol, after which it was incubated at 24 °C, with a 1,200ℓ/min. aeration rate and at 150 rpm for 66 hours.

The culture liquid obtained (1,150ℓ), after adjusting to pH 6.5, was added to a Hyflo super cell and the subjected to filtration and water washing, yielding a filtrate (1,220ℓ). The filtrate, after adjustment to pH 6.2, was passed through a column packed with IRC-50 ($Na^+$ type, 20ℓ). The column, after washing with water, was subjected to elution using a 0.5M hydrochloric acid solution (200ℓ) as an eluent. The eluate, after adjusting to pH 5.6, was passed through a column packed with Diaion SP-207 (20ℓ) and then subjected to elution with water (120ℓ). The eluate was concentrated to 2ℓ; the concentrate was passed through a column packed with CG-50 ($NH_4{}^+$ type, 3ℓ). Active fractions were then eluted using a 0.4 ~ 0.6M brine (40 ℓ) as an eluent.

TAN-749A, B, C and D fractions appeared in the first half of the chromatogram and a TAN-749A fraction appeared in the last half.

Each resulting fraction was subjected to chromatography using activated charcoal (1.2ℓ or 2.0ℓ) as the packing, and eluted with an 8% isobutanol water solution (4ℓ or 10ℓ). The fraction containing TAN-749A alone was concentrated and lyophilized, yielding TAN-749A (47.5g).

Three separate lots (corresponding to 3,450ℓ of the initial culture liquid) or the fraction containing TAN-749A, B, C and D, obtained by the same process, were concentrated in a lump, yielding a concentrate. The concentrate (2ℓ) was then subjected to column chromatography using CG-50 ($NH_4{}^+$, 3ℓ) as the packing. The column, after washing with a 0.2M brine, was subjected to elution using a 0.5~0.8M brine (40ℓ). A fraction containing TAN-749B and D appeared in the first half of the chromatogram and one containing TAN-749A and C appeared in the last half. The fraction containing TAN-749A and C was subjected to chromatography using activated charcoal as the packing and desalted. The eluate was concentrated and lyophilized, yielding a TAN-749A powder (20g) containing a small quantity of TAN-749C.

The fraction containing TAN-749B and D was subjected to chromatography using activated charcoal as the packing and desalted. The eluate was subjected to column chromatography using CM-Sephadex C-25

($Na^+$ type, 1ℓ) as the packing and a 0.2M brine as an eluent. The eluate was concentrated, and the concentrate was subjected to reversed-phase HPLC for separation [Mobile phase: 5% methanol/0.02M phosphoric acid buffer solution (pH 3.0)], yielding two fractions, i.e. a fraction containing TAN-749B alone and one containing TAN-749B and D. The fraction containing TAN-749B alone was subjected to chromatography using CM-Sephadex and then activated charcoal as packings, yielding TAN-749B (3.05g). The fraction containing TAN-749B and D was concentrated and then subjected to HPLC again. The fraction containing TAN-749D alone was then concentrated. The concentrate was passed through a column packed with IRA-402 ($Cℓ^-$ type, 10mℓ) and the column was washed with water. The eluate and the wash solution were subjected to chromatography using activated charcoal for desalting, yielding TAN-749D (15.5mg).

The TAN-749A powder (3g) containing a small quantity of TAN-749C, obtained above, was subjected to chromatography using CM-Sephadex, Amberlite CG-50 (Rohm & Haas Co., U.S.A.) and then activated charcoal as packings to increase the ratio of TAN-749C content. The resulting powder with a high TAN-749C content was purified via two repetitions of HPLC under the conditions shown above, yielding TAN-749C (20.2mg).

Reference example 3

XXII

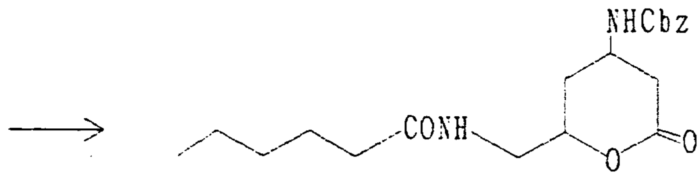

XXVII

To a solution of Compound XXII (0.5 g) in water (10 mℓ) were added THF(10 mℓ), $NaHCO_3$ (0.48 g) and benzyloxycarbonyl chloride(0.49 g), and the mixture was stirred at room temperture for 1.5 hours. To the reaction solution was added a small volume of pyridine to decompose excess amount of benzyloxycarbonyl chloride, followed by addition of 30 mℓ of ethyl acetate. With 2N HCℓ, the pH was adjusted to 2. The aqueous layer was separated, and the aqueous layer was subjected to extraction with ethyl acetate. The organic layers were combined, washed with a saturated brine, and then dried over magnesium sulfate, followed by concentration under reduced pressure. The concentrate was dissolved in 10 mℓ of methylene chloride. To the solution was added 0.57 g of WSC, then the reaction was allowed to proceed at room temperature for one hour. The reaction solution was washed with water, then dried over magnesium sulfate, followed by concentration. To the concentrate was added ether, then precipitating crystals were collected by filtration to afford Compound XXVII (0.51 g) as colorless needles, m.p. 111-112°C.

$$IR\ \nu_{max}^{KBr}\ cm^{-1}\ :\ 1732,\ 1680,\ 1638,\ 1535$$

| Elemental Analysis for $C_{20}H_{28}N_2O_5$ : | | | |
|---|---|---|---|
| Calcd. | C, 63.81; | H, 7.50; | N, 7.44 |
| Found | C, 63.93; | H, 7.44; | N, 7.48 |

Reference example 4

The dihydrochloride of compound I (approx. 200mg) was dissolved in water (20mℓ) and sodium bicarbonate (0.73g) and benzoyl chloride (0.175mℓ) were added, after which the solution was stirred at room temperature. With the disappearance of benzoyl chloride and pH reduction in the reaction mixture, both benzoyl chloride and triethylamine were added properly so that the reaction mixture was maintained at a pH value of approx. 8.3. Some 5 hours later, the reaction liquid was washed twice with ethyl acetate (35mℓ), adjusted to pH 2.0 with 2N HCℓ, and washed 3 times with ethyl acetate (30mℓ). The washing, after adjusting to pH 6.7 with 3N sodium hydroxide, was concentrated and adsorbed to a column packed with

Diaion HP-20 (50 ∼ 100 mesh, 20mℓ). The column was washed with water (120mℓ), after which the adsorbed concentrate was eluted with a 5% methanol water solution, a 20% methanol water solution, a 50% methanol water solution and 50% methanol-0.008N HCℓ (each 60mℓ) sequentially to fractionate into 20mℓ portions of the eluate. Each fraction was subjected to high performance liquid chromatography [Mobile phase: 50% methanol/0.01M phosphoric acid solution (pH 3)]; the fractions exhibiting a single peak were combined together and concentrated, after which the concentrate was lyophilized, yielding the hydrochloride of compound V in the form of a white powder (167mg).
Optical rotation: $[\alpha]_D^{23}$ - 40.7° (c = 0.46, water)

| Elemental analysis ($C_{22}H_{31}N_5O_4 \cdot HC\ell \cdot 1.0H_2O$) | | | | |
|---|---|---|---|---|
| Calcd.<br>Found | C; 54.60,<br>C; 54.52, | H; 7.08,<br>H; 6.92, | N; 14.47,<br>N; 14.41, | Cℓ; 7.33<br>Cℓ; 7.66 |

Reference example 5

The dihydrochloride of compound I (1.25g, 83% purity) was dissolved in 50% dioxane-water (50mℓ) and both triethylamine (0.5mℓ) and BOC-ON (1.1g) were added. The mixture was stirred at room temperature for 5.5 hours while adding triethylamine to maintain pH of the mixture at approx. 8.5. The reaction mixture, after adjustment to pH 7.2 with 2N HCℓ, was concentrated to remove dioxane. Water (200mℓ) was added, after which the concentrate was washed with ethyl acetate-ethyl ether (1:1, 200mℓ). The organic layer was separated and further extracted with water (150mℓ). The extract was combined with the water layer and concentrated. The concentrate, after adjustment to pH 6.8, was passed through a column packed with Diaion HP-20 (50 ∼ 100 mesh, 70mℓ). Elution was then carried out with water (210mℓ), 50% methanol-water (210mℓ) and 50% methanol-1/200 N HCℓ (280mℓ) sequentially to fractionate into 70mℓ portions of the eluate. Each fraction was analyzed by high performance liquid chromatography [Mobile phase: 50% methanol/0.01M phosphoric acid solution (pH 3)]. The fractions exhibiting a single peak were combined together and concentrated, after which the concentrate was lyophilized, yielding the hydrochloride of compound VI (834 mg).
Optical rotation: $[\alpha]_D^{25}$ - 23.7° (c = 0.52, water)

| Elemental analysis ($C_{20}H_{35}N_5O_5 \cdot HC\ell \cdot 1.5H_2O$) | | | | |
|---|---|---|---|---|
| Calcd.<br>Found | C; 49.12,<br>C; 49.08, | H; 8.04,<br>H; 8.07, | N; 14.32,<br>N; 14.44, | Cℓ; 7.25<br>Cℓ; 7.26 |

Reference example 6

The dihydrochloride of compound I (776mg) was dissolved in 3% sodium bicarbonate water solution (40mℓ) and carbobenzoxy chloride (798μℓ) was added, after which the solution was stirred at room temperature for 5 hours. The reaction mixture, after adjustment to pH 2, was diluted with water (50mℓ) and washed with ethyl acetate (100mℓ x 2). The aqueous layer, after adjustment to pH 4.5, was concentrated and subjected to column chromatography using Diaion HP-20 (50 ∼ 100 mesh, 40mℓ) as the packing. After washing with water (100mℓ) and then with 10% methanol-water (100mℓ), the concentrate was subjected to fractional elution using sequentially 50% methanol-water (100mℓ) and 50% methanol-1/200 N HCℓ - (150mℓ). Each fraction was analyzed by high performance liquid chromatography [Mobile phase: 60% methanol/0.01M phosphoric acid solution (pH 3)]. The fractions exhibiting a single peak were combined together and concentrated. The concentrate was then lyophilized, yielding the hydrochloride of compound VII (728mg).
Optical rotation: $[\alpha]_D^{22}$ - 41.7° (c = 0.55, water)

| Elemental analysis ($C_{23}H_{33}N_5O_5 \cdot HCl \cdot 0.5H_2O$) | | | | |
|---|---|---|---|---|
| Calcd.<br>Found | C; 54.70,<br>C; 55.02, | H; 6.99,<br>H; 6.85, | N; 13.87,<br>N; 14.06, | Cℓ; 7.02<br>Cℓ; 7.29 |

Reference example 7

The dihydrochloride of compound I (20.0g, approx. 97% purity) was dissolved in water (500mℓ) and 10% palladium-carbon (2.0g) was added, after which the solution was stirred at room temperature in hydrogen flow for some 4 hours. The reaction mixture was subjected to filtration to remove the catalyst; the filtrate was concentrated and lyophilized, yielding the dihydrochloride of compound VIII in the form of a white powder (19.0g).

Optical rotation: $[\alpha]_D^{23.5}$ − 5.2° (c = 0.60, water)

| Elemental analysis ($C_{15}H_{31}N_5O_3 \cdot 2HCl \cdot 1.0H_2O$) | | | | |
|---|---|---|---|---|
| Calcd.<br>Found | C; 42.86,<br>C; 42.88, | H; 8.39,<br>H; 8.84, | N; 16.66,<br>N; 16.75, | Cℓ; 16.87<br>Cℓ; 17.26 |

Reference example 8

The dihydrochloride of compound II (1.04g, 94% purity) was dissolved in water (100mℓ) and 10% palladium-carbon (104mg) was added, after which the solution was stirred at room temperature in hydrogen flow for some 80 minutes. The reaction mixture was subjected to filtration to remove the catalyst; the filtrate, after concentration, was passed through a column packed with activated charcoal (70mℓ). Sequential elution was then carried out using water (350mℓ) and then an 8% isobutanol water solution (500 mℓ) as eluents to fractionate into 70mℓ portions of the eluate. Each fraction was analyzed by high performance liquid chromatography [Mobile phase: 25% methanol/0.01M phosphoric acid solution (pH 3)]. The fractions exhibiting a single peak were combined together and concentrated, after which the concentrate was lyophilized, yielding the dihydrochloride of compound IX in the form of a white powder (899mg).
Optical rotation: $[\alpha]_D^{24}$ + 28.9° (c = 0.57, water)

| Elemental analysis ($C_{16}H_{33}N_5O_3 \cdot 2HCl \cdot 0.5H_2O$) | | | | |
|---|---|---|---|---|
| Calcd.<br>Found | C; 45.18,<br>C; 44.96, | H; 8.53,<br>H; 8.82, | N; 16.46,<br>N; 16.46, | Cℓ; 16.67<br>Cℓ; 17.10 |

Reference example 9

The dihydrochloride of compound VIII (19.3g, 80% purity) was dissolved in 50% dioxane-water (400mℓ) and both triethylamine (7.65mℓ) and BOC-ON (13.5g) were added. The mixture was stirred at room temperature for 5 hours, while triethylamine was added to maintain a pH value of approx. 8.5. The reaction mixture, after adjustment to pH 6.5 with 2N HCℓ, was concentrated to remove dioxane. The concentrate was diluted with water (900mℓ) and washed with ethyl acetate-ethyl ether (1:1, 800mℓ). The organic layer was separated and then further extracted with water (500mℓ). The extract was combined with the water layer and concentrated, after which the concentrate was adjusted to pH 5.6 and then passed through a column packed with Diaion HP-20 (50 ∼ 100 mesh, 450mℓ). Sequential elution was then carried out using a series of water (1.35ℓ), 50% methanol-water (1.35ℓ) and 50% methanol-1/200 N HCℓ (1.8ℓ) to fractionate into 450mℓ portions of the eluate. Each fraction was analyzed by high performance liquid chromatography [Mobile phase: 60% methanol/0.01M phosphoric acid solution (pH 3)]. The fractions exhibiting a single peak were combined together and concentrated, after which the concentrate was lyophilized, yielding the

hydrochloride of compound X (13.4g).
Optical rotation: $[\alpha]_D^{25}$ - 13.3° (c = 0.67, water)

| Elemental analysis ($C_{20}H_{39}N_5O_5 \cdot HC\ell$) | | | | |
|---|---|---|---|---|
| Calcd.<br>Found | C; 51.55,<br>C; 51.22, | H; 8.65,<br>H; 9.06, | N; 15.03,<br>N; 14.82, | Cℓ; 7.61<br>Cℓ; 7.64 |

Reference example 10

The dihydrochloride of compound IX (700mg) was dissolved in a 50% dioxane-water solution (28mℓ) and both triethylamine (0.28mℓ) and BOC-ON (455mg) were added. The mixture was then stirred at room temperature for some 8 hours while a pH value of approx. 8.8 was maintained using triethylamine. The reaction mixture was concentrated to remove dioxane. The concentrate, after dilution with water (100mℓ), was washed twice with ether-hexane (5:1, 100mℓ). The organic layer was separated and extracted with water (150mℓ). The extract was combined with the water layer; the mixture, after adjustment to pH 7, was concentrated and then adsorbed to Diaion HP-20 (50 ~ 100 mesh, 30mℓ). Sequential elution was then carried out using a series of water, a 20% methanol water solution, a 50% methanol water solution and 50% methanol-0.005N dilute HCℓ water solution (each 120mℓ) to fractionate into 20mℓ portions of the eluate. Each fraction was analyzed by high performance liquid chromatography [Mobile phase: 60% methanol/0.01M phosphoric acid solution (pH 3)]. The fractions exhibiting a single peak were combined together and concentrated. The concentrate was then lyophilized, yielding the hydrochloride of compound XI in the form of a white powder (487mg).
Optical rotation: $[\alpha]_D^{23}$ + 23.1° (c = 0.38, water)

| Elemental analysis ($C_{21}H_{41}N_5O_5 \cdot HC\ell \cdot H_2O$) | | | | |
|---|---|---|---|---|
| Calcd.<br>Found | C; 51.57,<br>C; 51.40, | H; 8.86,<br>H; 9.05, | N; 14.32,<br>N; 14.21, | Cℓ; 7.25<br>Cℓ; 7.21 |

Reference example 11

The dihydrochloride of compound VIII (202mg) was dissolved in a 0.03M phosphate buffer solution (pH 7.0, 100mℓ). After the addition of bacterial cells (10g) of Pseudomonas acidovorans IFO 13582, the solution was shaken at 37°C for 15 hours. The reaction mixture was centrifuged; the resulting supernatant, after adjustment to pH 7.0, was passed through a column packed with Amberlite CG-50 (100 ~ 200 mesh, $H^+$ type, Rohm & Hass, USA, 40mℓ). After washing the column with a series of water (200mℓ) and 0.01N HCℓ (160mℓ), fractional elution was carried out using 0.02N HCℓ as the eluent. Each fraction was analyzed by high performance liquid chromatography [Mobile phase: 30% acetonitrile/0.01M octanesulfonate-0.02M phosphoric acid solution (pH 3)]. The fractions whose main constituent was Compound 12, i.e. the desired product, were combined together and concentrated. The concentrate was then lyophilized, yielding a crude powder (147mg). This crude powder was dissolved in a small amount of water and passed through a column packed with Diaion HP-20 (50 ~ 100 mesh, 40mℓ). Fractional elution was then carried out. Each fraction was analyzed by high performance liquid chromatography [Mobile phase: 30% acetonitrile/0.01M octanesulfonate-0.02M phosphoric acid solution )pH 3)]. The fractions exhibiting a single peak were combined together and concentrated, after which the concentrate was lyophilized, yielding the trihydrochloride of compound XII (118mg).
Optical rotation: $[\alpha]_D^{25}$ - 7.6° (c = 0.45, water)

| Elemental analysis ($C_9H_{21}N_5O_2 \cdot 3HC\ell \cdot 2H_2O$) | | | | |
|---|---|---|---|---|
| Calcd.<br>Found | C; 28.70,<br>C; 28.58, | H; 7.49,<br>H; 7.19, | N; 18.59,<br>N; 18.32, | Cℓ; 28.23<br>Cℓ; 28.41 |

Reference example 12

The hydrochloride of compound X (10.0g) was dissolved in a 0.03M phosphate buffer solution (ph 7.0, 5.0ℓ). After the addition of bacterial cells (500g) of Pseudomonas acidovorance, the solution was shaken at 37°C for 15 hours. The reaction mixture was centrifuged; the supernatant, after adjusting to pH 7.3, was passed through a column packed with IRC-50 ($Na^+$ type, 1ℓ). After washing the column with water (4ℓ), fractional elution was carried out using sequentially 0.5M brine (8ℓ) and 1.0M brine (5ℓ). Each fraction was analyzed by high performance liquid chromatography [Mobile phase: 15% methanol/0.01M phosphoric acid solution (pH 3)]. The fractions exhibiting a single peak were combined together and passed through a column packed with charcoal powder (0.8ℓ). After washing the column with water (2ℓ), elution was carried out using a series of 8% isobutanol-water (4ℓ) and 8% isobutanol-1/100 N HCℓ (3ℓ). The eluate, after concentration, was lyophilized, yielding the dihydrochloride of compound XIII (6.82g).
Optical rotation: $[\alpha]_D^{22}$ - 1.6° (c = 0.90, water)

| Elemental analysis ($C_{14}H_{29}N_5O_4 \cdot 2HCℓ \cdot 0.5H_2O$) | | | | |
|---|---|---|---|---|
| Calculated<br>Found | C; 40.68,<br>C; 40.62, | H; 7.80,<br>H; 8.40, | N; 16.94,<br>N; 17.04, | Cℓ; 17.15<br>Cℓ; 17.76 |

Reference example 13

The hydrochloride of compound XI (400mg) was dissolved in a 0.03M phosphate buffer solution (pH 7.0, 200mℓ). After adding bacterial cells (18g) of Pseudomonas acidovorans, the solution was shaken at 37°C for 25 hours. The reaction mixture was centrifuged and the supernatant was passed through a column packed with IRC-50 ($NH_4^+$ type, 30mℓ). Elution was then carried out using water (100 mℓ), a 0.5M saline solution (150mℓ) and 1.0M brine (100mℓ) sequentially to fractionate into 20mℓ portions of the eluate. Each resulting fraction was analyzed by high performance liquid chromatography [Mobile phase: 15% methanol/0.01M phosphoric acid solution (pH 3)]. The fractions exhibiting a single peak were combined together and concentrated. The concentrate was passed through a column packed with activated charcoal (20mℓ), after which it was eluted with water (100mℓ) and then with an 8% isobutanol-water solution (100mℓ) to fractionate into 20mℓ portions of the eluate. Each fraction was analyzed by high performance liquid chromatography [Mobile phase: 15% methanol/0.01M phosphoric acid solution (pH 3)]. The fractions exhibiting a single peak were combined together and concentrated. The concentrate was then lyophilized, yielding the dihydrochloride of compound XIV in the form of a white powder (271mg).
Optical rotation: $[\alpha]_D^{21}$ + 4.0° (c = 0.55, water)

| Elemental analysis ($C_{15}H_{31}N_5O_4 \cdot 2HCℓ \cdot 0.5H_2O$) | | | | |
|---|---|---|---|---|
| Calcd.<br>Found | C; 42.16,<br>C; 42.23, | H; 8.02,<br>H; 8.61, | N; 16.39,<br>N; 16.33, | Cℓ; 16.59<br>Cℓ; 16.78 |

Reference example 14

The dihydrochloride of compound I (50.2g, 83% purity) was dissolved in 2N hydrochloric acid (500mℓ) and refluxed in an oil bath for 15 minutes under heating at 130°C. The refluxed solution was concentrated to evaporate hydrochloric acid and diluted with water (60mℓ), after which it was adjusted to pH 6.8 with 1N aqueous sodium hydroxide and concentrated. The concentrate was then passed through a column packed with Diaion HP-20 (50 ~ 100 mesh, 10ℓ) and eluted with water (3ℓ) and then with a 10% methanol water solution (5ℓ) to fractionate 1ℓ portions of the eluate. Each fraction was analyzed by high performance liquid chromatography [Mobile phase: 20% methanol/0.01M phosphoric acid solution (pH 3)], after which the fractions exhibiting a single peak were combined together, concentrated, and lyophilized, yielding compound XV in the form of a powder (1.79g).
Optical rotation: $[\alpha]_D^{21}$ - 22.3° (c = 0.52, water)

| Elemental analysis ($C_{12}H_{20}N_2O_4 \cdot 0.5H_2O$) | | | |
|---|---|---|---|
| Calcd. | C; 54.33, | H; 7.98, | N; 10.56 |
| Found | C; 53.81, | H; 8.11, | N; 10.46 |

Reference example 15

The dihydrochloride of compound II (1.25g, 86% purity) was dissolved in 2N hydrochloric acid (125mℓ) and refluxed in an oil bath for 18 hours under heating at 124°C. The reaction mixture was then cooled to room temperature and concentrated to evaporate hydrochloric acid. The concentrate, after diluting with water, was adjusted to pH 6.8 with 1N aqueous sodium hydroxide. The diluted solution, after concentration, was passed through a column packed with Diaion HP-20 (50 ~ 100 mesh, 150mℓ) and eluted sequentially with water (500mℓ), a 10% methanol water solution, a 20% methanol water solution and a 40% methanol water solution (each 450mℓ) to fractionate into 150mℓ portions of the eluate. Each fraction was analyzed by high performance liquid chromatography [Mobile phase: 25% methanol/0.01M phosphoric acid solution (pH 3)]. The fractions exhibiting a single peak were combined together and concentrated, after which the concentrate was lyophilized, yielding compound XVI in the form of a white powder (449mg).
Optical rotation: $[\alpha]_D^{24}$ + 84.7° (c = 0.42, water)

| Elemental analysis ($C_{13}H_{22}N_2O_4 \cdot 1.0H_2O$) | | | |
|---|---|---|---|
| Calcd. | C; 54.15, | H; 8.39, | N; 9.72 |
| Found | C; 54.55, | H; 8.15, | N; 9.72 |

Reference example 16

Compound XV (3.4g) was dissolved in a 50% acetone-water solution (114mℓ) and both triethylamine (7.35mℓ) and BOC-ON (3.92g) were added, after which the solution was stirred at room temperature for about 5 hours. After evaporation of acetone, the reaction mixture was adjusted to pH approx. 8.8 by the addition of sodium bicarbonate (1.2g) and then washed with ethyl ether (100mℓ) 3 times. The water layer, after adjusting to pH 2.0 with 1N HCℓ, was extracted 4 times with ethyl acetate (100mℓ). The extracts were combined together, washed twice with brine (100mℓ), dried with anhydrous sodium sulfate, and then concentrated to dryness, yielding a colorless, oily substance, which was crystallized from an ethyl acetate-ethyl ether-hexane yielding compound XVII in the form of white crystals (4.12g). Melting point: 128.5°C
Optical rotation: $[\alpha]_D^{26}$ - 26.5° (c = 0.50, methanol)

| Elemental analysis ($C_{13}H_{28}N_2O_6$) | | | |
|---|---|---|---|
| Calcd. | C; 57.29, | H; 7.92, | N; 7.86 |
| Found | C; 57.34, | H; 7.72, | N; 7.98 |

Reference example 17

Compound XV (600mg) was dissolved in water (60mℓ) and 10% palladium-carbon (60mg) was added, after which the solution was stirred at room temperature in hydrogen flow for 3.5 hours. The reaction mixture was subjected to filtration, after which the filtrate was adjusted to pH 7.0 and concentrated. The concentrate was subjected to column chromatography using Diaion HP-20 (50 ~ 100 mesh, 180mℓ) as the packing. After washing the column with water (720mℓ), fractional elution was carried out using 15% methanol-water (540mℓ) and then 25% methanol-water (540 mℓ) as eluents. Each fraction was analyzed by high performance liquid chromatography [Mobile phase: 40% methanol/0.01M phosphoric acid solution (pH 3)]. The fractions exhibiting a single peak were combined together and concentrated, after which the concentrate was lyophilized, yielding compound XVIII in the form of a powder (416mg).
Optical rotation: $[\alpha]_D^{22}$ - 13.9° (c = 0.51, water)

| Elemental analysis ($C_{12}H_{24}N_2O_4$) | | | |
|---|---|---|---|
| Calcd. | C; 55.36, | H; 9.29, | N; 10.76 |
| Found | C; 55.22, | H; 9.20, | N; 10.87 |

Reference example 18

Compound XVII (383mg) was dissolved in a solution of 0.1N NaOH (10.7mℓ) in water (30mℓ), and 10% palladium-carbon (40mg) was added. The mixture was stirred at room temperature in hydrogen atmosphere for 5 hours. The reaction mixture was subjected to filtration, after which the filtrate was adjusted to pH 7.0 and concentrated. The concentrate was subjected to column chromatography using Diaion HP-20 (50 ~ 100 mesh, 30mℓ) as the packing. After washing the column with water (120mℓ), fractional elution was carried out using 10% methanol-water (60mℓ) and then 50% methanol-water (200mℓ). Each fraction was analyzed by high performance liquid chromatography [Mobile phase: 65% methanol/0.01M phosphoric acid solution (pH 3)]. The fractions exhibiting a single peak were combined together and concentrated, after which the concentrate was lyophilized, yielding the sodium salt of compound XX (356mg).
Optical rotation: $[\alpha]_D^{23}$ - 7.7° (c = 0.48, water)

| Elemental analysis ($C_{17}H_{31}N_2O_6Na$) | | | |
|---|---|---|---|
| Calcd. | C; 53.39, | H; 8.17, | N; 7.33 |
| Found | C; 53.06, | H; 8.01, | N; 7.39 |

Reference example 19

Compound XVI (706mg) was dissolved in water (70mℓ) and 10% palladium-carbon (70mg) was added. The mixture was then stirred at room temperature in a hydrogen flow for about 1 hour. The reaction liquid was subjected to filtration to separate the catalyst. The filtrate, after concentration, was crystallized from a water-acetone system, yielding compound XIX in the form of white crystals (647mg).
Melting point: 204°C (decomposition)
Optical rotation: $[\alpha]_D^{21}$ + 31.8° (c = 0.57, water)

| Elemental analysis ($C_{13}H_{26}N_2O_4$) | | | |
|---|---|---|---|
| Calcd. | C; 56.91, | H; 9.55, | N; 10.21 |
| Found | C; 56.80, | H; 9.82, | N; 10.13 |

Reference example 20

Compound XVIII (320mg) was dissolved in a 3% sodium bicarbonate water solution (25mℓ) and carbobenzoxy chloride (332μℓ) was added, after which the mixture was stirred at room temperature for 6 hours. The reaction mixture, after adjusting to pH 2, was extracted with ethyl acetate. After washing with water, the organic layer was dried with anhydrous sodium sulfate and then concentrated. Ethyl etherhexane was added to the concentrate, yielding compound XXII in the form of a powder (389mg).
Optical rotation: $[\alpha]_D^{22}$ 0° (c = 0.49, methanol)

| Elemental analysis ($C_{20}H_{30}N_2O_6$) | | | |
|---|---|---|---|
| Calcd. | C; 60.90, | H; 7.67, | N; 7.10 |
| Found | C; 60.85, | H; 7.53, | N; 7.15 |

Reference example 21

Compound XIX (468mg) was dissolved in a 50% acetone water solution (16mℓ) and both triethylamine (0.95mℓ) and BOC-ON (504mg) were added, after which the mixture was stirred at room temperature for about 3 hours. After evaporating acetone and triethylamine by concentration, the reaction mixture was adjusted to pH 8.4 by adding sodium bicarbonate (160mℓ) and water (20mℓ). The diluted solution was washed 3 times with ethyl ether (30mℓ), after which it was adjusted to pH 2.2 with 2N HCℓ and then extracted 3 times with ethyl acetate (40mℓ). The ethyl acetate layers were combined, washed twice with saturated brine (20mℓ), dried over anhydrous sodium sulfate, and then concentrated to dry, yielding a colorless, oily substance. This substance was then crystallized from an ether-hexane system, yielding compound XXI in the form of white crystals (423mg).
Melting point: 102 ~ 103°C
Optical rotation: $[\alpha]_D^{23}$ + 36.0° (c = 0.45, methanol)

| Elemental analysis ($C_{18}H_{34}N_2O_6$) | | | |
|---|---|---|---|
| Calcd.<br>Found | C; 57.73,<br>C; 57.81, | H; 9.15,<br>H; 9.21, | N; 7.48<br>N; 7.47 |

Reference example 22

The dihydrochloride of compound I (1.94g) was dissolved in 2N HCℓ (193mℓ) and refluxed for 6 hours under heating. After cooling, the reaction mixture was washed 3 times with chloroform (200mℓ), concentrated to evaporate hydrochloric acid, and diluted with water (55mℓ). The solution was passed through a column packed with Dowex 50W-X2 ($H^+$ type, 50 ~ 100 mesh, 100mℓ) and eluted with a series of water (300mℓ), 0.5N HCℓ, 0.8N HCℓ, 1.0N HCℓ, 1.2N HCℓ and 1.5N HCℓ (each 400mℓ) to fractionate into 100mℓ portions of the eluate. Each fraction was analyzed by thin-layer chromatography, after which the fractions containing the desired compound were combined together and concentrated. The concentrate was diluted with water to make 30mℓ and again passed through a column packed with Dowex 50W-X2 ($H^+$ type, 50 ~ 100 mesh, 30mℓ). The effluent was further eluted with a series of 0.8N HCℓ, 0.9N HCℓ and 1.0N HCℓ (each 150mℓ) to fractionate into 30mℓ tions of the eluate. Each fraction was analyzed in the same procedure as above. The fractions exhibiting a single spot were combined together, concentrated, and then lyophilized, yielding the dihydrochloride of compound XXIII in the form of a powder (301mg).
Optical rotation: $[\alpha]_D^{25}$ - 18.3° (c = 0.85, water)

| Elemental analysis ($C_6H_{14}N_2O_3 \cdot 2HC\ell$) | | | | |
|---|---|---|---|---|
| Calcd.<br>Found | C; 30.65,<br>C; 30.30, | H; 6.86,<br>H; 7.13, | N; 11.92,<br>N; 12.07, | Cℓ; 30.16<br>Cℓ; 30.57 |

Reference example 23

Compound XVI (310mg) was dissolved in 2N HCℓ (31mℓ) and refluxed for 6 hours under heating. After cooling, the reaction mixture was washed 3 times with chloroform (40mℓ) and concentrated to evaporate hydrochloric acid. The concentrate, after diluting with water (18mℓ), was passed through a column packed with Dowex 50W-X2 ($H^+$ type, 50 ~ 100 mesh, 17mℓ) and eluted with a series of water (50mℓ), a 0.2% aqueous ammonia (50mℓ), a 0.3% aqueous ammonia (60mℓ) and a 0.4% aqueous ammonia (60mℓ) to fractionate into 17mℓ portions of the eluate. Each fraction was analyzed by thin-layer chromatography. The fractions exhibiting a single spot were combined together, concentrated, and then lyophilized, yielding Compound 24 in the form of a powder (174mg). 38mg of the powder was dissolved in water (3.8mℓ), adsorbed to Dowex 50W-X2 ($H^+$ type, 50 ~ 100 mesh, 5mℓ), and eluted with a series of water (15mℓ), 0.5N HCℓ, 0.8N HCℓ, 0.9N HCℓ and 1.0N HCℓ (each 20mℓ) to fractionate into 5mℓ portions of the eluate. Each fraction was analyzed by thin-layer chromatography. The fractions exhibiting a single spot were combined together, concentrated, and then lyophilized, yielding the dihydrochloride of compound XXIV (39mg).
Optical rotation: $[\alpha]_D^{23}$ - 2.7° (c = 0.58, water)

| Elemental analysis ($C_7H_{16}N_2O_3 \cdot 2HCℓ$) | | | | |
|---|---|---|---|---|
| Calcd.<br>Found | C; 34.02,<br>C; 33.81, | H; 6.53,<br>H; 7.81, | N; 11.34,<br>N; 11.20, | Cℓ; 28.69<br>Cℓ; 29.52 |

Reference example24

The sodium salt of compound XX (280mg) was dissolved in a 0.03M phosphate buffer solution (pH 7.0, 140mℓ) and bacterial cells (14g) of Pseudomonas acidovorans were added, after which the mixture was shaken at 37°C for 20 hours. The reaction mixture was centrifuged; the supernatant, after adjusting to pH 7.0, was concentrated. The concentrate was subjected to column chromatography using Diaion HP-20 (50 ~ 100 mesh, 140mℓ) as the packing and eluted with a series of water (900mℓ) and 5% methanol-water (500mℓ). Each fraction was analyzed by high performance liquid chromatography [Mobile phase: 25% methanol/0.01M phosphoric acid solution (pH 3)]. The fractions exhibiting a single peak were combined together and concentrated. The concentrate was then lyophilized, yielding compound XXV in the form of a white powder (145mg).
Optical rotation: $[\alpha]_D^{23}$ + 10.3° (c = 0.48, water)

| Elemental analysis ($C_{11}H_{22}N_2O_5$) | | | |
|---|---|---|---|
| Calcd.<br>Found | C; 50.37,<br>C; 49.91, | H; 8.45,<br>H; 8.54, | N; 10.68<br>N; 10.57 |

Reference example 25

Compound XXI (390mg) was suspended in a 0.03M phosphate buffer solution (pH 7.0, 200mℓ) and bacterial cells (40g) of Pseudomonas acidovorans were added, after which the mixture was shaken at 37°C for 18 hours. The reaction mixture was centrifuged; the supernatant, after adjusting to pH 7.0, was concentrated. The concentrate was subjected to column chromatography using Diaion HP-20 (50 ~ 100 mesh, 140mℓ) as the packing and then eluted with a series of water (700mℓ) and 5% methanol-water (700mℓ) to fractionate into 140mℓ portions of the eluate. Each fraction was analyzed by high performance liquid chromatography [Mobile phase: 25% methanol/0.01M phosphate solution (pH 6.3)]. The fractions exhibiting a single peak were combined together and concentrated. The concentrate was then lyophilized, yielding compound XXVI in the form of a white powder (94mg).

Optical rotation: $[\alpha]_D^{21.5}$ + 19.3° (c = 0.45, water)

| Elemental analysis ($C_{12}H_{24}N_2O_5 \cdot 0.5H_2O$) | | | |
|---|---|---|---|
| Calcd.<br>Found | C; 50.51,<br>C; 50.53, | H; 8.83,<br>H; 8.71, | N; 9.82<br>N; 9.82 |

Reference example 26

The hydrochloride of compound VI (9.5mg) was dissolved in 1N aqueous sodium hydroxide (0.95mℓ) and stirred at room temperature for 60 hours. After the completion of the reaction, the reaction mixture was diluted and analyzed by high performance liquid chromatography [Mobile phase: 60% methanol/0.01M phosphoric acid solution (pH 3)]: it was found compound XVII was produced in an amount of 3.8mg.

Reference example 27

The hydrochloride of compound X (111mg) was dissolved in water (3.7mℓ) and sodium hydroxide (188mg) was added, after which the mixture was stirred at 60°C for about 16 hours. The reaction mixture, after adjusting to pH 6.7 with HCℓ, was diluted with water to make 13mℓ and subjected to high performance liquid chromatography [Mobile phase: 70% methanol/0.01M phosphoric acid solution (pH 3)] for analysis and quantitative determination: it was found that compound XXV and compound XX were produced in amounts of 9mg and 32mg, respectively.

Reference example 28

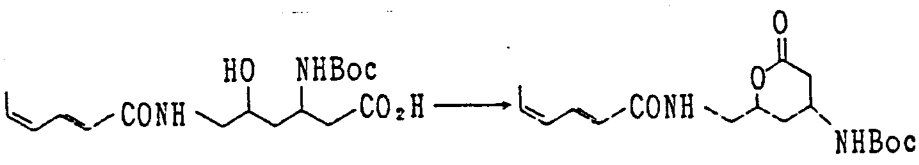

XVII XXVIII

In 100 mℓ of $CH_2Cl_2$ was dissolved Compound XVII (10.7 g). To the solution was added WSC (6.0 g). One hour later, the reaction mixture was washed with water, and dried over $MgSO_4$. The solution was concentrated under reduced pressure, and the concentrate was subjected to a silica gel column chromatography ($SiO_2$,250 g) using ethyl acetate hexane = 5 : 1 as an eluent to give Compound XXVIII (10 g) as colorless needles, m.p.156 to 157°C.
I R $\nu_{max}$ (K B r) $cm^{-1}$:3310,1733,1680,1535

| Elemental Analysis $C_{17}H_{26}N_2O_5$ | | | |
|---|---|---|---|
| Calcd. | C ,60.34; | H ,7.74; | N ,8.28 |
| Found | C ,60.59; | H ,7.52; | N ,8.00 |

Reference example 29

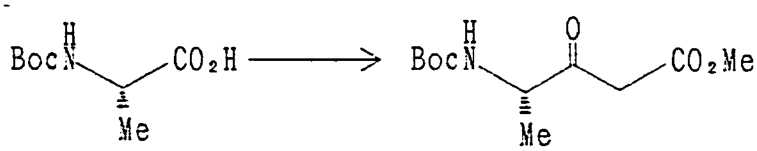

XXIX

A suspension of potassium salt of monomethyl malonate (23.4 g) and magnesium chloride (9.2 g) in anhydrous THF (150 mℓ) was stirred at 50°C for 4 hours. On the other hand, to a solution of L-Boc-alanine-(18.9 g) in anhydrous THF (200 mℓ) was added 1,1'-carbonyldimidazole(CDI) (19.4 g), and the mixture was stirred at room temperature for one hour. The solution was added to the above-mentioned suspension, and the mixture was stirred at room temperature for 14 hours, followed by distilling off the solvent. To the residue was added ethyl acetate, and the mixture was washed with 0.5N hydrochloric acid, water, a saturated aqueous solution of sodium hydrogencarbonate and water, successively, and dried ($Na_2SO_4$). The solvent was distilled off, and the residue was purified by means of a silica gel (150 g) column chromatography(hexane : ethyl acetate = 3:1 → 1:1) to afford 16.6 g of methyl (4S)-t-butoxycarbonylamino-3-oxopentanoate (XXIX) as colorless crystals. Recrystallization from isopropylether-hexane afforded colorless needles, m.p.57-58°C.
IR $\nu_{max}$(Nujol)$cm^{-1}$ : 3350, 1750, 1720, 1680

NMR(90MHz,$CDC\ell_3$)ppm : 1.35(3H,d,J=7Hz), 1.43(9H,s), 3.55(2H,s), 3.73(3H,s), 4.35(1H,m), 5.15(1H,b)
$[\alpha]_D^{26}$ -53.7° (c=0.99, methanol)

| Elemental Analysis for $C_{11}H_{19}NO_5$: | | | |
|---|---|---|---|
| Calcd. | C,53.87; | H,7.81; | N,5.71 |
| Found | C,53.78; | H,7.78; | N,5.71 |

Reference example 30-35 (Table 4 )

By a process similar to that in Reference example 29, chain-elongation reaction was conducted using N-protected amino acid to afford compounds of Reference examples 30-35. The respective yields and some of their physico-chemical properties are shown in Table 4.

Table 4

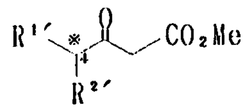

| Ref.Ex. No. | R¹′ R²′ (cpd. No.) | Steric Config. ($C_4$ Pos.) | Yield (%) | m.p. (℃) | IR $\nu_{max}$ $cm^{-1}$ |
|---|---|---|---|---|---|
| 30 | NHCbz Me <br> XXX | S | 81 | oil | (Neat) <br> 3340, 1750, 1720, 1690 |
| 31 | NHBoc Me <br> XXXI | R | 65 | 58 – 59 | (Nujol) <br> 3350, 1750, 1720, 1680 |
| 32 | NHCbz Me <br> XXXII | R | 65 | oil | (Neat) <br> 3340, 1750, 1720, 1690 |
| 33 | NHBoc Me <br> XXXIII | RS | 60 | 48 – 52 | (Nujol) <br> 3350, 1750, 1720, 1680 |
| 34 | NHBoc H <br> XXXIV | — | 89 | oil | (Neat) <br> 3400, 2980, 2940, 1770-1680 |
| 35 | NHCbz H <br> XXXV | — | 75 | 51 – 52 | (KBr) <br> 3380, 2940, 1760, 1740, 1690 |

Reference example 36

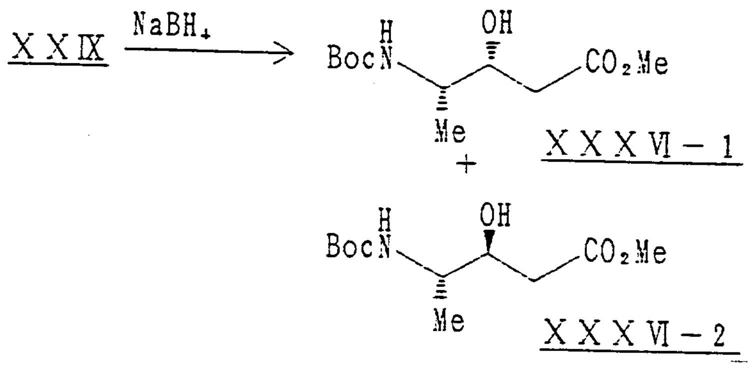


XXIX → XXXVI-1 + XXXVI-2

To a methanolic solution of Compound XXIX (250 mg) was added sodium borohydride(38 mg) at -78 °C, and the mixture was stirred for 15 minutes. To the reaction mixture was added 1N hydrochloric acid to adjust the pH at 7, which was then concentrated. To the concentrate was added ethyl acetate, and the mixture was washed with water and was then dried($Na_2SO_4$), followed by distilling off the solvent. The residue was purified by means of a silica gel (15 g) column chromatography(hexane→hexane: ethyl acetate = 3:2) to afford a mixture of alcohol compounds (XXXVI-1, XXXVI-2) (the mixture ratio was confirmed to be about 4:1 by NMR spectrum) (240 mg) as colorless crystals.

NMR(90MHz,$CDCl_3$)ppm : 1.14, 1.21 (total 3H, each d (ca.4:1), J = 7Hz), 1.43(9H,s), 2.46, 250(total 2H, each d (ca.4:1), J = 6Hz), 3.4(1H,J = 4Hz), 3.70(3H,s), 3.7(1H,m), 4.0(1H,m), 4.85(1H,b)

This product was recrystallized twice from isopropyl ether to afford the main product(XXXVI-1) as colorless prisms, m.p.92-93 °C. $[\alpha]_D^{25}$ -9.3 ° (c = 1.0, methanol)

IR and NMR spectra of this product were in grood agreement with those of the Compound (XXXVIII) obtained in Reference example 38.

Reference example 37

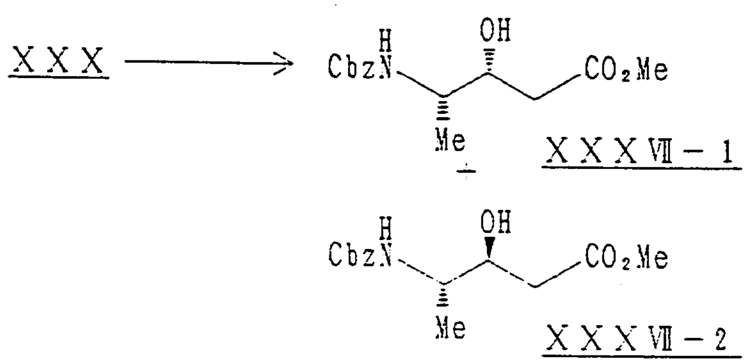

XXX → XXXVII-1 + XXXVII-2

In a manner similar to that of Reference example 36, Compound(XXX) was subjected to reduction by using sodium borohydride to afford a mixture of alcohol compounds(XXXVII-1, XXXVII-2)(the mixture ratio was confirmed to be about 4:1 by NMR spectrum) as an oily product(95%).

NMR(90MHz,$CDCl_3$)ppm : 1.15, 1.22(total 3H, each d (ca.4:1)), 2.46, 2.50(total 2H, each d, J = 6Hz), 3.16-(1H,d,J = 4Hz), 3.70(3H,s), 3.7(1H,m), 4.05(1H,m), 5.0(1H,m), 5.10(2H,s), 7.36(5H,s)

Reference example 38

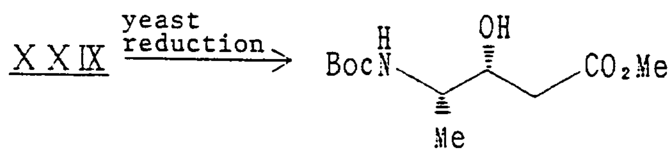


XXXVIII(≡ XXXVI-1)

To a solution of saccharose(4 g) in water (50 mℓ) was added baker's yeast (dry yeast, Oriental Yeast Industry, K.K.)(2.0 g), and the mixture was stirred at room temperature for one hour. To this mixture were added the Compound XXIX(0.50 g) obtained in Reference example 29 and ethanol(1 mℓ), and the mixture was stirred at room temperature(15-30 °C) for 5 days, to which was added celite, followed by filtration. The celite layer was washed with ethyl acetate. The filtrate was extracted with ethyl acetate. The extract and the washing were combined, washed with water and dried($Na_2SO_4$), followed by distilling off the solvent. The residue was purified by means of a silica gel(20 g) column chromatography(hexane → hexane : ethyl acetate = 3:2) to afford Compound XXXVIII(0.43g) as colorless crystals, m.p.87-89 °C. $[\alpha]_D^{22}$ -9.0(c = 0.80, methanol) Recrystallization of these crystals from isopropyl ether - hexane once afforded colorless prisms, showing the following physical constants. Melting point : 91-92 °C

IR $\nu_{max}$(Nujol)$cm^{-1}$: 3400, 3350, 1735, 1680

NMR(90MHz,$CDC\ell_3$)ppm : 1.14(3H,d,J = 7Hz), 1.43(9H,s), 2.46(2H, d,J = 6Hz), 3.30(1H,d,J = 4Hz), 3.70-(3H,s), 3,7(1H,m), 4.0 (1H,m), 4.7(1H,b)

$[\alpha]_D^{25}$ -9.5 ° (c = 1.06, methanol)

| Elemental Analysis for $C_{11}H_{21}NO_5$ : | | | |
|---|---|---|---|
| Calcd.<br>Found | C, 53.43;<br>C, 53.30; | H, 8.56;<br>H, 8.52; | N, 5.66<br>N, 5.60 |
| Erythro:threo ratio in this product was found to be 99:1 from 400MHz $^1$H-NMR(judged from the signal of Me group). | | | |

Reference Example 39-44 (Table 5 )

In a manner similar to that of Reference example 38, the Compounds XXX-XXXV obtained in Reference examples 30-35 were subjected to reduction using dry yeast to afford compounds of Reference example 39-44. The reaction conditions, yields and some of the physico-chemical properties of the products are shown in Table 5.

Table 5

Reduction of 4-substituted amino-3-oxobutyric acid derivative by yeast

| Ref.Ex. No. | Starting Material No. | Product | Product No. | Reaction time | Yield (%) (Recovery %) | m.p. (℃) | $[\alpha]_D$ (Methanol ℃, C %) | I R $\nu_{max}$ $cm^{-1}$ |
|---|---|---|---|---|---|---|---|---|
| 39 | XXX | 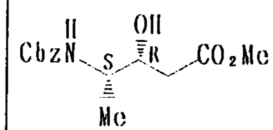 | XXXIX | 7 day | 13 (35) | 80 − 82 | −2.8° (26, 0.5) | (Nujol) 3320 1740 1690 |
| 40 | XXXI | 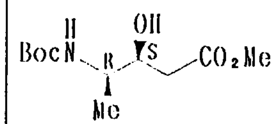 | XL | 5 day | 86 | 89 − 90 | +9.3° (22, 0.75) | (Nujol) 3400 3350 1735 1680 |
| 41 | XXXII | 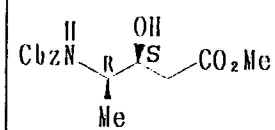 | XLI | 4 day | 23 (77) | 82 − 85 | +2.9° (25, 0.68) | (Nujol) 3320 1740 1690 |

| Ref.Ex. No. | Material No. | Product No. | Reaction time | Yield (%) (Recovery %) | m.p. (℃) | $[\alpha]_D$ (Methanol ℃, C %) | IR $\nu_{max}$ $cm^{-1}$ |
|---|---|---|---|---|---|---|---|
| 42 | XXXIII | 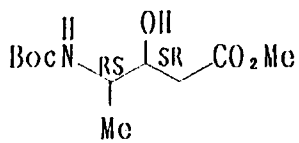 XLII | 5 days | 82 | 65 − 67 | 0° (22, 0.715) | (Nujol) 3400 3350 1735 1680 |
| 43 | XXXIV | 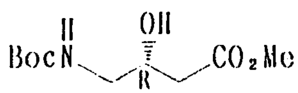 XLIII | 6 hours | 95 | 63 − 64 | − 1.2° (26, 1.025) | (Nujol) 3420 1735 1670 |
| 44 | XXXV | 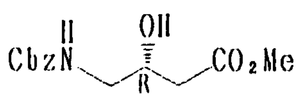 XLIV | 16 hours | 88 | 33 − 34 | − 2.6° (23, 1.135) | (Liquid) 3350 1720 |

Reference example 45

XXXVIII 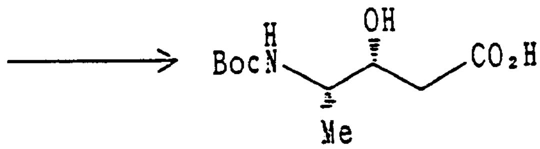

XLV

To a solution of Compound XXXVIII (7.0 g) in a mixture of dioxane(40 mℓ) and water (20 mℓ) was added, while ice-cooling under stirring, 1N NaCH(35 mℓ) over a period of 1.5 hours. The whole mixture was stirred at room temperature for 3.5 hours. To the reaction mixture was added water, and the mixture was washed with ethyl acetate. Using a 10% aqueous solution of potassium hydrogensulfate, the aqueous layer was acidified under ice-cooling (pH 2-3), and extracted with ethyl acetate. The extract solution was dried ($Na_2SO_4$), followed by distilling off the solvent to afford Compound XLV as colorless crystals. Recrystallization from hexane - ethyl acetate afforded colorless prisms(6.2 g), m.p.103-104 °C.

IR $\nu_{max}$(Nujol)$cm^{-1}$ : 3350, 1710, 1680

NMR(90MHz,$CDC\ell_3$)ppm : 1.15(3H,d,J = 7Hz), 1.43(9H,s), 2.50(2H, d,J = 6Hz), 3.73(1H,m), 4.0(1H,m), 4.90-(1H,b), 6.33(1H,b)

$[\alpha]_D^{26}$ -9.8 ° (c = 1.015, methanol)

| Elemental Analysis for $C_{10}H_{19}NO_5$ | | | |
|---|---|---|---|
| Calcd. | C, 51.49; | H, 8.21; | N, 6.00 |
| Found | C, 51.50; | H, 8.15; | N, 6.02 |

Reference examples 46-48 (Table 6)

In a manner similar to that of Reference example 45, Compounds XXXIX, XLIII and XLIV were subjected to alkaline hydrolysis to obtain compounds of Reference examples 46-48. The respective yields and some of the physico-chemical properties of the products are shown in Table 6.

Table 6

| Ref.Ex. No. | Compound No. | Yield (%) | m.p. (℃) | $[\alpha]_D$ (Methanol ℃, C%) | IR $\nu_{max}^{Nujol}$ $cm^{-1}$ |
|---|---|---|---|---|---|
| 46 | 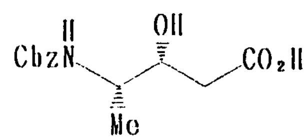 XLVI | 91 | 83−84 | −4.2° (26, 1.025) | 3320 1720 1680 |
| 47 | 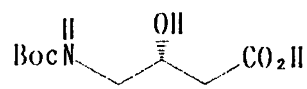 XLVII | 96 | 96−97 | −3.2° (23, 0.75) | 3490 3220 1690 |
| 48 | 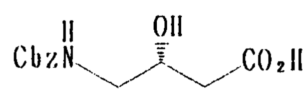 XLVIII | 90 | 81−82 | −5.4° (23, 0.67) | 3560 3340 1690 |

Reference example 49

XLVIII

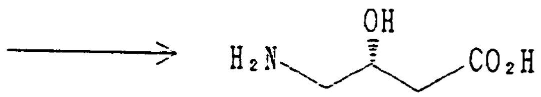

XLIX

A mixture of Compound XLVIII (0.25 g), 10% Pd-C(50 mg) and methanol (10 mℓ) was stirred under hydrogen at room temperature for 3 hours. The reaction mixture was filtrated, and the catalyst was washed with aqueous metanol(10%). The filtrate and the washing were combined, and the solvent was distilled off to afford 4-amino-3-hydroxybutyric acid (XLIX) (0.11 g) as colorless prisms, m.p.222-223 °C(decomp.).
IR $\nu_{max}$(Nujol)$cm^{-1}$ : 3125, 1650, 1570
NMR(90MHz, $D_2O$)ppm : 2.53(2H,d,J = 7Hz), 2.9-3.4(2H,m), 4.3(1H,m)
$[\alpha]_D^{23}$ -19.4 ° (c = 0.635, water)

| Elemental Analysis for $C_4H_9NO_3$ : | | | |
|---|---|---|---|
| Calcd. | C, 40.33; | H, 7.62; | N, 11.76 |
| Found | C, 40.26; | H, 7.70; | N, 11.66 |

Reference example 50

XLV

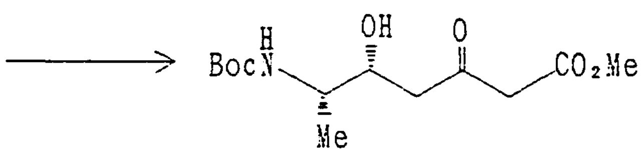

L

A suspension of potassium salt of monomethyl malonate (21.2 g) and magnesium chloride(8.4 g) in anhydrous THF (150mℓ) was stirred at 50 °C for 16.5 hours. On the other hand, to a solution of Compound XLV(10.0 g) in anhydrous THF (120 mℓ) was added, while stirring under ice-cooling, a solution of CDI(7.7g) in anhydrous THF(200 mℓ), over a period of one hour The whole mixture was stirred for further 1.5 hours under ice-cooling. To the solution was added the above-mentioned suspension, and the mixture was stirred at room temperature for 16.5 hours, followed by distilling off the solvent. To the residue was added ethyl acetate, which was washed with water, a saturated aqueous solution of sodium hydrogencarbonate and water, successively, dried ($Na_2SO_4$) and the solvent was evaporated. The residue was purified by means of a silica gel (120 g) column chromatography(ethyl acetate) to afford methyl(5R,6S)-6-t-butoxycarbonylamino-5-hydroxy-3-oxopentanoate (L) as colorless crystals. Recrystallization from ether afforded colorless needles (7.0 g), m.p.83-85 °C.
IR $\nu_{max}$(Nujol)$cm^{-1}$ : 3450, 1750, 1710, 1680
NMR(90MHz,CDCℓ3)ppm : 1.13(3H,d,J = 7Hz), 1.43(9H,s), 2.67(2H, d,J = 6Hz), 3.50(2H,s), 3.73(3H,s), 4.05-(1H,m), 4.76(1H,d, J = 8Hz)
$[\alpha]_D^{26}$ -5.9 ° (c = 1.035, methanol)

| Elemental Analysis for $C_{13}H_{23}NO_6$ : | | | |
|---|---|---|---|
| Calcd. | C, 53.97; | H, 8.01; | N, 4.84 |
| Found | C, 54.05; | H, 8.04; | N, 4.85 |

Reference examples 51-53 (Table 7)

In a manner similar to that in Reference example 50, the compounds obtained in Reference examples 46-48 were subjected to chain-elongation reaction to obtain compounds of Reference examples 51-53. The respective yields and some of the physico-chemical properties of the prodcuts are shown in Table 7.

Table 7

| Ref.Ex. No. | Compound No. | Yield (%) | m.p. (℃) | I R $\nu_{max}$ $cm^{-1}$ |
| --- | --- | --- | --- | --- |
| 51 | 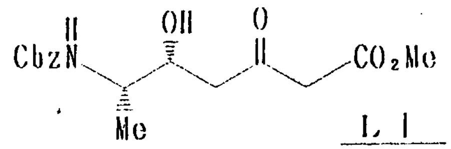 L I | 60 | 104 − 105 | (Nujol) 3300 1750 1720 1690 |
| 52 | 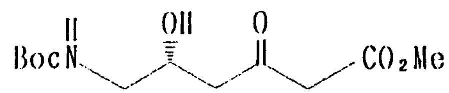 L II | 72 | oily product | (Liquid) 3400 1770− 1690 |
| 53 | 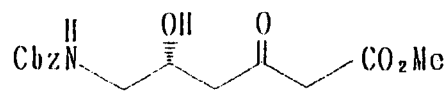 L III | 65 | oily 'product | (Liquid) 3400 1760− 1680 |

Reference example 54

L 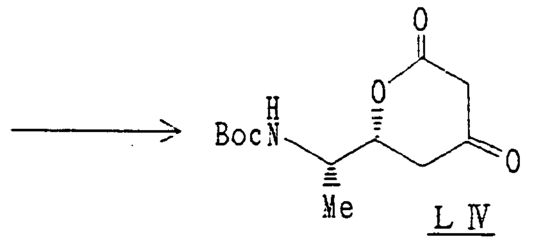 LIV

To a solution of Compound L(4.85 g) in THF(100 mℓ) was added 1N-NaOH(18.5 mℓ), while ice-cooling under stirring, over a period of 15 minutes. This mixture was stirred for 1.5 hours at room temperature, and concentrated. To the concentrate was added water, and the mixture was washed with ethyl acetate. The aqueous layer was acidified (pH about 2) with a 10% aqueous solution of potassium hydrogensulfate, and extracted with ethyl acetate. The extract was washed with water, then dried ($Na_2SO_4$), and the solvent was evaporated to afford (6R,1'S)-6-[(1'-t-butoxycarbonylamino)ethyl]-2,4-dioxotetrahydrofuran(LIV) (4.32 g) as colorless crystals, m.p.144-145°C.
IR $\nu_{max}$(Nujol)$cm^{-1}$: 3380, 1750, 1735, 1720, 1685
NMR(90MHz,$CDCℓ_3$)ppm : 1.26(3H,d,J=7Hz), 3.8(1H,m), 4.76(1H,m)
$[\alpha]_D^{23}$ +2.4° (c=0.63, methanol)

| Elemental Analysis for $C_{12}H_{19}NO_5$ | | | |
|---|---|---|---|
| Calcd. | C, 56.02; | H, 7.44; | N, 5.44 |
| Found | C, 56.23; | H, 7.49; | N, 5.44 |

Reference Example 55-57 (Table 8)

In a manner similar to that of Reference example 54, the compounds obtained in Reference examples 51-53 were allowed to react with 1N-NaOH to afford compounds of Reference example 55-57. The respective yeilds and some of the physico-chemical properties of the products are shown in Table 8.

Table 8

| Ref.Ex. No. | Compound No. | | yield (%) | m.p. (℃) | $[\alpha]_D$ (Solvent ℃, C %) | I R $\nu_{max}^{Nujol}$ $cm^{-1}$ |
|---|---|---|---|---|---|---|
| 55 | 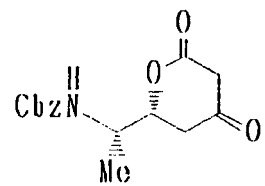 | L V | 99 | 142 − 144 | + 5.4° (Methanol 23, 0.815) | 3370 1755 1720 1695 |
| 56 | 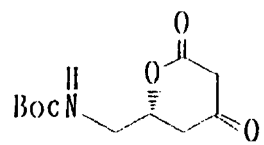 | L VI | 90 | 133 − 134 | + 58.8° (Ethanol 24, 0.495) | 3370 2630 1690 |
| 57 | 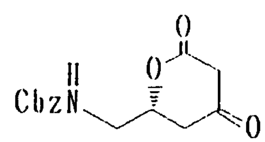 | L VII | 92 | 102 − 103 | + 49.3° (Methanol 26, 0.56) | 3350 1725 1695 |

Reference example 58

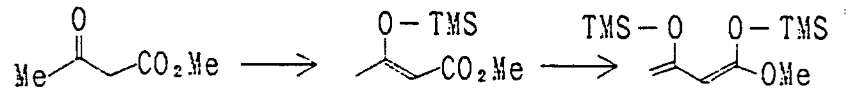

L VIII a

TMS = SiMe3

A suspension of anhydrous zinc chloride(16 g) in triethylamine(226 g) was stirred at room temperature for 2 hours, to which was added a solution of methyl acetoacetate(416.5 g) in ether(1 ℓ). To the mixture was then added, under ice-cooling and stirring, trimehtylsilylchloride(584 g). The reaction mixture was stirred at room temperature for 16 hours, then precipitating sediment was filtered off. The filtrate was purified by distillation to afford trimethylsilyloxy compound (LVIIIa) (b.p.50-60 °C/2.5mmHg, 638 g) as a colorless liquid. A solution of LVIII(68.5 g) in anhydrous THF(70 mℓ) was added, at -78 °C under stirring, to a solution of LDA[prepared from n-BuLi(1.6M hexane solution, 250 mℓ) and diisopropylamine(40.5 g)] in anhydrous THF-(500 mℓ), over a period of hour. The reaction mixture was stirred at -78 °C for 15 minutes and at room temperature for 2.5 hours, and, then, resulting precipitates were filtered off. The filtrate was concentrated under reduced pressure. To the concentrate was further added hexane, and the precipitates were filtered off, and the filtrate was again concentrated. The concentrate was purified by distillation to afford trimethylsilyloxydiene compound (LVIII) (b.p.65-70 °C/1 mmHg, 89.5 g) as a pale yellow liquid product.

Reference example 59

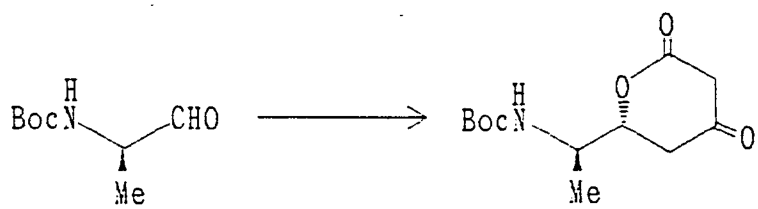

L IX

To a solution of Boc-D-alaninal(25 g) in anhydrous $CH_2C\ell_2$ (280 mℓ) were added, under ice-cooling and stirring, tris (6,6,7,7,8,8,8-heptafluoro-2,2-dimethyl-3,5-octanedionate) europium[Eu(fod)$_3$](75 mg) and Compound LVIII(94 g). This mixture was stirred at 5-8 °C for 3 days, to which was added a saturated aqueous solution of potassium hydrogensulfate to suspend the reaction, followed by extraction with an aqueous solution of sodium carbonate. The extract was washed with $CH_2C\ell_2$, to which was added potassium hydrogensulfate to render the solution acidic. The mixture was extracted with ethyl acetate. The extract was washed with water, dried($MgSO_4$), and concentrated. The concentrate was crystallized from chloroform-carbon tetrachloride to afford (6R,1'R)-6-[('-t-butoxycarbonylamino)ethyl]-2,4-dioxotetrahydrofuran(LIX)(16.0 g) as colorless crystals, m.p.146-148 °C.
IR $\nu_{max}$(KBr)$cm^{-1}$ : 3360, 2980, 1675, 1615, 1580
NMR(90MHz,$CDC\ell_3$)ppm : 1.35(3H,d,J = 7Hz), 1.43(9H,s), 2.60(1H, d,J = 4Hz), 2.68(1H,s), 3.48(2H,q), 3.75-4.18(1H,m),4.50-4.95(2H,m)
$[\alpha]_D^{23}$ + 117.6 ° (c = 0.25, chloroform)

| Elemental Analysis for $C_{12}H_{19}NO_5$ : | | | |
|---|---|---|---|
| Calcd. | C, 56.02; | H, 7.44; | N, 5.44 |
| Found | C, 55.88; | H, 7.47; | N, 5.43 |

Reference examples 60-67 (Table 9)

In a manner similar to that of Reference example 59, an aldehyde derivative of corresponding α - amino acid was allowed to react with trimethylsilyloxydiene(LVIII) in the presence of a catalyst to afford compounds of Reference examples 60-67. The catalysts, yields and some of the physico-chemical properties of the products are shown in Table 9.

Table 9

| Ref.Ex. No. | Compound No. | Catalyst | Yield (%) | m.p. (°C) | $[\alpha]_D$ (Solvent °C C %) | I R $\nu_{max}$ $cm^{-1}$ |
|---|---|---|---|---|---|---|
| 60 | 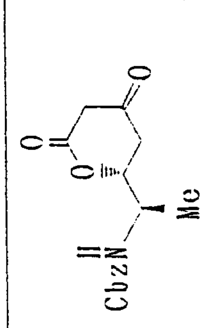 LX | $Eu(fod)_3$ | 18 1) | 75 − 80 | +89.7° (DMSO 27 0.31) | (KBr) 3290 1680 1655 1565 1545 |
| 61 | 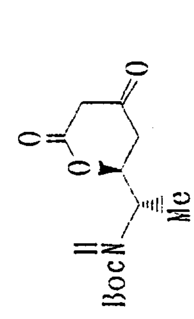 LXI | $Eu(fod)_3$ | 41 | 147 − 149 | −114.1° (Chloroform 23 0.44) | (KBr) 3360 2980 1675 1615 1580 |
| 62 | 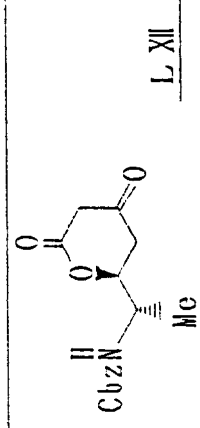 LXII | $Eu(fod)_3$ | 20 1) | 70 − 85 | −81.1° (DMSO 26 0.355) | (KBr) 3290 1680 1655 1565 1545 |

| Ref.Ex. No. | Compound No. | | Catalyst | Yield (%) | m.p. (℃) | $[\alpha]_D$ (Solvent ℃ C %) | IR $\nu_{max}$ $cm^{-1}$ |
|---|---|---|---|---|---|---|---|
| 63 | 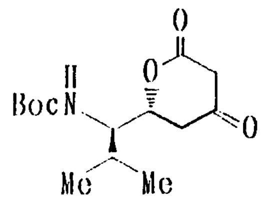 | L X III | $Eu(fod)_3$ | 27 1) | oily product | — 2) | — 2) |
| 64 | 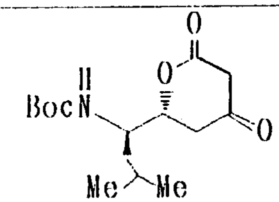 | L X IV | $SnCl_2$ | 17 1) | 155 − 159 | + 106.2° (Chloroform 24 0.565) | (KBr) 3330 2940 1695 1655 1500 |
| 65 | 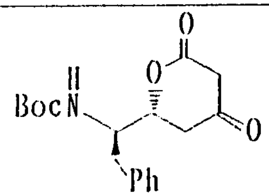 | L X V | $Eu(fod)_3$ | 21 | 146 − 148 | + 3.9° (Chloroform 23 0.44) | (KBr) 3300 2970 1710 1665 1580 |

| Ref. Ex. No. | Compound No. | | Catalyst | Yield (%) | m.p. (℃) | $[\alpha]_D$ (Solvent ℃ C %) | IR $\nu_{max}$ $cm^{-1}$ |
|---|---|---|---|---|---|---|---|
| 66 | 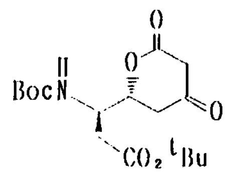 | LXVI | $SnCl_2$ | 10 [1] | 140 − 142 | +79.3° (Chloroform 24 0.28) | (Neat) 3420 1740-1650 1615 1500 |
| 67 | 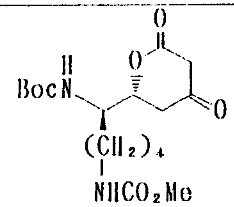 | LXVII | $SnCl_2$ | 20 [1] | oily product | —— [2] | (Neat) 3320 2920 1720-1680 |

1) Corresponding amino acid aldehyde was prepared by subjecting corresponding amino acid alcohol to oxidation with pyridine-$SO_3$/DMSO, and used in situ. Yields are shown in terms of the overall yields from alcohol.

2) not determined

Example 1

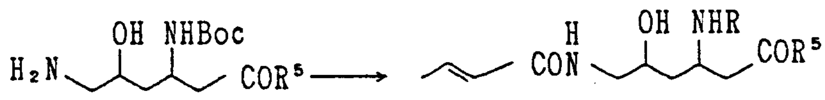

XIII

1a:R=Boc

1b:R=H

$R^5$ =
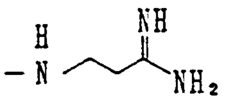

Step A (XIII → 1a)

In DMF (3 mℓ) was suspended dihydrochloride of Compound XIII (250 mg). To the suspension were added under ice-cooling $Et_3N$ (0.124 mℓ), crotonic acid (61 mg), HOBT (96 mg) and DCC (146 mg). The mixture was stirred for 30 minutes, then for six hours at room temperature. The reaction mixture was subjected to filtration. The filtrate was concentrated under reduced pressure. To the residue was added water (50 mℓ) The solution, after its pH being adjusted to 2.5 with 1 N HCℓ, was washed with ethyl acetate (30 mℓ x 3). The aqueous layer, after its pH being adjusted to 5.7 with 1N NaOH, was concentrated to a volume of about 30 mℓ. The concentrate was subjected to a column chromatography using Diaion HP-20 (50-100 mesh, 18 mℓ). The column was washed with water (50 mℓ), and then eluted with 10% methanol-water (70 mℓ), 50% methanol-water (50 mℓ)-N/200 hydrochloric acid (90 mℓ), successively to collect the respective fractions. Each fraction was subjected to analysis by means of high performance liquid chromatography [mobile phase : 40% methanol - 0.01M phosphoric acid solution (pH 3)]. Fractions showing single peak were collected, concentrated and then lyophilized to obtain hydrochloride of Compound 1a (228 mg) as white powder.

| Elemental Analysis : $C_{18}H_{33}N_5O_5 \cdot HCl \cdot 0.5H_2O$ | | | | |
|---|---|---|---|---|
| Calcd.<br>Found | C, 48.59;<br>C, 48.91; | H, 7.93;<br>H, 7.97; | N, 15.74;<br>N, 16.06; | Cl, 7.97<br>Cl, 8.01 |

Step B (1a → 1b)

In $CF_3CO_2H$(TFA) (1.8 mℓ) was dissolved hydrochloride of Compound 1a (180 mg), and the solution was left standing for 30 minutes at room temperature. The solvent was distilled off under reduced pressure, and the residue was treated with ether to obtain powder. The powder was dissolved in water (45 mℓ), and the solution was allowed to pass through Amberlite IRA-402 ($C\ell^-$ type) (15 mℓ). The resin was eluted with water (15 mℓ). The eluate was concentrated, followed by lyophilization to obtain dihydrochloride of Compound 1b (148 mg) as white powder.
IR $\nu_{max}$ (KBr) $cm^{-1}$ : 3250,3070,1660,1550

| Elemental Analysis : $C_{13}H_{25}N_5O_3 \cdot 2HCl \cdot 0.5H_2O$ | | | | |
|---|---|---|---|---|
| Calcd.<br>Found | C, 40.95;<br>C, 41.21; | H, 7.40;<br>H, 7.52; | N, 18.37;<br>N, 18.36; | Cl, 18.60<br>Cl, 18.60 |

Examples 2 - 45 (Table 10)

By the procedure similar to Example 1, dihydrochloride of Compound (XIII) was subjected to acylation (step A) by using various carboxylic acid ($R'CO_2H$) or its derivatives, then to deprotection reaction (Step B) to obtain compounds of Examples 2 - 45. The reaction conditions, yields and some of the physico-chemical properties of the products are shown in Table 10.

Table 10

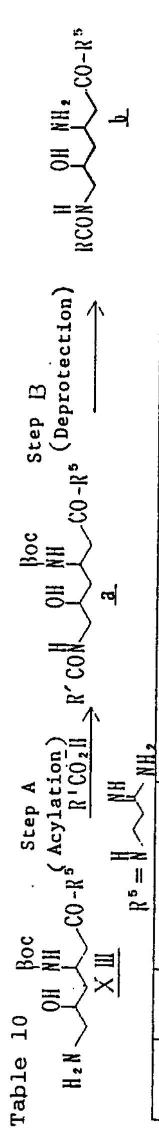


XIII, a, b

| Example No. | Step | Starting Compd. → Product | Reaction Conditions | Yield Material → Product | IR $\nu_{max}^{KBr}$ $cm^{-1}$ | Molecular Formula of Product Elemental Analysis (Calcd.) |
|---|---|---|---|---|---|---|
| 2 | A | XIII → 2a; R' = Ph | R'COCl (852mg)<br>$NaHCO_3$ (3.0g)<br>$H_2O$ (100mℓ)<br>r.t.[1] 6hr, pH~8<br>neutralization · washing with EtOAc<br>CHP-20P[2], desalting<br>lyophilization | 507mg (; Purity 69%) → 276.7mg | 1640 | $C_{21}H_{33}N_5O_5 \cdot HCl \cdot 0.9H_2O$<br>C 51.71 (51.67)<br>H 7.34 ( 7.39)<br>N 14.48 (14.35)<br>Cl 7.84 ( 7.26) |
| | B | 2a → 2b; R = Ph | TFA (2.0mℓ)<br>r.t., 1.0hr.<br>evap.[3] washing with EtOAc<br>Dowex 1×2[4]<br>lyophilization | 193.8mg → 204mg | 1640 | $C_{16}H_{25}N_5O_3 \cdot 2HCl \cdot 1.6H_2O$<br>C 44.14 (43.96)<br>H 7.11 ( 6.96)<br>N 15.92 (16.02)<br>Cl 22.18 (16.22) |

1) r.t.: room temp., 2) CHP-20P(Polystyrene resin, Diaion, Mitsubishi Chemical Industries, Ltd.)
3) evap.: solv. evaporation 4) DoweX 1×2 (anion exchange resin, Dow Chemical)
(same abbreviations are used hereinafter)

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 3 | A | XIII → 3a<br>R′ = $Me_2N$— 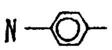 — | R′COOH (114.6mg)<br>HOBT (93.8mg)<br>DCC (143.2mg)<br>$Et_3N$ (96 μl)<br>DMF (7.5ml)<br>r.t. 14hr<br>evap. disolution in DMF, addition of $H_2O$ filtration, washing with EtOAc adjusted to pH 3 (1N-HCl)<br>HP-20 lypohilization | 234mg → 238mg (purity 70%) | 1650 | $C_{23}H_{28}N_6O_5 \cdot 2HCl$<br>C —[2] (50.93)<br>H — ( 5.76)<br>N — (15.49) |
| | B | 3a → 3b<br>R = $Me_2N$— 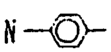 — | TFA (0.5ml)<br>r.t. 35min<br>evap. washing with $Et_2O$<br>IRA-402($Cl^-$)[3], CHP-20<br>lyophilization | 238mg (crude) → 30mg | 1660 | $C_{18}H_{30}N_6O_3 \cdot 3HCl \cdot H_2O$<br>C 42.60 (42.74)<br>H 6.62 ( 6.97)<br>N 16.29 (16.61) |

1) HP-20 (Polystyrene resin, Diaion, Mitsubishi Chemical Industries, Ltd.) 2) not measured
3) IRA-402($Cl^-$) (anion exchange resin, Amberlite, Rohm & Haas Co.) (same abbreviations are used hereinafter)

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 4 | A | XIII → 4a<br>R′ = Ph 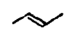 | $R'CO_2H$ (111.3mg)<br>HOBT (101.5mg)<br>DCC (155mg)<br>$Et_3N$ (0.13ml)<br>DMF (8.4ml)<br>r.t. 22hr.<br>(hereinafter same as 3A) | 253mg → 264mg | 1660 | $C_{23}H_{35}N_5O_5 \cdot HCl \cdot 0.8H_2O$<br>C 53.99 (53.91)<br>H 7.47 ( 7.40)<br>N 13.70 (13.67)<br>Cl 7.44 ( 6.92) |
| | B | 4a → 4b<br>R = Ph 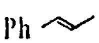 | TFA (2.0ml)<br>r.t. 30min<br>evap. washing with $Et_2O$<br>IRA-402($Cl^-$)<br>lyophilization | 219mg → 182mg | 1670 | $C_{18}H_{27}N_6O_3 \cdot 2HCl \cdot 0.7H_2O$<br>C 48.26 (48.37)<br>H 7.02 ( 6.85)<br>N 15.62 (15.67)<br>Cl 16.52 (15.86) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 5 | A | XIII<br>→<br>5a<br>R′ = 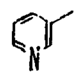 | $R'CO_2H$ (93mg)<br>HOBT (102mg)<br>DCC (156mg)<br>$Et_3N$ (0.13mℓ)<br>DMF (9.0mℓ)<br>r.t. 24hr<br>hereinafter same as 3A | 255mg<br>→<br>106mg | 1650 | $C_{20}H_{32}N_6O_4 \cdot 2HCl \cdot H_2O$<br>C 46.58 (46.97)<br>H 7.24 (7.09)<br>N 16.33 (16.43) |
| | B | 5a<br>→<br>5b<br>R = 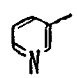 | TFA (1.0mℓ)<br>r.t. 30min<br>evap. washing with $Et_2O$<br>IRA-402($Cl^-$)<br>lyophilization | 86mg<br>→<br>79mg | 1660 | $C_{15}H_{20}N_6O_3 \cdot 3HCl \cdot 0.1H_2O$<br>C 40.40 (40.25)<br>H 6.28 (6.13)<br>N 18.51 (18.71)<br>Cl 22.33 (23.76) |

| Example No. | Step | Starting Compd. → Product | Reaction Conditions | Yield Material → Product | IR $\nu_{max}^{KBr}$ $cm^{-1}$ | Molecualr Formula of Prodcut<br>Elemental Analysis(Calcd.) |
|---|---|---|---|---|---|---|
| 6 | A | XIII → 6a<br>R′ = 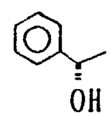 | $R'CO_2H$ (85mg)<br>HOBT (75.5mg)<br>DCC (115mg)<br>$Et_3N$ (97 μℓ)<br>DMF (6.0mℓ)<br>r.t. 29 hr<br>hereinafter same as 3A | 188mg → 56.5mg | 1670 | $C_{22}H_{35}N_5O_6 \cdot HCl \cdot 0.5H_2O$<br>C 51.75 (51.91)<br>H 7.44 ( 7.33)<br>N 16.38 (16.51) |
| | B | 6a → 6b<br>R = 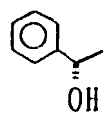 | TFA (0.4mℓ)<br>rt. 30min<br>evap. washing with $Et_2O$<br>IRA-402($Cl^-$)<br>lyophilization | 86mg → 72mg | 1660 | $C_{17}H_{27}N_5O_4 \cdot 2HCl \cdot 0.5H_2O$<br>C 45.70 (45.64)<br>H 6.91 ( 6.76)<br>N 15.67 (15.65)<br>Cl 16.30 (15.85) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 7 | A | XIII<br>→<br>7a<br>R′ = Me 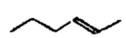 | $R'CO_2H$ (97mg)<br>HOBT (115mg)<br>DCC (176mg)<br>$Et_3N$ (148μℓ)<br>DMF (4mℓ)<br>0°C 0.5hr, r.t. 7h<br>washing with EtOAc<br>HP-20<br>lyophilization | 300mg<br>→<br>265mg | —[1]) | $C_{20}H_{37}N_5O_5 \cdot HCl \cdot 0.5H_2O$<br>C 50.53 (50.79)<br>H 8.49 ( 8.31)<br>N 14.71 (14.81) |
| | B | 7a<br>→<br>7b<br>R = Me 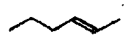 | TFA (2mℓ)<br>r.t. 0.5hr<br>treatment with $Et_2O$<br>IRA-402($Cl^-$)<br>lyophilization | 248mg<br>→<br>208mg | 3250<br>3060<br>1660<br>1545 | $C_{15}H_{29}N_5O_3 \cdot 2HCl \cdot 0.5H_2O$<br>C 43.69 (44.01)<br>H 7.90 ( 7.88)<br>N 16.68 (17.11)<br>Cl 17.12 (17.32) |

[1]) not measured (hereinafter, same abbreviation is used hereinafter)

| Example No. | Step | Starting Compd. → Product | Reaction Conditions | Yield Material → Product | I R $\nu_{max}^{KBr}$ $cm^{-1}$ | Molecualr Formula of Product Elemental Analysis (Calcd.) |
|---|---|---|---|---|---|---|
| 8 | A | X III → 8a<br>R′ = $HO_2C$ 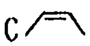 | maleic anhydride (302mg)<br>$NaHCO_3$(3.0g)<br>$H_2O$ 100 mℓ<br>r.t. 1hr. pH8<br>neutralization, HP-20<br>lyophilization | 588mg<br>(purity 69%) →<br>296mg | — | $C_{18}H_{31}N_5O_7 \cdot 1.5H_2O$<br>C 47.06 (47.36)<br>H 7.68 ( 7.51)<br>N 15.18 (15.34) |
| | B | 8a → 8b<br>R = $HO_2C$ 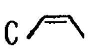 | TFA (0.5 mℓ)<br>r.t. 30min<br>evap. washing with $Et_2O$<br>IRA-402($Cl^-$)<br>lyophilization | 143mg<br>→<br>128mg | — | $C_{13}H_{23}N_5O_4 \cdot 2HCl \cdot 1.3H_2O$<br>C 38.44 (38.30)<br>H 6.27 ( 6.33)<br>N 17.42 (17.18)<br>Cl 17.00 (17.39) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 9 | A | XIII<br>→<br>9a<br>R' = Me 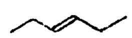 | $R'CO_2H$ (101 μℓ)<br>HOBT (115mg)<br>DCC (176mg)<br>$Et_3N$ (148 μℓ)<br>DMF (4mℓ)<br>0℃ 0.5h, r.t. 7hr<br>washing with EtOAc<br>HP-20<br>lyophilization | 300mg<br>→<br>280mg | — | $C_{20}H_{37}N_5O_5 \cdot HCl \cdot 0.5H_2O$<br>C 50.84 (50.79)<br>H 8.38 ( 8.31)<br>N 14.61 (14.81) |
| | B | 9a<br>→<br>9b<br>R = Me 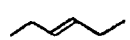 | TFA (2mℓ)<br>r.t. 0.5hr<br>treatment with $Et_2O$<br>IRA-402($Cl^-$)<br>lyophilization | 264mg<br>→<br>223mg | 3270<br>3060<br>1640<br>1540 | $C_{15}H_{29}N_5O_3 \cdot 2HCl \cdot 0.5H_2O$<br>C 43.95 (44.01)<br>H 8.20 ( 7.88)<br>N 17.18 (17.11)<br>Cl 16.89 (17.32) |

| Example No. | Step | Starting Compd. → Product | Reaction Conditions | Yield material → Product | I R $\nu_{max}^{KBr}$ $cm^{-1}$ | Molecular Formula of Product<br>Elemental Analysis (Calcd.) |
|---|---|---|---|---|---|---|
| 10 | A | <u>X III</u> → <u>10a</u><br>$R' = -(CH_2)_{14}CH_3$ | $R'CO_2H$ (218mg)<br>HOBT (115mg)<br>DCC (176mg)<br>$Et_3N$ (148 μℓ)<br>DMF (5mℓ)<br>0°C 1hr, r.t. 25h<br>Washing with EtOAc<br>HP-20<br>lyophilization | 300mg → 275mg | — | $C_{30}H_{59}N_5O_5 \cdot HCl \cdot H_2O$<br>C 57.66 (57.72)<br>H 10.19 (10.01)<br>N 11.08 (11.22) |
| | B | <u>10a</u> → <u>10b</u><br>$R = -(CH_2)_{14}CH_3$ | TFA (2mℓ)<br>r.t. 0.5hr<br>treatment with $Et_2O$<br>IRA-402($Cl^-$)<br>lyophilization | 258mg → 195mg | 3270<br>3070<br>2920<br>2855<br>1640<br>1540 | $C_{26}H_{51}N_5O_3 \cdot 2HCl \cdot H_2O$<br>C 53.74 (53.56)<br>H 9.62 ( 9.89)<br>N 12.15 (12.49)<br>Cl 11.83 (12.65) |

| Example No. | Step | Starting Compd. → Product | Reaction Conditions | Yield Material → Product | IR $\nu_{max}^{KBr}$ $cm^{-1}$ | Molecular Formula of product Elemental Analysis (Calcd.) |
|---|---|---|---|---|---|---|
| 11 | A | XIII → 11a<br>R' = 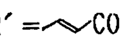$CO_2CHPh_2$ | $R'CO_2H$ (338mg)<br>HOBT (162mg)<br>DCC (247mg)<br>$Et_3N$ (209 μℓ)<br>DMF (5mℓ)<br>0℃ 0.5hr, r.t.7.5h<br>washing with $EtOAc-Et_2O$<br>HP-20<br>lyophilization | 422mg → 552mg | — | $C_{30}H_{41}N_5O_7 \cdot HCl \cdot H_2O$<br>C 56.32 (56.46)<br>H 6.88 ( 6.95)<br>N 11.05 (10.97) |
| | B | 11a → 11b<br>R = 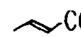$CO_2CHPh_2$ | 0.01N HCl (30mℓ)<br>Concentration<br>to dryness (6 times)<br>HP-20<br>lyophilization | 300mg → 214mg | 3250<br>3060<br>1710<br>1660<br>1540 | $C_{26}H_{33}N_5O_5 \cdot 2HCl \cdot H_2O$<br>C 52.93 (53.24)<br>H 6.37 ( 6.36)<br>N 12.16 (11.94)<br>Cl 13.17 (12.09) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 12 | B | 11a → 12b<br>R = 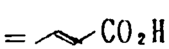 | TFA (2ml)<br>r.t. 30min<br>treatment with $Et_2O$<br>IRA-402($Cl^-$)<br>lyophilization | 220mg → 129mg | 3370<br>3060<br>1640<br>1550 | $C_{13}H_{23}N_5O_5 \cdot HCl \cdot H_2O$<br>C 40.35 (40.68)<br>H 6.68 ( 6.83)<br>N 17.91 (18.25)<br>Cl 13.41 ( 9.24) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 13 | A | XIII<br>→<br>13a<br>R′ = $HO_2C$ 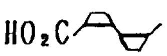 | (Z,Z) – Muconic acid (226.5mg)<br>HOBT (172mg)<br>DCC (263mg)<br>$Et_3N$ (0.22mℓ)<br>DMF (21mℓ)<br>r.t. 16 hr<br>hereinafter same as 3A | 429.7mg<br>→<br>125mg | — | $C_{20}H_{33}N_5O_7 \cdot 2H_2O$<br>C 48.99 (48.87)<br>H 7.68 (7.59)<br>N 14.03 (14.25) |
| | B | 13a<br>→<br>13b<br>R = $HO_2C$ 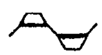 | TFA (0.3mℓ)<br>r.t. 30min<br>evap. washing with $Et_2O$<br>IRA-402($Cl^-$)<br>lyophilization | 109mg<br>→<br>92mg | 1750<br>1700–<br>1640 | $C_{15}H_{25}N_5O_5 \cdot 2HCl \cdot 2.0H_2O$<br>C 39.07 (38.80)<br>H 6.76 (6.73)<br>N 14.81 (15.08)<br>Cl 15.99 (15.27) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis(Calcd.) |
|---|---|---|---|---|---|---|
| 14 | A | XIII → 14a<br>R′ = 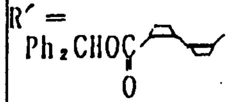 | $RCO_2H$ (188mg)<br>HOBT (82.2mg)<br>DCC (125.5mg)<br>$Et_3N$ (0.105mℓ)<br>DMF (6.8mℓ)<br>r.t. 15 hr<br>Hereinafter same as 3A | 205mg → 268mg | — | $C_{33}H_{43}N_6O_7 \cdot HCl \cdot 1.5H_2O$<br>C 57.93 (57.84)<br>H 6.70 ( 6.91)<br>N 10.28 (10.22) |
| | B | 14a → 14b<br>R = 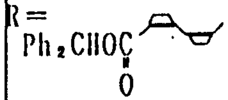 | 0.5N HCl (20mℓ)<br>i-BuOH(1.0mℓ)<br>EtOH (2.0mℓ)<br>r.t. 28 hr<br>0°C 15hr<br>neutralization, HP-20<br>lyophilization | 208mg → 104mg | 1700<br>1660 | $C_{28}H_{36}N_5O_6 \cdot 2HCl \cdot 2.4H_2O$<br>C 52.75 (52.73)<br>H 6.73 ( 6.61)<br>N 11.09 (10.98)<br>Cl 11.50 (11.12) |

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 15 | A | XIII → 15a<br>R' = $ClCH_2CH(NHBoc)-$ *D[1] | R'$CO_2H$ (224mg)<br>EEDQ (281mg)<br>DMF (3mℓ)<br>r.t. 18hr<br>evaporation of DMF<br>CHP-20P<br>50% MeOH-$H_2O$,<br>lyophilization | 331mg → 130mg | 1690<br>1650 (sh)<br>1520 | $C_{22}H_{41}N_8O_7 \cdot HCl \cdot 0.5H_2O$<br>C 48.11 (48.30)<br>H 7.99 (7.92)<br>N 15.08 (15.36) |
| | B | 15a → 15b<br>R = $ClCH_2CH(NH_2)-$ *D | TFA (5mℓ)<br>r.t. 40min<br>evap. washing with $ET_2O$<br>IRA-402($Cl^-$)<br>lyophilization | 120mg → 102mg | 1690<br>1645<br>1510 | $C_{12}H_{25}ClN_8O_3 \cdot 3HCl \cdot 0.5H_2O$<br>C 31.52 (31.66)<br>H 6.21 (6.42)<br>N 18.23 (18.46) |

1) *D: D-compound (hereinafter the same applies)

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 16 | A | XIII<br>→<br>16a<br>R′ = 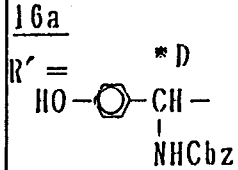 | $R'CO_2H$ (301mg)<br>EEDQ (281mg)<br>DMF (3mℓ)<br>r.t. 18hr<br>evap. washing with $Et_2O$<br>CHP-20P, 50% $MeOH-H_2O$<br>lyophilization | 331mg<br>→<br>130mg | 1715 (sh)<br>1690<br>1650<br>1515 | $C_{30}H_{42}N_8O_8 \cdot HCl \cdot H_2O$<br>C 53.88 (53.85)<br>H 6.92 ( 6.78)<br>N 12.31 (12.56) |
| | B | 16a<br>→<br>16b<br>R = 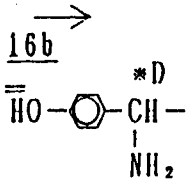 | 1) 10% Pd-C(120mg)<br>MeOH(5mℓ), $H_2$<br>3hr. evap.<br>2) TFA (3mℓ)<br>r.t. 40min, evap.<br>IRA-402($Cl^-$)<br>lyophilization | 120mg<br>→<br>23mg | 1690<br>1645<br>1520 | $C_{17}H_{28}N_8O_4 \cdot 3HCl \cdot H_2O$<br>C 40.00 (40.21)<br>H 6.52 ( 6.55)<br>N 16.23 (16.55) |

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 17 | A | X III → 17a<br>R' = 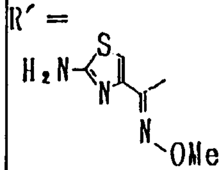 | R'$CO_2$H (402mg)<br>HOBT (271mg)<br>DCC (413mg)<br>DMF(3mℓ), 1 5 hr, r.t.<br>evap. CHP-20P column<br>50% MeOH-$H_2O$<br>lyophilization | 331mg → 500mg | 1705<br>1680<br>1515 | $C_{20}H_{34}O_6N_6S \cdot HCl \cdot 1.5H_2O$<br>C 41.25 (41.55)<br>H 6.70 ( 6.63)<br>N 19.09 (19.38) |
| | B | 17a → 17b<br>R = 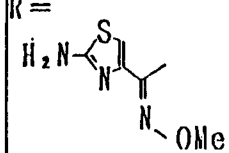 | TFA (3mℓ)<br>r.t. 1hr<br>evap. washing with $Et_2O$<br>CHP-20P, $H_2O$<br>lyophilization | 500mg → 200mg | 1690<br>1520 | $C_{15}H_{20}N_8O_4 \cdot 3HCl \cdot 2H_2O$<br>C 34.05 (34.13)<br>H 6.18 ( 6.30)<br>N 21.12 (21.23) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 18 | A, B | XIII → 18b<br>R = $Ph_2CHO_2C$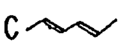 | $R'CO_2H$ (185mg)<br>HOBT (81mg)<br>DCC (124mg)<br>24hr, r.t. evap. 50% extraction with 50% EtOH<br>CHP-20P, 1/100N · HCl<br>50% EtOH,<br>lyophilization | 202mg → 237mg | 1715<br>(sh)<br>1690<br>1650<br>1620<br>1550 | $C_{28}H_{35}N_8O \cdot 2HCl \cdot H_2O$<br>C 61.20 (61.31)<br>H 7.05 (7.17)<br>N 12.63 (12.77) |
| 19 | B | 18b → 19b<br>R = $HO_2C$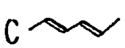 | THA (2ml)<br>r.t. 30min, evap.<br>IRA-402(Cl⁻), $H_2O$<br>lyophilization | .138mg → 78mg | 1730 – 1610<br>1550 | $C_{16}H_{26}N_6O_6 \cdot 2CF_3CO_2H \cdot H_2O$<br>C 38.07 (37.94)<br>H 4.85 (4.86)<br>N 11.86 (11.65) |

| Example No. | Step | Starting Compd. → Product | Rection Conditions | Yield Material → Product | I R $\nu_{max}^{KBr}$ $cm^{-1}$ | Molecular Formula of Product / Elemental Analysis (Calcd.) |
|---|---|---|---|---|---|---|
| 20 | A | XIII → 20a<br>R′ = 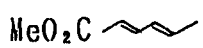 | $R'CO_2H$ (52mg)<br>$Et_3N$ (63μℓ)<br>EEDQ (89mg)<br>DMF (3mℓ)<br>r.t.20hr., 60℃,10hr<br>evap. 1N-HCl(pH3.0)<br>CHP-20P, 20% $EtOH/H_2O$<br>lyophilization | 122mg → 107mg | 1720<br>1690<br>1655<br>1530 | $C_{21}H_{35}N_5O_7 \cdot HCl \cdot 0.5H_2O$<br>C 49.11 (48.98)<br>H 7.35 ( 7.24)<br>N 13.39 (13.60) |
| | B | 20a → 20b<br>R = 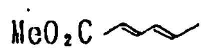 | TFA (2mℓ)<br>0℃, 30min<br>evap. IRA-402($Cl^-$)<br>$H_2O$,<br>lyophilization | 106mg → 93mg | 1720 (sh)<br>1690<br>1650 | $C_{18}H_{27}N_6O_5 \cdot 2HCl \cdot 0.5H_2O$<br>C 42.52 (42.58)<br>H 6.56 ( 6.70)<br>N 15.23 (15.52) |

| Example No. | Step | Starting Compd. → Product | Reaction Conditions | Yield Material → Product | IR $\nu_{max}^{KBr}$ $cm^{-1}$ | Molecular Formula of Product : Elemental Analysis (Calcd.) |
|---|---|---|---|---|---|---|
| 21 | A | XIII → 21a<br>R' = $nC_8H_{17}O_2C$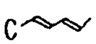 | R'$CO_2H$ (84mg)<br>EEDQ (89mg)<br>$Et_3N$ (63μℓ)<br>DMF (3mℓ)<br>60℃, 6hr, evap.<br>1N-HCl(pH3.0), CHP-20P<br>50% EtOH-$H_2O$<br>lyophilization | 122mg → 110mg | 1710 (sh)<br>1690<br>1655<br>1535 | $C_{28}H_{40}N_8O_7$ · HCl · 1.5$H_2O$<br>C 53.04 (53.28)<br>H 8.70 ( 8.46)<br>N 10.89 (11.10) |
| | B | 21a → 21b<br>R = $nC_8H_{17}O_2C$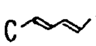 | TFA (2mℓ)<br>0℃, 45min<br>evap. IRA-402(Cl⁻)<br>$H_2O$<br>lyophilization | 108mg → 69mg | 1720 (sh)<br>1690<br>1655 | $C_{23}H_{41}N_5O_5$ · 2HCl · 2$H_2O$<br>C 47.62 (47.91)<br>H 7.97 ( 8.22)<br>N 11.96 (12.15) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 22 | A | XIII → 22a<br>R' =<br>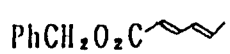 | R'$CO_2H$ (77mg)<br>EEDQ (89mg)<br>$Et_3N$ (63μℓ)<br>DMF (3mℓ)<br>60°C, 4hr, evap.<br>1N-HCl(pH3.0), CHP-20P<br>40% EtOH-$H_2O$<br>lyophilization | 122mg → 124mg | 1710 (sh)<br>1690<br>1655<br>1625<br>1520 | $C_{27}H_{30}N_8O_7 \cdot HCl \cdot H_2O$<br>C 54.22 (54.04)<br>H 6.88 ( 7.05)<br>N 11.73 (11.67) |
| | B | 22a → 22b<br>R =<br>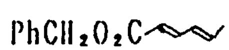 | TFA (2mℓ)<br>0°C, 30min, evap.<br>IRA-402($Cl^-$), $H_2O$<br>conc., CHP-20P,<br>20% EtOH-$H_2O$<br>lyophilization | 123mg → 100mg | 1710 (sh)<br>1690<br>1650<br>1550 | $C_{22}H_{31}N_5O_6 \cdot 2HCl \cdot 1.5H_2O$<br>C 48.59 (48.44)<br>H 6.22 ( 6.65)<br>N 12.73 (12.84) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 23 | A | XIII → 23a<br>R' = 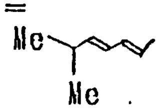 | R'$CO_2H$ (62mg)<br>EEDQ (119mg)<br>$Et_3N$ (84 μℓ)<br>DMF (3mℓ)<br>55°C, 5hr, evap.<br>1N-HCl (pH2.5), CHP-20P<br>50% EtOH-$H_2O$<br>lyophilization | 162mg → 129mg | 1710 (sh)<br>1690<br>1650<br>1535 | $C_{22}H_{30}N_6O_5 \cdot HCl \cdot H_2O$<br>C 52.13 (52.01)<br>H 8.22 ( 8.33)<br>N 13.80 (13.78) |
| | B | 23a → 23b<br>R = 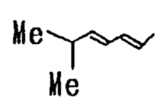 | TFA (2mℓ)<br>r.t, 40min, evap.<br>IRA-402($Cl^-$), $H_2O$<br>conc., CHP-20P,<br>15% EtOH-$H_2O$<br>lyophilization | 128mg → 85mg | 1690<br>1655<br>1545 | $C_{17}H_{31}N_6O_3 \cdot 2HCl \cdot 1.5H_2O$<br>C 45.13 (45.03)<br>H 8.09 ( 8.00)<br>N 15.25 (15.45) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 24 | A | <u>XIII</u> → <u>24a</u><br>R′ = 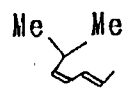 | R′$CO_2$H (62mg)<br>EEDQ (119mg)<br>$Et_3N$ (84 μℓ)<br>DMF (3mℓ)<br>55°C, 5hr, evap.<br>1N-HCl(pH2.5), CHP-20P<br>50% EtOH-$H_2O$<br>lyophilization | 162mg → 127mg | 1710 (sh)<br>1690<br>1650<br>1530 | $C_{22}H_{30}N_6O_6$ · HCl · 1.2$H_2O$<br>C 51.69 (51.64)<br>H 8.21 ( 8.35)<br>N 13.57 (13.69) |
| | B | <u>24a</u> → <u>24b</u><br>R = 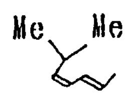 | TFA (2mℓ)<br>r.t, 50min, evap.<br>IRA-402(Cl⁻), $H_2O$<br>conc., CHP-20P,<br>15% EtOH-$H_2O$<br>lyophilization | 126mg → 93mg | 1690<br>1655<br>1545 | $C_{17}H_{31}N_5O_3$ · 2HCl · 1.5$H_2O$<br>C 44.92 (45.03)<br>H 8.50 ( 8.00)<br>N 15.33 (15.45) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 25 | A | <u>XIII</u><br>→<br><u>25a</u><br>R′ =<br>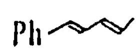 | R′$CO_2$H (77mg)<br>EEDQ (119mg)<br>$Et_3N$ (84 μℓ)<br>DMF (3mℓ)<br>60°C, 6hr, evap.<br>1N-HCl(pH3.0), CHP-20P<br>50% EtOH-$H_2O$<br>lyophilization | 162mg<br>→<br>151mg | 1710 (sh)<br>1690<br>1650<br>1610<br>1530 | $C_{25}H_{37}N_5O_6 \cdot HCl \cdot 1.5H_2O$<br>C 54.51 (54.49)<br>H 7.36 ( 7.50)<br>N 12.88 (12.71) |
| | B | <u>25a</u><br>→<br><u>25b</u><br>R =<br>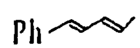 | TFA (2mℓ)<br>r.t, 1hr, evap.<br>IRA-402($Cl^-$), $H_2O$<br>conc., CHP-20P,<br>15% EtOH-$H_2O$<br>lyophilization | 150mg<br>→<br>96mg | 1690<br>1645<br>1610<br>1540 | $C_{20}H_{29}N_5O_3 \cdot 2HCl \cdot 1.5H_2O$<br>C 49.10 (49.28)<br>H 7.03 ( 7.03)<br>N 14.02 (14.37) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 26 | A | XIII → 26a<br>R' = 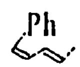 | R'$CO_2H$ (77mg)<br>EEDQ (119mg)<br>$Et_3N$ (84μℓ)<br>DMF (3mℓ)<br>55℃, 10hr, evap.<br>1N-HCl(pH2.5), CHP-20P<br>50% EtOH-$H_2O$<br>lyophilization | 162mg → 171mg | 1690<br>1650<br>1530 | $C_{25}H_{37}N_5O_5 \cdot HCl \cdot H_2O$<br>C 55.18 (55.39)<br>H 7.57 (7.44)<br>N 12.86 (12.92) |
| | B | 26a → 26b<br>R = 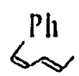 | TFA (2mℓ)<br>r.t, 1hr, evap.<br>IRA-402($Cl^-$), $H_2O$<br>lyophilization | 170mg → 104mg | 1690<br>1645<br>1605<br>1545 | $C_{20}H_{29}N_5O_3 \cdot 2HCl \cdot 1.5H_2O$<br>C 49.05 (49.28)<br>H 7.15 (7.03)<br>N 14.20 (14.37) |

| Example No. | Step | Starting Compd. ⟶ Product | Reaction Conditions | Yield Material ⟶ Product | IR $\nu_{max}^{KBr}$ $cm^{-1}$ | Molecular Formula of Product<br>Elemental Analysis (Calcd.) |
|---|---|---|---|---|---|---|
| 27 | A | <u>XIII</u><br>⟶<br><u>27a</u><br>R′ =<br>$F_2CHSCH_2$- | $R'CO_2H$ (213mg)<br>HOBT (162mg)<br>DCC (248mg)<br>$Et_3N$ (210mg)<br>DMF (3mℓ)<br>r.t. 24hr, evap.<br>0.5N-HCl (pH3.0),<br>CHP-20P, 30% EtOH-$H_2O$<br>lyophilization | 404mg<br>⟶<br>600mg | 1710<br>(sh)<br>1680<br>1530 | $C_{17}H_{31}F_2N_5O_5S \cdot HCl$<br>C — (41.50)<br>H — ( 6.56)<br>N — (14.23) |
| | B | <u>27a</u> ⟶ <u>27b</u><br>R =<br>$F_2CHSCH_2$- | TFA (6mℓ)<br>r.t. 1hr, evap.<br>IRA-402($Cl^-$), $H_2O$<br>conc., CHP-20P, $H_2O$<br>lyophilization | 580mg<br>⟶<br>362mg | 1690<br>1675<br>1520 | $C_{12}H_{20}F_2N_6O_3S \cdot 2HCl \cdot H_2O$<br>C 32.64 (32.29)<br>H 6.02 ( 6.10)<br>N 16.00 (15.69) |

| Example No. | Step | Starting Compd. → Product | Reaction Conditions | Yield Material → Product | IR $\nu_{max}^{KBr}$ $cm^{-1}$ | Molecular Formula of Product Elemental Analysis (Calcd.) |
|---|---|---|---|---|---|---|
| 28 | A | XIII → 28a R' = [1] *S *D 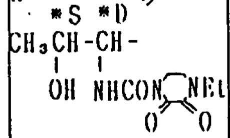 | $R'CO_2H$ (517mg) HOBT (243mg) DCC (372mg) $Et_3N$ (310mg) DMF (5mℓ) r.t. 40hr, evap. 1/2N-HCl (pH3.0), CHP-20P, 20% $MeOH-H_2O$ lyophilization | 606mg → 300mg | 1710, 1670, 1515 | $C_{25}H_{44}N_8O_9 \cdot HCl \cdot 1.5H_2O$ C 45.04 (45.21) H 7.40 (7.28) N 16.74 (16.87) |
| | B | 28a → 28b R = *S *D 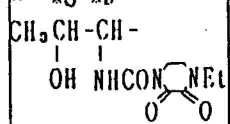 | TFA (3mℓ) r.t. 1hr, evap IRA-402($Cl^-$), $H_2O$ conc., CHP-20P 20% $MeOH-H_2O$ lyophilization | 280mg → 127mg | 1690 1665 1520 | $C_{20}H_{36}N_8O_7 \cdot 2HCl \cdot 1.5H_2O$ C 40.23 (40.00) H 6.59 (6.86) N 18.42 (18.66) |

[1] *S: steric configuration shown by R or S (The same shall apply hereinafter.)

*D: steric configuration shown by D or L (The same shall apply hereinafter.)

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 29 | A | XIII<br>→<br>29a<br>R' = Me<br>CbzNHNCH$_2$- | R'$CO_2H$ (429mg)<br>HOBT (243mg)<br>DCC (371mg)<br>$Et_3N$ (310mg)<br>DMF (5ml)<br>r.t, 72hr, evap.<br>0.5N-HCl(pH3.0),<br>CHP-20P, 50% MeOH-$H_2O$<br>lyophilization | 606mg<br>→<br>900mg | 1710<br>(sh)<br>1670<br>1530 | $C_{25}H_{41}N_7O_7 \cdot HCl \cdot H_2O$<br>C 49.58 (49.54)<br>H 7.16 (7.32)<br>N 16.20 (16.18) |
| | B | 29a → 29b<br>R = Me<br>CbzNHNCH$_2$- | TFA (6ml)<br>r.t. 1hr, evap<br>IRA-402($Cl^-$), $H_2O$<br>conc., CHP-20P<br>20% MeOH-$H_2O$<br>lyophilization | 880mg<br>→<br>492mg | 1700<br>1670<br>1520 | $C_{20}H_{33}N_7O_5 \cdot 2HCl \cdot 2H_2O$<br>C 43.10 (42.86)<br>H 7.01 (7.01)<br>N 17.53 (17.49) |

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 30 | B | <u>29b</u> → <u>30b</u><br>R = $H_2NN(Me)CH_2$- | $H_2O$ (5 ml)<br>10% Pd-C (150mg)<br>$H_2$, 1hr, filtration<br>IRA-402($Cl^-$), $H_2O$<br>lyophilization | 280mg → 234mg | 1690<br>1660<br>1530 | $C_{12}H_{27}N_7O_3 \cdot 3HCl \cdot 2H_2O$<br>C 31.10 (31.14)<br>H 7.20 (7.40)<br>N 21.11 (21.19) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 31 | A | XIII → 31a<br>R′ =<br>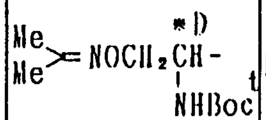 | $R'CO_2H$ (580mg)<br>HOBT (270mg)<br>DCC (412mg)<br>$Et_3N$ (310mg)<br>DMF (5mℓ)<br>r.t., 24hr, evap.<br>1N-HCl(pH3.0), CHP-20P<br>50% MeOH-$H_2O$<br>lyophilization | 606mg → 600mg | 1710<br>(sh)<br>1690<br>1650<br>1520 | $C_{25}H_{47}N_7O_8 \cdot HCl \cdot H_2O$<br>C 47.84 (47.80)<br>H 7.92 (8.02)<br>N 15.66 (15.61) |
| | B | 31a → 31b<br>R =<br>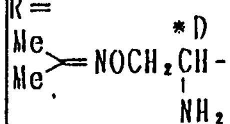 | TFA (6mℓ)<br>r.t. 1hr, evap.<br>IRA-402($Cl^-$), $H_2O$<br>conc., CHP-20P, $H_2O$<br>lyophilization | 580mg → 348mg | 1690<br>1645<br>1520 | $C_{16}H_{31}N_7O_4 \cdot 3HCl \cdot H_2O$<br>C 35.69 (35.97)<br>H 7.12 (7.24)<br>N 19.28 (19.58) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 32 | A | XIII → 32a<br>R′ =<br>$H_2NCOCH_2CH-$ *L [1]<br>(C=O; CH–$NHBoc^t$) | R′$CO_2H$ (447mg)<br>HOBT (243mg)<br>DCC (371mg)<br>$Et_3N$ (350mg)<br>DMF (5mℓ)<br>r.t, 4days, evap.<br>0.5N-HCl(pH3.0),<br>CHP-20P, 50% $MeOH-H_2O$<br>lyophilization | 606mg<br>→<br>800mg | 1705<br>1685<br>1520 | $C_{23}H_{43}N_7O_9 \cdot HCl \cdot 0.5H_2O$<br>C 45.39 (45.50)<br>H 7.52 (7.47)<br>N 16.01 (16.15) |
| | B | 32a → 32b<br>R =<br>$H_2NCOCH_2CH-$ *L<br>(C=O; CH–$NH_2$) | TFA (6mℓ)<br>r.t. 1hr, evap.<br>IRA-402($Cl^-$), $H_2O$<br>conc., CHP-20P, $H_2O$<br>lyophilization | 780mg<br>→<br>490mg | 1690<br>1650<br>1520 | $C_{13}H_{27}N_7O_5 \cdot 3HCl \cdot 2H_2O$<br>C 30.64 (30.81)<br>H 6.59 (6.76)<br>N 19.26 (19.35) |

[1] *L: L-compound (The same shall apply hereinafter.)

| Example No. | Step | Starting Compd. → Product | Reaction Conditions | Yield Material → Product | IR $\nu_{max}^{KBr}$ $cm^{-1}$ | Molecular Formula of Product / Elemental Analysis (Calcd.) |
|---|---|---|---|---|---|---|
| 33 | A | <u>X III</u> → <u>33a</u><br>R′ = $ClCH_2CONHCO-CH_2CH(NHBoc)-$ (*L) | $R'CO_2H$ (535mg)<br>HOBT (223mg)<br>DCC (341mg)<br>$Et_3N$ (310mg)<br>DMF (5mℓ)<br>r.t. 3days, evap.<br>0.5N-HCl (pH3.0),<br>CHP-20P, 50% MeOH-$H_2O$<br>lyophization | 606mg → 200mg | 1710,<br>1680,<br>1520 | $C_{26}H_{44}ClN_7O_{10}\cdot HCl\cdot 0.5H_2O$<br>C 43.82 (43.93)<br>H 6.90 (6.78)<br>N 14.18 (14.34) |
| | B | <u>33a</u> → <u>33b</u><br>R = $ClCH_2CONHCO-CH_2CH(NH_2)-$ (*L) | TFA (2mℓ)<br>r.t. 1hr, evap.<br>IRA-402($Cl^-$), $H_2O$<br>conc., CHP-20P, $H_2O$<br>lyophilization | 198mg → 138mg | 1690<br>1520 | $C_{16}H_{20}ClN_7O_8\cdot 3HCl\cdot 0.5H_2O$<br>C 32.09 (32.39)<br>H 5.95 (5.80)<br>N 17.43 (17.63) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 34 | A | XIII → 34a<br>R' = $H_2NSO_2CH_2CH(NHCbz)-$ (*L) | R'$CO_2H$ (340mg)<br>HOBT (152mg)<br>DCC (232mg)<br>$Et_3N$ (233mg)<br>DMF (5ml)<br>r.t. 4days, evap.<br>0.5N-HCl (pH3.0),<br>CHP-20P, 50% MeOH-$H_2O$<br>lyophilization | 404mg → 103mg | 1710 (sh)<br>1680<br>1520 | $C_{28}H_{40}N_7O_9S \cdot HCl \cdot H_2O$<br>C 44.93 (44.87)<br>H 6.63 (6.48)<br>N 14.48 (14.65) |
| | B | 34a → 34b<br>R = $H_2NSO_2CH_2CH(NHCbz)-$ (*L) | TFA (2ml)<br>r.t. 1hr, evap.<br>IRA-401 ($Cl^-$), $H_2O$<br>conc., CHP-20P,<br>20% MeOH-$H_2O$<br>lyophilization | 100mg → 48mg | 1690<br>1520 | $C_{20}H_{32}N_7O_7S \cdot 2HCl \cdot H_2O$<br>C 39.82 (39.67)<br>H 5.81 (5.99)<br>N 16.40 (16.16) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 35 | A | X III<br>→<br>35a<br>R' =<br>Me 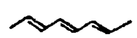 | $R'CO_2H$ (33mg)<br>$Et_3N$ (50 μℓ)<br>HOBT (37mg)<br>DCC (56mg)<br>DMF (2mℓ)<br>r.t., 20hr, evap.<br>1N-HCl (pH3.0),<br>CHP-20P, 50% EtOH-$H_2O$<br>lyophilization | 97mg<br>→<br>121mg | 1710<br>1690<br>1650<br>1545 | $C_{22}H_{37}N_5O_5 \cdot HCl \cdot H_2O$<br>C 52.10 (52.22)<br>H 7.77 ( 7.97)<br>N 13.91 (13.84) |
| | B | 35a → 35b<br>R =<br>Me 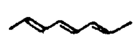 | TFA (2mℓ)<br>r.t. 1.5hr, evap<br>CHP-20P, 30% EtOH-$H_2O$,<br>conc., IRA-401($Cl^-$)<br>$H_2O$<br>lyophilization | 116mg<br>→<br>54mg | 1690<br>1650<br>1600<br>1550 | $C_{17}H_{19}N_5O_3 \cdot 2HCl \cdot 1.5H_2O$<br>C 46.14 (46.27)<br>H 5.36 ( 5.48)<br>N 15.90 (15.87) |

| Example No. | Step | Starting Compd. → Product | Reaction Conditions | Yield Material → Product | IR $\nu_{max}^{KBr}$ $cm^{-1}$ | Molecular Formula of Product Elemental Analysis (Calcd.) |
|---|---|---|---|---|---|---|
| 36 | A, B | <u>XIII</u> → <u>36b</u> R, R′ = $PhCH_2OCO$ 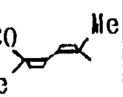 | $R'CO_2H$ (202mg)<br>EEDQ (149mg)<br>$Et_3N$ (105 $\mu\ell$)<br>DMF (3 $m\ell$)<br>60°C, 6hr, evap.<br>CHP-20P, 1/100N-HCl - 50%, EtOH<br>lyophilization | 202mg → 140mg | 1710 (sh)<br>1690<br>1640<br>1605<br>1530 | $C_{30}H_{39}N_5O_5 \cdot 2HCl \cdot 1.5H_2O$<br>C 55.14 (55.47)<br>H 6.75 ( 6.83)<br>N 10.85 (10.78) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 37 | A | XIII → 37a<br>R′ = 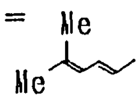 | R′$CO_2$H (61mg)<br>HOBT (65mg)<br>DCC (99mg)<br>$Et_3N$ (84μℓ)<br>r.t., 24hr, evap.<br>1N-HCl (pH3.0),<br>CHP-20P, 40% EtOH-$H_2O$<br>lyophilization | 162mg → 226mg | 1710<br>(sh)<br>1650<br>1530 | $C_{21}H_{37}N_5O_5$ · HCl · 0.5$H_2O$<br>C 52.11 (52.00)<br>H 8.01 (8.10)<br>N 14.59 (14.44) |
| | B | 37a → 37b<br>R = 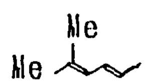 | EtOH (10mℓ)<br>1N-HCl (1mℓ)<br>evap. CHP-20P, $H_2O$<br>lyophilization | 226mg → 138mg | 1685<br>1645<br>1530 | $C_{18}H_{20}N_6O_3$ · 2HCl · 2.5$H_2O$<br>C 42.27 (42.02)<br>H 7.98 (7.93)<br>N 15.49 (15.31) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 38 | A | XIII<br>→<br>38a<br>R′ = 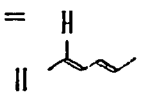 | R′$CO_2H$ (59mg)<br>HOBT (41mg)<br>DCC (124mg)<br>$Et_3N$ (105μℓ)<br>DMF (2mℓ);<br>r.t., 28hr, evap.<br>1N-HCl (pH2.5),<br>CHP-20P, 30% EtOH-$H_2O$<br>lyophilization | 202mg<br>→<br>125mg | 1710<br>(sh)<br>1690<br>1650<br>1630<br>1545 | $C_{19}H_{33}N_5O_5 \cdot HCl \cdot 2H_2O$<br>C 47.01 (47.15)<br>H 7.99 (7.91)<br>N 14.51 (14.47) |
| | B | 38a → 38b<br>R = 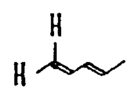 | TFA (1.5mℓ)<br>r.t., 1hr, evap.<br>CHP-20P, $H_2O$<br>lyophilization | 124mg<br>→<br>99mg | 1670<br>1550 | $C_{14}H_{25}N_5O_5 \cdot 2CF_3CO_2H$<br>C 37.62 (37.57)<br>H 5.80 (5.43)<br>N 12.09 (12.17) |

| Example No. | Step | Starting Compd. ⟶ Product | Reaction Conditions | Yield Material ⟶ Product | I R $\nu_{max}^{KBr}$ $cm^{-1}$ | Molecular Formula of Product<br>Elemental Analysis (Calcd.) |
|---|---|---|---|---|---|---|
| 39 | A | XIII<br>⟶<br>39a<br>R' = 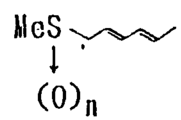<br>n=0:n=1=40:60 | $R'CO_2H$ (160mg)<br>HOBT (124mg)<br>DCC (189mg)<br>$Et_3N$ (168 μℓ)<br>DMF (3mℓ)<br>r.t., 20hr, evap.<br>1N-HCl (pH2.5),<br>CHP-20P, 40% EtOH-$H_2O$<br>lyophilization | 303mg<br>⟶<br>420mg | 1710<br>(sh)<br>1695<br>1660<br>1640 | $C_{21}H_{37}N_5O_6S \cdot HCl$ (n = 0)<br>$C_{21}H_{37}N_5O_6S \cdot HCl$ (n = 1)<br>——— |
| | B | 39a ⟶ 39b<br>R = 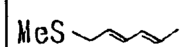 | TFA (2mℓ)<br>r.t., 1hr, evap.<br>CHP-20P, 5% EtOH-$H_2O$,<br>conc., IRA-402($Cl^-$),<br>$H_2O$, lyophilization | 420mg<br>⟶<br>130mg | 1695<br>1670<br>1655<br>1550 | $C_{19}H_{36}N_6O_5S \cdot 2HCl \cdot H_2O$<br>(isolate only sulfide)<br>C 41.18 (41.56)<br>H 7.19 ( 7.19)<br>N 15.02 (15.14) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 40 | A | X III → 40a<br>R′ =<br>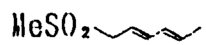 | $R'CO_2H$ (84mg)<br>HOBT (27mg)<br>DCC (91mg)<br>$Et_3N$ (84 μℓ)<br>DMF (2mℓ)<br>r.t., 16hr, evap.<br>1N-HCl (pH2.5),<br>CHP-20P, 20% EtOH-$H_2O$<br>lyophilization | 162mg → 225mg | 1710<br>(sh)<br>1690<br>1660<br>1540 | $C_{21}H_{37}N_5O_7S \cdot HCl \cdot 1.5H_2O$<br>C 44.53 (44.48)<br>H 7.16 ( 7.29)<br>N 12.18 (12.35) |
| | B | 40a → 40b<br>R =<br>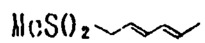 | TFA (2mℓ)<br>r.t., 1hr, evap.<br>CHP-20P, $H_2O$, conc.<br>IRA-402($Cl^-$), $H_2O$<br>lyophilization | 220mg → 176mg | 1690<br>1660<br>1630<br>1550 | $C_{16}H_{29}N_5O_6S \cdot 2HCl \cdot 1.5H_2O$<br>C 38.11 (38.17)<br>H 6.66 ( 6.81)<br>N 13.79 (13.91) |

| Example No. | Step | Starting Compd. ⟶ Product | Reaction Conditions | Yield Material ⟶ Product | IR $\nu_{max}^{KBr}$ $cm^{-1}$ | Molecular Formula of Product<br>Elemental Analysis (Calcd.) |
|---|---|---|---|---|---|---|
| 41 | A | XIII<br>⟶<br>41a<br>R' = 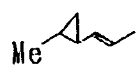 | R'$CO_2H$ (76mg)<br>HOBT (34mg)<br>DCC (124mg)<br>$Et_3N$ (105μl)<br>DMF (2ml)<br>r.t., 3days, evap.<br>1N-HCl(pH2.5),<br>CHP-20P, 40% EtOH-$H_2O$<br>lyophilization | 203mg<br>⟶<br>231mg | 1710<br>(sh)<br>1690<br>1660<br>1530 | $C_{21}H_{37}N_5O_5 \cdot HCl \cdot H_2O$<br>C 50.89 (51.06)<br>H 8.03 (8.16)<br>N 14.26 (14.18) |
| | B | 41a ⟶ 41b<br>R = 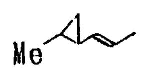 | TFA (2ml)<br>anisole (2ml)<br>0°C, 1.5hr, evap.<br>CHP-20P, $H_2O$, conc.<br>IRA-402($Cl^-$), $H_2O$<br>lyophilization | 230mg<br>⟶<br>134mg | 1690<br>1655<br>1545 | $C_{16}H_{29}N_5O_3 \cdot 2HCl \cdot H_2O$<br>C 44.41 (44.65)<br>H 7.90 (7.73)<br>N 15.94 (16.27) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 42 | A | XIII → 42a<br>R′ = 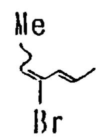 | $R'CO_2H$ (92mg)<br>HOBT (27mg)<br>DCC (99mg)<br>$Et_3N$ (84 μl)<br>DMF (2ml)<br>r.t., 16hr., evap.<br>CHP-20P, 40% EtOH-$H_2O$<br>lyophilization | 162mg → 215mg | 1710<br>1650<br>1530 | $C_{20}H_{31}BrN_5O_5 \cdot HCl \cdot 0.5H_2O$<br>C 43.49 (43.68)<br>H 6.52 (6.60)<br>N 12.86 (12.74) |
| | B | 42a → 42b<br>R = 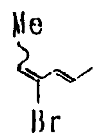 | TFA (2ml)<br>r.t., 40min, evap.<br>CHP-20P, 5% EtOH-$H_2O$.<br>conc., IRA-402($Cl^-$),<br>$H_2O$, lyophilization | 213mg → 142mg | 1690<br>1655<br>1550 | $C_{15}H_{26}BrN_5O_3 \cdot 2HCl \cdot 0.5H_2O$<br>C 36.92 (37.05)<br>H 5.90 (6.01)<br>N 14.21 (14.40) |

| Example No. | Step | Starting Compd. → Product | Reaction Conditions | Yield Material → Product | I R $\nu_{max}^{KBr}$ $cm^{-1}$ | Molecular Formula of Product Elemental Analysis (Calcd.) |
| --- | --- | --- | --- | --- | --- | --- |
| 43 | A | XIII → 43a<br>R' = 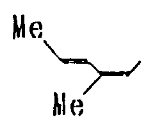 | R'$CO_2H$ (61mg)<br>HOBT (27mg)<br>DCC (99mg)<br>$Et_3N$ (84 μℓ)<br>DMF (2mℓ)<br>r.t., 3days., evap.<br>1N-HCl(pH2.5),<br>CHP-20P, 30% EtOH-$H_2O$<br>lyophilization | 162mg → 187mg | 1710<br>(sh)<br>1690<br>1640<br>1530 | $C_{21}H_{37}N_5O_5 \cdot HCl \cdot H_2O$<br>C 51.14 (51.06)<br>H 8.23 ( 8.16)<br>N 14.13 (14.18) |
| | B | 43a → 43b<br>R = 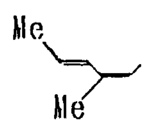 | TFA (2mℓ)<br>r.t., 1hr, evap.<br>CHP-20P, 5% EtOH-$H_2O$,<br>conc.; IRA-402($Cl^-$),<br>$H_2O$, lyophilization | 186mg → 71mg | 1690<br>1650<br>1640 | $C_{10}H_{20}N_5O_5S \cdot 2HCl \cdot H_2O$<br>C 36.60 (36.38)<br>H 6.01 ( 6.11)<br>N 14.19 (14.14) |

| Example No. | Step | Starting Compd. → Product | Reaction Conditions | Yield Material → Product | IR $\nu_{max}^{KBr}$ $cm^{-1}$ | Molecular Formula of Product<br>Elemental Analysis (Calcd.) |
|---|---|---|---|---|---|---|
| 44 | A | <u>XIII</u><br>→<br><u>44a</u><br>R′ = 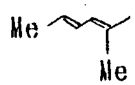 | $R'CO_2H$ (61mg)<br>HOBT (27mg)<br>DCC (99mg)<br>$Et_3N$ (84μℓ)<br>r.t., 16hr., evap.<br>CHP-20P, 30% $EtOH$-$H_2O$<br>lyophilization | 162mg<br>→<br>174mg | 1710<br>(sh)<br>1690<br>1650<br>1530 | $C_{21}H_{37}N_5O_5 \cdot HCl \cdot 1.5H_2O$<br>C 50.00 (50.14)<br>H 8.13 (8.22)<br>N 14.02 (13.92) |
| | B | <u>44a</u> → <u>44b</u><br>R = 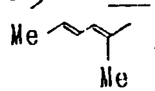 | TFA (2mℓ)<br>r.t., 1hr, evap.<br>CHP-20P, 5% $EtOH$-$H_2O$,<br>conc., IRA-402($Cl^-$),<br>$H_2O$, lyophilization | 172mg<br>→<br>133mg | 1690<br>1650<br>1590<br>1530 | $C_{10}H_{20}N_6O_9 \cdot 2HCl \cdot H_2O$<br>C 36.40 (36.38)<br>H 6.11 (6.11)<br>N 14.19 (14.14) |

| Example No. | Step | Starting Compd. → Product | Reaction Conditions | Yield Material → Product | I R $\nu_{max}^{KBr}$ $cm^{-1}$ | Molecular Formula of Product<br>Elemental Analysis (Calcd.) |
|---|---|---|---|---|---|---|
| 45 | A | XIII · 2HCl → IIIa<br>R′ = 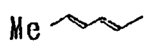 | R′$CO_2H$ (307mg)<br>$Et_3N$ (480μl)<br>HOBT (369mg)<br>DCC (563mg)<br>DMF (10ml)<br>0°C 1hr, r.t., 8hr,<br>evapo. of DMF, HP-20,<br>lyophilization | 964mg<br>→<br>999mg | 3270<br>1660<br>1515 | $C_{20}H_{35}N_5O_6$ · HCl · 0.5$H_2O$<br>C 50.83 (51.00)<br>H 8.01 ( 7.92)<br>N 14.44 (14.87)<br>Cl 7.57 ( 7.53) |
| | B | IIIa → IIIb<br>R = 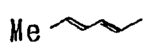 | TFA (5ml)<br>r.t., 0.5hr<br>treatment with $Et_2O$<br>IRA-402($Cl^-$), $H_2O$<br>lyophilization | 853mg<br>→<br>725mg | 3250<br>1660<br>1550 | $C_{15}H_{27}N_5O_3$ · 2HCl · 0.5$H_2O$<br>C 44.35 (44.23)<br>H 7.83 ( 7.42)<br>N 17.28 (17.19)<br>Cl 17.59 (17.41) |

Example 46

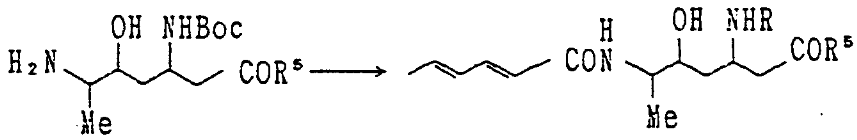

XIV

IVa : R = Boc

IV : R = H

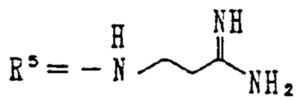

Step A (XIV → IVa)

To a suspension of dihydrochloride of Compound XIV (180 mg) in DMF (2 mℓ) were added under ice-cooling $Et_3N$ (86μℓ), sorbic acid (56 mg), HOBT (67 mg) and DCC (103 mg). The reaction was allowed to proceed in a manner similar to the Step A of Example 1 to obtain hydrochloride of Compound IVa (178 mg) as white powder.
IR $\nu_{max}$ (KBr) $cm^{-1}$:3260,1660,1510

| Elemental Analysis : $C_{20}H_{35}N_5O_5 \bullet HCl \bullet H_2O$ | | | | |
|---|---|---|---|---|
| Calcd.<br>Found | C, 51.06;<br>C, 51.00; | H, 8.16;<br>H, 8.27; | N, 14.18;<br>N, 14.27; | Cl, 7.18<br>Cl, 7.36 |

Step B (IVa → IV)

In TFA (1.5 mℓ) was dissolved hydrochloride of Compound IVa (145 mg). The reaction was allowed to proceed in a manner similar to the Step B of Example 1 to obtain dihydrochloride of Compound IV (126 mg) as white powder. This product was in complete agreement in the data of physico-chemical properties with Compound IV of natural origin.

Example 47

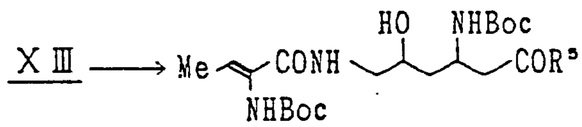

47a

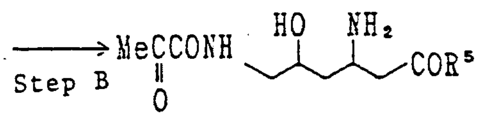


47b

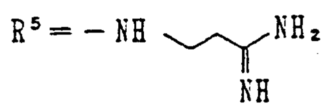

Step A (XIII → 47a)

A mixture of N-Boc-L-vinylglycine (362 mg), HOBT (243 mg), DCC (372 mg) and $CH_2Cl_2$ (4 mℓ) was stirred at room temperature for one hour, followed by filtration. To the filtrate were added XIII (606 mg), $Et_3N$ (310 mg) and DMF (3 mℓ), and the reaction was allowed to proceed at room temperature for 40 hours. The reaction mixture was concentrated under reduced pressure, followed by adjusting to pH 3.0 with 1N-HCℓ. The resultant was subjected to a CHP-20P column chromatography. Elution using 30% methanol-water, followed by freeze-drying afforded Compound 47a (600 mg) as white powder. IR $\nu_{max}$ (KBr) $cm^{-1}$:1680,1520

Step B (47a → 47b)

In TFA (3 mℓ) was dissolved Compound 47a (600 mg), and the solution was stirred at room temperature for one hour, followed by distilling off TFA under reduced pressure. The residue was dissolved in a small volume of water, and the solution was allowed to pass through Amberlite IRA-402 ($Cl^-$ type) resin (10 mℓ), followed by elution with 40 mℓ of water. The eluate was subjected to a CHP-20P column chromatography. Elution with water and lyophilization gave dihydrochloride of 47b (200 mg) as white powder.
IR $\nu_{max}$ (KBr) $cm^{-1}$:1700,1640,1525

| Elemental Analysis : $C_{13}H_{25}N_4O_4 \cdot 2HCl \cdot 3H_2O$ | | | |
|---|---|---|---|
| Calcd. | C, 36.45; | H, 7.77; | N, 13.08 |
| Found | C, 36.40; | H, 7.62; | N, 12.94 |

Example 48

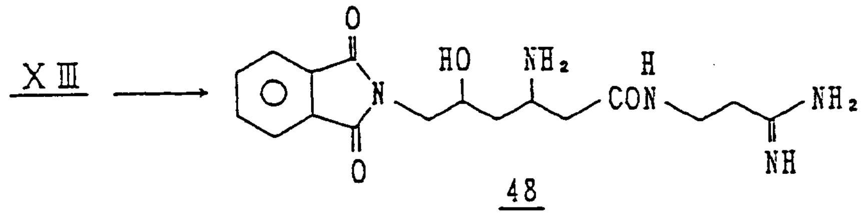


48

In DMF (2 mℓ) was dissolved dihydrochloride of Compound XIII (404 mg). To the solution were added $Et_3N$ (250 mg) and N-carboethoxyphthalimide (219 mg), and the reaction was allowed to proceed at room temperature for 20 hours, followed by distilling off DMF under reduced pressure. To the residue was added TFA (3 mℓ), and the mixture was stirred at room temperature for one hour, followed by concentration under reduced pressure. To the concentrate was added water. Insolubles were removed by filtraiton. The filtrate was allowed to pass through Amberlite IRA-402 ($Cl^-$ type) (15 mℓ). The resin was eluted with water (15 mℓ), then the eluate was concentrated. The concentrate was subjected to a column chromatography using Diaion CHP-20P (50 to 100 mesh, 30 mℓ). Elution with 20% ethanol-water, followed by lyophilization gave dihydrochloride of Compound 48 (278 mg) as white powder.
IR $\nu_{max}$ (KBr) $cm^{-1}$:1710,1690,1650,1540

| Elemental Analysis : $C_{17}H_{23}N_5O_4 \cdot 2HCl \cdot 2.5H_2O$ | | | |
|---|---|---|---|
| Calcd. | C, 42.60; | H, 6.31; | N, 14.61 |
| Found | C, 42.60; | H, 6.24; | N, 14.42 |

Example 49

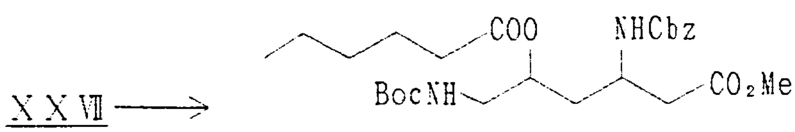


49a

To a solution of Compound XXVII(3.8 g) in methylenechloride (100 ml) was added pyridine (3.2 g). To the mixture was added, while vigorously stirring under cooling with acetone-dry ice, pulverized phosphorus pentachloride (4.16 g), followed by stirring for 1.5 hours on an ice-water bath. To the resultant was added methanol (10 mℓ) under cooling with acetone-dry ice. The mixture was stirred for 45 minutes on an ice-water bath, to which was added water(20 mℓ), followed by stirring for 30 minutes. To the reaction mixture was added a saturated aqueous solution of sodium hydrogencarbonate to neutralize. The organic layer was then separated and dried over magnesium sulfate, which was concentrated to distill off pyridine. To the concentrate were added methylene chloride (4 mℓ) and di-tert-butyl bicarbonate (4.4 g), and the mixture was stirred at room temperature for 20 hours. To the reaction mixture were added ethyl acetate and water, and the mixture was shaken. The ethyl acetate layer was taken and dried over magnesium sulfate, followed by concentration under reduced pressure. The concentrate was subjected to a silica gel($SiO_2$ 100 g) column chromatography, eluting with n-hexane : ethyl acetate = 2:1, to afford Compound 49a (3.6 g) as a pale yellow foamy product.

$$IR\,\nu_{max}^{KBr}\,cm^{-1}: 1680 \text{ to } 1740, 1510$$

| Elemental Analysis for $C_{26}H_{40}N_2O_8$ : | | | |
|---|---|---|---|
| Calcd.<br>Found | C, 61.40;<br>C, 61.58; | H, 7.93;<br>H, 8.13; | N, 5.51<br>N, 5.63 |

49a
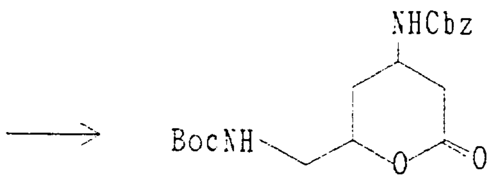
49b

To a solution of Compound 49a(1.29 g) in methanol (16 mℓ) was added 1N NaOH (8 mℓ), and the mixture was stirred at room temperature for one hour. Methanol was distilled off under reduced pressure. To the residue was added a 5% aqueous solution of $KHSO_4$ to adjust the pH to 3, followed by saturation with sodium chloride. Then precipitating oily substance was extracted with methylene chloride, and the extract was dried over magnesium sulfate, followed by concentration to a volume of about 20 mℓ. To the concentrate was added WSC(0.72 g), and the reaction was allowed to proceed at room temperature for one hour. The reaction mixture was washed with water, dried over magnesium sulfate and concentrated under reduced pressure. The concentrate was purified by means of a silica gel($SiO_2$ 30 g) column, eluting with ethyl acetate : n-hexane = 1:1, to afford Compound 49b(0.65 g) as colorless needles, m.p.136-137 ° C.

$$IR\,\nu^{KBr}_{max}\ cm^{-1}:\ 1740,\ 1700,\ 1520$$

| Elemental Analysis for $C_{19}H_{26}N_2O_6$ | | | |
|---|---|---|---|
| Calcd.<br>Found | C, 60.30;<br>C, 60.35; | H, 6.93;<br>H, 6.88; | N, 7.40<br>N, 7.41 |

Example 50

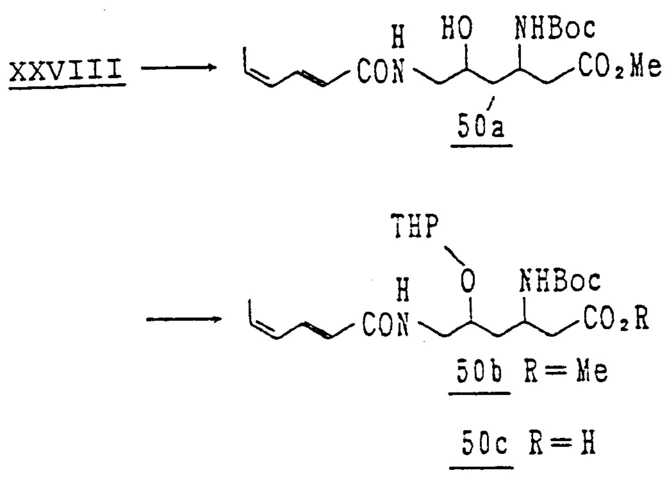

XXVIII

50a

50b R=Me

50c R=H

In methanol (70 mℓ) was dissolved Compound XXVIII (3.4 g). To the solution was added a 28% NaOMe-MeOH solution (0.3 mℓ), and the reaction was allowed to proceed at room temperature for one hour. The reaction mixture was neutralized with acetic acid, which was then concentrated under reduced pressure. The concentrate was dissolved in ethyl acetate, and the solution was washed with brine, and dried over $MgSO_4$. The resultant was concentrated under reduced pressure to give Compound 50a (3.5 g) as a white foamy substance.
IR $\nu_{max}$ (KBr) $cm^{-1}$:1740,1715,1670,1540

| Elemental Analysis : $C_{18}H_{30}N_2O_6$ | | | |
|---|---|---|---|
| Calcd.<br>Found | C, 58.36;<br>C, 58.60; | H, 8.16;<br>H, 8.10; | N, 7.56<br>N, 7.26 |

In dihydropyran (30 mℓ) was dissolved Compound 50a(6.2 g). To the solution was added under ice-cooling a catalytic amount of anhydrous p-TsOH, then the reaction was allowed to proceed at room temperature for 15 hours. To the reaction mixture was added ethyl acetate, and the mixture was washed with an aqueous solution of sodium hydrogencarbonate and a saturated brine, and dried over $MgSO_4$. The resultant was concentrated under reduced pressure, and the concentrate was subjected to a silica gel (60 g) column chromatography, followed by elution with ethyl acetate : hexane = 3 : 1 to give Compound 50b (2.9 g) as a viscous oily substance. IR $\nu_{max}$ (KBr) $cm^{-1}$:1740,1715,1665,1520

In DMSO (8 mℓ) was dissolved Compound 50b (2.4 g). To the solution was added 1N-NaOH (7 mℓ), and the reaction was allowed to proceed at room temeprature for 24 hours. To the reaction mixture was added a 5% aqueous solution of $KHSO_4$ to adjust the pH to 3, and then extracted with $CH_2Cl_2$. The $CH_2Cl_2$ layer was washed with water, which was dried over $MgSO_4$, and then concentrated under reduced pressure to give Compound 50c (1.8 g) as a white foamy substance.
IR $\nu_{max}$ (KBr) $cm^{-1}$:1725(sh),1715,1660, 1530

Example 51

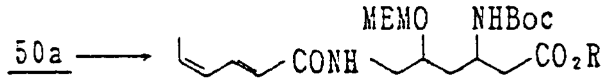

51a R=Me

51b R=H

To a solution of Compound 50a (3.5 g) in $CH_2Cl_2$ (30 mℓ) were added iso-$Pr_2NEt$ (2 g) and methoxyethoxymethylchloride (1.9 g). The reaction was allowed to proceed at room temperature for 20 hours. The reaction mixture was washed with water, dried over $MgSO_4$ and concentrated under reduced pressure. The concentrate was subjected to a silica gel(50g) column chromatography, followed by elution with ethyl acetate - hexane = 5 : 1 to give Compound 51a (3.7 g) as a viscous oily substance.
IR $\nu_{max}$ (KBr) $cm^{-1}$:1740,1715,1665,1530

To a solution of Compound 51a (3.7 g) in a mixture of DMSO (4 mℓ) and MeOH (4 mℓ) was added under ice-cooling 1N-NaOH (14 mℓ), followed by stirring at room temperature for one hour. To the reaction mixture was added a 5% aqueous solution of $KHSO_4$ to bring its pH to 3.0 to allow an oily substance to precipitate, which was extracted with ether. The ether layer was washed with water, dried over $MgSO_4$, and concentrated under reduced pressure to give Compound 51b (3.4 g) as a white foamy substance.
IR $\nu_{max}$ (KBr) $cm^{-1}$:1725(sh),1715,1665,1535

| Elemental Analysis : $C_{21}H_{38}N_2O_8$ | | | |
|---|---|---|---|
| Calcd. | C, 56.74; | H, 8.16; | N, 6.30 |
| Found | C, 56.40; | H, 8.42; | N, 6.11 |

Example 52

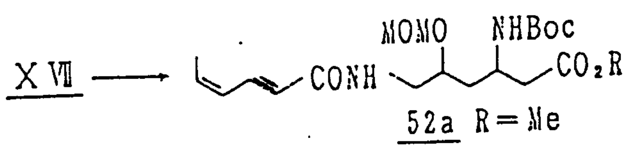

52a R=Me

52b R=H

To a solution of Compound XVII(1.43 g) in $CH_2Cl_2$ (40 mℓ) were added under ice-cooling $Et_3N$(0.62 mℓ) and $tBuPh_2SiCl$ (1.15 mℓ). The mixture was stirred at room temperature for 30 minutes, then concentrated under reduced pressure. The concentrate was dissolved in ethyl acetate, washed with water and dried over $MgSO_4$. The resultant was concentrated under reduced pressure to give silylester of carboxylic acid (2.6 g) as a white foamy substance.

This silylester(1.63 g) was dissolved in $CH_2Cl_2$ (20 mℓ), to which were added under ice-cooling iso-$Pr_2NEt$(2.27 mℓ) and methoxymethylchloride (0.99 mℓ), followed by allowing the reaction to proceed at room temperature for 21 hours. The resultant was concentrated under reduced pressure, and the concentrate was dissolved in ethyl acetate, washed with water and dried over $MgSO_4$, followed by concentration under reduced pressure. The concentrate was subjected to a silica gel (35 g) column chromatography, followed by developing ethyl acetate hexane = 1 : 1 to give Compound 52a(1.4 g) as a colorless oily substance.
NMR (90MHz, $CDCl_3$), ppm:1.13(9H,s),1.43(9 H,s),1.86(3H,d,6Hz),3.38(3H,s),7.2~7.8(10 H,m).

To a solution of Compound 52a (1.4 g) in MeOH(30 mℓ) was added KF(300 mg). The mixture was stirred at room temperature for 15 minutes, and concentrated under reduced pressure. The concentrate was

dissolved in ether, and the solution was extracted with an aqueous solution of sodium hydrogencarbonate twice. The aqueous layer was adjusted to pH 3.5 with a 5% aqueous solution of $KHSO_4$ under ice-cooling, and extracted with ethyl acetate. The extract was washed with brine, dried over $MgSO_4$, and concentrated under reduced pressure to give Compound 52b(850 mg) as a white foamy substance.
NMR (90MHz, $CDCl_3$)ppm:1.41(9H,s),1.84(3H, d,J = 6Hz),3.38(3H,s),9.05(1H,br).

| Elemental Analysis $C_{19}H_{32}N_2O_8 \cdot 0.5H_2O$ | | | |
|---|---|---|---|
| Calcd. | C, 53.64; | H, 7.82; | N, 6.58 |
| Found | C, 53.29; | H, 7.90; | N, 6.44 |

Example 53

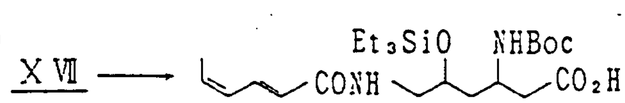

53

To a solution of Compound XVII(179 mg) in $CH_2Cl_2$(5 mℓ) were added with stirring under ice-cooling $Et_3N$(279μℓ) and $Et_3SiCl$ (335μℓ), and the reaction was allowed to proceed at room temperature for 16 hours. The reaction mixture was concentrated under reduced pressure, and the concentrate was dissolved in ethyl acetate. The solution was washed once with each of water and brine, dried over $MgSO_4$, and concentrated under reduced pressure to give Compound 53(210 mg) as a colorless oily substance.
NMR (90MHz, $CDCl_3$)ppm:0.97(9H,t,J = 7Hz), 0.4~0.9(6H,m), 1.43(9H,s),1.86(3H,d,J = 6Hz)

Example 54

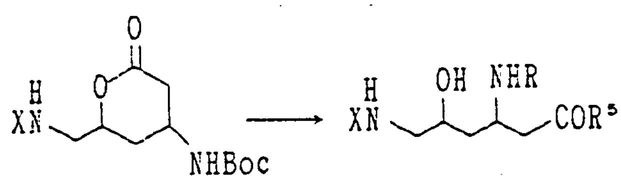

XXVIII

54a : R = Boc

54b : R = H

(X= 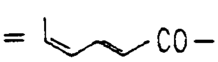 , $R^5 = -HN(CH_2)_4NH_2$)

Step A XXVIII - 54a)

To a solution of Compound XXVIII (250 mg) in $CH_2Cl_2$ (10 mℓ) was added tetramethylenediamine(0.7 mℓ), and the mixture was stirred at room temperature for one hour. The solvent was distilled off under reduced pressure. To the residue was was added water (50 mℓ), and the pH of the aqueous solution was adjusted to 2.0 with 1N HCl followed by washing with ethyl acetate(25 mℓ). To the aqueous layer was added 1N NaOH to adjust the pH to 5.5, followed by concentration to a volume of about 40 mℓ. The concentrate was subjected to a Diaion HP-20(50 to 100 mesh, 20 mℓ) column chromatography. The column

was washed with water (80 mℓ), followed by fractional elution with 50% methanol-water (60 mℓ), and 50% methanol-N/200 hydrochloric acid (100 mℓ), successively. Each fraction was subjected to analysis by means of high performance liquid chromatography [mobile phase:55%methanol/0.01M phosphoric acid solution(pH 3)]. Fractions showing a single peak were collected and concentrated, followed by lyophilization to yield hydrochloride of Compound 54a (308 mg) as a white powdery product.

| Elemental Analysis : $C_{21}H_{38}N_4O_5 \cdot HCl$ | | | | |
|---|---|---|---|---|
| Calcd. | C, 54.48; | H, 8.49; | N, 12.10; | Cl, 7.66 |
| Found | C, 54.00; | H, 8.71; | N, 12.11; | Cl, 7.59 |

Step B (54a → 54b)

A solution of Compound 54a hydrochloride(250 mg) in TFA (2 mℓ) was left standing at room temperature for 30 minutes. The solvent was distilled off under reduced pressure, and the residue was treated with ether to give a powdery substance, which was dissolved in water (60 mℓ), followed by allowing to pass through an Amberlite IRA-402($Cl^-$ type, 20 mℓ) column. The column was washed with water and eluted. The washing and eluate were combined and concentrated, followed by lyophilization to give Compound 54b dihydrochloride (218 mg) as a white powdery product.
IR : $\nu_{max}$ (KBr) $cm^{-1}$:3260,2940,1640,1540

| Elemental Analysis : $C_{16}H_{30}N_4O_3 \cdot 2HCl \cdot 0.5H_2O$ | | | | |
|---|---|---|---|---|
| Calcd. | C, 47.06; | H, 8.15; | N, 13.72; | Cl, 17.36 |
| Found | C, 47.37; | H, 8.19; | N, 13.71; | Cl, 17.31 |

Examples 55 - 66 (Table 11)

By the procedure similar to Example 54, Compound XXVIII was subjected to amidation by using various amines (R'$NH_2$) to obtain corresponding amido compounds (Step A), followed by deprotection reaction (Step B) to obtain compounds of Examples 55 - 66. The reaction conditions, yields and some of the physico-chemical properties of the products are shown in Table 11.

Table 11

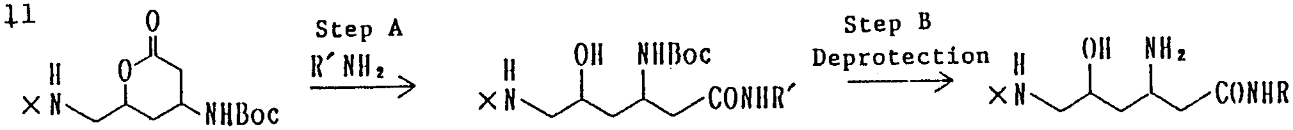


XXVIII a b

X = 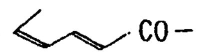


| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 55 | A | XXVIII → 55a<br>R′ = $-(CH_2)_3NH_2$ | R′$NH_2$ (0.6ml)<br>$CH_2Cl_2$(10ml)<br>r.t. 1hr<br>HP-20<br>lyophilization | 250mg → 278mg | — | $C_{20}H_{36}N_4O_6 \cdot HCl \cdot 0.5H_2O$<br>C 52.73 (52.45)<br>H 8.38 ( 8.36)<br>N 12.47 (12.23)<br>Cl 7.76 ( 7.74) |
| | B | 55a → 55b<br>R = $-(CH_2)_3NH_2$ | TFA (2ml)<br>r.t. 0.5hr<br>treatment with $Et_2O$<br>IRA-402($Cl^-$)<br>lyophilization | 240mg → 207mg | 3260<br>2930<br>1645<br>1550 | $C_{16}H_{20}N_4O_5 \cdot 2HCl \cdot 0.5H_2O$<br>C 46.18 (45.69)<br>H 8.07 ( 7.92)<br>N 14.39 (14.21)<br>Cl 18.17 (17.98) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 56 | A | XXVIII<br>→<br>56a<br>$R' = -(CH_2)_2NH_2$ | $R'NH_2$ (1.6ml)<br>$CH_2Cl_2$(10ml)<br>r.t. 1hr<br>HP-20<br>lyophilization | 800mg<br>→<br>898mg | — | $C_{10}H_{34}N_4O_8 \cdot HCl$<br>C 52.23 (52.47)<br>H 8.21 ( 8.11)<br>N 12.51 (12.88)<br>Cl 8.07 ( 8.15) |
| | B | 56a<br>→<br>56b<br>$R = -(CH_2)_2NH_2$ | TFA (2ml)<br>r.t. 0.5hr<br>treatment with $Et_2O$<br>IRA-402($Cl^-$)<br>lyophilization | 200mg<br>→<br>177mg | 3250<br>3000<br>1640<br>1540 | $C_{14}H_{20}N_4O_3 \cdot 2HCl \cdot 0.5H_2O$<br>C 44.47 (44.21)<br>H 7.57 ( 7.69)<br>N 14.99 (14.73)<br>Cl 18.63 (18.64) |

| Example No. | Step | Starting Compd. → Product | Reaction conditions | Yield Material → Product | IR $\nu_{max}^{KBr}$ $cm^{-1}$ | Molecular Formula Elemental Analysis (Calcd.) |
|---|---|---|---|---|---|---|
| 57 | A | XXVIII → 57a<br>$R' = -CH_2\underset{\mid \atop NH_2}{CH}CO_2H$ | $R'NH_2 \cdot HCl$ (156mg)<br>$Et_3N$ (1.5ml)<br>MeOH (15ml)<br>50℃, 20hr<br>HP-20<br>lyophilization | 250mg → 271mg | — | $C_{20}H_{34}N_4O_7 \cdot H_2O$<br>C 52.00 (52.16)<br>H 8.02 (7.88)<br>N 12.02 (12.17) |
| | B | 57a → 57b<br>$R = -CH_2\underset{\mid \atop NH_2}{CH}CO_2H$ | TFA (1.5ml)<br>r.t. 0.5hr<br>treatment with $Et_2O$<br>IRA-402($Cl^-$)<br>lyophilization | 142mg → 121mg | 3250<br>2930<br>1620<br>1530 | $C_{15}H_{20}N_4O_5 \cdot HCl \cdot H_2O$<br>C 45.62 (45.40)<br>H 7.33 (7.36)<br>N 14.20 (14.12)<br>Cl 10.06 (8.93) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 58 | A | XXVIII<br>→<br>58a<br>R′ = $-(CH_2)_2-$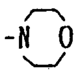 | R′ $NH_2$ (110 μℓ)<br>$CH_2Cl_2$ (15mℓ)<br>treatment with<br>MeOH-$Et_2O$ | 250mg<br>→<br>311mg | 3300<br>2940<br>1650 | $C_{23}H_{40}N_4O_6 \cdot HCl$<br>C — (54.70)<br>H — ( 8.18)<br>N — (11.09) |
| | B | 58a<br>→<br>58b<br>R = $-(CH_2)_2-$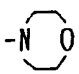 | TFA (2mℓ)<br>r.t. 0.5hr<br>treatment with $Et_2O$<br>IRA-402($Cl^-$)<br>lyophilization | 282mg<br>→<br>270mg | 3260<br>2930<br>1650<br>1535 | $C_{18}H_{32}N_4O_4 \cdot 2HCl \cdot 0.5H_2O$<br>C 48.19 (48.00)<br>H 8.01 ( 7.83)<br>N 12.35 (12.44)<br>Cℓ 15.62 (15.74) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 59 | A | XXVIII<br>→<br>59a<br>R′ = $-(CH_2)_2-$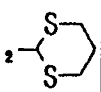 | $R'NH_2$ (∼10mg)<br>MeOH (3.0ml)<br>$Et_3N$ (0.11ml)<br>r.t. 62hr<br>50°C, 5hr<br>evap.<br>crystallization(MeOH-EtOAc) | 100mg<br>→<br>11mg | – | $C_{23}H_{30}N_3O_5S_2$<br>C 54.86 (55.06)<br>H 7.80 ( 7.84)<br>N 8.29 ( 8.38) |
| | B | 59a<br>→<br>59b<br>R = $-(CH_2)_2-$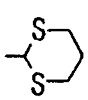 | TFA (0.5ml)<br>r.t. 30min<br>evap, washing with $Et_2O$<br>IRA-402($Cl^-$)<br>HP-20<br>lyophilization | 33mg<br>→<br>18mg | 3300<br>1650 | $C_{18}H_{31}N_3O_3S_2 \cdot HCl \cdot 2H_2O$<br>C 42.20 (42.43)<br>H 7.19 ( 7.12)<br>N 8.15 ( 8.25) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 60 | A | XXVIII<br>→<br>60a<br>R' =<br>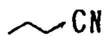 | R'$NH_2$ (214 μℓ)<br>$Et_3N$ (213 μℓ)<br>MeOH (2mℓ)<br>r.t. 24hr<br>50°C, 12hr<br>evap.<br>$SiO_2$ column ($CHCl_3$:EtOAc:MeOH=4:5:1) | 196mg<br>→<br>236mg | 3330<br>2250<br>1705(sh)<br>1690<br>1665(sh)<br>1650<br>1550 | $C_{20}H_{32}N_4O_6$<br>C — (58.81)<br>H — ( 7.90)<br>N — (13.72) |
| | B | 60a<br>→<br>60b<br>R =<br>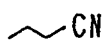 | TFA (1mℓ)<br>r.t. 30min, evap.<br>IRA-402($Cl^-$), $H_2O$<br>conc., CHP-20P<br>20% EtOH-$H_2O$,<br>lyophilization | 70mg<br>→<br>50mg | 2250<br>1655<br>1610<br>1550 | $C_{15}H_{24}N_4O_3 \cdot HCl \cdot H_2O$<br>C 49.35 (49.65)<br>H 7.80 ( 7.50)<br>N 15.23 (15.44) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 61 | A, B | XXVIII → 61.6<br>R = 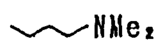 | R′$NH_2$ (95 μℓ)<br>MeOH (1mℓ)<br>r.t. 24hr, evap.<br>TFA (1mℓ)<br>r.t. 40min<br>evap.<br>IRA-401($C\ell^-$), $H_2O$<br>concentration<br>CHP-20P, $H_2O$<br>lyophilization | 51mg<br>→<br>62mg | 1650<br>1610(sh)<br>1550<br>1470 | $C_{17}H_{32}N_4O_3 \cdot 2HC\ell \cdot 1.5H_2O$<br>C 46.16 (46.36)<br>H 8.62 (8.47)<br>N 12.43 (12.72) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 62 | A | XXVIII → 62a<br>R′ =<br>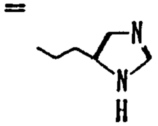 | $R'NH_2 \cdot 2HCℓ$ (166mg)<br>1.36M MeOLi/MeOH (1.32mℓ); MeOH (2mℓ)<br>reflux, 7hr, evap.<br>1N-HCℓ (pH3.0)<br>CHP-20P<br>30% EtOH-$H_2O$<br>lyophilization | 102mg<br>→<br>139mg | 1690<br>1650<br>1615<br>1540 | $C_{23}H_{35}N_5O_5 \cdot HCℓ \cdot 1.5H_2O$<br>C 55.41 (55.47)<br>H 7.20 ( 7.29)<br>N 14.00 (14.06) |
| | B | 62a → 62b<br>R =<br>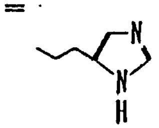 | TFA (2mℓ)<br>0°C, 30min<br>evap.<br>IRA-402(Cℓ⁻), $H_2O$<br>conc., CHP-20P<br>5% EtOH-$H_2O$<br>lyophilization | 139mg<br>→<br>143mg | 1670<br>1540 | $C_{17}H_{27}N_5O_3 \cdot 2HCℓ$<br>C 48.24 (48.34)<br>H 6.90 ( 6.92)<br>N 16.41 (16.58) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 63 | A | XXVIII → 63a<br>R' = 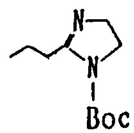 | R'NHCbz (174mg)<br>5% Pd-C (174mg)<br>MeOH (5mℓ)<br>$H_2$, 40min, evap.<br>MeOH (2mℓ)<br>60°C, 20hr, evap.<br>$SiO_2$ column ($CHCℓ_3$ : AcOEt : MeOH = 3:3:1) | 102mg<br>→<br>67mg | 1705<br>1650<br>1530 | $C_{27}H_{45}N_5O_7$<br>C — (58.78)<br>H — ( 8.22)<br>N — (12.69) |
| | B | 63a → 63b<br>R = 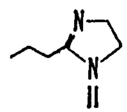 | TFA (2mℓ)<br>r.t. 1.5hr<br>evap.<br>IRA-402(Cℓ⁻), $H_2O$<br>conc., CHP-20P<br>$H_2O$<br>lyophilization | 89mg<br>→<br>54mg | 1655<br>1605<br>1545 | $C_{17}H_{20}N_8O_3 \cdot 2HCℓ$<br>C 44.39 (44.35)<br>H 7.82 ( 7.66)<br>N 15.49 (15.21) |

| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 64 | A, B | XXVIII → 64a<br>R′ =<br>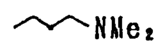<br>64a → 64b<br>R =<br>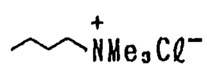 | R′$NH_2$ (189 μl)<br>MeOH (2ml)<br>r.t. 16hr, evap.<br>MeOH (2ml)<br>MeI (38 μl)<br>r.t. 6hr, evap.<br>TFA (2ml)<br>r.t. 1.5hr, evap.<br>CHP-20P, $H_2O$, conc.<br>IRA-402($Cl^-$), $H_2O$<br>lyophilization | 102mg<br>→<br>139mg | 1670<br>1655<br>1545 | $C_{18}H_{35}ClN_4O_3 \cdot HCl \cdot H_2O$<br>C 48.40 (48.54)<br>H 8.19 ( 8.60)<br>N 12.40 (12.58) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 65 | A | XXVIII → 65a<br>R′ =<br>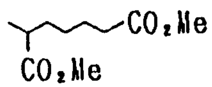 | $R'NH_2$ (204mg)<br>DMF (3mℓ)<br>$Et_3N$ (70μℓ)<br>110°C, 28hr extn.<br>with EtOAc, $SiO_2$ column<br>($CHCℓ_3$:EtOAc:MeOH = 40:55:5) | 170mg<br>→<br>176mg | 1740<br>1655<br>1545<br>1530 | $C_{26}H_{43}N_3O_9$<br>C — (57.66)<br>H — ( 8.00)<br>N — ( 7.76) |
| | B | 65a → 65b<br>R =<br>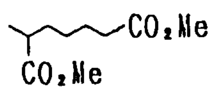 | TFA (2mℓ)<br>r.t. 1hr, evap.<br>IRA-402($Cℓ^-$), 50% MeOH-$H_2O$<br>lyophilization | 287mg<br>→<br>276mg | 1740<br>1670<br>1650<br>1550 | $C_{21}H_{35}N_3O_7 \cdot HCℓ$<br>C 52.38 (52.77)<br>H 7.69 ( 7.59)<br>N 8.61 ( 8.79) |

| Example No. | Step | Starting Compd. → Product | Reaction Conditions | Yield Material → Product | IR $\nu_{max}^{KBr}$ $cm^{-1}$ | Molecular Formula / Elemental Analysis (Calcd.) |
|---|---|---|---|---|---|---|
| 66 | B′ | 65a → 66b<br>R′ = 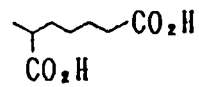 | MeOH (10 mℓ)<br>1N-NaOH (2.82 mℓ)<br>r.t. 4.5hr, evap.<br>1N-HCℓ(pH3.5)<br>CHP-20P<br>15% EtOH-$H_2O$<br>lyophilization | 245mg<br>→<br>153mg | 1710<br>1665<br>1655<br>1550 | $C_{19}H_{31}N_3O_7$<br>C 55.40 (55.19)<br>H 7.80 ( 7.56)<br>N 10.31 (10.16) |

Example 67

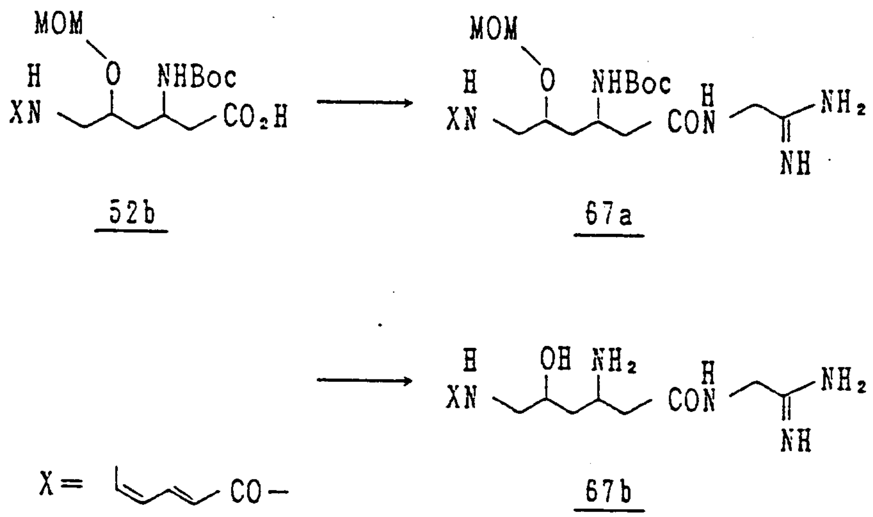

52b

67a

67b

Step A (52b → 67a)

To a solution of 52b(260 mg) in anhydrous THF(2 mℓ) were added HOBT(97 mg) and DCC(146 mg), and the mixture was stirred at room temperature for one hour. This reaction mixture was added, while stirring vigorously, to a mixture of 2 mℓ of $H_2O$ solution of 2-aminoacetoamidine•dihydrochloride(143mg) and sodium hydrogencarbonate(123 mg). The whole mixture was stirred at room temperature for 16 hours. Insolubles were filtered off, followed by distilling off THF under reduced pressure. To the residue was added 1N-HCl to adjust its pH to 4.0, which was subjected to a CHP-20P(wet 70 mℓ) column chromatography, followed by elution with 30% ethanol-water. The eluate was concentrated and lyophilized to give Compound 67a(291 mg) as a pale yellow powdery product.
I R $\nu_{max}$ (KBr) $cm^{-1}$:1710(sh),1685,1650 1530

Step B (67a → 67b)

To a suspension of 67a(290 mg) in anisole(2 mℓ) was added, under ice-cooling, TFA(3 mℓ). The mixture was stirred at room temperature for one hour, followed by concentration under reduced pressure. The concentrate was subjected to a CHP-20P(wet 20 mℓ) column chromatogrpahy. The column was eluted with water, and the objective fractions were combined, and concentrated under reduced pressure. The concentrate was allowed to pass through an Amberlite IRA-402 ($Cl^-$ type) (20 mℓ) column, and the column was washed with water(100 mℓ). The eluate was concentrated and lyophilized to give Compound 67b(133 mg) as a pale yellow foamy product.
IR $\nu_{max}$ (KBr) $cm^{-1}$:1690,1650,1540

| Elemental Analysis : $C_{14}H_{25}N_5O_3 \cdot 2HCl \cdot 0.5H_2O$ | | | |
|---|---|---|---|
| Calcd. | C, 42.75; | H, 7.18; | N, 17.81 |
| Found | C, 42.45; | H, 7.30; | N, 17.61 |

Examples 68 - 73 (Table 12)

Instead of Compound 52b were employed Compounds 50c, 51b and 53, and corresponding amido-compounds were obtained by procedures similar to Example 67 (Step A), followed by deprotection reaction (Step B) to obtain compounds of Examples 68 - 73. The reaction conditions, yields and some of the physico-chemical properties of the products are shown in Table 12.

Table 12

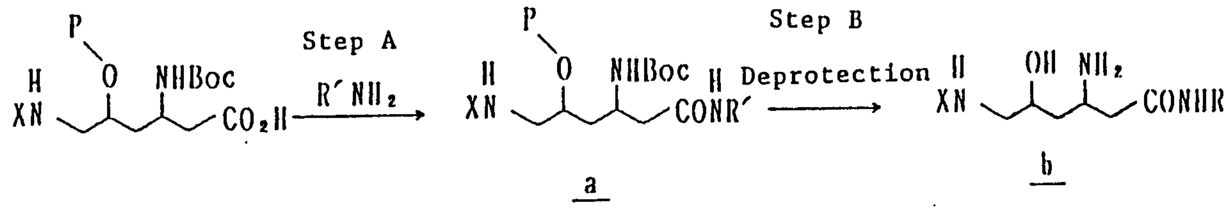


a

b

50c : P = THP

51b : P = MEM

53 : P = $Et_3Si$

X = 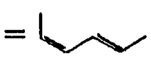 CO−

| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 68-1 | A | 50c → 68-1a<br>R′ = <br>Me<br>\|<br>$-NCH_2CO_2CH_2Ph$<br>P = THP | $R'\overset{+}{N}H_3 \cdot P\text{-}Tso^-$(183mg)<br>HOBT(68mg), DCC(103mg)<br>$Et_3N$(0.07mℓ)<br>$CH_2Cl_2$ (3mℓ), r.t. 4hr<br>evap. $SiO_2$ column<br>(EtOAc:MeOH = 5:1) | 220mg<br>→<br>193mg | 1735<br>1710<br>1660<br>1530 | $C_{32}H_{48}N_4O_8$<br>C — (62.32)<br>H — ( 7.84)<br>N — ( 9.08) |
| | B | 68-1a → 68b<br>R = <br>Me<br>\|<br>$-NCH_2CO_2Na$ | TFA (3mℓ)<br>r.t. 40min, evap.<br>MeOH(2mℓ), 1 N-NaOH<br>(0.6mℓ), r.t. 1hr, evap.<br>CHP-20P, $H_2O$, lyophilization | 247mg<br>→<br>110mg | 1690(sh)<br>1655<br>1590<br>1450 | $C_{16}H_{26}N_4O_6Na \cdot H_2O$<br>C 47.00 (47.12)<br>H 7.01 ( 7.12)<br>N 14.65 (14.65) |
| 68-2 | A | 51b → 60-2a<br>R′ = <br>Me<br>\|<br>$-NCH_2CO_2CH_2Ph$<br>P = MEM | $R'\overset{+}{N}H_3 \cdot P\text{-}Tso^-$(145mg)<br>HOBT(49mg), DCC(75mg)<br>$Et_3N$(50mg), THF(3mℓ)<br>r.t. 20hr, evap.<br>$SiO_2$ column<br>(EtOAc:MeOH = 5:1) | 155mg<br>→<br>83mg | 1740<br>1710<br>1665<br>1523 | $C_{31}H_{48}N_4O_9$<br>C — (59.98)<br>H — ( 7.79)<br>N — ( 9.03) |

| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 69-1 | A | 50c → 69-1a<br>R′ =<br>$-CH_2CO_2Me$<br>P = THP | $R'\overset{+}{N}H_3Cl^-$ (251mg)<br>HOBT(135mg), DCC(206mg)<br>$Et_3N$(220mg)<br>DMF (3ml), r.t. 2day,<br>evap. $SiO_2$ column<br>(EtOAc:MeOH = 10:1) | 440mg<br>→<br>220mg | 1740<br>1705<br>1680<br>1530 | $C_{25}H_{41}N_3O_8$<br>C — (58.69)<br>H — ( 8.08)<br>N — ( 8.21) |
| 69-2 | A | 51b → 69-2a<br>R′ =<br>$-CH_2CO_2Me$<br>P = THP | $R'\overset{+}{N}H_3Cl^-$ (172mg)<br>$Et_3N$(0.234ml)<br>DCC (145mg)<br>$CH_2Cl_2$(3ml), r.t. 20hr<br>conc., $SiO_2$ column<br>(EtOAc) | 305mg<br>→<br>200mg | 1745<br>1710(sh)<br>1690<br>1660<br>1530 | $C_{24}H_{41}N_3O_9$<br>C — (55.91)<br>H — ( 8.01)<br>N — ( 8.15) |
| | B | 69-2a → 69b<br>R =<br>$-CH_2CO_2Na$ | TFA (2ml)<br>r.t. 40min, evap.<br>MeOH(2ml), 1 N-NaOH<br>(0.6ml), r.t. 1hr, evap.<br>CHP-20P, $H_2O$, lyophilization | 198mg<br>→<br>70mg | 1690(sh)<br>1660<br>1610<br>1400 | $C_{14}H_{22}N_3O_6Na \cdot 0.5H_2O$<br>C 46.29 (46.67)<br>H 6.60 ( 6.43)<br>N 11.38 (11.66) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 70 | A | 51b → 70a<br>R′ =<br>*DL[1]<br>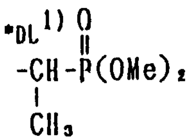<br>P = MEM | R′-NHCbz · HCℓ (396mg)<br>10% Pd-C (300mg)<br>$CH_2Cl_2$ (15mℓ), $H_2$, 20min<br>filtn.conc., addn. of THF (5mℓ)<br>HOBT (96mg), DCC (147mg)<br>$Et_3N$ (300mg)<br>r.t. 24hr<br>conc., $SiO_2$ column<br>(EtOAc:MeOH = 5:1) | 305mg<br>→<br>242mg | 1710<br>1665<br>1530<br>1030 | $C_{26}H_{40}N_3O_{10}P$<br>C — (51.80)<br>H — (8.00)<br>N — (7.25) |
| | B | 70a → 70b<br>R =<br>*DL<br>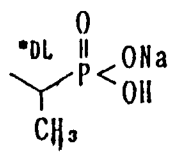 | $CH_3CN$ (3mℓ)<br>NaI (276mg)<br>$Me_3SiCl$ (0.22mℓ)<br>r.t. 3.5hr, evap.<br>1N-NaOH (pH9.0)<br>CHP-20P, 20% MeOH · $H_2O$<br>lyophilization | 240mg<br>→<br>36mg | 1650<br>1540<br>1070 | $C_{14}H_{26}N_3O_8NaP \cdot H_2O$<br>C 41.40 (41.69)<br>H 6.75 (6.75)<br>N 10.23 (10.42) |

1) *DL : Racemic compounds (hereinafter the same applies)

| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 71 | A | 51b → 71a<br>R′ =<br>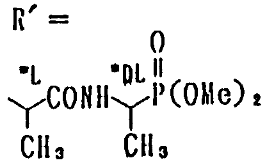<br>P = MEM | R′-NHCbz (294mg)<br>Pd-black (200mg)<br>THF (10mℓ)<br>$H_2$, r.t. 40min, filtn.<br>HOBT (101mg)<br>DCC (154mg)<br>r.t. 24hr, filtn., evap.<br>$SiO_2$ column<br>(EtOAc:MeOH = 5:1) | 321mg<br>→<br>250mg | 1710(sh)<br>1690(sh)<br>1655<br>1530<br>1030 | $C_{20}H_{51}N_4O_{11}P$<br>C — (51.68)<br>H — ( 7.90)<br>N — ( 8.61) |
| | B | 71a → 71b<br>R =<br>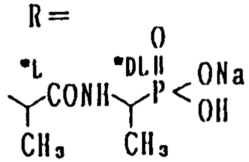 | $CH_3CN$ (3mℓ)<br>NaI (254mg)<br>$Me_3SiC\ell$ (0.20mℓ)<br>r.t. 3.5hr, evap.<br>1N-NaOH (pH9.0)<br>CHP-20P, 20% MeOH-$H_2O$<br>lyophilization | 248mg<br>→<br>45mg | 1650<br>1545<br>1060 | $C_{17}H_{30}N_4O_7NaP \cdot 1.5H_2O$<br>C 42.14 (42.24)<br>H 6.76 ( 6.88)<br>N 11.49 (11.59) |

| Example No. | Step | Starting Compd. → Product | Reaction Conditions | Yield Material → Product | IR $\nu_{max}^{KBr}$ $cm^{-1}$ | Molecular Formula<br>Elemental Analysis (Calcd.) |
|---|---|---|---|---|---|---|
| 72 | A | 53 → 72a<br>R′ =<br>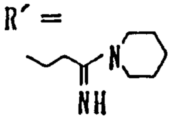<br>P = $Et_3Si$ | ①R′-NHCbz · HCl(165mg)<br>$H_2O$ (3ml), 1N-HCl<br>(0.54ml), 5% Pd-C<br>(150mg), $H_2$, r.t. 50min,<br>filtn., $NaHCO_3$(pH8.0)<br>②53, THF(5ml), HOBT<br>(102mg), DCC(165mg)<br>r.t. 2hr<br>①+②(0°C, pH8($NaHCO_3$))<br>r.t. 2hr, 1N-HCl(pH4.0),<br>evap. washing with EtOAc<br>conc. of aq. layer, CHP-20P<br>30% EtOH-$H_2O$, lyophil. | 353mg<br>→<br>113mg | 1710(sh)<br>1690<br>1660<br>1630<br>1550 | $C_{25}H_{43}N_5O_6 \cdot HCl \cdot H_2O$<br>C 54.58 (54.78)<br>H 8.14 ( 8.46)<br>N 12.70 (12.78) |
| | B | 72a → 72b<br>R =<br>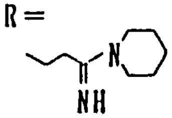 | TFA (1.5ml)<br>r.t. 1hr, evap.<br>CHP-20P twice 10%EtOH<br>conc., IRA-402($Cl^-$), $H_2O$<br>lyophilization | 112mg<br>→<br>81mg | 1665<br>1620<br>1540 | $C_{20}H_{35}N_5O_3 \cdot 2HCl \cdot H_2O$<br>C 49.40 (49.58)<br>H 8.01 ( 8.11)<br>N 14.40 (14.46) |

| Example No. | Step | Starting Compd. → Product | Reaction Conditions | Yield Material → Product | IR $\nu_{max}^{KBr}$ $cm^{-1}$ | Molecular Formula Elemental Analysis (Calcd.) |
|---|---|---|---|---|---|---|
| 73 | A | 53 → 73a<br>R′= 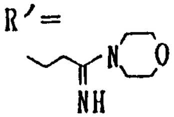<br>P=$Et_3Si$ | ① R′-NHCbz · HCl(213mg)<br>$H_2O$ (5ml)<br>1N-HCl(0.64ml)<br>5% Pd-C(200mg)<br>$H_2$, r.t. 50min, filtn.<br>$NaHCO_3$(pH8)<br>② 53, THF(7ml)<br>HOBT(135mg)<br>DCC(227mg), r.t., 2hr<br>① + ②(0°C, pH8($NaHCO_3$))<br>r.t. 2hr, 1N-HCl(pH3.0),<br>filtn., conc.<br>EtOAc(10ml)<br>extraction twice with $H_2O$<br>CHP-20P, 30% EtOH-$H_2O$<br>lyophilization | 471mg<br>→<br>172mg | 1710(sh)<br>1690<br>1660<br>1630<br>1530 | $C_{24}H_{41}N_5O_8 \cdot HCl$<br>C — (54.20)<br>H — ( 7.64)<br>N — (14.44) |
| | B | 73a → 73b<br>R= 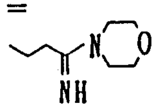 | TFA (2ml)<br>r.t. 1hr, evap.<br>CHP-20P, 10% EtOH-$H_2O$<br>conc., IRA-402($Cl^-$), $H_2O$<br>lyophilization | 171mg<br>→<br>95mg | 1670<br>1615<br>1545 | $C_{18}H_{33}N_5O_4 \cdot 2HCl \cdot H_2O$<br>C 47.00 (46.91)<br>H 7.35 ( 7.67)<br>N 14.26 (14.40) |

Example 74

XVII

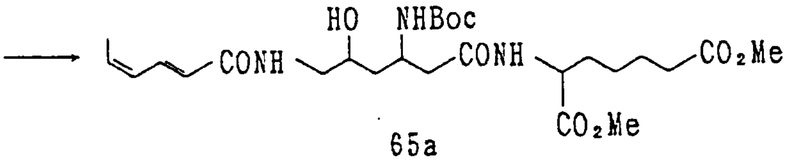

65a

To a solution of Compound XVII(179 mg) in $CH_2Cl_2$(5 mℓ) were added, under ice-cooling, $Et_3N$(279μℓ) and $Me_3SiCl$(253 μℓ). The mixture was stirred at room temperature for 20 hours. To the reaction mixture was added ice-water. The mixture was extracted twice with $CH_2Cl_2$ quickly, the extract was dried over $MgSO_4$, and concentrated under reduced pressure. To the concentrate were added $CH_2Cl_2$ (10 mℓ), HOBT-(68 mg) and DCC(124 mg). The mixture was stirred at room temperature for 30 minutes, to which was then added DL-α-aminopimelic acid methyl ester(122 mg). The reaction was allowed to proceed for 20 hours. The reaction mixture was subjected to filtration to remove insolubles. The filtrate was concentrated under reduced pressure. The concentrate was subjected to a silica gel(18 g) column chromatography, followed by elution with chloroform:ethyl acetate:methanol = 40:55:5 to obtain Compound 65a(112 mg) as a white powdery product. This product was in agreement with the compound obtained in Example 65A in the physico-chemical data.

Example 75

50a

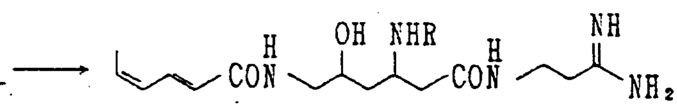

75a (≡ VI): R = Boc

75b (≡ I): R = H

Step A (50a → 75a)

In methanol(2.0 mℓ) were dissolved Compound 50a (57.8 mg) and 2-aminoethylamidine•dihydrochloride(30 mg). To the solution was added at room temperature t-butoxypotassium (29 mg, 90% purity). The mixture was stirred for 19 hours, to which was poured a saturated aqueous solution of ammonium chloride(10 mℓ), then the aqueous layer was washed with ether and ethyl acetate. The aqueous layer, after adjustng the pH thereof to 5 to 6, was concentrated. The concentrate was allowed to adsorb onto a column of Diaion HP-20(50 to 100 mesh, 5 mℓ). The column was washed with water followed by elution with 20% aqueous methanol, 50% aqueous methanol and 50% methanol-0.01N hydrochloric acid (each 25 mℓ), successively to fractionate into 5 mℓ each portions. Each fraction was subjected to analysis by means of high performance liquid chromatography[mobile phase: 25% acetonitrile-0.01M phosphoric acid buffer(pH3.0)]. Fractions showing a single peak were combined and concentrated, followed by lyophilization to obtain hydrochloride of Compound 75a (53 mg). Comparison of this product with N-Boc compound (VI) of I of natural origin by means of a high performance liquid chromatography [mobile phase; the same as above] for analysis revealed that both were in complete agreement with each other.

Step B (75a → 75b)

A solution of Compound 75a hydrochloride(50 mg) in TFA (0.5 mℓ) was subjected to a reaction similar to that of Step B in Example 1 to yield Compound 75b dihydrochloride (38 mg) as a white powdery product. which was in agreement with Compound I of natural origin in the physico-chemical properties.

Example 76

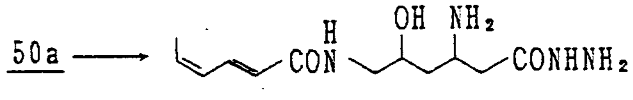

76

To a solution of Compound 50a(251 mg) in methanol (8.0 mℓ) was added hydrazine-hydrate(0.325 mℓ), and the mixture was stirred at room temperature for 40 minutes. The solvent was distilled off under reduced pressure. To the residue was added water, whose pH was adjusted to 3 to 4 with dilute hydrochloric acid, followed by washing twice with ether. To the aqueous layer was added 1N-NaOH to adjust the pH to 8 to 9, followed by extraction three times with ethyl acetate-isobutanol (3:1). Organic layers were combined and washed with saturated brine, and concentrated under reduced pressure. To the concentrate was further added water, then isobutanol was distilled off azeotropically, followed by lyophilization to obtain N-Boc compound of Compound 76 as a white powdery product(177 mg). This compound(122.8 mg) was dissolved in TFA(0.5 mℓ), and the solution was left standing at room temperature for 30 minutes, followed by removing TFA under reduced pressure. To the residue was added ether. The resultant solid material was washed with ether, and dissolved in water (15 mℓ). The aqueous solution was allowed to pass through the resin of Amberlite IRA-402($Cl^-$ type, 50 to 100 mesh, 10 mℓ), which was eluted with water(30 mℓ). The solution which was passed through the resin and the eluate were combined and concentrated and lyophilized to obtain dihydrochloride of Compound 76 as a white powdery product(105 mg)
IR $\nu_{max}$(KBr)$cm^{-1}$: 1700 to 1500

| Elemental Analysis : $C_{12}H_{22}N_4O_3 \cdot 2HCl$ | | | | |
|---|---|---|---|---|
| Calcd. | C,41.99; | H,7.05; | N,16.32; | Cl,20.66 |
| Found | C,42.23; | H,7.23; | N,16.07; | Cl,18.31 |

Example 77

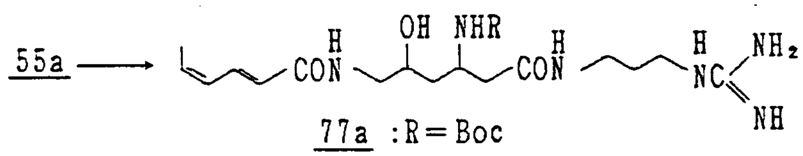

77a :R=Boc

77b :R=H

Step A (55a → 77a)

In water (0.6 mℓ) was dissolved sulfuric acid S-methyl isothiourea(117 mg), to which was added, under ice-cooling, 1N-KOH(0.255 mℓ). To this solution was added Compound 55a (70 mg), and the reaction was allowed to proceed at room temperature for 20 hours, followed by purification by means of a CHP-20P column chromatography. Fractions eluted with 40% ethanol-water were combined, concentrated, and lyophilized to yield Compound 77a (55 mg) as a white powdery product.
IR $\nu_{max}$(KBr)$cm^{-1}$: 1710 to 1610, 1550

Step B (77a → 77b)

In TFA(1 mℓ) was dissolved Compound 77a(55 mg). The solution was stirred at room temperature for 30 minutes, and concentrated under reduced pressure. The concnetrate was dissolved in a small volume of

water, which was allowed to pass through Amberlite IRA-402($Cl^-$ type)(5 mℓ), followed by elution with water (40 mℓ). The eluate was concentrated and subjected to a CHP-20P column chromatography, using water as an eluent. The eluate was concentrated and then lyophilized to obtain dihydrochloride of Compound 77b (30 mg) as a pale yellow resinous powdery product.
IR $\nu_{max}$ (KBr)$cm^{-1}$: 1690 to 1610, 1550

| Elemental Analysis : $C_{16}H_{30}N_6O_3 \cdot 2HCl \cdot 0.5H_2O$ | | | |
|---|---|---|---|
| Calcd. | C,44.04; | H,7.62; | N,19.26 |
| Found | C,44.00; | H,7.82; | N,19.01 |

Example 78

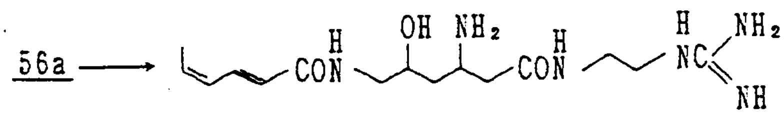

78

Employing Compound 56a(500 mg) and sulfuric acid S-methylisothiourea(450 mg), a similar reaction to that in Step A of Example 77 was conducted to obtain N-Boc compound of Compound 78a(291 mg) as a white powdery product. This compound(275 mg) was dissolved in TFA(2 mℓ), which was subjected to a reaction similar to that in Step B of Example 77 to obtain dihydrochloride of Compound 78(241 mg) as a white powdery product.
IR $\nu_{max}$(KBr)$cm^{-1}$: 3270, 1645, 1530

| Elemental Analysis : $C_{15}H_{28}N_6O_3 \cdot 2HCl \cdot 0.5H_2O$ | | | | |
|---|---|---|---|---|
| Calcd. | C,42.66; | H,7.40; | N,19.90; | Cl,16.79 |
| Found | C,42.79; | H,7.56; | N,19.78; | Cl,16.45 |

Example 79

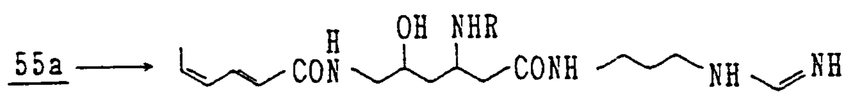

79a :R=Boc

79b :R=H

Step A (55a → 79a)

Compound 55a (103 mg) obtained in Example 55A was dissolved in water (2 mℓ). To the solution were added, under ice-cooling, benzylformimidate hydrochloride(129 mg) and 1N-KOH(0.75 mℓ), followed by stirring at room temperature for 3 hours. To the resultant was added, under ice-cooling, 1N-HCl to adjust the pH to 2.0. The resultant was shaken together with ethyl acetate. The aqueous layer was concentrated under reduced pressure, and the residue was subjected to a CHP-20P column chromatography. The fraction eluted with 20% ethanol-water was lyophilized to obtain Compound 79a(60 mg) as a white powdery product.
IR $\nu_{max}$(KBr) $cm^{-1}$: 1715, 1665, 1650, 1545

Step B (79a → 79b)

In TFA(1 mℓ) was dissolved Compound 79a(60 mg). The reaction was allowed to proceed at room temperature for 30 minutes, and concentrated under reduced pressure. The concentrate was dissolved in water, and the aqueous solution was allowed to pass through a column of Amberlite IRA-402($Cl^-$ type) resin (6 mℓ), followed by elution with water(25 mℓ). The eluate was concentrated under reduced pressure, and the concentrate was subjected to a CHP-20P column chromatography. The fraction eluted with water was lyophilized to obtain dihydrochloride of Compound 79b(48 mg) as a pale yellow resinous powdery product.
IR $\nu_{max}$ (KBr) $cm^{-1}$ : 3270, 1710, 1670 to 1610, 1550

| Elemental Analysis : $C_{16}H_{29}N_5O_3 \cdot 2HCl \cdot H_2O$ | | | |
|---|---|---|---|
| Calcd. | C,44.65; | H,7.73; | N,16.27 |
| Found | C,44.65; | H,7.61; | N,16.00 |

Example 80

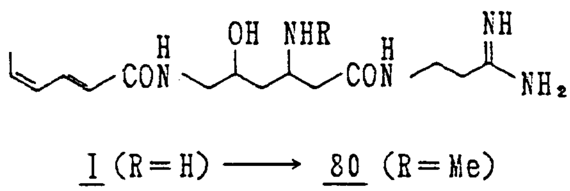


To a solution of dihydrochloride of Compound I (443 mg) in methanol(5 mℓ) was added 37% formaline-(86μℓ), and the mixture was stirred for one hour, to which were added acetic acid(0.5 mℓ) and sodium cyanoborohydride(95 mg). The whole mixture was stirred for 5 hours, which was left standing overnight. The resultant was concentrated under reduced pressure, and the concentrate was dissolved in water, followed by purification by means of an Amberlite XAD-2 column. Fractions eluted with water were combined and allowed to pass through activated charcoal and Amberlite IRA-401($Cl^-$ type), and the aqueous solution was concentrated. The concentrate was lyophilized to obtain dihydrochloride of Compound 80(101 mg) as a white powdery product.
IR $\nu_{max}$ (KBr)$cm^{-1}$: 1690, 1650, 1545, 1435

| Elemental Analysis : $C_{16}H_{29}N_5O_3 \cdot 2HCl$ | | | |
|---|---|---|---|
| Calcd. | C,46.60; | H,7.58; | N,16.98 |
| Found | C,46.60; | H,7.29; | N,16.78 |

Example 81

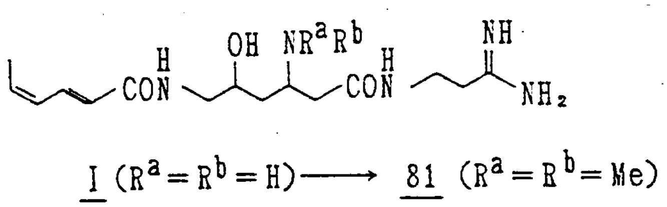


To a solution of dihydrochloride of Compound I(443 mg) in methanol(5 mℓ) was added $Et_3N$(154μℓ), followed by adding 37% formalin(150μℓ) and stirring for 30 minutes. To the resultant were added acetic acid(0.5 mℓ) and sodium cyanoborohydride(126 mg), and the mixture was stirred for 2 hours. To the

resultant was further added sodium cyanoborohydride (63 mg), and the mixture was stirred for one hour, which was left standing overnight. The same work-up of reaction mixture as that in Example 80 afforded dihydrochloride of Compound 81(342 mg).
IR $\nu_{max}$ (KBr)$cm^{-1}$: 1690, 1650, 1545

| Elemental Analysis : $C_{17}H_{31}N_5O_3 \cdot 2HCl \cdot 0.5H_2O$ | | | |
|---|---|---|---|
| Calcd. | C,46.90; | H,7.87; | N,16.09 |
| Found | C,46.80; | H,7.90; | N,16.05 |

Example 82

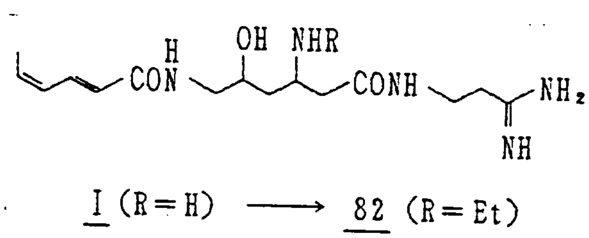


To a solution of dihydrochloride of Compound I(222 mg) in methanol (5 mℓ) was added a 80% aqueous solution of acetaldehyde(60μℓ). The mixture was stirred for 30 minutes, to which were added, under ice-cooling, acetic acid(0.25 mℓ) and sodium cyanoborohydride(63 μg). The temperature of the mixture was allowed to revert to room temperature. The mixture was stirred for 4 hours, left standing overnight, and concentrated under reduced pressure. The concentrate was allowed to pass through Amberlite IRA-401($Cl^-$ type), followed by purification by means of an Amberlite XAD-2 column chromatography. Fractions eluted with water were combined and concentrated, and the concentrate was lyophilized to obtain dihydrochloride of Compound 82(190 mg) as a white powdery product.
IR $\nu_{max}$ (KBr)$cm^{-1}$: 1690, 1650, 1545

| Elemental Analysis : $C_{17}H_{31}N_5O_3 \cdot 2HCl \cdot 0.5H_2O$ | | | |
|---|---|---|---|
| Calcd. | C,46.90; | H,7.87; | N,16.09 |
| Found | C,46.75; | H,7.99; | N,15.85 |

Example 83

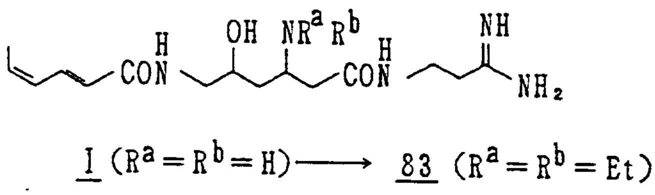


To a solution of dihydrochloride of Compound I(443 mg) in methanol (8 mℓ) was added a 80% aqueous solution of acetaldehyde(175μℓ), and the mixture was stirred for 30 minutes. To the resultant were added, under ice-cooling, acetic acid (0.5 mℓ) and sodium cyanoborohydride(157 mg). The temperature of the mixture was allowed to revert to room temperature. The mixture was then stirred for 2 hours. To the mixture were further added twice every two hours a 80% aqueous solution of acetaldehyde(105μℓ each) and sodium cyanoborohydride(95 mg each). The whole mixture was left standing overnight, and concentrated under reduced pressure. The concentrate was dissolved in a small volume of water. The pH of the solution

was adjusted to 1.5 with 1N hydrochloric acid. The solution was subjected to an activated charcoal column chromatography for purification. Fractions eluted with a 30% aqueous solution of ethanol and with a 50 % aqueous solution of ethanol were combined, concentrated, and lyophilized to obtain dihydrochloride of Compound 83 (276 mg) as a white powdery product.
IR $\nu_{max}$ (KBr)$cm^{-1}$: 1690, 1670, 1650, 1440, 1190

| Elemental Analysis : $C_{19}H_{35}N_5O_3 \cdot 2HCl \cdot 3H_2O$ | | | |
|---|---|---|---|
| Calcd. | C,44.88; | H,8.52; | N,13.77 |
| Found | C,44.80; | H,8.32; | N,13.89 |

Example 84

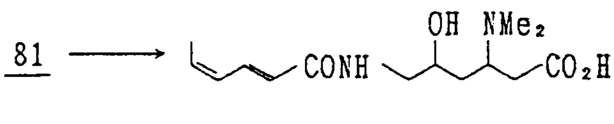

84

A solution of dihydrochloride of Compound 81(220 mg) in 2N hydrochloric acid (20 mℓ) was stirred for 10 minutes on an oil bath (bath temperature : 120 °C), and concentrated under reduced pressure. The concentrate was dissolved in water, to which was added sodium hydrogencarbonate to adjust the pH to 7.0, followed by purification by means of an Amberlite XAD-2 column chromatography. Fractions eluted with 15% ethanol - water were combined, concentrated, and lyophilized to obtain Compound 84(63 mg) as a white powdery product.
IR $\nu_{max}$ (KBr)$cm^{-1}$: 1650, 1600, 1390

| Elemental Analysis : $C_{14}H_{24}N_2O_4 \cdot 1.2H_2O$ | | | |
|---|---|---|---|
| Calcd. | C,54.96; | H,8.70; | N,9.15 |
| Found | C,54.77; | H,8.72; | N,9.37 |

Example 85A

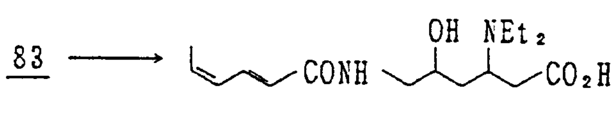

85

A solution of dihydrochloride of Compound 83(196 mg) in 2N hydrochloric acid (20 mℓ) was stirred for 20 minutes on an oil bath(bath temperature : 120 °C), and work-up in a manner similar to that in Example 84 afforded Compound 85(29 mg).
IR $\nu_{max}$ (KBr)$cm^{-1}$: 1670, 1610, 1540, 1430, 1390, 1270

| Elemental Analysis : $C_{16}H_{28}N_2O_4 \cdot H_2O$ | | | |
|---|---|---|---|
| Calcd. | C,58.16; | H,9.15; | N,8.48 |
| Found | C,58.00; | H,9.10; | N,8.19 |

Example 85B

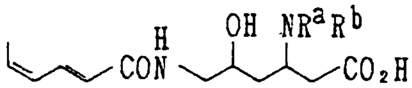

XV ($R^a = R^b = H$) ⟶ 85 ($R^a = R^b = Et$)

To a solution of sodium salt of Compound xv(195 mg) in methanol(5 mℓ) was added a 80% aqueous solution of acetaldehyde, and the mixture was stirred for 15 minutes. To the mixture were added, while stirring under ice-cooling, acetic acid(0.5 mℓ) and sodium cyanoborohydride(116 mg). The whole mixture was, after reverting to room temperature, stirred for 1.5 hour. To the mixture were added each poriton of a 80% aqueous solution of acetaldehyde(100μℓ) and sodium cyanoborohydride(88 mg) five times at an interval of one hour. The mixture was left standing overnight and concentrated under reduced pressure. The concentrate was dissolved in a small volume of water. To the solution was added sodium hydrogencarbonate to adjust the pH to 6.5, followed by purification by means of an Amberlite XAD-2 column chromatography. Fractions eluted with 15% ethanol - water were combined, concentrated, and lyophilized to obtain Compound 85(192 mg) as a white powdery product. This product was in agreement with the compound obtained in Example 85A in physico-chemical data.

Example 86

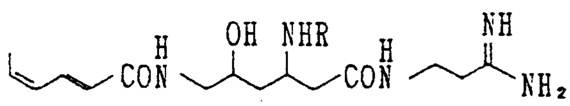

I (R=H)

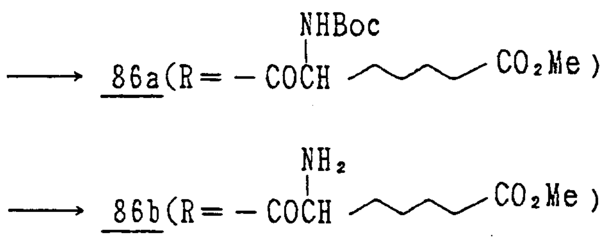

86a

86b

To a suspension of dihydrochloride of Compound I(399 mg) in DMF(5 mℓ) were added monomethylester of 2-t-butyloxycarbonylaminopimelic acid(290 mg), HOBT(61 mg), DCC(227 mg) and $Et_3N$(250μℓ). The mixture was stirred at room temperature for 16 hours and at 40°C for 3 hours. DMF was distilled off under reduced pressure, and the residue was suspended in water, to which was added 1N HCl to adjust the pH to 2.5, followed by purificaiton by means of an Amberlite XAD-2 column chromatography. Fractions eluted with 30% ethanol - water were combined, concentrated and lyophilized to give hydrochloride of Compound 86a(570 mg).

The hydrochloride of Compound 86a(569 mg) was suspended in TFA(3 mℓ), which was stirred at room temperature for one hour. The solvent was distilled off under reduced pressure. The residue was dissolved in water, followed by purification by means of an Amberlite XAD-2 column chromatography. Fractions eluted

with water and 5% ethanol were combined, concentrated and lyophilized to obtain ditrifluoroacetic acid salt of Compound 86b(390 mg).
IR $\nu_{max}$ (KBr)cm$^{-1}$: 3290, 1670, 1545, 1200

| Elemental Analysis : $C_{23}H_{40}N_6O_6F_6 \cdot 2CF_3CO_2H \cdot H_2O$ | | | |
|---|---|---|---|
| Calcd.<br>Found | C,43.67;<br>C,43.25; | H,5.97;<br>H,6.21; | N,11.32<br>N,11.02 |

Example 87

86b

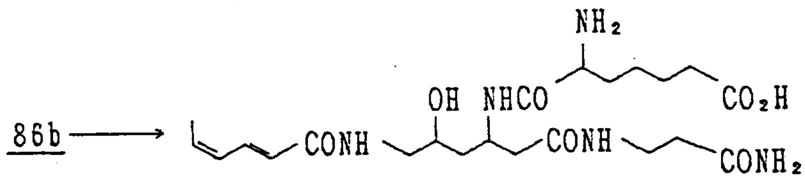

87

To a solution of 2TFA salt of Compound 86b(288 mg) in water(2 mℓ) was added 1N NaOH(1.59 mℓ), and the mixture was stirred for 3 hours. To the mixture was added, under ice-cooling, 1N HCl to adjust the pH to 5.0, followed by purification by means of an Amberlite XAD-2 column chromatography. Fractions eluted with 5% ethanol - water were combined, concentrated and lyophilized to obtain Compound 87 (158 mg).
IR $\nu_{max}$ (KBr)cm$^{-1}$: 1670, 1545, 1410, 1275, 1200

| Elemental Analysis : $C_{22}H_{37}N_5O_7 \cdot 2H_2O$ | | | |
|---|---|---|---|
| Calcd. | C,50.86;<br>C,50.70; | H,7.95;<br>H,7.78; | N,13.48<br>N,13.62 |

Example 88

XVII

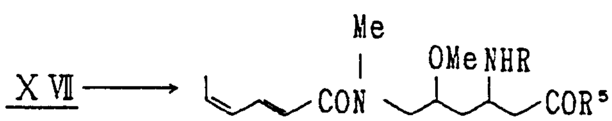

88a : R = Boc, $R^5$ = OH

88b : R = H, $R^5$ = 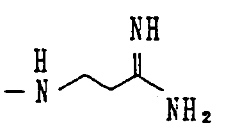

To a suspension of 60% oily sodium hydride (200 mg) in anhydrous THF(5 mℓ) was added, under ice-cooling in argon streams, a solution of Compound XVII(356 mg) in anhydrous THF. The mixture was stirred for 15 minutes, to which was added methyl iodide(374μℓ). The mixture was, after reverting the temperature

thereof to room temperature, stirred for 4 hours. The solvent was distilled off under reduced pressure. To the residue were added a small volume of methanol and the ice-water, followed by washing with ethyl acetate. To the aqueous layer thus obtained was added 1N HCl to render its pH to 3.0. The mixture was extracted with ethyl acetate three times, and the extract was washed with brine, dried ($MgSO_4$), and evaporated under reduced pressure. The residue was prurified by means of a silica gel column chromatography. Fractions eluted with ethyl acetate : acetic acid = 100 : 1 were combined and concentrated to obtain Compound 88a(132 mg).

NMR(90MHz, $CDCl_3$)ppm:1.43(9H,s),1.5-2.0(2H,m),1.87(3H,d,J=6Hz),2.55(2H,d,J=5 Hz),3.04,3.16(3H,s),3.2-3.7(3H,m),3.41(3H,s), 3.8-4.3(1H,m),5.5(1H,br.),5.7-7.9(4H,m).

To a solution of Compound 88a(132 mg) in anhydrous THF (2 mℓ) were added HOBT(51 mg) and DCC(78 mg), and the mixture was stirred for 1.5 hour. To a solution of dihydrochloride of 3-aminopropioamidine(111 mg) in water (2 mℓ) was added sodium hydrogencarbonate(102 mg), and the mixture was stirred. To the mixture was added the above-mentioned active ester solution. The mixture was stirred at room temperature for 15 hours, and insolubles were removed by filtration, followed by concentration of the filtrate. The concentrate was dissolved in ethanol. To the concentrate was then added, under ice-cooling, TFA(3 mℓ). The temperature of the mixture was reverted to room temperature, and the mixture was stirred for one hour. The solvent was distilled off under reduced pressure, and the residue was purified by means of an Amberlite XAD-2 column chromatography. Fracitons eluted with water and 5% ethanol - water were combined and concentrated. The concentrate was allowed to pass through a column of Amberlite IRA-401($Cl^-$ type). The column was subjected to elution with water. The eluate was concentrated and lyophilized to obtain dihydrochloride of Compound 88b(82 mg) as a white powdery product.

IR $\nu_{max}$ (KBr)$cm^{-1}$: 1690, 1635, 1560, 1480, 1435, 1100

| Elemental Analysis : $C_{17}H_{31}N_5O_3 \cdot 2HCl \cdot 0.5H_2O$ | | | |
|---|---|---|---|
| Calcd. | C,46.90; | H,7.87; | N,16.09 |
| Found | C,46.60; | H,7.99; | N,16.00 |

Example 89

XVII

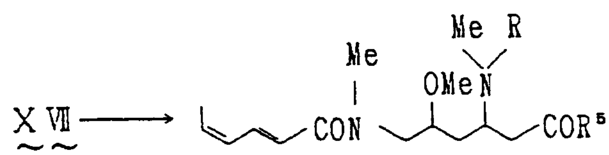

89a : R = Boc, $R^5$ = OH

89b : R = Boc, $R^5$ =

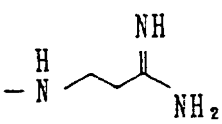

89c : R = H, $R^5$ =

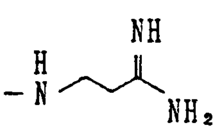

To a solution of Compound XVII(282 mg) in anhydrous THF (10 mℓ) was added, in argon streams, 60% sodium hydride in oil(221 mg), and the mixture was stirred for 5 hours. To the mixture was then added

methyl iodide(345µℓ), and the mixture was left standing at room temperature for 3 days. To the reaction mixture was added methanol(2 mℓ), and the mixture was concentrated under reduced pressure. The concentrate was dissolved in cold water, followed by washing with ethyl acetate. To the aqueous layer was added a 5% aqueous solution of potassium hydrogensulfate to adjust the pH to 3.0, followed by extracting with ethyl acetate three times, washing with brine and drying (over $MgSO_4$). The solvent was distilled off under reduced pressure. The residue was purified by means of a silica gel column chromatography. Fractions eluted with ethyl acetate - acetic acid = 100 : 1 were combined and concentrated to obtain Compound 89a(235 mg).

NMR (90MHz, $CDCl_3$)ppm:1.47(9H,s),1.5-2.0(2H,m),1.88(3H,d,J=6Hz),2.3-2.8(2H,m), 2.75(3H,s),3.06,3.17-(3H,s),3.2-3.7(3H,m), 3.39(3H,s),4.3-4.8(1H,m),5.7-7.9(4H,m).

To a solution of Compound 89a (176 mg) in anhydrous THF (2 mℓ) were added HOBT(68 mg) and DCC(104 mg), and the mixture was stirred at room temperature for one hour. To a solution of dihydrochloride of 3-aminopropioamidine(147 mg) in water(2 mℓ) was added sodium hydrogencarbonate(115 mg), and the mixture was stirred, to which was added the above-mentioned active ester solution. The mixture was stirred at room temperature for 19 hours. Insolubles were filtered off, and the filtrate was concentrated. To the concentrate was added 1N HCl to adjust the pH to 3.0, followed by purication by means of an Amberlite XAD-2 column chromatography. Fractions eluted with 30% ethanol - water were combined, concentrated and lyophilized to obtain hydrochloride of Compound 89b(135 mg) as a white powdery product.

IR $\nu_{max}$ (KBr)$cm^{-1}$: 1690, 1650, 1400

To hydrochloride of Compound 89b(134 mg) was added, while stirring under ice-cooling, TFA(2 mℓ). The temperature of the mixture was reverted to room temperature, and the mixture was stirred for 40 minutes. The solvent was distilled off under reduced pressure. The residue was dissolved in cold water, followed by purification by means of an Amberlite XAD-2 column chromatography. Fractions eluted with water and 5% ethanol were combined and concentrated. The concentrate was allowed to pass through Amberlite IRA-401($Cl^-$ type). Elution was conducted with water, and the eluate was concentrated, followed by lyophilization to obtain dihydrochloride of Compound 89c(83 mg) as a white powdery product.

IR $\nu_{max}$(KBr)$cm^{-1}$: 1690, 1645, 1570, 1415, 1100

| Elemental Analysis : $C_{18}H_{33}N_5O_3 \cdot 2HCl \cdot 1.3H_2O$ | | | |
|---|---|---|---|
| Calcd. | C,46.61; | H,8.17; | N,15.10 |
| Found | C,46.41; | H,8.07; | N,14.81 |

Example 90

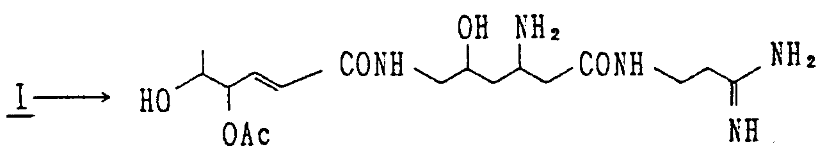

90

To a suspension of dihydrochloride of Compound I(223 mg) in acetic acid (3 mℓ) was added, while stirring under ice-cooling, m-chloroperbenzoic acid(148 mg). The mixture was stirred at room temperature for 3 hours, which was then left standing for 3 days. The solvent was distilled off under reduced pressure, and the residue was dissolved in water, followed by purification by means of an Amberlite XAD-2 column. Fractions eluted with water were combined and concentrated. The concentrate was allowed to pass through Amberlite IRA-401($Cl^-$ type). The eluate was concentrated, followed by lyophilization to obtain dihydrochloride of Compound 90(109 mg) as a white powdery product.

IR $\nu_{max}$ (KBr)$cm^{-1}$: 1735, 1690, 1670 (sh), 1650, 1550, 1235

| Elemental Analysis : $C_{17}H_{31}N_5O_6 \cdot 2HCl \cdot 1.5H_2O$ | | | |
|---|---|---|---|
| Calcd.<br>Found | C,40.72;<br>C,40.61; | H,7.24;<br>H,7.10; | N,13.97<br>N,13.86 |

Example 91

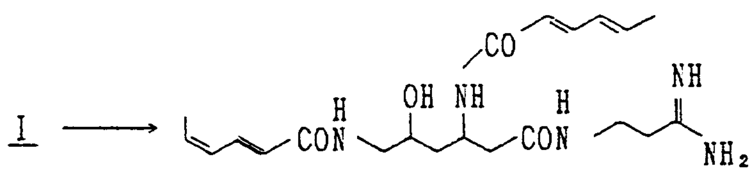
I

91

To a solution of Compound I(295 mg) in DMF(8.0 ml) were added sorbic acid(97 mg) and $Et_3N$(0.149 mℓ). To the mixture were added, under ice-cooling, HOBT(116 mg) and DCC(178 mg), followed by stirring for 30 minutes. The mixture was stirred at room temperature for 20 hours, followed by distilling off DMF under reduced pressure. To the residue was added water(20 mℓ), to which was added 1N HCl to adjust the pH to 1.9. The mixture was washed with ethyl acetate (12 mℓ) three times. To the aqueous layer was added 1N NaOH to adjust the pH to 6.4, followed by concentration under reduced pressure to a volume of about 1 mℓ. The concentrate was subjected to a Diaion HP-20(50-100 mesh, 20 mℓ) column chromatography. Elution was conducted using water(100 mℓ), 20% methanol-water(60 mℓ), 50% methanol-water(100 ml) and 50% methanol-0.01N HCl(100 ml), successively, followed by fractionating at 20 ml each portion. Each fraction was subjected to analysis by means of a high performance liquid chromatography [mobile phase : 25% acetonitrile/0.01 M phosphate buffer (pH3.0)]. Fractions showing single peak were combined and concentrated, followed by lyophilization to obtain hydrochloride of Compound 91(190 mg) as a white powdery product.

| Elemental Analysis : $C_{21}H_{33}N_5O_4 \cdot HCl \cdot 3.0H_2O$ | | | | |
|---|---|---|---|---|
| Calcd.<br>Found | C,49.45;<br>C,49.44; | H,7.90;<br>H,7.33; | N,13.73;<br>N,14.14; | Cl,6.95<br>Cl,6.95 |

Example 92

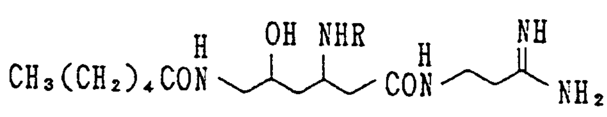

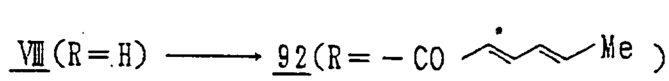
VIII(R=H) 92(R= −CO Me )

In DMF were dissolved Compound VIII(307.3 mg), sorbic acid (103 mg) and $Et_3N$(0.16 mℓ). To the mixture were added, under ice-cooling, HOBT(124 mg) and DCC(189 mg), successively. The mixture was stirred for 30 minutes, then for 16 hours at room temperature. The reaction mixture was concentrated, to which was added dilute hydrochloric acid (25 mℓ) to adjust the pH to 1.8, followed by washing with ethyl acetate(13 mℓ) three times. To the mixture was added 1N-NaOH to adjust the pH to 6.7. The mixture was

concentrated, and the concentrate was subjected to a Diaion HP-20(50 to 100 mesh, 20 mℓ) column chromatography, followed by elution with water(100 mℓ), 10%, 20%, 50% aqueous methanol(each 60 mℓ) and 50% methanol - 0.01N HCl(60 mℓ), successively to fractionate into 20 mℓ each portion. Each fraction was subjected to analysis by means of a high performance liquid chromatography[mobile phase : 26% acetonitrile/0.01M phosphate buffer(pH 3.0)]. Fractions exhibiting single peak were combined and concentrated, followed by lyophilization to obtain hydrochloride of Compound 92(302 mg) as a white powdery product.

| Elemental Analysis : $C_{21}H_{37}N_5O_4 \cdot HCl \cdot 1.5H_2O$ | | | | |
|---|---|---|---|---|
| Calcd. | C,51.79; | H,8.49; | N,14.38; | Cl,7.28 |
| Found | C,51.58, | H,8.51; | N,14.10; | Cl,7.48 |

Example 93

92 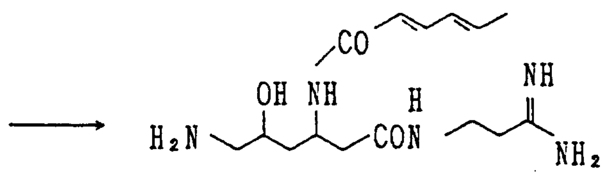

93

In a 0.03M phosphate buffer solution(pH 7.0, 100 mℓ) was dissolved monohydrochloride of Compound 92(186 mg). To the solution were added cells of Pseudomonas•acidovorans IFO 13582(10 g), and the mixture was shaken at 37°C for 14 hours. The reaction solution was subjected to centrifugation. The supernatant was allowed to pass through a column of IRC-50 ($NH_4^+$ ,14mℓ), followed by elution with water, 0.5M, 1.0M, 1.5M and 0.2M brine (each 70 mℓ), successively, fractionating into 14 mℓ each portion. Each fraction was subjected to analysis by means of a high performance liquid chromatography[mobile phase : 6% acetonitrile/0.01M phosphate buffer solution(pH 3.0)]. Fractions exhibiting single peak were combined and concentrated. The concentrate was allowed to pass through a column of activated charcoal (10 mℓ), followed by elution with water (50 mℓ) and 8% isobutanol water(60 mℓ), fractionating into 10 mℓ each portion. Each fraction was subjected to a high performance liquid chromatography[mobile phase : the same as above]. Fractions exhibiting single peak were combined and concentrated under reduced pressure followed by lyophilization to obtain dihydrochloride of Compound 93(99 mg) as a white powdery product.

| Elemental Analysis : $C_{15}H_{27}N_5O_3 \cdot 2HCl \cdot 1.0H_2O$ | | | | |
|---|---|---|---|---|
| Calcd. | C,43.27; | H,7.50; | N,16.82; | Cl,17.03 |
| Found | C,43.27; | H,8.23; | N,16.85; | Cl,17.03 |

Example 94

I

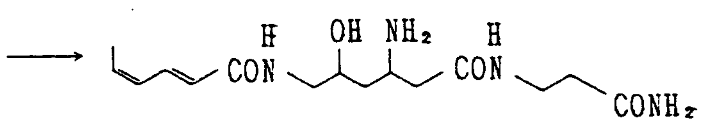

94

A solution of dihydrochloride of Compound I(170 mg) in water (30 mℓ) was subjected to a Dowex 1 x 2-(OH⁻ type, 50 to 100 mesh, 15 mℓ) column chromatography. The column was washed with water. The solution which passed through the column and the washing were combined and concentrated, followed by lyophilization to obtain Compound 94 as a crude powdery product(141 mg). The crude powder was dissolved in water (30 mℓ), and the solution was subjected to an Amberlite CG-50(H⁺ type, 100 to 200 mesh, 15 mℓ) column chromatography. The column was wahsed with water(45 mℓ) and 0.5% aqueous ammonia(75 mℓ), successively, followed by elution with 2% aqueous ammonia(45 mℓ). The eluate was concentrated and then lyophilized to obtain Compound 94 as a white powdery product(100 mg).
IR $\nu_{max}$(KBr)$cm^{-1}$: 3320, 1660, 1550

| Elemental Analysis : $C_{15}H_{26}N_4O_4 \cdot H_2O$ | | | |
|---|---|---|---|
| Calcd.<br>Found | C,52.31;<br>C,52.62; | H,8.19;<br>H,7.36; | N,16.27<br>N,15.60 |

Example 95

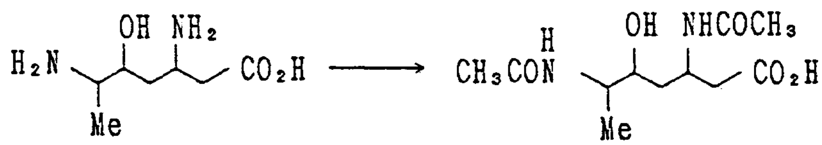

XXIV

95

To a solution of Compound XXIV(108 mg) in a 3% aqueous solution of sodium hydrogencarbonate(15 mℓ) was added acetic anhydride(0.17 mℓ). The mixture was stirred at room temperature for 3 hours, followed by addition of Dowex 50W-X2($H^+$,10 mℓ). The mixture was stirred for further 30 minutes. The resin was separated by filtration and washed with water(40 mℓ). The filtrate and the washing were combined and concentrated under reduced pressure, followed by lyophilization to obtain Compound 95 as a crude powdery product(123 mg). This crude product was subjected to a silica gel column chromatography, using as eluents ethyl acetate:methanol = 7:1,5:1,4:1 and 3:1 (each 60 mℓ) successively, to fractionate into 12 mℓ each portion. Each fraction was subjected to analysis by means of a silica gel thin layer chromatography-(mobile phase : ethyl acetate : methanol = 3:1). Fractions exhibiting single spot were combined and concentrated to dryness to obtain Compound 95 as a white powdery product(69 mg), which was crystallized from acetone to obtain Compound 95 as colourless crystals (44 mg).

| Elemental Analysis : $C_{11}H_{20}N_2O_5$ | | | |
|---|---|---|---|
| Calcd.<br>Found | C,50.76;<br>C,50.68; | H,7.74;<br>H,7.80; | N,10.76<br>N,10.50 |

Example 96

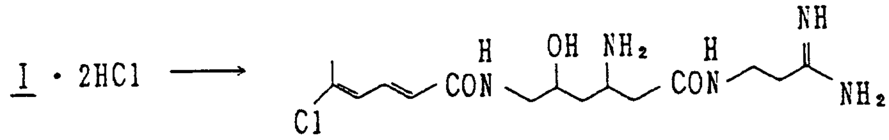

96

To a solution of dihydrochloride of Compound I(196 mg) in water(1.8 mℓ) and dioxane(5.2 mℓ) was added at room temperature N-bromosuccinimide(87 mg), and the mixture was stirred for 60 minutes. Dioxane was distilled off under reduced pressure, and the reaction mixture was allowed to pass through Amberlite CG-50(type I$^+$, $NH_4$,20 mℓ), elution being conducted with water, 0.1M, 0.2M, 0.3M, 0.4M, 0.5M, 0.6M and 0.8M brine (each 60 mℓ) and 1.0M and 1.5M brine (each 200 mℓ), fractionating into 20 mℓ each portion. Each fraction was subjected to analysis by means of high performance liquid chromatography [mobile phase : 25% methanol/0.01M phosphate buffer(pH 3.0)]. Fractions exhibiting substantially single peak were combined and concentrated under reduced pressure. The concentrate was subjected to a Diaion HP-20(50 to 100 mesh, 15 mℓ) column chromatography, eluting with water(100 mℓ) to fractionate into 15 mℓ each portion. Each fraction was subjected to analysis by means of high performance liquid chromatography[mobile phase : the same as above]. Fractions exhibiting single peak were combined and concentrated under reduced pressure, followed by lyophilization to obtain dihydrochloride of Compound 96-(45 mg) as a white powdery product.

| Elemental Analysis : $C_{15}H_{26}N_5O_3Cl \cdot 2HCl \cdot 8.0H_2O$ | | | |
|---|---|---|---|
| Calcd. | C,31.23; | H,7.69; | N,12.14 |
| Found | C,31.32; | H,5.97; | N,12.06 |

Examples 97 - 120 (Table 13)

By the procedure similar to Example 1, dihydrochloride of Compound (XIII) was subjected to acylation (Step A) by using various carboxylic acid (R'$CO_2$H) or its derivatives, then to deprotection reaction (Step B) to obtain compounds some of the physico-chemical properties of the products are shown in Table 13.

Table 13

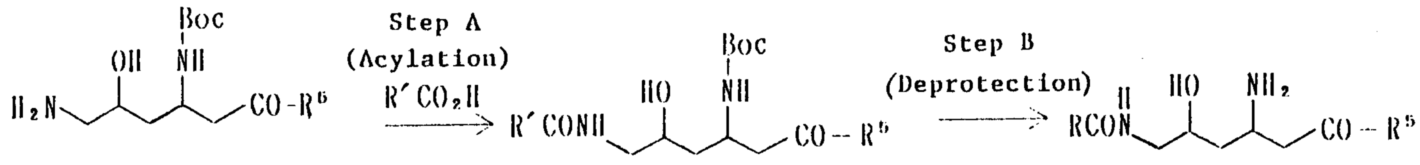


XIII a b

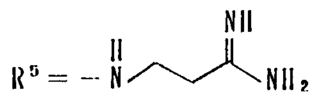

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 97 | A | XIII → 97a<br>R′ = 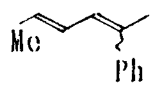 | R′$CO_2H$ (113mg)<br>HOBT (34mg)<br>DCC (124mg)<br>$Et_3N$ (105μℓ)<br>DMF (3mℓ)<br>r.t. 20hr<br>(hereafter same as 3A) | 202mg → 252mg | 1710(sh)<br>1690<br>1650<br>1540 | $C_{26}H_{39}N_5O_5 \cdot HCl \cdot H_2O$<br>C 55.86 (56.16)<br>H 7.88 (7.61)<br>N 12.31 (12.59) |
| | B | 97a → 97b<br>R = 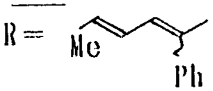 | TFA (2mℓ)<br>r.t. 30min<br>evap. $Et_2O$ washing<br>IRA-402($Cl^-$), HP-20<br>Lyophilization | 240mg → 181mg | 1690<br>1650<br>1530 | $C_{21}H_{31}N_5O_3 \cdot 2HCl \cdot 1.5H_2O$<br>C 50.02 (50.30)<br>H 7.44 (7.24)<br>N 13.67 (13.97) |

| Ex. No. | Step | Starting Compd. → Prodcut Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 98 | A | X III<br>→<br>98a<br>R′ = 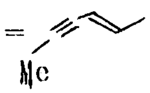 | $R'CO_2H$ (6.6mg)<br>HOBT (34mg)<br>DCC (124mg)<br>$Et_3N$ (105μℓ)<br>DMF (3mℓ)<br>r.t. 15hr<br>(hereafter same as 3A) | 202mg<br>→<br>235mg | 1710(sh)<br>1690<br>1650<br>1610<br>1540 | $C_{26}H_{33}N_5O_5 \cdot HCl \cdot 0.5H_2O$<br>C 57.65 (57.72)<br>H 6.74 ( 6.52)<br>N 12.88 (12.94) |
| | B | 98a<br>→<br>98b<br>R = 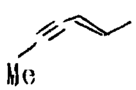 | TFA (2mℓ)<br>anisole (2mℓ)<br>r.t. 1 hr<br>(hereafter same as 97B) | 235mg<br>→<br>180mg | 1690<br>1650<br>1610<br>1545 | $C_{15}H_{26}N_5O_3 \cdot 2HCl \cdot H_2O$<br>C 43.19 (43.48)<br>H 7.32 ( 7.05)<br>N 16.68 (16.90) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 99 | A | XIII → 99a<br>R′ = 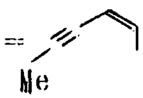 | $R'CO_2H$ (66mg)<br>HOBT (34mg)<br>DCC (124mg)<br>$Et_3N$ (105μℓ)<br>DMF (3mℓ)<br>r.t. 20hr<br>(hereafter same as 3A) | 202mg<br>→<br>225mg | 1710(sh)<br>1690<br>1650<br>1530 | $C_{20}H_{33}N_5O_6 \cdot HCl \cdot H_2O$<br>C 56.51 (56.77)<br>H 6.88 ( 6.60)<br>N 12.49 (12.73) |
| | B | 99a → 99b<br>R = 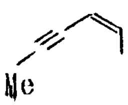 | TFA (2mℓ)<br>anisole (2mℓ)<br>0℃→r.t. 1hr<br>(hereafter same as 97B) | 224mg<br>→<br>169mg | 1690<br>1645<br>1545 | $C_{15}H_{25}N_5O_3 \cdot 2HCl \cdot 1.5H_2O$<br>C 42.28 (42.56)<br>H 7.41 ( 7.14)<br>N 16.27 (16.54) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 100 | A | XIII → 100a<br>R' = 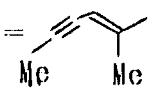 | $R'CO_2H$ (60mg)<br>HOBT (27mg)<br>DCC (100mg)<br>$Et_3N$ (84 μℓ)<br>DMF (2mℓ)<br>r.t. 3days<br>(hereafter same as 3A) | 162mg → 165mg | 1710(sh)<br>1695<br>1650<br>1535 | $C_{21}H_{35}N_5O_6 \cdot HC\ell \cdot 0.5H_2O$<br>C 51.95 (52.22)<br>H 7.96 ( 7.72)<br>N 14.23 (14.50) |
| | B | 100a → 100b<br>R = 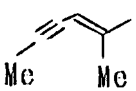 | TFA (2mℓ)<br>anisole (2mℓ)<br>0℃→r.t. 1 hr<br>(hereafter same as 97B) | 164mg → 116mg | 1690<br>1640<br>1600<br>1535 | $C_{16}H_{27}N_5O_3 \cdot 2HC\ell \cdot H_2O$<br>C 44.59 (44.86)<br>H 7.56 ( 7.29)<br>N 16.09 (16.35) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
| --- | --- | --- | --- | --- | --- | --- |
| 101 | A | XIII → 101a<br>R' = 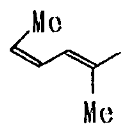 | $R'CO_2H$ (61mg)<br>HOBT (27mg)<br>DCC (100mg)<br>$Et_3N$ (84μℓ)<br>DMF (2mℓ)<br>r.t. 3days<br>(hereafter same as 3A) | 162mg<br>→<br>158mg | 1710(sh)<br>1690<br>1650<br>1530 | $C_{21}H_{37}N_5O_6 \cdot HCl \cdot 0.5H_2O$<br>C 51.72 (52.00)<br>H 8.34 ( 8.10)<br>N 14.21 (14.44) |
| | B | 101a → 101b<br>R = 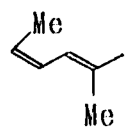 | TFA (2mℓ)<br>0℃→r.t. 75min<br>(hereafter same as 97B) | 157mg<br>→<br>106mg | 1690<br>1650<br>1540 | $C_{16}H_{29}N_5O_3 \cdot 2HCl \cdot H_2O$<br>C 44.39 (44.65)<br>H 8.31 ( 8.05)<br>N 14.48 (14.71) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 102 | A | XIII<br>→<br>102a<br>R′ = 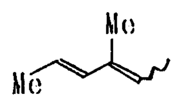 | $R'CO_2H$ (61mg)<br>HOBT (27mg)<br>DCC (100mg)<br>$Et_3N$ (84μℓ)<br>DMF (2mℓ)<br>r.t. 3days<br>(hereafter same as 3A) | 162mg<br>→<br>184mg | 1710(sh)<br>1690<br>1650<br>1530 | $C_{21}H_{37}N_6O_6 \cdot HC\ell \cdot H_2O$<br>C 50.79 (51.06)<br>H 8.44 ( 8.16)<br>N 14.00 (14.18) |
| | B | 102a<br>→<br>102b<br>R = 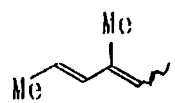 | TFA (2mℓ)<br>0℃→r.t. 1 hr<br>(hereafter same as 97B) | 183mg<br>→<br>72mg | 1690<br>1650<br>1540 | $C_{16}H_{20}N_5O_3 \cdot 2HC\ell \cdot 1.5H_2O$<br>C 43.49 (43.74)<br>H 8.09 ( 7.80)<br>N 15.68 (15.94) |

| Ex. No. | Step | Starting Compd. → Product Compd. | Reaction Conditions | Yield Material → Product | IR $\nu_{max}^{KBr}$ $cm^{-1}$ | Molecualr Formula Analysis (Calcd.) |
|---|---|---|---|---|---|---|
| 103 | A | XIII → 103a<br>R′ = 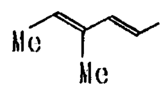 | $R'CO_2H$ (61mg)<br>HOBT (27mg)<br>DCC (100mg)<br>$Et_3N$ (84μℓ)<br>DMF (2mℓ)<br>r.t. 15hr<br>(hereafter same as 3A) | 162mg → 197mg | 1710(sh)<br>1690<br>1650<br>1605<br>1540 | $C_{21}H_{37}N_5O_6 \cdot HCl \cdot 0.5H_2O$<br>C 51.74 (52.00)<br>H 8.32 ( 8.10)<br>N 14.18 (14.44) |
| | B | 103a → 103b<br>R = 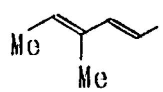 | TFA (2mℓ)<br>(hereafter same as 97B) | 196mg → 147mg | 1690<br>1650<br>1600<br>1550 | $C_{16}H_{29}N_5O_3 \cdot 2HCl \cdot H_2O$<br>C 44.39 (44.65)<br>H 7.93 ( 7.73)<br>N 16.03 (16.27) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 104 | A | XIII<br>→<br>104a<br>R′ = 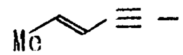 | $R'CO_2H$ (39mg)<br>HOBT (27mg)<br>DCC (87mg)<br>$Et_3N$ (73μℓ)<br>DMF (2mℓ)<br>r.t. 23hr<br>(hereafter same as 3A) | 142mg<br>→<br>164mg | 1710(sh)<br>1690<br>1630<br>1530 | $C_{20}H_{33}N_5O_6 \cdot HCl \cdot H_2O$<br>C 50.01 (50.26)<br>H 7.81 (7.59)<br>N 14.42 (14.65) |
| | B | 104a<br>→<br>104b<br>R = 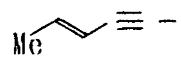 | TFA (2mℓ)<br>0°C → r.t. 1.5hr<br>(hereafter same as 97B) | 164mg<br>→<br>119mg | 1680<br>1630<br>1610<br>1540 | $C_{15}H_{25}N_5O_3 \cdot 2HCl \cdot 1.5H_2O$<br>C 42.29 (42.56)<br>H 7.36 (7.14)<br>N 16.28 (16.54) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 105 | A | XIII → 105a<br>R' = 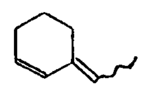 | $R'CO_2H$ (53mg)<br>HOBT (27mg)<br>DCC (89mg)<br>$Et_3N$ (81 μℓ)<br>DMF (2mℓ)<br>r.t. 3days<br>(hereafter same as 3A) | 154mg → 173mg | 1710(sh)<br>1690<br>1640<br>1530 | $C_{22}H_{37}N_5O_5 \cdot HCl \cdot 0.5H_2O$<br>C 52.96 (53.16)<br>H 8.05 ( 7.91)<br>N 13.88 (14.09) |
| | B | 105a → 105b<br>R = 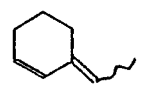 | TFA (2mℓ)<br>r.t. 1hr<br>(hereafter same as 97B) | 173mg → 94mg | 1690<br>1650<br>1630<br>1540 | $C_{17}H_{29}N_5O_3 \cdot 2HCl \cdot H_2O$<br>C 45.88 (46.16)<br>H 7.76 ( 7.52)<br>N 15.64 (15.83) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 106 | A | XIII<br>→<br>106a<br>R' = 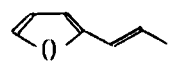 | $R'CO_2H$ (105mg)<br>HOBT (90mg)<br>DCC (150mg)<br>$Et_3N$ (0.06ml)<br>DMF (3 ml)<br>r.t. 20hr<br>(hereafter same as 3A) | 200mg<br>→<br>220mg | 1690<br>1670<br>1665<br>1550 | $C_{21}H_{33}N_5O_6 \cdot HCl \cdot H_2O$<br>C 49.63 (49.85)<br>H 7.38 (7.17)<br>N 13.60 (13.84) |
| | B | 106a<br>→<br>106b<br>R = 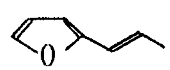 | 2N-HCl (3ml)<br>r.t. 4hr<br>Activated-C column(3g)<br>After washing with $H_2O$(100 ml)<br>Eluted with 10%MeOH-$H_2O$<br>Lyophilization | 220mg<br>→<br>70mg | 1690<br>1665<br>1650<br>1560 | $C_{16}H_{25}N_5O_4 \cdot 2HCl \cdot H_2O$<br>C 43.29 (43.45)<br>H 6.75 (6.61)<br>N 15.68 (15.83) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 107 | A | XIII → 107a<br>R' = 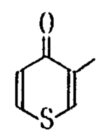 | $R'CO_2H$ (120mg)<br>HOBT (90mg)<br>DCC (150mg)<br>$Et_3N$ (0.06mℓ)<br>DMF (3mℓ)<br>r.t. 20hr<br>(hereafter same as 3A) | 200mg<br>→<br>250mg | 1690<br>1670<br>1650<br>1540 | $C_{20}H_{31}N_5O_8 \cdot HC\ell \cdot 0.5H_2O$<br>C 46.39 (46.64)<br>H 6.69 ( 6.46)<br>N 13.48 (13.60) |
| | B | 107a → 107b<br>R = 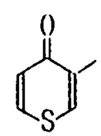 | 2N-HCl (3mℓ)<br>r.t. 4hr<br>(hereafter same as 106B) | 250mg<br>→<br>75mg | 1690<br>1660<br>1650<br>1535 | $C_{16}H_{23}N_5O_4S \cdot 2HC\ell \cdot H_2O$<br>C 38.89 (39.13)<br>H 6.16 ( 5.91)<br>N 15.03 (15.21) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 108 | A | X III → 108a<br>R′ = 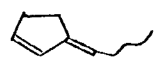 | $R'CO_2H$ (50mg)<br>HOBT (27mg)<br>DCC (100mg)<br>$Et_3N$ (84 μℓ)<br>DMF (2mℓ)<br>r.t. 23hr<br>(hereafter same as 3A) | 162mg<br>→<br>170mg | 1710(sh)<br>1690<br>1645<br>1525 | $C_{21}H_{35}N_5O_5 \cdot HCl \cdot H_2O$<br>C 51.03 (51.26)<br>H 8.01 ( 7.78)<br>N 14.00 (14.23) |
| | B | 108a → 108b<br>R = 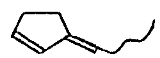 | THF (2mℓ)<br>r.t. 1hr<br>(hereafter same as 97B) | 169mg<br>→<br>101mg | 1690<br>1650<br>1545 | $C_{16}H_{27}N_5O_3 \cdot 2HCl \cdot H_2O$<br>C 44.63 (44.86)<br>H 7.47 ( 7.29)<br>N 16.21 (16.35) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 109 | A | XIII<br>→<br>109a<br><br>R′ = 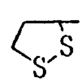 $(CH_2)_4$- | $R'CO_2H$ (80mg)<br>HOBT (45mg)<br>DCC (85mg)<br>$Et_3N$ (0.35mℓ)<br>DMF (2mℓ)<br>r.t. 24hr<br>(hereafter same as 3A) | 100mg<br>→<br>130mg | 1690<br>1640<br>1540 | $C_{22}H_{41}N_5O_5S_2 \cdot HC\ell \cdot 0.5H_2O$<br>C 46.48 (46.75)<br>H 7.89 ( 7.67)<br>N 12.11 (12.39) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 110 | A | <u>XIII</u><br>→<br><u>110a</u><br>R′ = 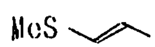 | $R'CO_2H$ (90mg)<br>HOBT (90mg)<br>DCC (150mg)<br>$Et_3N$ (0.06mℓ)<br>DMF (3mℓ)<br>r.t. 24hr<br>(hereafter same as 3A) | 200mg<br>→<br>200mg | 1680<br>1590<br>1450 | $C_{18}H_{33}N_5O_5S \cdot HCl \cdot H_2O$<br>C 44.22 (44.48)<br>H 7.69 ( 7.47)<br>N 14.21 (14.41) |
| | B | <u>110a</u><br>→<br><u>110b</u><br>R = 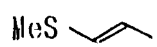 | TFA (2mℓ)<br>r.t. 20min<br>Distn. off TFA, activ. C chrom.<br>Eltn. with 10%MeOH-$H_2O$<br>Lyophilization | 100mg<br>→<br>60mg | 1680<br>1580<br>1440 | $C_{13}H_{25}N_5O_3S \cdot 2CF_3CO_2H \cdot H_2O$<br>C 35.22 (35.36)<br>H 5.26 ( 5.06)<br>N 12.01 (12.13) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 111 | A | XIII<br>→<br>111a<br><br>R′ =$CH_3CH_2O$ 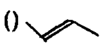 | $R'CO_2H$ (90mg)<br>HOBT (90mg)<br>DCC (150mg)<br>$Et_3N$ (0.06mℓ)<br>DMF (3mℓ)<br>r.t. 24hr<br>(hereafter same as 3A) | 200mg<br>→<br>200mg | 1690<br>1660<br>1600<br>1550 | $C_{19}H_{35}N_5O_6$ · HCℓ · 0.5$H_2O$<br>C 47.85 (48.05)<br>H 8.03 ( 7.85)<br>N 14.51 (14.74) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 112 | A | XIII → 112a<br>R′ = 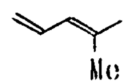 | $R'CO_2H$ (50mg)<br>HOBT (27mg)<br>DCC (100mg)<br>$Et_3N$ (84 μℓ)<br>DMF (2mℓ)<br>r.t. 3days<br>(hereafter same as 3A) | 162mg<br>→<br>177mg | 1710(sh)<br>1690<br>1650<br>1530 | $C_{20}H_{35}N_5O_6 \cdot HCl \cdot 0.5H_2O$<br>C 50.74 (51.00)<br>H 8.19 ( 7.92)<br>N 14.72 (14.87) |
| | B | 112a → 112b<br>R = 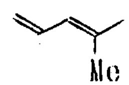 | TFA (2mℓ)<br>0℃→r.t. 1.5hr<br>(hereafter same as 97B) | 176mg<br>→<br>161mg | 1690<br>1650<br>1530 | $C_{15}H_{27}N_5O_3 \cdot 2HCl \cdot H_2O$<br>C 43.08 (43.27)<br>H 7.66 ( 7.50)<br>N 16.68 (16.82) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 113 | A | X III → 113a<br>R' = 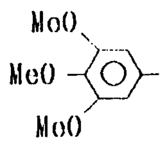 | R'$CO_2$H (318mg)<br>HOBT (180mg)<br>DCC (300mg)<br>$Et_3N$ (0.1mℓ)<br>DMF (6mℓ)<br>r.t. 24hr<br>(hereafter same as 3A) | 400mg<br>→<br>490mg | 1690<br>1650<br>1550 | $C_{24}H_{30}N_5O_8 \cdot HC\ell \cdot H_2O$<br>C 49.47 (49.69)<br>H 7.51 ( 7.30)<br>N 11.92 (12.07) |
| | B | 113a → 113b<br>R = 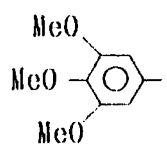 | 2N-HCl (5mℓ)<br>r.t. 3hr<br>Activ.-C chrom.<br>Eltn.with 10%MeOH<br>Lyophilization | 490mg<br>→<br>200mg | 1690<br>1650<br>1540 | $C_{19}H_{31}N_5O_8 \cdot 2HC\ell \cdot H_2O$<br>C 44.01 (44.19)<br>H 6.99 ( 6.83)<br>N 13.33 (13.56) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 114 | A | <u>X III</u> → <u>114a</u><br>R′ = 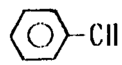 $CH_2$- | $R'CO_2H$ (408mg)<br>HOBT (350mg)<br>DCC (615mg)<br>$Et_3N$ (0.2mℓ)<br>DMF (10mℓ)<br>r.t. 24hr<br>(hereafter same as 3A) | 800mg<br>→<br>900mg | 1690<br>1650<br>1620<br>1540 | $C_{22}H_{35}N_5O_6 \cdot HC\ell \cdot 0.5H_2O$<br>C 53.13 (53.38)<br>H 7.69 ( 7.53)<br>N 14.00 (14.15) |
| | B | <u>114a</u> → <u>114b</u><br>R = 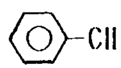 $CH_2$- | 1N-HCl (20mℓ)<br>Activ.-C chromato.<br>Eltn.with 10%MeOH<br>Lyophilization | 900mg<br>→<br>550mg | 1690<br>1650<br>1630<br>1540<br>1480 | $C_{17}H_{27}N_5O_3 \cdot 2HC\ell \cdot H_2O$<br>C 46.18 (46.37)<br>H 7.24 ( 7.10)<br>N 15.77 (15.90) |

| Ex. No. | Step | Starting Compd. → Product Compd. | Reaction Conditions | Yield Material → Product | IR $\nu_{max}^{KBr}$ $cm^{-1}$ | Molecuar Formula Analysis (Calcd.) |
|---|---|---|---|---|---|---|
| 115 | A | XIII<br>→<br>115a<br>R' = 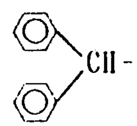 | $R'CO_2H$ (635mg)<br>HOBT (350mg)<br>DCC (617mg)<br>$Et_3N$ (0.28ml)<br>DMF (10ml)<br>r.t. 24hr<br>(hereafter same as 3A) | 800mg<br>→<br>1020mg | 1690<br>1650<br>1550 | $C_{28}H_{30}N_5O_5 \cdot HCl \cdot H_2O$<br>C 57.76 (57.97)<br>H 7.48 ( 7.30)<br>N 11.92 (12.07) |
| | B | 115a<br>→<br>115b<br>R = 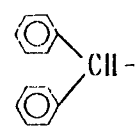 | 1N-HCl (20ml)<br>r.t. 4hr<br>Activ.-C Chromato.<br>Eltn.with 10%MeOH<br>Lyophilization | 1020mg<br>→<br>650mg | 1690<br>1640<br>1550<br>1495 | $C_{23}H_{31}N_5O_3 \cdot 2HCl \cdot 1.5H_2O$<br>C 45.17 (45.44)<br>H 7.40 ( 7.18)<br>N 15.30 (15.58) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
| --- | --- | --- | --- | --- | --- | --- |
| 116 | A | XIII → 116a<br>R′ = 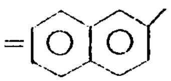 | R′$CO_2H$ (650mg)<br>HOBT (440mg)<br>DCC (760mg)<br>$Et_3N$ (0.34mℓ)<br>DMF (15mℓ)<br>r.t. 24hr<br>(hereafter same as 3A) | 1000mg → 1000mg | 1690<br>1630<br>1550 | $C_{25}H_{35}N_5O_5 \cdot HCl \cdot 0.5H_2O$<br>C 56.25 (56.54)<br>H 7.21 ( 7.02)<br>N 13.03 (13.19) |
| | B | 116a → 116b<br>R = 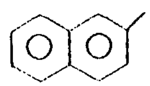 | 1N-HCl (20mℓ)<br>Active.-C chromato.<br>Eltn.with 10%MeOH<br>Lyophilization | 1000mg → 500mg | 1690<br>1630<br>1540 | $C_{20}H_{27}N_5O_3 \cdot 2HCl \cdot H_2O$<br>C 50.19 (50.42)<br>H 6.80 ( 6.56)<br>N 14.54 (14.70) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 117 | A | XIII → 117a<br>R′ = 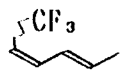 | R′$CO_2$H (67mg)<br>HOBT (54mg)<br>DCC (99mg)<br>$Et_3N$ (84μℓ)<br>DMF (2mℓ)<br>r.t. 16hr<br>(hereafter same as 3A) | 162mg → 228mg | 1710(sh)<br>1690<br>1675<br>1640<br>1530 | $C_{20}H_{32}F_3N_5O_5 \cdot HCl \cdot H_2O$<br>C 44.77 (44.99)<br>H 6.87 ( 6.61)<br>N 13.03 (13.12) |
| | B | 117a → 117b<br>R = 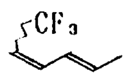 | TFA (2mℓ)<br>0℃ → r.t. 1hr<br>(hereafter same as 97B) | 228mg → 163mg | 1690<br>1635<br>1550 | $C_{15}H_{24}F_3N_5O_3 \cdot 2HCl \cdot 1.5H_2O$<br>C 37.28 (37.59)<br>H 6.32 ( 6.10)<br>N 14.39 (14.61) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 118 | A | XIII → 118a<br>R' = 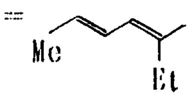 | $R'CO_2H$ (67mg)<br>HOBT (27mg)<br>DCC (99mg)<br>$Et_3N$ (84μℓ)<br>DMF (2mℓ)<br>r.t. 16hr<br>(hereafter same as 3A) | 162mg<br>→<br>161mg | 1710(sh)<br>1685<br>1650<br>1520 | $C_{22}H_{30}N_5O_5 \cdot HCl \cdot 0.5H_2O$<br>C 52.71 (52.95)<br>H 8.40 ( 8.28)<br>N 13.88 (14.03) |
| | B | 118a → 118b<br>R = 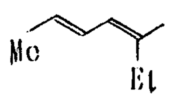 | TFA (2mℓ)<br>0℃→r.t. 1hr<br>(hereafter same as 97B) | 160mg<br>→<br>101mg | 1690<br>1645<br>1530 | $C_{17}H_{31}N_5O_3 \cdot 2HCl \cdot H_2O$<br>C 45.87 (46.05)<br>H 7.89 ( 7.73)<br>N 15.70 (15.79) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 119 | A | XIII<br>→<br>119a<br>R′ = 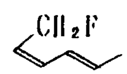 | $R'CO_2H$ (83mg)<br>HOBT (34mg)<br>DCC (130mg)<br>$Et_3N$ (105μℓ)<br>DMF (3mℓ)<br>r.t. 16hr<br>(hereafter same as 3A) | 203mg<br>→<br>234mg | 1710(sh)<br>1690<br>1660<br>1530 | $C_{20}H_{34}FN_5O_6 \cdot HCl \cdot H_2O$<br>C 47.98 (48.24)<br>H 7.64 ( 7.49)<br>N 13.87 (14.06) |
| | B | 119a<br>→<br>119b<br>R = 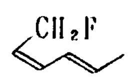 | TFA (2mℓ)<br>0℃→r.t. 1hr<br>(hereafter same as 97B) | 233mg<br>→<br>153mg | 1690<br>1655<br>1540 | $C_{15}H_{26}FN_5O_3 \cdot 2HCl \cdot H_2O$<br>C 41.25 (41.48)<br>H 7.17 ( 6.96)<br>N 16.01 (16.12) |

| Ex. No. | Step | Starting Compd. → Product Compd. | Reaction Conditions | Yield Material → Product | IR $\nu_{max}^{KBr}$ $cm^{-1}$ | Molecular Formula Analysis : (Calcd.) |
|---|---|---|---|---|---|---|
| 120 | A | XIII → 120a<br>R' = 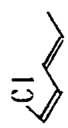 | $R'CO_2H$ (106mg)<br>HOBT (108mg)<br>DCC (206mg)<br>THF (3mℓ)<br>r.t. 1.5hr<br>XIII, $NaHCO_3$ (51mg)<br>$H_2O$(3mℓ)soln.+above active ester<br>0℃→r.t. 1.5hr, 1N-HCl 3drops, HP-20 column purificn. | 162mg → 201mg | 1710(sh)<br>1690<br>1655<br>1530 | $C_{19}H_{32}ClN_5O_5 \cdot HCl \cdot 0.5H_2O$<br>C 46.23 (46.44)<br>H 7.11 ( 6.97)<br>N 14.08 (14.25) |
| | B | 120a → 120b<br>R = 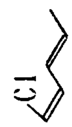 | TFA (2mℓ)<br>0℃→r.t. 1hr<br>(hereafter same as 97B) | 201mg → 147mg | 1690<br>1655<br>1610<br>1545 | $C_{14}H_{24}ClN_5O_3 \cdot 2HCl \cdot H_2O$<br>C 41.82 (41.90)<br>H 7.21 ( 7.03)<br>N 17.38 (17.45) |

Examples 121 - 123 (Table 14)

By the procedure similar to Example 54, Compound (XXVIII) was subjected to amidation by using various amines ($R'NH_2$) to obtain corresponding amido compounds (Step A), followed by deprotection

reaction (Step B) to obtain compounds of Examples 121 - 123. The reaction conditions, yields and some of the physico-chemical properties of the products are shown in Table 14.

Table 14

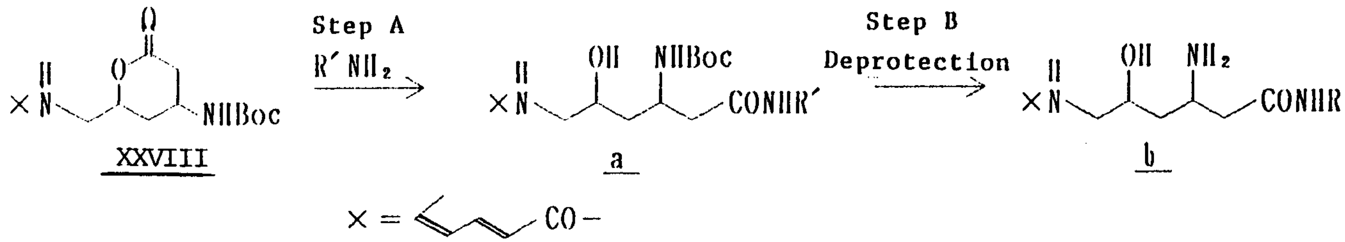


XXVIII a b

| Ex. No. | Step | Starting Compd. → Product Comps. | Reaction Conditons | Yield Material → Product | IR $\nu_{max}^{KBr}$ $cm^{-1}$ | Molecular Formula Analysis (Calcd.) |
|---|---|---|---|---|---|---|
| 121 | A | XXVIII → 121a R′ = 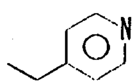 | R′$NH_2$ (275mg) MeOH (10ml) r.t. 7days conc., $SiO_2$ column chromato $CHCl_3$:EtoAc:MeOH = 3:3:1 elution | 576mg → 758mg | 1710(sh) 1690 1655 | $C_{23}H_{34}N_4O_6$ C 61.88 (61.86) H 7.65 (7.67) N 12.62 (12.55) |
| | B | 121a → 121b R = 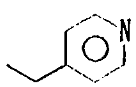 | TFA (3ml) 0°C→r.t. 1hr hereafter same as 97B | 358mg → 322mg | 1665 1650 1630 1600 1540 | $C_{10}H_{20}N_4O_3 \cdot 2HCl \cdot H_2O$ C 49.38 (49.43) H 7.13 (6.91) N 12.65 (12.81) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 122 | A | XXVIII<br>→<br>122a<br>R′ = 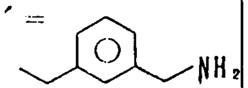 | R′$NH_2$ (2270mg)<br>MeOH (20mℓ)<br>r.t. 18hr<br>conc., $SiO_2$ column chromato<br>EtoAc:MeOH = 5:1 eltn. | 1128mg<br>→<br>700mg | 1710(sh)<br>1695<br>1655<br>1525 | $C_{25}H_{38}N_4O_5$<br>C 63.11 (63.27)<br>H 8.18 ( 8.07)<br>N 11.74 (11.81) |
| | B | 122a<br>→<br>122b<br>R = 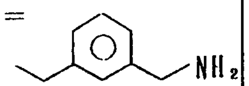 | Conc. HCl (1mℓ)<br>MeOH (4mℓ)<br>0℃ → r.t. 1hr<br>HP-20 column chromato.<br>Eltn. with $H_2O$, lyophilization | 500mg<br>→<br>334mg | 1655<br>1610<br>1540 | $C_{20}H_{30}N_4O_3 \cdot 2HCl \cdot H_2O$<br>C 51.49 (51.61)<br>H 7.51 ( 7.36)<br>N 12.00 (12.04) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 123 | A | XXVIII → 123a<br>R′ = 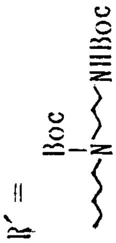 | R′$NH_2$ (1036mg)<br>MeOH (5mℓ)<br>r.t. 3days<br>conc., silica gel column chromato.<br>Eltn. with EtOAc | 677mg<br>→<br>900mg | 1710(sh)<br>1690<br>1655<br>1540 | $C_{34}H_{61}N_5O_9$<br>C 59.67 (59.71)<br>H 8.29 ( 8.99)<br>N 10.09 (10.24) |
| | B | 123a → 123b<br>R = 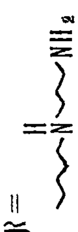 | TFA (4mℓ)<br>0°C → r.t. 1hr<br>(hereafter same as 97B) | 800mg<br>→<br>400mg | 1655<br>1620<br>1530 | $C_{19}H_{37}N_5O_3 \cdot 3HCl \cdot 2H_2O$<br>C 42.96 (43.14)<br>H 8.52 ( 8.38)<br>N 13.08 (13.24) |

Examples 124 - 130 (Table 15)

By the procedure similar to Example 67, Compound 52b was subjected to amidation (Step A) by using various amines (R'$NH_2$) or its derivatives, then to deprotection reaction (Step B) to obtain compounds of

Examples 124 - 130. The reaction conditions, yields and some of the physico-chemical properties of the products are shown in Table 15.

Table 15

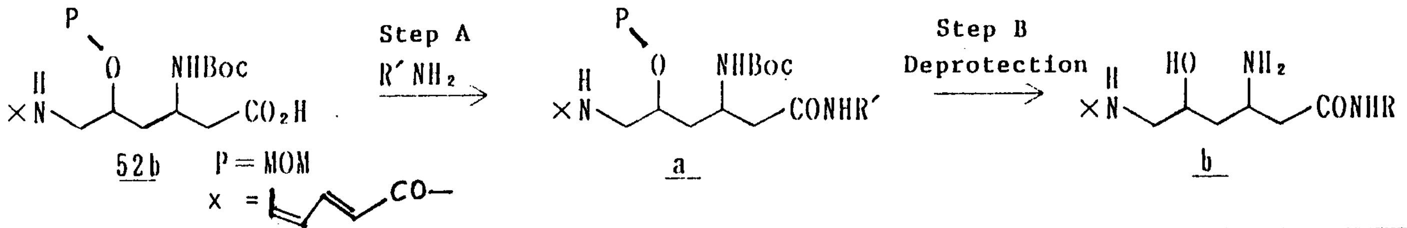


52b P = MOM

x =

a

b

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 124 | A | 52b → 124a<br>R′ = 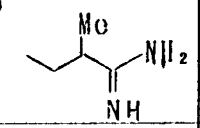 | R′$NH_2$ · 2HCl (130mg)<br>$NaHCO_3$(95mg), $H_2O$(3mℓ)<br>52b, HOBT(75mg), DCC (114mg), THF(2mℓ)<br>0℃→r.t. 15hr stir.<br>pH3.0(1N-HCl), HP-20<br>Lyophilization | 220mg → 253mg | 1710(sh)<br>1690<br>1660<br>1650<br>1540 | $C_{23}H_{41}N_5O_6$ · HCl · 0.5$H_2O$<br>C 52.20 (52.21)<br>H 8.40 ( 8.19)<br>N 13.03 (13.24) |
| | B | 124a → 124b<br>R = 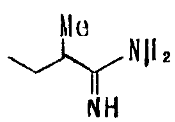 | TFA (3mℓ)<br>0℃→r.t. 1.5hr<br>(hereafter same as 97B) | 252 mg → 135 mg | 1690<br>1660<br>1650<br>1540 | $C_{16}H_{20}N_5O_3$ · 2HCℓ · $H_2O$<br>C 44.45 (44.65)<br>H 7.88 ( 7.73)<br>N 16.09 (16.27) |

| Ex. No. | Step | Starting Compd. → Product Compd. | Reaction Condtions | Yield Material → Product | IR $\nu_{max}^{KBr}$ $cm^{-1}$ | Molecular Formula Analysis (Calcd.) |
|---|---|---|---|---|---|---|
| 125 | A | 52b → 125a R′ = 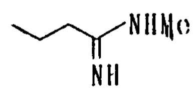 | R′$NH_2$ · 2HCl (130mg) $NaHCO_3$(95mg), $H_2O$ (3mℓ) 52b, HOBT(75mg), DCC (114mg), THF(2mℓ) 0℃→r.t. 20hr pH3.0(1N-HCl), HP-20 Lyophilization | 220mg → 270mg | 1710 1690 1650 1535 | $C_{23}H_{41}N_5O_6$ · HCℓ · $H_2O$ C 51.17 (51.34) H 8.41 ( 8.24) N 12.89 (13.02) |
| | B | 125a → 125b R = 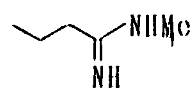 | TFA (3mℓ) 0℃→r.t. 1.5hr (hereafter same as 97b) | 270mg → 139mg | 1680 1650 1540 | $C_{10}H_{20}N_5O_3$ · 2HCℓ · 1.5$H_2O$ C 43.60 (43.74) H 7.91 ( 7.80) N 15.77 (15.94) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 126 | A | 52b → 126a<br>R' = 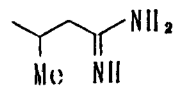 | $R'NH_2 \cdot 2HCl$ (105mg)<br>$NaHCO_3$(101mg), $H_2O$(3mℓ)<br>52b, HOBT(60mg), DCC (91mg), THF(2mℓ)<br>0℃→r.t. 2hr<br>pH3.0(1N-HCl), HP-20<br>Lyophilization | 176mg<br>→<br>184mg | 1710(sh)<br>1690<br>1650<br>1530 | $C_{23}H_{41}N_5O_8 \cdot HCl \cdot H_2O$<br>C 51.09 (51.34)<br>H 8.48 ( 8.24)<br>N 12.88 (13.02) |
| | B | 126a → 126b<br>R = 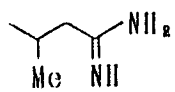 | TFA (2mℓ)<br>0℃→r.t. 1hr<br>(hereafter same as 97B) | 183mg<br>→<br>96mg | 1685<br>1650<br>1540 | $C_{18}H_{20}N_5O_3 \cdot 2HCl \cdot H_2O$<br>C 44.39 (44.65)<br>H 7.91 ( 7.73)<br>N 16.07 (16.27) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 127 | A | 52b → 127b<br>R′ = 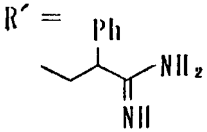 | $R'NH_2 \cdot 2HCl$ (142mg)<br>$NaHCO_3$(76mg), $H_2O$(2mℓ)<br>52b, HOBT(60mg), DCC (91mg), THF(2mℓ)<br>0°C → r.t. 2hr<br>EtoAc:THF(1:1) extn.<br>Concn.<br>TFA (2mℓ)<br>0°C → r.t. 1.5hr<br>(hereafter same as 97B) | 176mg<br>→<br>138mg | 1690<br>1650<br>1540 | $C_{21}H_{31}N_6O_3 \cdot 2HCl \cdot 1.5H_2O$<br>C 50.12 (50.30)<br>H 7.38 (7.24)<br>N 13.79 (13.97) |

| Ex. No. | Step | Starting Compd. → Product Compd. | Reaction Conditons | Yield Material → Product | IR $\nu_{max}^{KBr}$ $cm^{-1}$ | Molecular Formula Analysis (Calcd.) |
|---|---|---|---|---|---|---|
| 128 | A | 52b → 128a R′ = 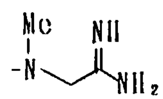 | R′ $NH_2$ · 2HCl (105mg) $NaHCO_3$(101mg), $H_2O$(2mℓ) 52b, HOBT(60mg), DCC (91mg), THF(2mℓ) 0℃→r.t. 20hr pH3.5(1N-HCl), HP-20 Lyophilization | 176mg → 91mg | 1710 1690 1530 | $C_{22}H_{40}N_8O_8$ · HCℓ · 0.5$H_2O$ C 49.70 (49.85) H 8.17 (7.99) N 15.66 (15.85) |
| | B | 128a → 128b R = 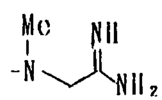 | TFA (2mℓ) 0℃→r.t. 1hr concn., HP-20 Lyophilization | 91mg → 51mg | 1685 1200 1130 | $C_{15}H_{28}N_8O_3$ · 2$CF_3CO_2H$ · $H_2O$ C 38.70 (38.91) H 5.69 (5.50) N 14.13 (14.33) |

| Ex. No. | Step | Starting Compd. → Product Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 129 | A | 52b → 129a<br>R′ = 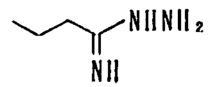 | $R'NH_2 \cdot 2HCl$ (176mg)<br>$NaHCO_3$(84mg), $H_2O$(3mℓ)<br>52b, HOBT(81mg), DCC (124mg), THF(3mℓ)<br>0℃→r.t. 2hr<br>pH3.5(1N-HCl), HP-20<br>Lyophilization | 220mg<br>→<br>254mg | 1710<br>1690<br>1650<br>1530 | $C_{22}H_{40}N_6O_6 \cdot HCl \cdot H_2O$<br>C 48.89 (49.02)<br>H 8.19 ( 8.04)<br>N 15.44 (15.59) |
| | B | 129a → 129b<br>R = 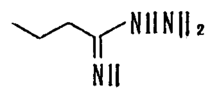 | TFA (3mℓ)<br>0℃→r.t. 75min<br>(hereafter same as 97B) | 253mg<br>→<br>108mg | 1690(sh)<br>1675<br>1650<br>1610<br>1540 | $C_{15}H_{28}N_6O_3 \cdot 2HCl \cdot H_2O$<br>C 41.59 (41.77)<br>H 7.69 ( 7.48)<br>N 19.32 (19.48) |

| Ex. No. | Step | Starting Compd. → Prodcut Compd. | Reaction Conditions | Yield Material → Product | IR $\nu_{max}^{KBr}$ $cm^{-1}$ | Molecular Formula Analysis (Calcd.) |
|---|---|---|---|---|---|---|
| 130 | A | 52b → 130a<br>R′ = 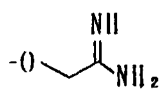 | $R'NH_2 \cdot 2HBr$ (151mg)<br>$NaHCO_3$(84mg), $H_2O$(3mℓ)<br>52b, HOBT(75mg), DCC (114mg), THF(3mℓ)<br>r.t. 20hr<br>pH3.0(1N-HCl), HP-20<br>Lyophilization | 220mg → 91.5mg | 1710(sh)<br>1690<br>1520<br>1360 | $C_{21}H_{37}N_5O_7 \cdot HCl \cdot 0.5H_2O$<br>C 48.57 (48.79)<br>H 7.82 ( 7.60)<br>N 13.39 (13.55) |
| | B | 130a → 130b<br>R = 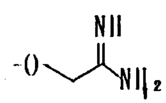 | TFA (2mℓ)<br>0℃ → r.t. 1hr<br>(hereafter same as 97B) | 91mg → 17mg | 1690<br>1650<br>1610 | $C_{14}H_{26}N_6O_4 \cdot 2HCl \cdot H_2O$<br>C 40.01 (40.20)<br>H 7.12 ( 6.99)<br>N 16.58 (16.74) |

Example 131

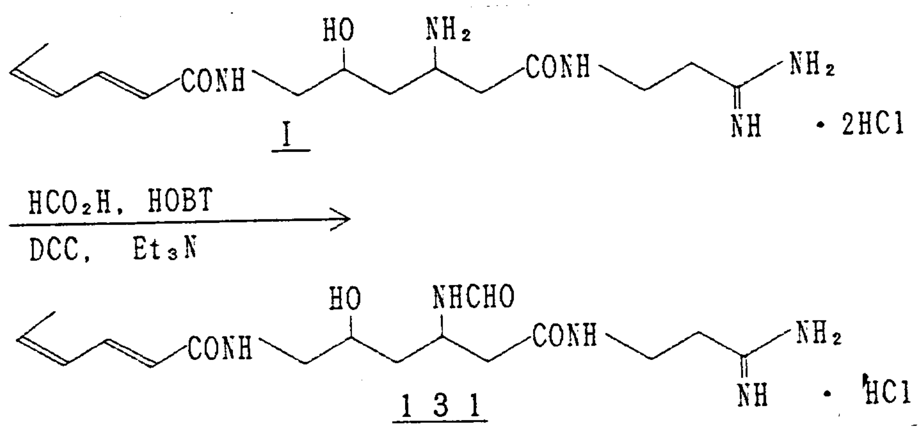


I

131

To a solution of Compound I (222 mg) in DMF (2 mℓ) was added $Et_3N$ (105 μℓ). To the mixture were added, 5 minutes later, $HCO_2H$ (35 mg), HOBT (102 mg) and DCC (155 mg), followed by allowing the reaction to proceed at room temperature for 4 days. The reaction mixture was concentrated under reduced pressure. The concentrate was passed through an IRA-401 ($Cℓ^-$) resin column (20 mℓ), followed by elution with water (100 mℓ). The eluate was concentrated and purified by means of an HP-20 column and eluted with 5% EtOH-$H_2O$. The eluate was lyophilized to afford Compound 131 (38 mg) as a pale yellow powdery product.

$$IR\nu_{max}^{KBr}\ cm^{-1}:\ 1690,\ 1650,\ 1540$$

| Elemental analysis for: $C_{16}H_{27}N_5O_4$ • HCℓ • 1.5 $H_2O$ | | | |
|---|---|---|---|
| Calcd. | C, 46.10; | H, 7.49; | N, 16.80 |
| Found | C, 45.88; | H, 7.62; | N, 16.63 |

Example 132

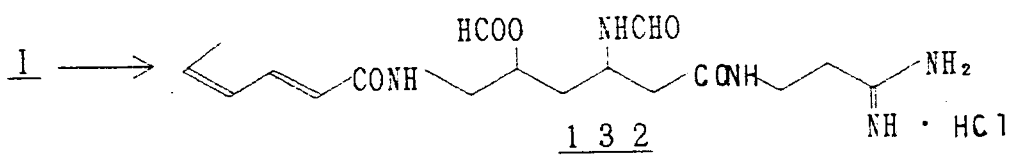

I

132

To a solution of Compound I (222 mg) in $HCO_2H$ (4 mℓ) was added $AC_2O$ (2 mℓ) under ice-cooling, and the mixture was stirred at room temperature for 2.5 hours, followed by concentration under reduced pressure. The concentrate was dissolved in water, which was passed through an IRA-401 ($Cℓ^-$) column (20 mℓ), then eluted with water (150 mℓ). The eluate was concentrated, followed by purification by means of an HP-20(70 mℓ) column chromatography, eluting with 5% EtOH-$H_2O$. The eluate was lyophilized to afford Compound 132 as colorless powder.

$$IR\nu_{max}^{KBr}\ cm^{-1}:\ 1720,\ 1690,\ 1660,\ 1540$$

| Elemental analysis for: $C_{17}H_{27}N_5O_5 \cdot HC\ell \cdot H_2O$ | | | |
|---|---|---|---|
| Calcd. | C, 46.84; | H, 6.94; | N, 16.07 |
| Found | C, 46.66; | H, 7.19; | N, 15.89 |

Example 133

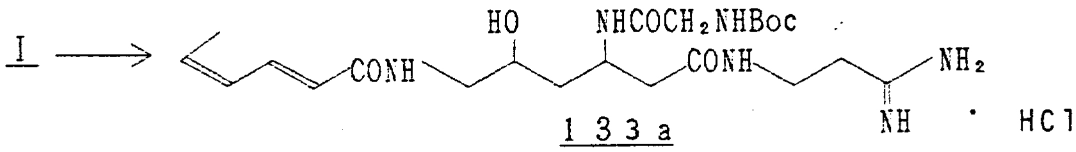

133a

To a suspension of Compound I (177 mg) in DMF (2 mℓ) was added $Et_3N$ (84 μℓ), and the mixture was stirred for 5 minutes. To the reaction mixture were added N-Boc•glycine (84 mg), HOBT (27 mg) and DCC (100 mg), and the reaction was allowed to proceed at room temperature for 3 days. DMF was distilled off under reduced pressure, then the residue was dissolved in a small volume of water, to which was added 1N-HCℓ to adjust the pH at 3, followed by purification by means of an HP-20 (70 mℓ) column chromatography, eluting with 30% EtOH-$H_2O$. The eluate was lyophilized to afford Compound 133a (194 mg) as colorless powder.

$$IR\nu_{max}^{KBr}\ cm^{-1}:\ 1710\ (sh),\ 1690,\ 1660,\ 1550$$

| Elemental analysis for: $C_{22}H_{38}N_6O_6HC\ell \cdot 0.5H_2O$ | | | |
|---|---|---|---|
| Calcd. | C, 50.04; | H, 7.64; | N, 15.92 |
| Found | C, 49.88; | H, 7.77; | N, 15.81 |

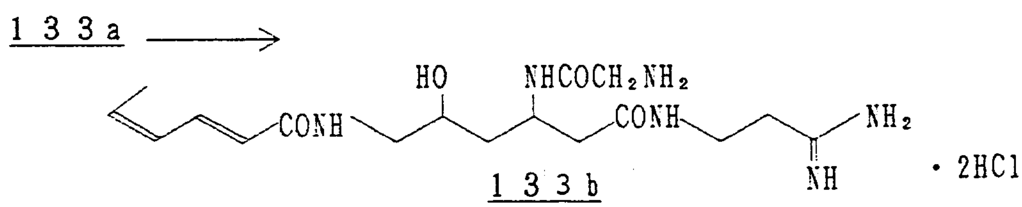

133b

To Compound 133a (193 mg) was added, under ice-cooling, TFA (2 mℓ), and the mixture was stirred at room temperature for one hour, followed by concentration under reduced pressure. The residue was dissolved in a small volume of water, which was purified by means of an HP-20 (70 mℓ) column, eluting with 5% EtOE-$H_2O$. Eluate fractions containing the desired compound were combined and concentrated. The concentrate was passed through an IRA-401 ($C\ell^-$) (20 mℓ) column, followed by elution with water (60 mℓ). The eluate was lyophilized to afford Compound 133b (129 mg) as pale yellow powder.

$$IR\nu_{max}^{KBr}\ cm^{-1}:\ 1690,\ 1655,\ 1545$$

| Elemental analysis for: $C_{17}H_{30}N_6O_4 \cdot 2HC\ell \cdot H_2O$ | | | |
|---|---|---|---|
| Calcd. | C, 43.13; | H, 7.24; | N, 17.75 |
| Found | C, 42.96; | H, 7.48; | N, 17.52 |

Example 134

I

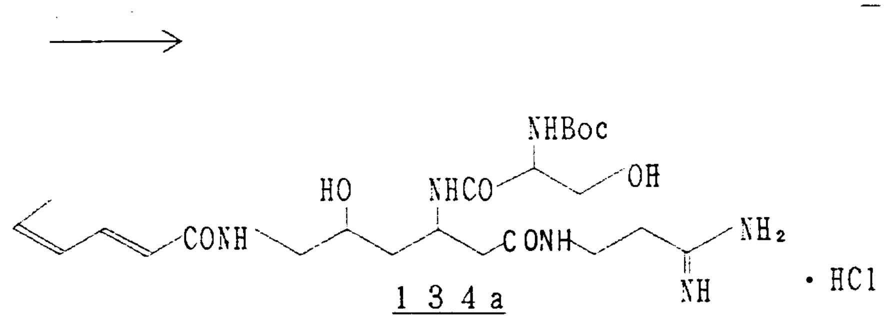

134a

To a solution of Compound I (177 mg) and DL-N-Boc•serine (99 mg) in DMF (2 mℓ) were added $Et_3N$-(84 μℓ), HOBT (27 mg) and/DCC (100 mg), followed by allowing the reaction to proceed for 20 hours. DMF was distilled off under reduced pressure, and the residue was purified by means of an HP-20 (70 mℓ) column, eluting with 30% EtOH-$H_2O$. The eluate was lyophilized to afford Compound 134a (240 mg) as colorless powder.

$$IR\nu_{max}^{KBr}\ cm^{-1}: 1710\ (sh), 1690, 1660, 1650, 1540$$

| Elemental analysis for: $C_{23}H_{40}N_6O_7 \cdot HC\ell \cdot H_2O$ | | | |
|---|---|---|---|
| Calcd. | C, 48.71; | H, 7.64; | N, 14.82 |
| Found | C, 48.50; | H, 7.78; | N, 14.73 |

134a

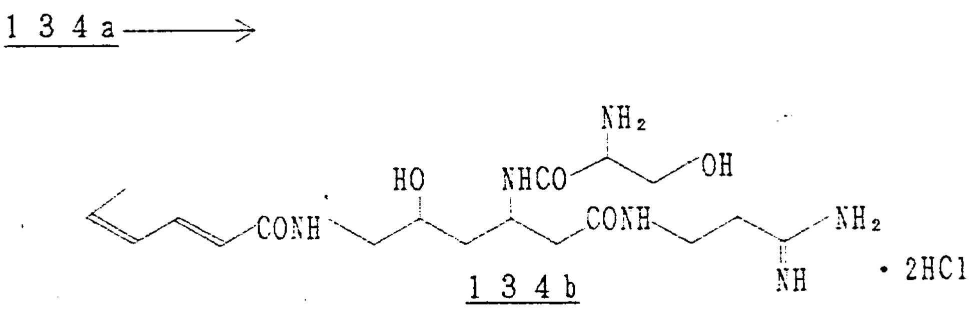

134b

To Compound 134a (240 mg) was added, under ice-cooling, TFA (2 mℓ), and the mixture was stirred at room temperature for one hour. The mixture was concentrated under reduced pressure, then the concentrate was subjected to an HP-20 (70 mℓ) column chromatography, eluting with 5% EtOE-$H_2O$. The eluate was concentrated, which was passed through an IRA-401 ($C\ell^-$) (20 mℓ) column, followed by elution with water (60 mℓ). The eluate was lyophilized to afford Compound 134b (123 mg) as colorless powder.

$$IR\nu_{max}^{KBr}\ cm^{-1}:\ 1690,\ 1650,\ 1545$$

| Elemental analysis for: $C_{18}H_{32}N_6O_5 \cdot 2HC\ell \cdot 1.5H_2O$ | | | |
|---|---|---|---|
| Calcd. | C, 42.19; | H, 7.28; | N, 16.40 |
| Found | C, 42.00; | H, 7.43; | N, 16.21 |

Example 135

1

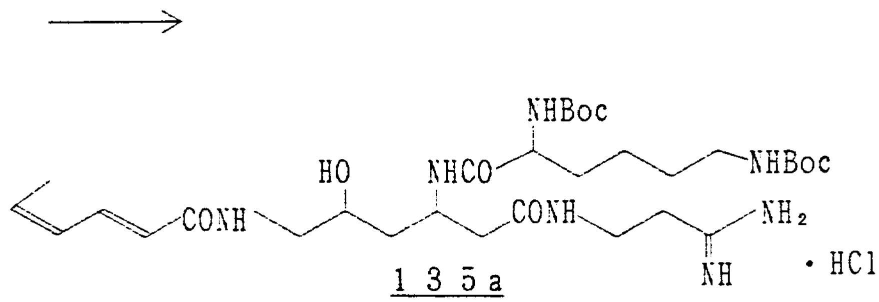

135a

To a solution of DL-$N^2$, $N^6$-diBoc-lysine (167 mg) in THF (2 m$\ell$) were added HOBT (65 mg) and DCC (99 mg), and the mixture was stirred at room temperature for 1.5 hours. On the other hand, Compound I - (177 mg) was dissolved in $H_2O$ (3 m$\ell$), to which was added sodium hydrogencarbonate (51 mg). To the mixture was added, while stirring under ice-cooling, the above-mentioned THF solution of active ester, followed by allowing the reaction to proceed at room temperature for 3 hours. To the reaction mixture was added under ice-cooling 1N-HC$\ell$ to adjust the pH at 3.0, followed by distilling off THF under reduced pressure. The residue was subjected to an HP-20 (60 m$\ell$) column chromatography, eluting with 40% EtOH-$H_2O$. The eluate was lyophilized to afford Compound 135a (245 mg) as colorless powder.

$$IR\nu_{max}^{KBr}\ cm^{-1}:\ 1710\ (sh),\ 1690,\ 1660,\ 1525$$

| Elemental analysis for: $C_{31}H_{55}N_7O_8 \cdot HC\ell \cdot 0.5H_2O$ | | | |
|---|---|---|---|
| Calcd. | C, 53.25; | H, 8.22; | N, 14.02 |
| Found | C, 53.19; | H, 8.29; | N, 13.82 |

135a

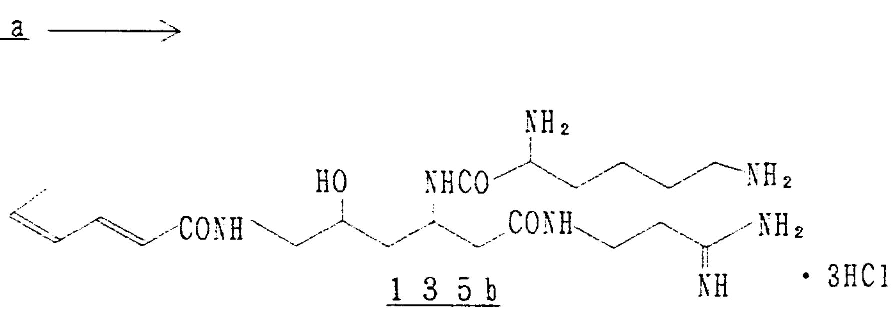

135b

To Compound 135a (244 mg) was added, under ice-cooling, TFA (3 mℓ), and the mixture was stirred for 1.5 hours, followed by concentration under reduced pressure. The concentrate was subjected to an HP-20 (60 mℓ) column chromatography, eluting with 5% EtOH-$H_2O$. The eluate was concentrated and passed through an IRA-401 ($Cℓ^-$) (20 mℓ) column, followed by elution with 60 mℓ of water. The eluate was lyophilized to afford Compound 135b (144 mg) as pale yellow powder.

$$IR\nu_{max}^{KBr}\ cm^{-1}:\ 1685,\ 1650,\ 1540$$

| Elemental analysis for: $C_{21}H_{39}N_7O_4 \cdot 3HCℓ \cdot 1.5H_2O$ | | | |
|---|---|---|---|
| Calcd. | C, 42.75; | H, 7.69; | N, 16.62 |
| Found | C, 42.64; | H, 7.81; | N, 16.49 |

Example 136

I

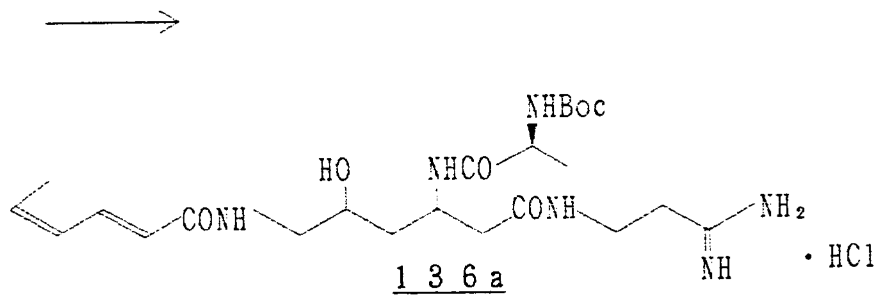

136a

To a solution of L-Boc-Ala (114 mg) in THF (2 mℓ) were added HOBT (81 mg) and DCC (124 mg), and the mixture was stirred at room temperature for one hour. On the other hand, Compound I (221 mg) was dissolved in $H_2O$ (2 mℓ). To the solution was added the THF solution of active ester as prepared above, followed by allowing the reaction to proceed at room temperature far 1.5 hours. The reaction mixture was adjusted to pH 3.0 with 1N-HCℓ, from which was distilled off THF under reduced pressure, followed by filtering off insolubles. The filtrate was subjected to an HP-20 (60 mℓ) column chroamtography. Fractions eluted with 30% EtOH-$H_2O$ were combined and lyophilized to afford Compound 136a (246 mg) as colorless powder.

$$IR\nu_{max}^{KBr}\ cm^{-1}:\ 1710\ (sh),\ 1690,\ 1655,\ 1540$$

| Elemental analysis for: $C_{23}H_{40}N_6O_6 \cdot HCl \cdot H_2O$ | | | |
|---|---|---|---|
| Calcd. | C, 50.13; | H, 7.86; | N, 15.25 |
| Found | C, 49.85; | H, 7.97; | N, 15.04 |

136a

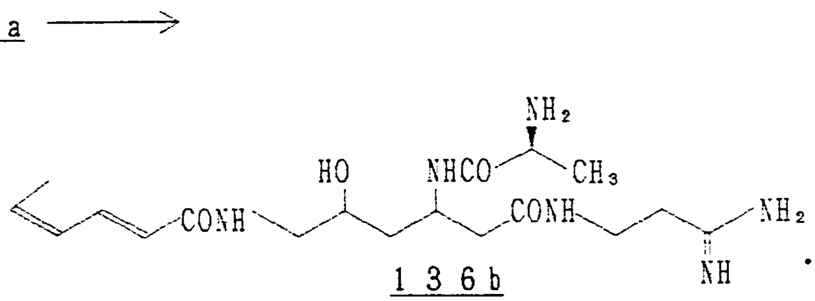

136b · 2HCl

To Compound 136a (201 mg) was added, under ice-cooling, TFA (2 mℓ), and the mixture was stirred at room temperature for one hour. The reaction mixture was concentrated under reduced pressure. The concentrate was subjected to an HP-20 (60 mℓ) column chromatography, eluting with 5% EtOH-$H_2O$. The eluate was concentrated and passed through an IRA-401 ($Cl^-$ column, eluting with water (60 mℓ), followed by lyophilization to afford Compound 136b (145 mg) as pale yellow powder.

$$IR\nu_{max}^{KBr} cm^{-1} : 1690, 1655, 1550$$

| Elemental analysis for: $C_{18}H_{32}N_6O_4 \cdot 2HCl \cdot H_2O$ | | | |
|---|---|---|---|
| Calcd. | C, 44.36; | H, 7.44; | N, 17.24 |
| Found | C, 44.29; | H, 7.61; | N, 17.11 |

Example 137

I

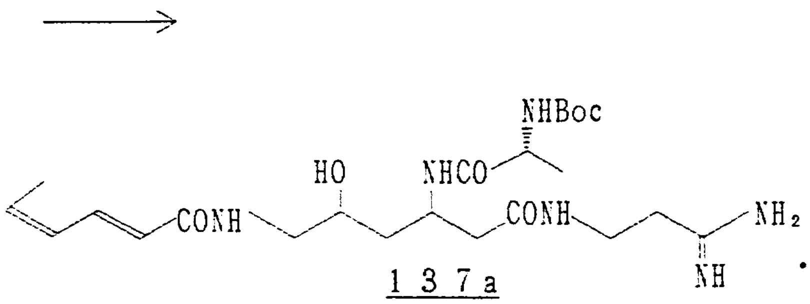

137a · HCl

To the solution of D-Boc-Ala(114 mg) in THF (2 mℓ) were added HOBT (81 mg) and DCC (124 mg), and the reaction was allowed to proceed at room temperature for one hour. On the other hand, Compound I (221 mg) was dissolved in water (2 mℓ), to which was added sodium hydrogencarbonate (84 mg). To the mixture was added, under ice-cooling, the THF solution of active ester prepared as above, followed by stirring at room temperature for 1.5 hours. The reaction mixture was adjusted to pH 3.0 with 1N-HCℓ under ice-cooling, followed by distilling off THF under reduced pressure. Insolubles were then filtered off. The filtrate was subjected to an HP-20 (60 mℓ) column chromatograpy. Fractions eluted with 30% EtOH-$H_2O$

were combined and lyophilized to afford Compound 137a (235 mg) as colorless powder.

$$IR\nu_{max}^{KBr}\ cm^{-1}:\ 1710\ (sh),\ 1690,\ 1665,\ 1650,\ 1530$$

| Elemental analysis for: $C_{23}H_{40}N_6O_6 \cdot HC\ell \cdot 0.5H_2O$ | | | |
|---|---|---|---|
| Calcd. | C, 50.96 | H, 7.81; | N, 15.50 |
| Found | C, 50.71 | H, 7.98; | N, 15.35 |

137a 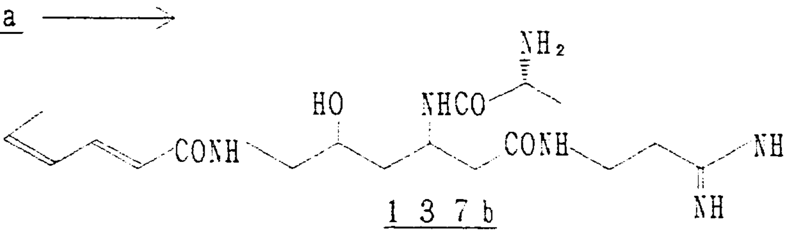

137b • 2HCl

Compound 137a (230 mg) was dissolved in THF (2 mℓ) under ice-cooling, and the reaction was allowed to proceed at room temperature for one hour, followed by concentration under reduced pressure. The concentrate was subjected to an HP-20 (60 mℓ) column chromatography, eluting with 5% EtOH-$H_2O$. The eluate was concentrated, passed through an IRA-40 ($C\ell^-$) (20 mℓ) column, eluted with water (60 mℓ) and lyophilized to afford Compound 137b (143 mg) as colorless powder.

$$IR\nu_{max}^{KBr}\ cm^{-1}:\ 1690-1640,\ 1545$$

| Elemental analysis for: $C_{18}H_{32}N_6O_4 \cdot 2HC\ell \cdot H_2O$ | | | |
|---|---|---|---|
| Calcd. | C, 44.36 | H, 7.44; | N, 17.24 |
| Found | C, 44.18; | H, 7.58; | N, 17.13 |

Example 138

I 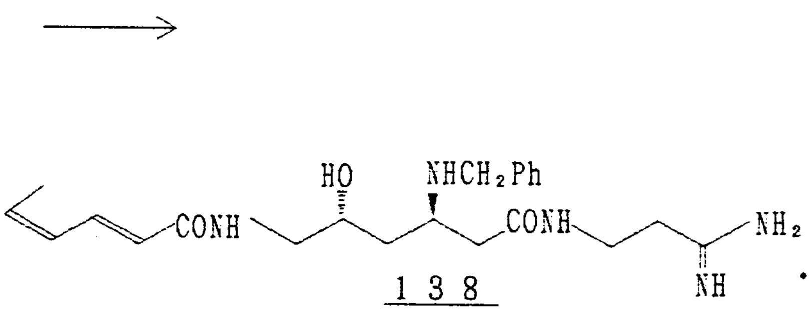

138 • 2HCl

To a solution of Compound I (844 mg) in MeOH (18 mℓ) was added benzaldehyde (0.485 mℓ), and the mixture was stirred at room temperature for 30 minutes. To the reaction mixture were added $NaBH_3CN$ (283

mg) and acetic acid (0.7 mℓ), and the mixture was stirred at room temperature for 3 hours, followed by further addition of benzaldehyde (0.254 mℓ) and $NaBH_3CN$ (157 mg). The whole mixture was stirred for further one hour, which was concentrated under reduced pressure. The concentrate was dissolved in water, which was adjusted to pH 3 with 1n HCℓ. The mixture was subjected to an HP-20 (350 mℓ) column chromatography, eluting with 20% $EtOH\text{-}H_2O$. The eluate was lyophilized to afford Compound 138 (667 mg) as colorless powder.

$$IR\nu_{max}^{KBr}\ cm^{-1}:\ 1680,\ 1640,\ 1530$$

| Elemental analysis for: $C_{22}H_{33}N_5O_3 \cdot 2HC\ell \cdot H_2O$ | | | |
|---|---|---|---|
| Calcd. | C, 52.17; | H, 7.36; | N, 13.83 |
| Found | C, 52.02; | H, 7.49; | N, 13.69 |

Example 139

I

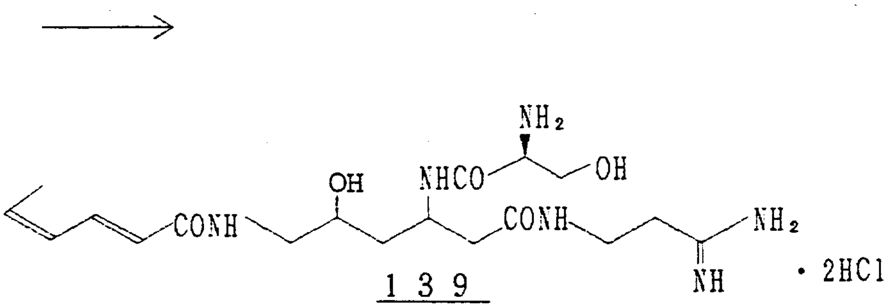

139

To a solution of L-Boc-Ser (154 mg) in THF (3 mℓ) were added DCC (155 mg) and HOBT (102 mg), and the mixture was stirred at room temperature for one hour. On the other hand, Compound I (234 mg) was dissolved in water (3 mℓ), to which was added sodium hydrogencarbonate (42 mg). To the solution was added the above-mentioned THF solution of active ester under ice-cooling, followed by stirring at room temperature for 2.5 hours. Insolubles were filtered off, and the filtrate was adjusted to pH 3.0, which was then purified by means of an HP-20 (60 mℓ) column, eluting with 5% $EtOH\text{-}H_2O$. Eluate fractions were combined and lyophilized to afford a BOC derivative (261 mg) as colorless powder. To the Boc derivative was added under ice-cooling, TFA (2 mℓ), and the mixture was stirred at room temperature for one hour, followed by concentration under reduced pressure. The concentrate was subjected to an HP-20 (60 mℓ) column chromatography, eluting with 5% $EtOH \cdot H_2O$. The eluate was concentrated and passed through an IRA-40 ($C\ell^-$) (20 mℓ) column, eluting with $H_2O$ (60 mℓ), followed by lyophilization to afford Compound 139 (200 mg) as pale yellow powder.

$$IR\nu_{max}^{KBr}\ cm^{-1}:\ 1690,\ 1650,\ 1550$$

| Elemental analysis for: $C_{18}H_{32}N_6O_5 \cdot 2HC\ell \cdot 1.5H_2O$ | | | |
|---|---|---|---|
| Calcd. | C, 42.19; | H, 7.28; | N, 16.40 |
| Found | C, 42.04; | H, 7.44; | N, 16.27 |

Example 140

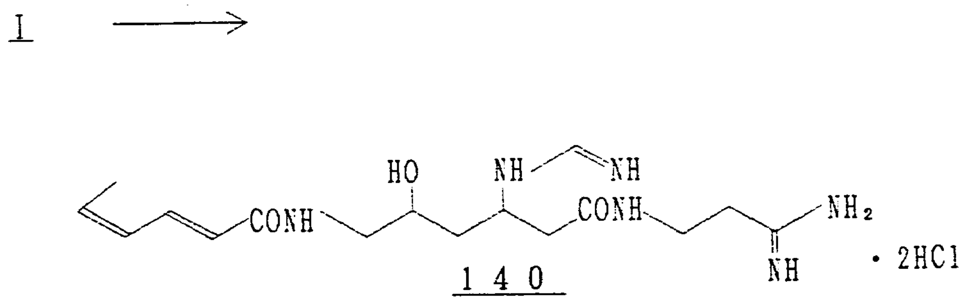

140

To a solution of Compound I (188 mg) in $H_2O$ (3 mℓ) were added, under stirring at room temperature, benzylformimidate•hydrochloride (103 mg) and sodium hydrogencarbonate (101 mg), then the reaction was allowed to proceed at room temperature for 3 hours. To the reaction mixture were further added benzylformimidate hydrochloride (138 mg) and $NaHCO_3$ (101 mg). Benzylformimidate hydrochloride (each 206 mg) and $NaHCO_3$ (each 101 mg) were further added to the mixture after 18 hours and 26 hours thereafter. Then, the reaction was further allowed to proceed for 15 hours. The reaction mixture was adjusted to pH 3 by the addition of 1N HCℓ under ice-cooling, followed by subjecting to an HP-20 (120 mℓ) column chromatography, eluting with 5% EtOH-$H_2O$. The eluate was lyophilized to afford Compound 140 - (77 mg) as colorless powder.

$$IR\nu_{max}^{KBr}\ cm^{-1}:\ 1690,\ 1650,\ 1540$$

| Elemental analysis for: $C_{16}H_{28}N_6O_3$•2HCℓ•$H_2O$ | | | |
|---|---|---|---|
| Calcd. | C, 43.34; | H, 7.27; | N, 18.95 |
| Found | C, 43.15; | H, 7.48; | N, 18.69 |

Example 141

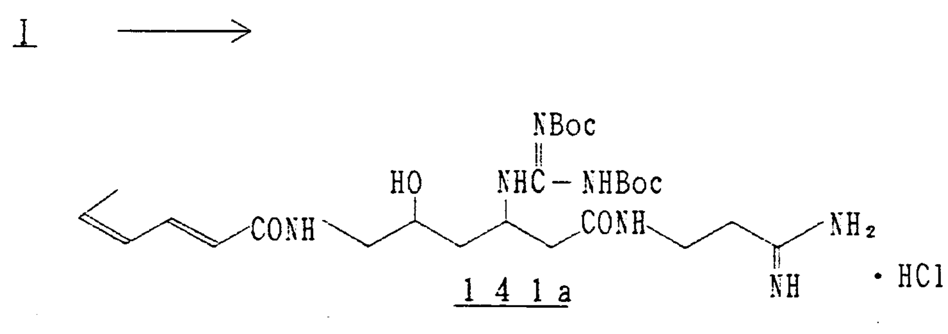

141a

To a solution of Compound I (600 mg) in DMF (5 mℓ) and water (1 mℓ) were added $Et_3N$ (300 mg) and N,N'-bis(tert-butoxycarbonyl)-S'-methylisothiourea (450 mg), and the mixture was stirred at 50°C for 8 hours. DMF was distilled off under reduced pressure, and the residue was adjusted to pH 3 with 1N HCℓ, which was subjected to an HP-20 (350 mℓ) column chromatography. Fractions eluted with 20% MeOH-$H_2O$ were combined and lyophilized to afford Compound 141a (400 mg) as colorless powder.

$IR\nu_{max}^{KBr}$ $cm^{-1}$ : 1710 (sh), 1690, 1655, 1540

| Elemental analysis for: $C_{26}H_{45}N_7O_5 \cdot HCl \cdot 0.5H_2O$ | | | |
|---|---|---|---|
| Calcd. | C, 53.74; | H, 8.15; | N, 16.87 |
| Found | C, 53.69; | H, 8.09; | N, 16.93 |

141a

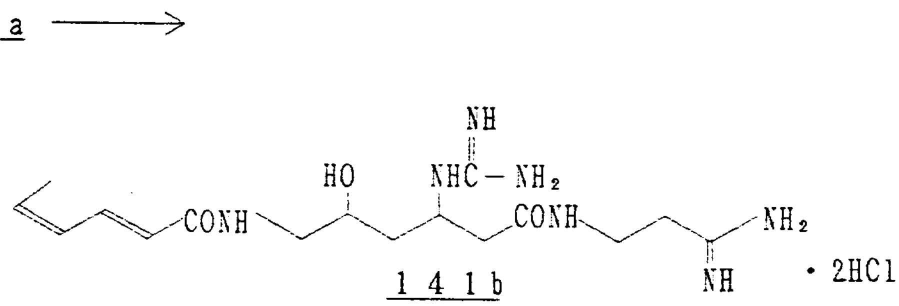

141b

Compound 141a (400 mg) was dissolved in 2N HCl (14 ml), and the reaction was allowed to proceed at room temperature for 3 hours. The reaction mixture was purified by means of an HP-20 (350 ml) column chromatography. Fractions eluted with 10% MeOH-$H_2O$ were combined and lyophilized to afford Compound 141b (250 mg) as colorless powder.

$IR\nu_{max}^{KBr}$ $cm^{-1}$ : 1690, 1650, 1530

| Elemental analysis for: $C_{16}H_{29}N_7O_3 \cdot 2HCl \cdot H_2O$ | | | |
|---|---|---|---|
| Calcd. | C, 41.73; | H, 7.26; | N, 21.39 |
| Found | C, 41.73; | H, 7.49; | N, 21.13 |

Example 142

I

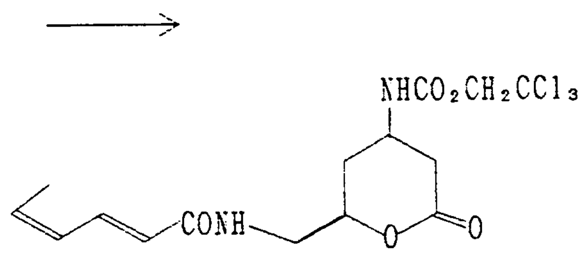

142

A soultion of Compound I (2.35 g) in 2N-HCl (200 ml) was heated under reflux for 40 minutes, followed by concentration to dryness under reduced pressure. The concentrate was dissolved in $H_2O$:THF (1:1) (100 ml), whose pH was adjusted to 9.0 with 2N-NaOH under ice-cooling, to which was added 2,2,2-trichloroethoxycarbonyl chloride (2.07 ml). The mixture was stirred at room temperature for one hour while

keeping the pH of the reaction mixture within the range of from 8.0 to 9.0. The reaction mixture was extracted with ethyl acetate, washed with saturated brine, followed by drying over anhydrous magnesium sulfate. The mixture was concentrated under reduced pressure, to which was added ether. Then precipitating crystals were collected by filtration to afford Compound 142 (1.31 g).

$$IR\nu_{max}^{KBr}\ cm^{-1}\ :\ 1745,\ 1725,\ 1665,\ 1540$$

| Elemental analysis for: $C_{15}H_{19}C\ell_3N_2O_5$ | | | |
|---|---|---|---|
| Calcd.<br>Found | C, 47.63;<br>C, 47.58; | H, 5.06;<br>H, 5.11; | N, 7.41<br>N, 7.38 |

Example 143

142

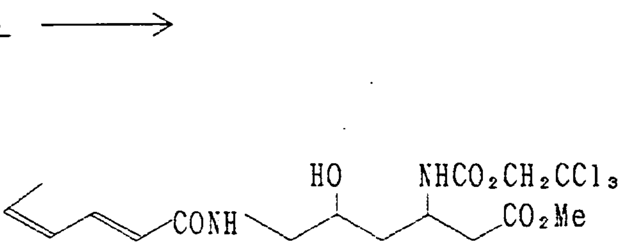

143

To a suspension of Compound 142 (743 mg) in MeOH (20 mℓ) was added a 28% MeONa/MeOH solution (0.3 mℓ), and the mixture was stirred at room temperature for 2 hours, to which was added acetic acid (0.3 mℓ), followed by concentration under reduced pressure. To the concentrate was added saturated brine, and the mixture was subjected to extraction with AcOEt-THF (2:1), followed by drying over magnesium sulfate. The mixture was concentrated under reduced pressure, and the concentrate was subjected to a silica gel (40 g) column chloromatography, eluting with EtoAc:CHCℓ₃ = 3:1, to afford Compound 143 (462 mg) as a colorless foamy product.

$$IR\nu_{max}^{KBr}\ cm^{-1}\ :\ 1740,\ 1720\ (sh),\ 1640,\ 1540$$

| Elemental analysis for: $C_{16}H_{23}C\ell_3N_2O_6$ | | | |
|---|---|---|---|
| Calcd.<br>Found | C, 43.12;<br>C, 43.30; | H, 5.20;<br>H, 5.14; | N, 6.28<br>N, 6.37 |

Example 144

142

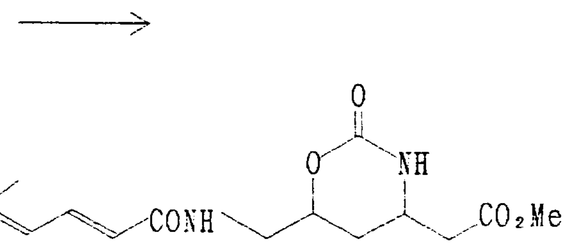

144

To a suspension of Compound 142 (414 mg) in MeOH (10 mℓ) was added a 28% NaOME/MeOH solution (0.3 mℓ), and the reaction was allowed to proceed at room temperature for 18 hours. To the mixture was added acetic acid (0.3 mℓ), which was concentrated under reduced pressure. The concentrate was subjected to a silica gel (35 g) column chromatography, eluting with EtoAc:$CHC\ell_3$:MeOH = 10:10:1, to afford Compound 144 (220 mg) as colorless cyrstals.

$$IR\nu_{max}^{KBr}\ cm^{-1}: 1730, 1710, 1675, 1550$$

m.p.: 141 - 143 °C

| Elemental analysis for: $C_{14}H_{20}N_2O_5$ | | | |
|---|---|---|---|
| Calcd.<br>Found | C, 56.75;<br>C, 56.62; | H, 6.80;<br>H, 6.93; | N, 9.45<br>N, 9.32 |

Example 145

143

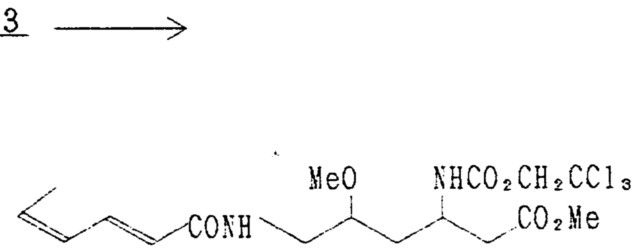

145

To a solution of Compound 143 (360 mg) in $CH_2C\ell_2$ (10 mℓ) was added $BF_3Et_2O/C\ell CH_2CH_2C\ell$ (0.5 mmol/mℓ) solution (0.34 mℓ). To the mixture was then added an excess of $CH_2N_2$/ether solution, followed by stirring for 15 minutes. The mixture was concentrated under reduced pressure, and the concentrate was dissolved in ethyl acetate. The solution was washed with an aqueous solution of sodium hydrogencarbonate and saturated brine in that order, followed by drying over $MgSO_4$. The mixture was concentrated under reduced pressure, which was then subjected to a silica gel (30 g) column chromatography, eluting with Hexane:EtOAc = 1:3, to afford Compound 145 as a colorless oily product.

$$IR\nu_{max}^{KBr}\ cm^{-1}\ :\ 1740,\ 1720,\ 1650,\ 1550$$

| Elemental analysis for: $C_{17}H_{25}C\ell_3N_2O_6$ | | | |
|---|---|---|---|
| Calcd.<br>Found | C, 44.41;<br>C, 44.32; | H, 5.48;<br>H, 5.37; | N, 6.09<br>N, 6.17 |

Example 146

145

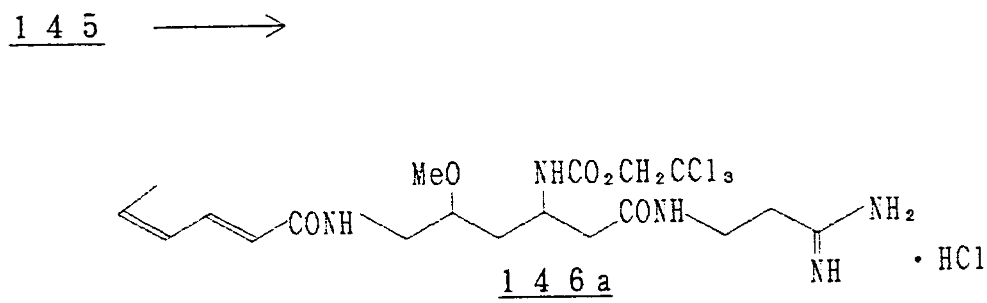

146a

To a solution of Compound 145 (175 mg) in EtOH (2 mℓ) was added 1N-KOH (0.76 mℓ), and the reaction was allowed to proceed at room temperature for 2.5 hours. The reaction mixture was concentrated under reduced pressure, whose pH was adjusted to 2.0 with 1N-HCℓ, followed by extraction with ethyl acetate. The ethyl acetate layer was washed with saturated brine, which was dried over $MgSO_4$, followed by concentration under reduced pressure. The concentrate was dissolved in THF (2 mℓ), to which were added HOBT (61 mg) and DCC (93 mg), and the mixture was stirred at room temperature for one hour.

On the other hand, 3-aminopropioamidine•2HCℓ(92 mg) was dissolved in water (2 m). To the solutiion was added $NaHCO_3$ (73 mg). To the mixture was added, while stirring under ice-cooling, the active ester solution prepared as above. The mixture was stirred at room temperature for 16 hours, which was concentrated under reduced pressure. To the concentrate was added 1N-HCℓ to adjsut the pH to 2.0. The mixture was subjected to an HP-20 (70 mℓ) column chromatography. The fractions eluted with 30% EtOH-$H_2O$ were combined and lyophilized to afford Compound 146a (79 mg) as colorless powder.

$$IR\nu_{max}^{KBr}\ cm^{-1}\ :\ 1720,\ 1695,\ 1550,\ 1390$$

| Elemental analysis for: $C_{19}H_{30}Cl_3N_5O_5HCl \cdot 0.5H_2O$ | | | |
|---|---|---|---|
| Calcd.<br>Found | C, 40.73;<br>C, 40.58; | H, 5.76;<br>H, 5.99; | N, 12.50<br>N, 12.38 |

146a

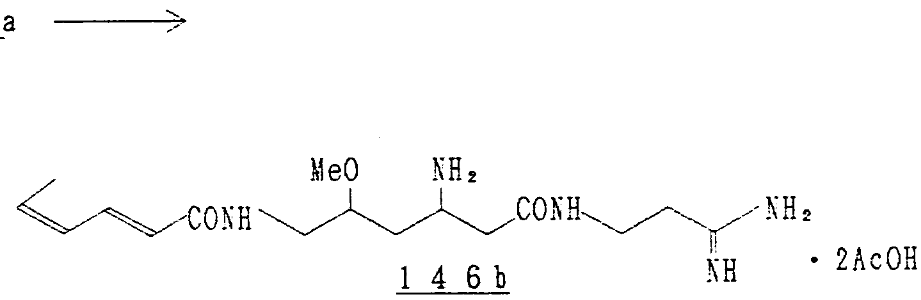

146b

To a solution of Compound 146a (78 mg) in AcOH (2 mℓ) was added zinc powder (100 mg), and the mixture was stirred at room temperature. In every 30 minutes, 100 mg each portion of zinc powder was added three times, followed by stirring for one hour. The reaction mixture was subjected to filtration and washed with water. The filtrate was concentrated under reduced pressure, followed by subjecting to an HP-20 (70 mℓ) column chromatography. The fractions eluted with 5% EtOH were combined and lyophilized to afford Compound 146b as pale yellow powder.

$$IR\nu_{max}^{KBr}\ cm^{-1}\ :\ 1665,\ 1630-1550,\ 1400$$

| Elemental analysis for: $C_{16}H_{29}N_5O_3 2AcOH \cdot H_2O$ | | | |
|---|---|---|---|
| Calcd.<br>Found | C, 50.30;<br>C, 50.09; | H, 8.23;<br>H, 8.48; | N, 14.66<br>N, 14.40 |

Example 147

144

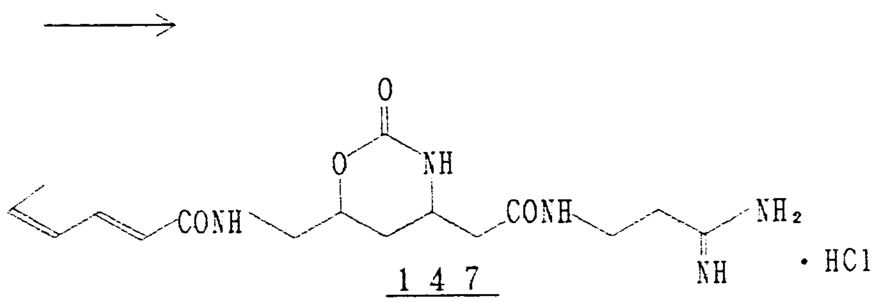

147

To a suspension of Compound 144 (163 mg) in EtOH (3 mℓ) was added 1N-KOH (1.1 mℓ), and mixture was stirred at room temperature for 30 minutes, followed by concentration under reduced pressure. To the concentrate was added 1N-HCℓ to adjsut the pH to 2.0, followed by saturation with sodium chloride. The mixture was extracted with AcOEt:THF (1:1) and dried over $MgSO_4$. The extract was concentrated under reduced pressure. The concentrate was dissolved in THF (2 mℓ), to which were added HOBT (81 mg) and DCC (124mg). The mixture was stirred at room temperature for 1.5 hours.

To a solution of 3-aminopropioamidine•2HCℓ salt (132 mg) in water (2 mℓ) was added $NaHCO_3$ (105 mg), and the mixture was cooled with ice. To this mixture was added, while stirring, the active ester solution prepared as above, and the mixture was stirred at room temperature for 6 hours, followed by allowing the reaction to proceed overnight. To the reaction mixture was added 1N-HCl to adjust the pH to 3.0, which was then subjected to filtration. The filtrate was concentrated under reduced pressure, and the concentrate was subjected to an HP-20 (70 mℓ) column chromatography. Elution was conducted with water, and eluate fractions were combined and lyophilized to afford Compound 147 (111 mg) as pale yellow powder.

$$IR\nu^{KBr}_{max}\ cm^{-1}\ :\ 1710\ (sh),\ 1690,\ 1655,\ 1545$$

| Elemental analysis for: $C_{16}H_{25}N_5O_4HC\ell \cdot H_2O$ | | | |
|---|---|---|---|
| Calcd. | C, 47.35; | H, 6.95; | N, 17.25 |
| Found | C, 47.11; | H, 7.20; | N, 17.00 |

Example 148

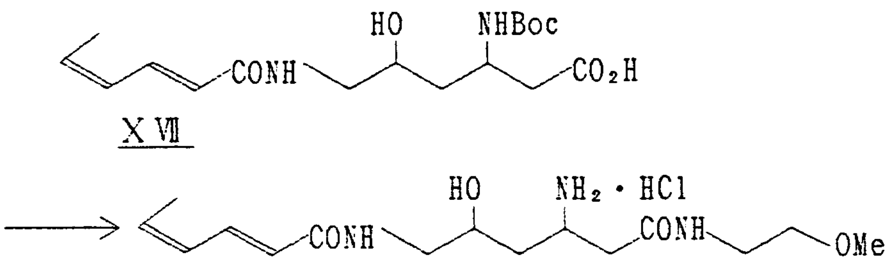

XVII

148

To a solution of Compound XVII (180 mg) in dry DMF were added $Et_3N$ (0.28 mℓ) and $Me_3SiC\ell$ (0.1 mℓ). The mixture was stirred for 10 minutes, to which was then added ethyl chlorocarbonate (0.1 mℓ). The mixture was stirred for 10 minutes, to which was further added 2-methoxyethylamine (0.075 mℓ), followed by stirring at room temperature for 30 minutes. The mixture was concentrated under reduced pressure, and the concentrate was dissolved in EtOAc. The solution was washed with an aqueous solution of sodium hydrogencarbonate and saturated brine, successively, followed by drying over anhydrous sodium sulfate. The solvent was distilled off. The residue was subejected to a silica gel (5 g) column chromatography, eluting with $CHC\ell_3$:MeOH:AcOEt = 85:10:5, to afford an N-Boc derivative of Compound 148 (70 mg). To this compound was added 2N-HCℓ (3 mℓ), and the reaction was allowed to proceed for 4 hours. The mixture was subjected to an activated charcoal (Shirasagi A, Takeda Chemical Industries, Ltd., Japan, 3 g) column chromatography. The fractions eluted with 60% EtOH-$H_2O$ to 80% EtOH-$H_2O$ were combined and lyophilized to afford Compound 148 (50 mg) as colorless powder.

$$IR\nu^{KBr}_{max}\ cm^{-1}\ :\ 1690,\ 1660,\ 1650$$

| Elemental analysis for: $C_{15}H_{27}N_3O_4HC\ell \cdot 0.5H_2O$ | | | |
|---|---|---|---|
| Calcd. | C, 50.20; | H, 8.15; | N, 11.71 |
| Found | C, 50.04; | H, 8.30; | N, 11.62 |

Example 149

52b

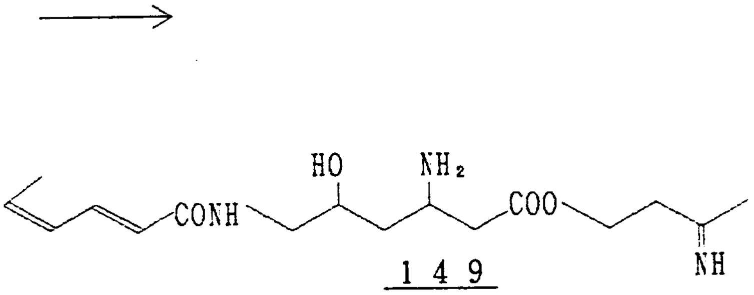

149 • $2CF_3CO_2H$

To a solution of Compound 52b (167 mg) in DMF (2 mℓ) was added 1,1'-carbonyldiimidazole (81 mg), and the mixture was stirred for 80 minutes in argon streams, followed by addition of 3-hydroxypropioamidine (78 mg). The reaction was allowed to proceed at room temperature for 18 hours. DMF was distilled off under reduced pressure. To the residue were added anisole (2 mℓ) and TFA (2 mℓ), and the mixture was stirred at room temperature for 1.5 hours. The mixture was concentrated under reduced pressure, and the concentrate was subjected to an HP-20 (70 mℓ) column chromatography, eluting with water. Eluate fractions were combined and lyophilized to afford Compound 149 (44 mg) as colorless powder.

$$IR\nu_{max}^{KBr}\ cm^{-1}: 1675, 1540$$

| Elemental analysis for: $C_{15}H_{26}N_4O_4 \cdot 2CF_3CO_2H$ | | | |
|---|---|---|---|
| Calcd.<br>Found | C, 40.50;<br>C, 40.27; | H, 5.19;<br>H, 5.31; | N, 9.94<br>N, 9.77 |

Example 150

I

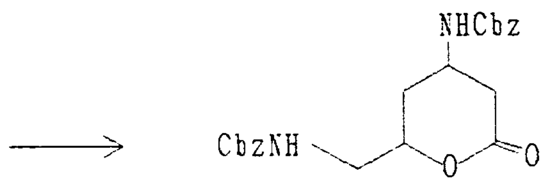

150

A mixture of Compound I (5.0 g) and 2N HCℓ (400 mℓ) was heated under reflux for 6 hours. The mixture was concentrated under reduced pressure, and the concentrate was dissolved in $H_2O$:THF (1:1) (100 mℓ). To the solution were added, while stirring under ice-cooling, carbobenzoxychloride (9.075 m) and 2N NaOH alternatively so that the pH was kept within the range of from 8.0 to 9.0. The mixture was stirred at room temperature for one hour, followed by extraction with EtOAc, and the EtOAc layer was washed with 1N HCℓ then with saturated brine, followed by drying over $MgSO_4$. The extract was concentrated under reduced pressure, and the concentrate was subjected to a silica gel (160 g) column chromatography, eluting with hexane:EtOAc = 3:2 to afford Compound 150 (3.302 g) as crystals.

$$IR\nu_{max}^{KBr}\ cm^{-1}\ :\ 1760,\ 1730-1690,\ 1540$$

| Elemental analysis for: $C_{22}H_{24}N_2O_6$ | | | |
|---|---|---|---|
| Calcd. | C, 60.07; | H, 5.87; | N, 6.79 |
| Found | C, 63.88; | H, 6.02; | N, 6.91 |

Example 151

150 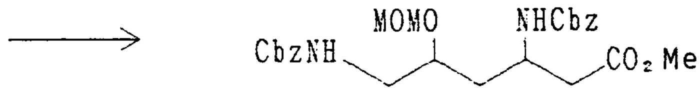

151

To a suspension of Compound 150 (1.65 g) in MeOH (30 mℓ) was added 28% NaOMe/MeOH solution (0.4 mℓ), and the reaction was allowed to proceed at room temperature for 30 minutes. To the reaction mixture was added acetic acid (0.4 mℓ), and the mixture was concentrated under reduced pressure. The concentrate was dissovled in EtOAc, and the solution was washed with water, then dried over $MgSO_4$. The solution was concentrated under reduced pressure, and the concentrate was dissolved in dry $CH_2Cℓ_2$ (25 mℓ). To the solution were added, under ice-cooling, diisopropylethylamine (2.79 mℓ) and methoxymethyl chloride (1.22 mℓ), and the reaction was allowed to proceed at room temperature for 3 days. The mixture was concentrated under reduced pressure, and the concentrate was dissolved in EtOAc. The solution was washed with water, dried over $MgSO_4$ and concentrated, followed by subjecting to a silica gel (30 g) column chromatography, eluting with EtOAc:hexane = 1:1 to afford Compound 151 (1.90 g) as a colorless oily product.

$$IR\nu_{max}^{KBr}\ cm^{-1}\ :\ 1730-1660,\ 1520$$

| Elemental analysis for: $C_{25}H_{32}N_2O_8$ | | | |
|---|---|---|---|
| Calcd. | C, 61.46; | H, 6.60; | N, 5.73 |
| Found | C, 61.57; | H, 6.55; | N, 5.84 |

Example 152

151 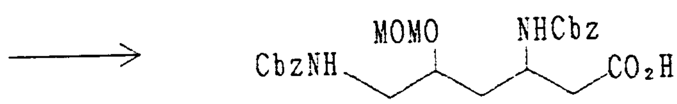

152

To a solution of Compound 151 (1.9 g) in EtOH (30 mℓ) was added 2N NaOH (4 mℓ), and the reaction was allowed to proveed at room temperature for one hour. The reaction mixture was concentrated under

reduced pressure, and the concentrate was dissolved in a small volume of water, whose pH was adjusted to 3.0 with 1N HCℓ, followed by extraction with EtOAc. The ethyl acetate layer was washed with saturated brine, followed by drying over $MgSO_4$. The solvent was distilled off under reduced pressure to afford Compound 152 (1.58 g) as a colorless oily product.

$$IR\nu^{KBr}_{max}\ cm^{-1}:\ 1730\ 1680,\ 1520$$

| Elemental analysis for: $C_{24}H_{30}N_2O_8$ | | | |
|---|---|---|---|
| Calcd. | C, 60.75; | H, 6.37; | N, 5.90 |
| Found | C, 60.88; | H, 6.39; | N, 5.78 |

Example 153

152

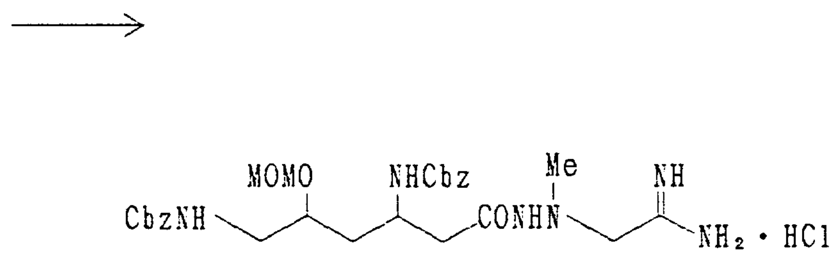

153a

To a solution of Compound 152 (285 mg) in THF (3 mℓ) were added HOBT (90 mg) and DCC (137 mg), and the mixture was stirred at room temperature for one hour. On the other hand, 2-(1-methyl-hydrazino)acetoamidine•2HCℓ salt (158 mg) was dissolved in water (3 mℓ), to which was added $NaHCO_3$ - (152 mg). To the mixture was added the active ester solution prepared as above, followed by stirring vigorously at room temperature for 6 hours. To the mixture was added, under ice-cooling, 1N HCℓ to adjust the pH to 3.0, followed by filtration. The filtrate was, after distilling off THF, subjected to an HP-20 (60 mℓ) column chromatography. The fraction eluted with 40% EtOH-$H_2O$ was lyophilized to afford Compound 153a (162 mg) as yellow powder

$$IR\nu^{KBr}_{max}\ cm^{-1}:\ 1725,\ 1700,\ 1525,\ 1450$$

| Elemental analysis for: $C_{27}H_{38}N_6O_7HC\ell \cdot 0.5H_2O$ | | | |
|---|---|---|---|
| Calcd. | C, 53.68; | H, 6.67; | N, 13.91 |
| Found | C, 53.60; | H, 6.84; | N, 13.78 |

153a

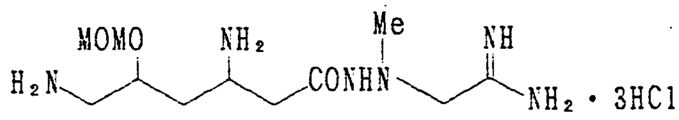

153b

To a solution of Compound 153a (161 mg) in water (3 mℓ) were added 10% Pd-C (100 mg), 1N HCℓ - (0.54 mℓ) and $H_2O$ (3 mℓ), and the mixture was stirred for 30 minutes under hydrogen. The catalyst was filtered off, and the filtrate was concentrated. The concentrate was subjected to an HP-20 (60 mℓ) column chromatography. The fraction eluted with $H_2O$ was lyophilized to afford Compound 153b (58 mg) as pale yellow powder.

$$IR\nu^{KBr}_{max}\ cm^{-1}\ :\ 1690,\ 1660\ (sh),\ 1480$$

| Elemental analysis for: $C_{11}H_{26}N_6O_3 3HC\ell \cdot 1.5H_2O$ | | | |
|---|---|---|---|
| Calcd. | C, 30.96; | H, 7.56; | N, 19.69 |
| Found | C, 30.76; | H, 7.84; | N, 19.42 |

153b

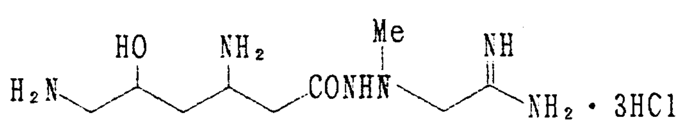

153c

To Compound 153b (84 mg) was added, under ice-cooling, TFA (2 mℓ), and the mixture was stirred at room temperature for one hour. TFA was distilled off, and the residue was dissolved in a small volume of water. The solution was subjected to pass through an IRA-401 ($C\ell^-$) (20 mℓ), eluting with water (50 mℓ), followed by lyophilization to afford Compound 153c (83 mg) as pale yellow powder.

$$IR\nu^{KBr}_{max}\ cm^{-1}\ :\ 1690,\ 1490,\ 1400$$

| Elemental analysis for: $C_9H_{22}N_6O_3 3HC\ell \cdot 1.5H_2O$ | | | |
|---|---|---|---|
| Calcd.<br>Found | C, 28.25;<br>C, 28.04; | H, 7.37;<br>H, 7.55; | N, 21.96<br>N, 21.76 |

Example 154

122a 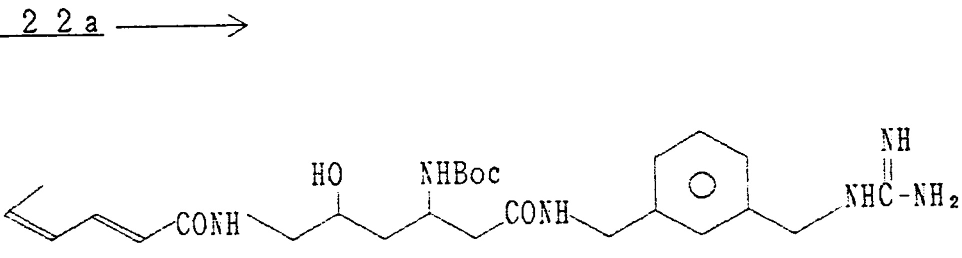

134a

To a solution of Compound 122a (700 mg) in EtOH (13 mℓ) was added S'-methyl isothiourea$\cdot$1/2$H_2SO_4$ (1.4 g). To the mixture was added, under ice-cooling, 1N NaOH (10 mℓ), followed by allowing the reaction to proceed at room temperature for 15 hours. EtOH was distilled off, and the residue was subjected to an HP-20 (350 mℓ) column chromatography. The fraction eluted with 50% MeOH-$H_2O$ was concentrated to afford Compound 154a (256 mg) as a colorless oily product.

$$IR\nu^{KBr}_{max}\ cm^{-1}: 1710\ (sh),\ 1690,\ 1655,\ 1530$$

| Elemental analysis for: $C_{26}H_{40}N_6O_5$ | | | |
|---|---|---|---|
| Calcd.<br>Found | C, 60.45;<br>C, 60.36; | H, 7.80;<br>H, 7.89; | N, 16.27<br>N, 16.42 |

154a 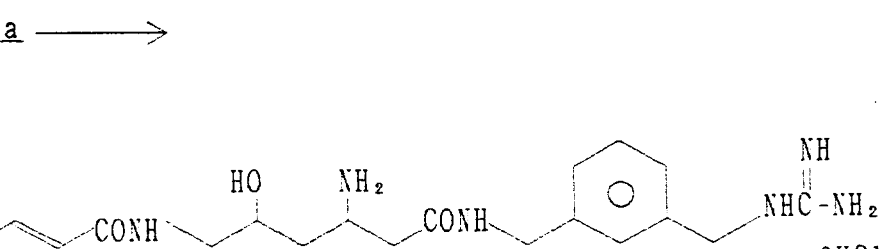

154b

To Compound 154a (256 mg) was aded, under ice-cooling,TFA (2 mℓ), and the mixture was stirred at room temperature for one hour, followed by distilling off TFA under reduced pressure. The residue was dissolved in a small volume of water, and the solution was allowed to pass through an IRA-401 ($C\ell^-$) (20 mℓ) column, followed by elution with water (60 mℓ). The eluate was lyophilized to afford Compound 154b - (243 mg) as colorless powder.

$$IR\nu_{max}^{KBr}\ cm^{-1}:\ 1660,\ 1610,\ 1540$$

| Elemental analysis for: $C_{21}H_{32}N_6O_3 2HCl \cdot 1.5H_2O$ | | | |
|---|---|---|---|
| Calcd.<br>Found | C, 48.84;<br>C, 48.62; | H, 7.22;<br>H, 7.38; | N, 16.27<br>N, 16.28 |

Example 155

XIII

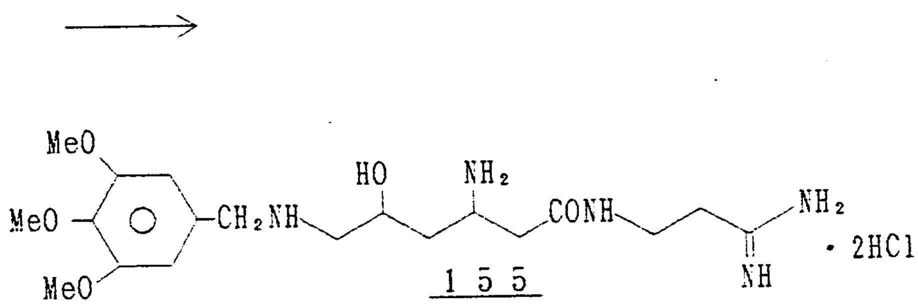

155

To a solution of Compound XIII (600 mg) in MeOH (15 mℓ) was added 3,4,5-trimethoxybenzaldehyde (600 mg), and the mixture was stirred at room temperature for 30 minutes. To the mixture were added, under ice-cooling, $NaBH_4CN$ (201 mg) and acetic acid (0.7 mℓ), and the mixture was stirred at room temperature for 15 hours. MeOH was distilled off under reduced pressure. To the residue was added 1N HCℓ to adjsut the pH to 2.5, which was shaken with ethyl acetate. The aqueous layer was separated and concentrated. To the concentrate was added 2N HCℓ (3 mℓ), and the reaction was allowed to proceed at room temperature for 3 hours, followed by purification by means of an activated charcoal (Shirasagi A, 10 g) column chromatography. The fraction eluted with 10% $MeOH-H_2O$ was lyophilized to afford Compound 155 (150 mg) as colorless powder.

$$IR\nu_{max}^{KBr}\ cm^{-1}:\ 1680,\ 1635,\ 1520$$

| Elemental analysis for: $C_{19}H_{33}N_5O_5 3HCl \cdot H_2O$ | | | |
|---|---|---|---|
| Calcd.<br>Found | C, 42.35;<br>C, 42.21; | H, 7.11;<br>H, 7.24; | N, 13.00<br>N, 12.89 |

Example 156

49b 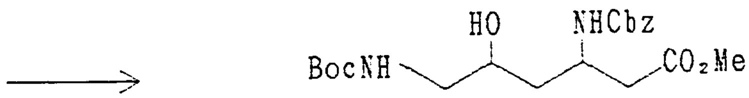

156

To a solution of Compound 49b (2.0 g) in methanol (50 mℓ) was added 28% NaOMe/MeOH solution (0.3 mℓ). The reaction was allowed to proceed for one hour. To the mixture was added acetic acid (0.3 mℓ), followed by concentration under reduced pressure. The concentrate was dissolved in ethyl acetate, washed with saturated brine and dried over magnesium sulfate. The resultant was concentrated under reduced pressure to afford Compound 156 (2.2 g) as colorless needles, m.p. 83 -84 ° C.

$$IR\nu_{max}^{KBr} cm^{-1} : 1730, 1705, 1670, 1550, 1520$$

| Elemental analysis for: $C_{20}H_{30}N_2O_7$ | | | |
|---|---|---|---|
| Calcd.<br>Found | C, 58.52;<br>C, 58.49; | H, 7.37;<br>H, 7.33; | N, 6.82<br>N, 6.87 |

Example 157

L 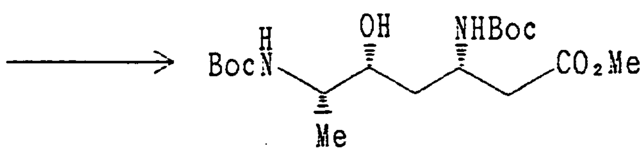

157

To a solution of Compound L(100 mg) in methanol(4 mℓ) were added molecular sieves 3A-(manufactured by Wako Pure Chemicals Industreis, Ltd., Japan) (excess) and ammonium acetate(0.2g). To the mixture was added a solution of sodium cyanoborohydride (70 mg) in methanol (0.7 ml) at room temperature under stirring, over a period of 2 hours. The mixture was stirred at room temperature for further one hour, followed by filtration using celite. The filtrate was concentrated. To the concentrate were added water and ethyl acetate, and the mixture was acidified (pH 3) under ice-cooling with a 10% aqueous solution of potassium hydrogensulfate, followed by making the mixture alkaline with an aqueous solution of potassium carbonate. To this mixture was added di-t-butyl dicarbonate(0.1 mℓ), and the whole mixture was stirred at room temperature for 4 hours. Then, the ethyl acetate layer was separated, and the aqueous layer was extracted with ethyl acetate. Organic layers were combined, washed with water and dried($Na_2SO_4$)-,followed by distilling off the solvent. The residue was purified by means of a silica gel(20 g) column chromatography (hexane→hexane:ethyl acetate = 2:1) to obtain methyl (3S,5R,6S)-3,6-di(t-butoxycarbonylamino)-5-hydroxyheptanoate(157) (35 mg) as colorless crystals, which were recrystallized from isopropyl ether to afford colorless prisms, m.p.126-127 ° C.
$IR\nu_{max}$(Nujol)$cm^{-1}$: 3490, 3380, 3360, 1725, 1690, 1660
NMR(90MHz,CDCℓ$_3$)ppm : 1.08(3H,d,J = 7Hz), 1.43(18H,s), 1.75 (2H,m), 2.60(2H,d,J = 5Hz), 3.45(1H,m), 3.68(3H,s), 4.05 (1H,m), 4.83(1H,d,J = 8Hz), 5.35(1H,d,J = 8Hz)

| Elemental Analysis for $C_{18}H_{33}N_2O_7$ : | | | |
|---|---|---|---|
| Calcd. | C, 55.51; | H, 8.54; | N, 7.19 |
| Found | C, 55.37; | H, 8.68; | N, 7.25 |

Example 158

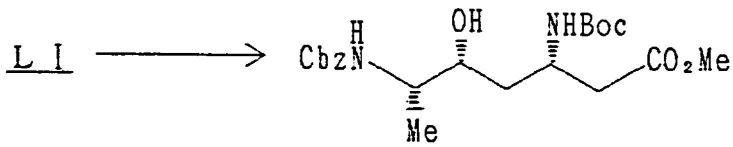

158

The Compound LI obtained in Reference Example 51 was subjected to a reaction and treatment in a manner similar to those of Example 157 to obtain Compound 158 as colorless crystals, m.p.102.5-103.5 ° C.
NMR(90MHz,CDCℓ3)ppm : 1.08(3H,d,7Hz), 1.43(9H,s), 1.75(2H,m), 2.55(2H,d,J = 7Hz), 3.45(1H,m), 3.68-(3H,s), 4.05(1H,m), 5.05(2H,s), 5.25(1H,m), 7.30(5H,s)

Example 159

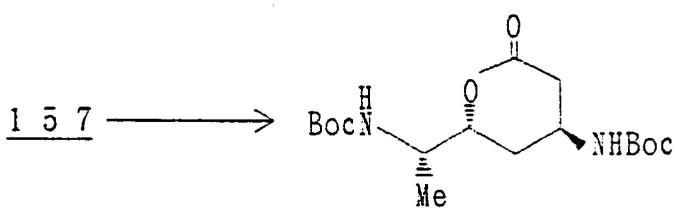

159

To a solution of Compound 157(30 mg) in THF(1 mℓ) was added 1N NaOH(0.1 mℓ), and the mixture was stirred at room temperature for 2.5 hours. The solvent was distilled off, and the residue was dissolved in water. The solution was acidified with a 10% aqueous solution of potassium hydrogensulfate, followed by extraction with ethyl acetate. The extract was washed with water, dried ($Na_2SO_4$), and the solvent was evaporated. The residue was dissolved in chloroform(2 mℓ). To the solution was added 1-ethyl-3-(3-dimethylaminopropyl)carbodiimide•hydrochloride(40 mg), and the mixture was stirred at room temperature for 30 minutes. The reaction mixture was washed with water, dried($Na_2SO_4$),followed by distilling off the solvent to give (4S,6R,1'S)-4-t-butoxycarbonylamino-6-[(1'-t-butoxycarbonylamino)ethyl]-2-oxotetrahydropyran(159)(24 mg) as colorless crystals. Recrystallization from ethyl acetate - isopropyl ether gave colorless prisms, m.p.143-145 ° C.
IR$\nu_{max}$(KBr)$cm^{-1}$: 3380, 2980, 2930, 1710, 1695, 1520
NMR(90MHz,CDCℓ3)ppm : 1.17(3H,d,J = 6Hz), 1.43(18H,s), 1.7-2.2 (3H,m), 2.7(1H,m), 3.7(1H,m), 4.1(1H,m), 4.5(1H,m), 5.0(2H,m)
Mass Spectrum : m/e 385($M^+$)

$$[\alpha]_D^{22.5°} -40.62°(c=0.485, CHC\ell_3)$$

Example 160

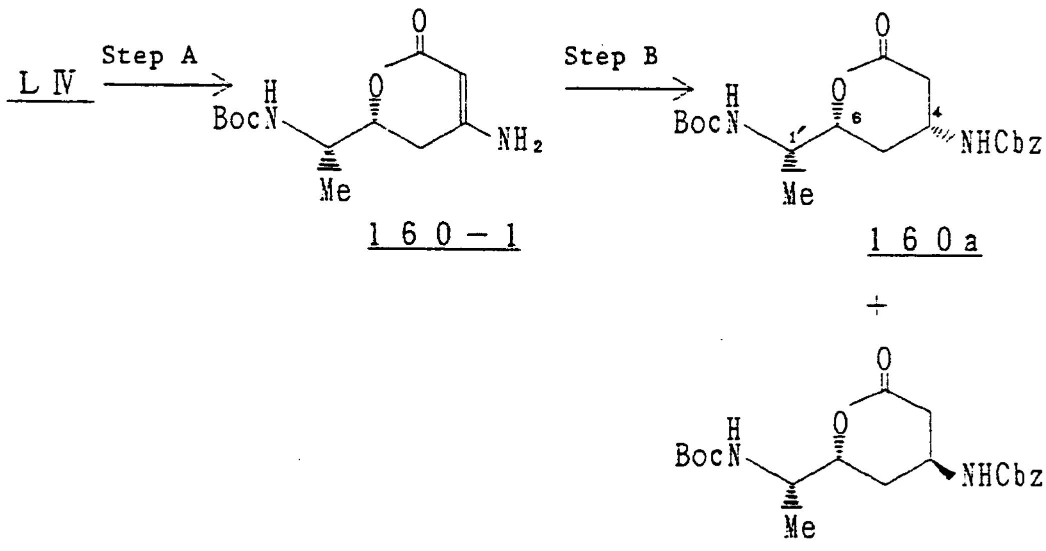


160-1

160a

+

160b

Step A

To a solution of Compound XIV(1.00 g) in acetic acid(10 mℓ) was added ammonium acetate(5.99 g), and the mixture was stirred at room temperature for 4 hours. To the reaction mixture was further added ammonium acetate(12.58 g), and the mixture was stirred at room temperature for 16.5 hours, followed by adding water and extracting with ethyl acetate. The aqueous layer was made alkaline with a saturated aqueous solution of sodium hydrogencarbonate, which was subjected to extraction with ethyl acetate. The extracts were combined, washed with an aqueous solution of sodium chloride, dried($Na_2SO_4$), followed by distilling off the solvent to leave a pale yellow oily product. To the oily product was added $CH_2C\ell_2$-iso$Pr_2O$ to obtain Compound 160-1(553 mg) as colorless crystals, m.p.170-172 °C.

IR$\nu_{max}$(KBr)$cm^{-1}$: 3360, 3140, 2960, 1670, 1640, 1570

NMR(90MHz, $CDC\ell_3$)ppm : 1.22(3H,d,J=7Hz), 1.43(9H,s), 2.00-2.76(2H,m), 3.83(1H,m), 4.23(1H,m), 4.60-(1H,b), 4.93(1H,s)

| Elemental Analysis : $C_{12}H_{20}N_2O_4$ | | | |
|---|---|---|---|
| Calcd. | C,56,24; | H,7.86; | N,10.93 |
| Found | C,56.00; | H,7.59; | N,10.70 |

Step B (method 1)

To a solution of Compound 160-1(80 mg) in THF(4 mℓ) were added acetic acid(4 mℓ) and a solution of sodium cyanoborohydride(59 mg) in THF(0.5 mℓ), and the mixture was stirred at room temperature for 3 hours. The reaction mixture was made alkaline(pH 11) with a saturated aqueous solution of sodium hydrogencarbonate and 1N-NaOH, followed by addition of ethyl acetate(30 mℓ) and benzyloxycarbonyl chloride(0.3 mℓ). The mixture was stirred at room temperature for 14.5 hours. The ethyl acetate layer was separated, dried($Na_2SO_4$),and the solvent was distilled off. The residue was purified by means of a silica gel (15 g) column chromatography(hexane: ethyl acetate = 1:1) to obtain a colorless oily product(49 mg) (this product was confirmed by the NMR spectrum as a mixture (about 1:1 ratio) of two isomers of 4-benzyloxycarbonylamino-6-[(1'-t-butoxycarbonylamino)ethyl]-2-oxotetrahydropyrans, i.e. (4R,6R,1'S)isomer-(160a) and (4S,6R,1'S) isomer(160b)). Recrystallization of this product from ether afforded Compound 160a-(11 mg) as colorless crystals, m.p. 133-134 °C.

IR$\nu_{max}$(KBr)$cm^{-1}$ : 3350, 2900, 1750, 1690, 1520

NMR(90MHz, CDCℓ₃)ppm : 1.14(3H,d,J=6Hz), 1.43(9H,s), 2.06-2.53(3H,m), 2.80-3.15(1H,m), 3.8(1H,m), 4.1(1H,m), 4.4(1H,m), 5.0(1H,m), 5.10(2H,s), 7.36(5H,s)
$[\alpha]_D^{22}$ -34.0° (c=0.65, ethanol)

| Elemental Analysis for $C_{20}H_{28}N_2O_6$ : | | | |
|---|---|---|---|
| Calcd.<br>Found | C,61.21;<br>C,61.01; | H,7.19;<br>H,7.30; | N,7.13<br>N,7.40 |

Step B (method 2)

To a solution of phosphoric acid(0.32 mℓ) in anhydrous THF(20 mℓ) were added phosphorus pentoxide(0.28 g), 5% Pt-C (0.8 g) and Compound 160-1(0.52 g), and the mixture was stirred at room temperature in hydrogen streams for 2 hours. The reaction solution was filtrated and washed with THF-(about 20 mℓ). The filtrate and the washing were combined, to which were added a saturated aqueous solution of sodium hydrogencarbonate(about 25 mℓ) and benzyloxycarbonyl chloride(1 mℓ), The mixture was stirred at room temperature for 3.5 hours, and THF was distilled off. The residue was subjected to extraction with ethyl acetate, and the extract was washed with water and dried($Na_2SO_4$), followed by distilling off the solvent. The residue was purified by means of a silica gel (25 g) column chromatography-(hexane : ethyl acetate = 1:1→2:3) to afford a colorless oily product(0.80 g) (this product is confirmed from the NMR data as a mixture of Compound 160a and Compound 160b at a ratio of about 3:1). This product was recrystallized twice from CHCℓ₃-isoPro₂O to afford Compound 160a(0.35 g) as colorless crystals. The physico-chemical properties of this product were in agreement with those of Compound 160a obtained in the method 1 described above.

Example 161

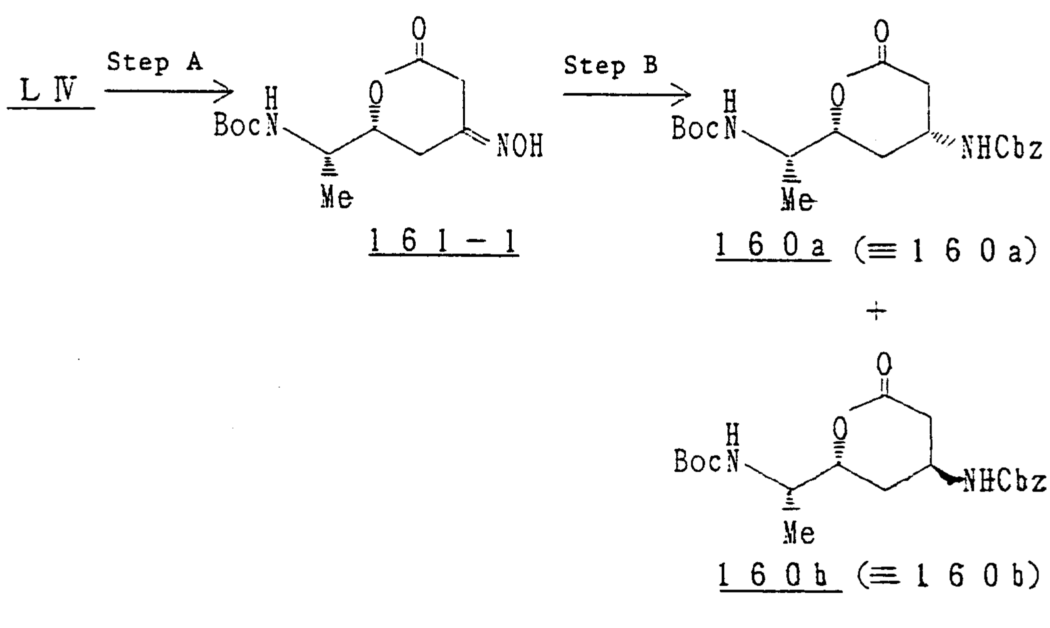

LIV
161-1
160a (≡160a)
+
160b (≡160b)

Step A

To a solution of Compound LIV(2.50 g) in methanol(30 mℓ) were added pyridine(1.05 mℓ) and hydroxylamine hydrochloride (1.01 g). The mixture was stirred at room temperature for 2.5 hours and concentrated. To the concentrate was added water, and precipitating colorless crystals were collected by filtration, washed with water and dried to afford (6R,1'S)-6-[(1'-t-butoxycarbonylamino)ethyl]-2,4-dioxotetrahydropyran 4-oxime(161-1)(2.26 g), m.p.187-189°C.
$IR\nu_{max}(KBr)cm^{-1}$ : 3300, 2950, 1720, 1680, 1515
NMR(90MHz,CDCℓ₃)ppm : 1.21(3H,d,J=7Hz), 1.43(9H,s), 2.33-2.95(2H,m), 3.36-3.65(2H,m), 3.93(1H,m),

4.50(1H,m), 5.0(1H,m)

| Elemental Analysis for $C_{12}H_{20}N_2O_5$ : | | | |
|---|---|---|---|
| Calcd. | C,52.93; | H,7.40; | N,10.29 |
| Found | C,52.89; | H,7.43; | N,10.11 |

Step B

Using Compound 161-1(0.67 g), reduction was conducted in a manner similar to that of Step B (Method 2) in Example 160 to thereby afford a mixture of Compound 161a and Compound 161b (about 3:1), which was recrystallized to give Compound 161a (0.39 g). (The physico-chemical properties of this product were in agreement with those of Compound 160a obtained in Example 160.

Example 162

161-1

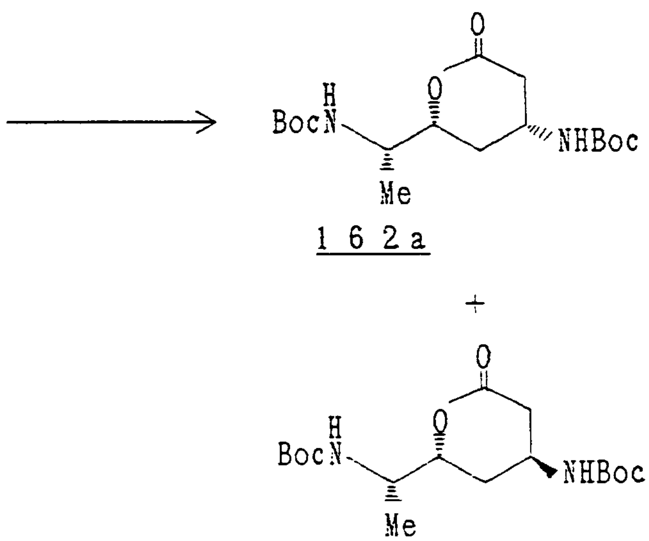

162a

+

162b

Using the Compound 161-1 obtained by Step A of Example 161, reduction was conducted in a manner similar to Step B (method 2) of Example 160 using Pt-C, and using di-t-butyl dicarbonate instead of benzyloxycarbonyl chloride, a mixture of 162a and 162b (about 3:1 composition ratio). Recrystallization afforded Compound 162a(40%), m.p.163-164.5 °C.
IR$\nu_{max}$(KBr)cm$^{-1}$: 3375, 2990, 1720, 1680, 1520
NMR(90MHz,$CDCl_3$)ppm : 1.14(3H,d,J=6Hz), 1.43(18H,s), 2.06-2.53(3H,m), 2.85-3.15(1H,m), 3.8(1H,m), 4.1(1H,m), 4.4(1H,m), 4.63(1H,m), 4.86(1H,m)

$$[\alpha]_D^{22.5} -32.37° (c=0.519, chloroform)$$

| Elemental Analysis for $C_{17}H_{30}N_2O_6$ : | | | |
|---|---|---|---|
| Calcd. | C,56.97; | H,8.44; | N,7.82 |
| Found | C,56.75; | H,8.21; | N,7.90 |

Examples 163-173 (Table 16)

By manners similar to those of Examples 161 and 162, using Compounds LVI and LIX-LXVII, compounds of Examples 163-173 were obtained via 1) enamination employing ammonium acetate or oximation employing hydroxylamine (Step A, A'), 2) catalytic reduction employing Pt-C or reduction employing sodium cyanoborohydride (Step B,B') and 3) reaction of protecting the resulting amino group with Boc group or cbz group (Step C,C'). Yields of isolated products and physico-chemical properties thereof are shown in Table 16.

Table 16

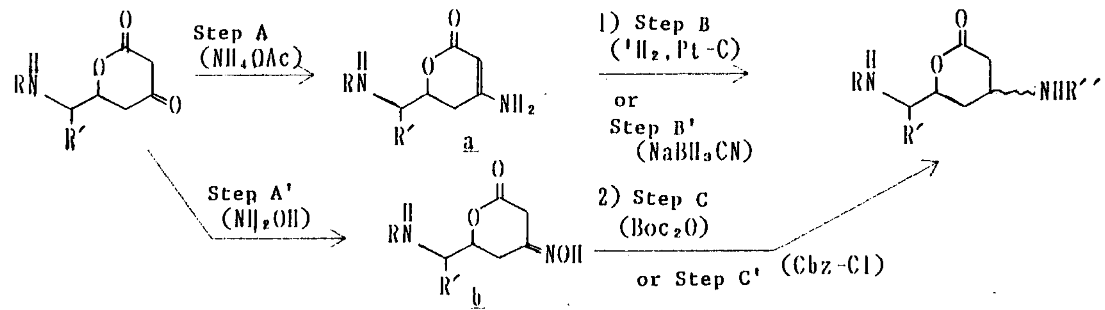


| Ex. No. | Step | Starting Compd. No. | Isolated Compound No. | Yield (%) | m.p. (℃) |
|---|---|---|---|---|---|
| 163 | A' | LVI | 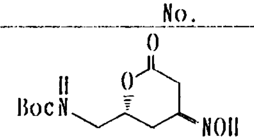 163-1 | 87 | 165-167 |
| | B' → C' | 163-1 | 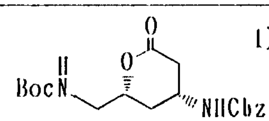 1) 163a (≡49b) | 41 | 135-137 |

1) The physico-chemical data of this product were in agreement with those of Compound 49b obtained in Example 49.

| Ex. No. | Step | Starting Compd. No. | Isolated Compound No. | Yield (%) | m.p. (℃) |
|---|---|---|---|---|---|
| 164 | A | LIX | 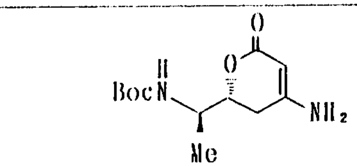 164-1 | 51 | 169-171 |
| | B → C′ | 164-1 | 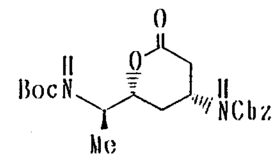 164a | 35 | 121-123 |
| 165 | B → C | 164-1 | 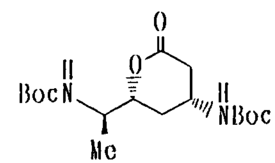 165a | 38 | 164-165 |

| Ex. No. | Step | Starting Compd. No. | Isolated Compound No. | Yield (%) | m.p. (℃) |
|---|---|---|---|---|---|
| 166 | A | LX | 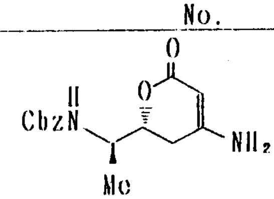 166-1 | 62 | 190-193 |
| | B′ → C | 166-1 | 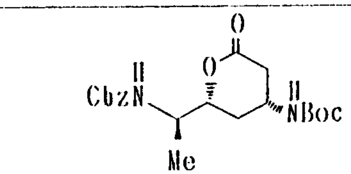 166a | 13 | 151-152 |
| | | | 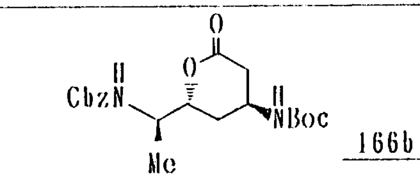 166b | 1 | 129-130 |

| Ex. No. | Step | Starting Compd.No. | Isolated Compound No. | Yield (%) | m.p. (℃) |
|---|---|---|---|---|---|
| 167 | A | L XI | 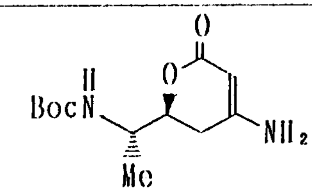 167－1 | 60 | 172－174 |
| | B → C′ | 167－1 | 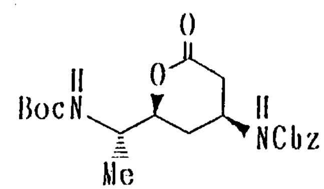 167a | 32 | 123－124 |

| Ex. No. | Step | Starting Compd. No. | Isolated Compound No. | Yield (%) | m.p. (℃) |
|---|---|---|---|---|---|
| 168 | A | L XII | 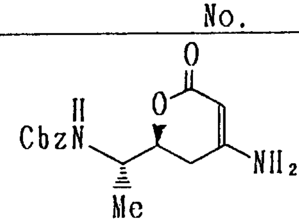 168-1 | 43 | 194-196 |
| | B′ → C | 168a | 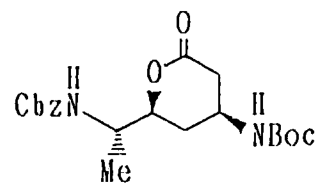 168a | 19 | 150-151 |
| 169 | A′ → B → C′ | L XIII | 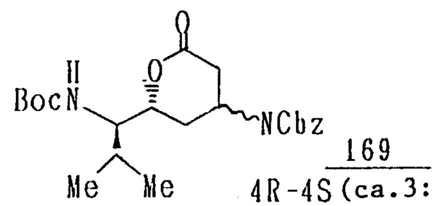 169 4R-4S (ca.3:1) | 63 | oily product 2) |

2) NMR(90MHz, $CDCl_3$)ppm: 0.97(6H, d, J=7Hz), 1.43(9H, s), 1.60-2.30(2H, m), 2.31(1H, dd, J=10, 18Hz), 2.95(1H, dd, J=6, 18Hz), 3.35(1H, t, J=10Hz), 3.90-4.60(3H, m), 4.76(1H, d, J=11Hz), 5.10(2H, s), 5.32(1H, d, J=8Hz), 7.35(5H, s)

| Ex. No. | Step | Starting Compd. No. | Isolated Compound No. | Yield (%) | m.p. (℃) |
|---|---|---|---|---|---|
| 170 | A′ → B → C′ | LXIV | 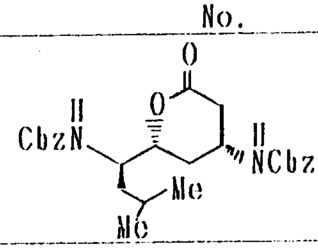 170a | 46 | 74 − 76 |
| 171 | A′ → B″ 3) → C′ | LXV | 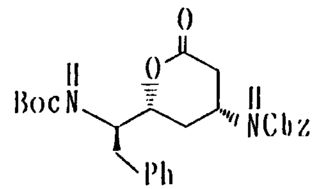 171a | 20 | Powder 4) (not determined yet) |
| | | | 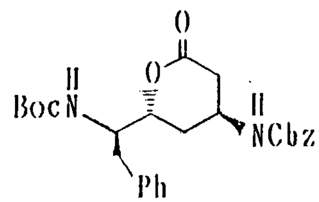 171b | 9 | 156 − 158 |

3) After oximinoation, reduction using 10%Pd-C was conducted in MeOH in the presence of $HCO_2NH_4$ was conducted to give an enamine compound, which was then reduced by using sodium cyanoborohydride.

4) NMR(90MHz, $CDCl_3$)ppm: 1.35(9H, s), 1.48-2.25(2H, m), 2.31(1H, dd, J = 9, 18Hz), 2.92(1H, dd, J = 6, 18Hz), 2.90(2H, d, J = 8Hz), 3.60-4.30(3H, m), 4.79(1H, d, J = 10Hz), 5.00-5.20(1H, m), 5.09 (2H, s), 7.25, 7.34(5H × 2, s)

| Ex. No. | Step: | Starting Compd. No. | Isolated Compound No. | Yield (%) | m.p. (℃) |
|---|---|---|---|---|---|
| 172 | A′ → B → C′ | L X VI | 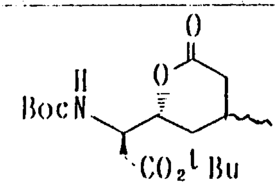 172 4R − 4S (ca.3:1) | 29 | oily product 5) |
| 173 | A′ → B → C′ | L X VII | 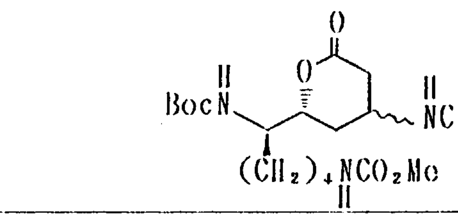 173 4R − 4S (ca.4:1) | 22 | oily product 6) |

5) NMR (90MHz, $CDCl_3$) ppm: 1.43 (18H, s), 1.60-2.35 (2H, m), 2.35 (1H, dd, J = 9, 15Hz), 2.42-2.74 (2H, m), 2.92 (1H, dd, J = 6, 15Hz), 3.50-4.51 (3H, m), 5.00 (1H, d, 7Hz), 5.40 (1H, d, J = 8Hz), 5.08 (2H, s), 7.34 (5H, s)

6) NMR (90MHz, $CDCl_3$) ppm: 1.42 (9H, s), 1.20-1.74 (6H, m), 1.74-2.35 (2H, m), 2.33 (1H, dd, J = 10, 18Hz), 2.96 (1H, dd, J = 6, 18Hz), 2.96-3.31 (2H, m), 3.64 (3H, s), 3.45-4.40 (2H, m), 4.65-5.32 (3H, m), 5.10 (2H, s), 7.34 (5H, s)

Example 174

XXVI

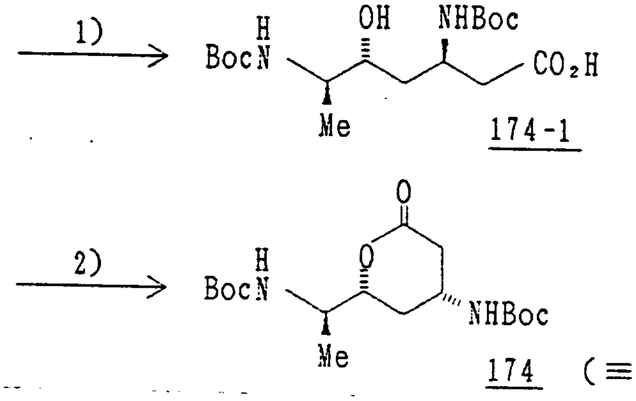


174-1

174 (≡ 165a)

1) Compound XXVI(40 mg) was dissolved in a mixture of dioxane(0.8 mℓ) and methanol(0.5 mℓ). To the solution were added at room temperature triethylamine(24μℓ) and BOC•ON(44 mg), successively. The mixture was stirred at room temperature for further 8 hours, to which was added a 10% aqueous solution of sodium hydrogencarbonate, followed by washing with ether. To the aqueous layer was added 2N hydrochloric acid to adjust the pH at 2, which was then extracted with ethyl acetate. The extract was washed with water, which was passed through a column(ϕ15 x 100mm) of anhydrous sodium sulfate. The effuluent was concentrated to afford Compound 174-1(53 mg) as an oily prodcut.
NMR(90MHz,$CDC\ell_3$)ppm : 1.20(3H,d,J=7Hz), 1.43(18H,s), 1.60(2H,n), 2.60(2H,d,J=5Hz), 3.60(2H,m), 4.20(1H,m), 5.60(2H,d,J=10Hz)
2) Compound 174-1(52 mg) was dissolved in dichloromethane (1.7 mℓ), to which was added 10-camphor sulfonic acid(3 mg). The mixture was stirred at room temperature for 50 minutes. To the reaction solution was added water, which was extracted with ethyl acetate. The extract solutions were combined, washed with water, then allowed to pass through a column of anhydrous sodium sulfate (ϕ15 x 80mm), and the effluent was concentrated to obtain an oily product, which was crystallized from ether to afford Compound 174(29 mg) as colorless crystals, m.p.163-164 °C.
$IR\nu_{max}(KBr)cm^{-1}$ : 3370, 2970, 1715, 1690, 1530
NMR(90MHz,$CDC\ell_3$)ppm : 1.28(3H,d,J=7Hz), 1.45(18H,s), 1.35-1.70, 2.05-2.35(2H,m), 2.30-(1H,dd,J=9,18Hz), 2.95(1H,dd,J=7.18Hz), 3.67-4.72(5H,m)

$$[\alpha]_D^{22.5} +19.6° (c=0.49, chloroform)$$

| Elemental Analysis for $C_{17}H_{30}N_2O_6$ : | | | |
|---|---|---|---|
| Calcd. | C,56.97; | H,8.44; | N,7.82 |
| Found | C,57.14; | H 8.38; | N,7.78 |

IR and NMR spectra of this product are in agreement with those of the Compound (165a) obtained in Example 165.

Example 175

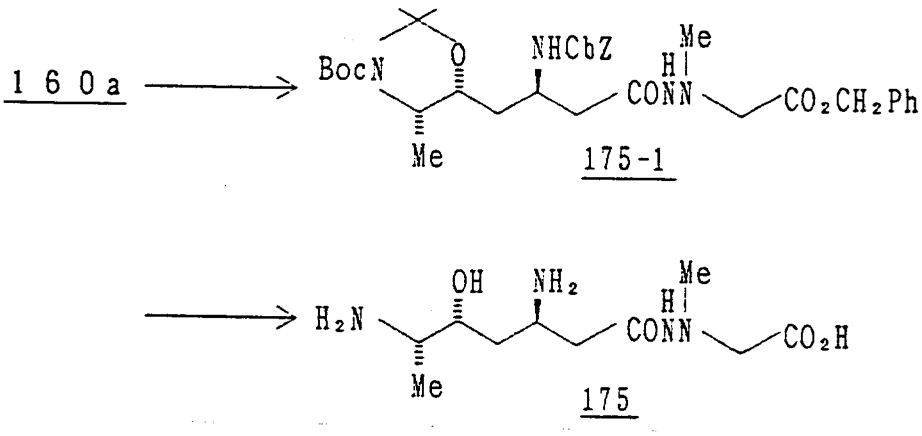

160a

175-1

175

To a solution of Compound 160a(185 mg) in methanol(20 mℓ) was added NaOMe(3 mg), and the mixture was stirred at room temperature for 5 hours, then the solvent was distilled off. To the residue were added $CH_2C\ell_2$(5 mℓ), pyridinium p-toluene sulfonate(5 mg), 2-methoxypropene(0.5 mℓ) and 2,2-dimethoxypropane(2 mℓ), and the mixture was stirred at room temperature for one hour. The solvent was distilled off. To the residue was added ethyl acetate, and the mixture was washed with water, dried($Na_2SO_4$), followed by distilling off the solvent. To the residue were added methanol(15 mℓ), THF(5 mℓ), water(5 mℓ) and 1N-NaOH(5 mℓ), and the mixture was stirred at room temperature for 2 hours, then the solvent was distilled off. To the residue was added water, and the mixture was washed with ether. The aqueous layer was acidified with a 10% aqueous solution of potassium hydrogensulfate, followed by extraction with ethyl acetate. The extract was dried($Na_2SO_4$), then the solvent was distilled off. To the residue were added anhydrous THF(10 mℓ), DCC(117 mg) and HOBT(87 mg), and the mixture was stirred for 3.5 hours. To the mixture was added a solution of 1-methylhydrazinoacetic acid(154 mg) in anhydrous THF(5 mℓ), and the mixture was stirred at room temperature for 15 hours, then the solvent was distilled off. To the residue was added ethyl acetate, and the mixture was washed with a saturated aqueous solution of sodium hydrogencarbonate and dried($Na_2SO_4$), followed by distilling off the solvent. The residue was purified by means of a silica gel(20 g) column chromatography(hexane-ethyl acetate = 1:1→ethyl acetate) to afford Compound 175-1(170 mg) as a colorless oily product.
[NMR(90MHz,$CDC\ell_3$)ppm : 1.05(3H,d,J=6Hz), 1.43(15H,b), 1.83(b), 2.45(b), 2.73(b), 3.57(b), 3.70(s), 4.10-(b), 5.08(2H,s), 5.16(2H,s), 5.96(b), 7.36(10H,s)]

To a solution of Compound 175-1(170 mg) in methanol(5 mℓ) and acetic acid(2.5 mℓ) was added 10%Pd-C(170 mg), and the mixture was stirred at room temperature for 6 hours in hydrogen streams. The solvent was filtered off and washed with methanol. The filtrate and the washing were combined, from which was distilled off the solvent. To the residue was added TFA(5 mℓ), and the mixture was stirred at room temperature for one hour, then the solvent was distilled off. To the residue was added water(0.5 mℓ), which was purified by means of a CG-50(50 mℓ) column chromatography(water → 0.5% aqueous ammonia). The eluate was concentrated and lyophilized to afford Compound(175)(65 mg) as a white powdery product.
IR$\nu_{max}$(KBr)$cm^{-1}$: 3700-2400, 1650, 1580, 1400, 1310
NMR(90MHz,$D_2O$)ppm : 1.30(3H,d,J=7Hz), 1.70(2H,m), 2.51(2H,d,J=6Hz), 2.72(3H,6s), 3.36-3.66(4H,m), 4.1(1H,m)
SIMS:m/e 263($M^+$)

Example 176

164a

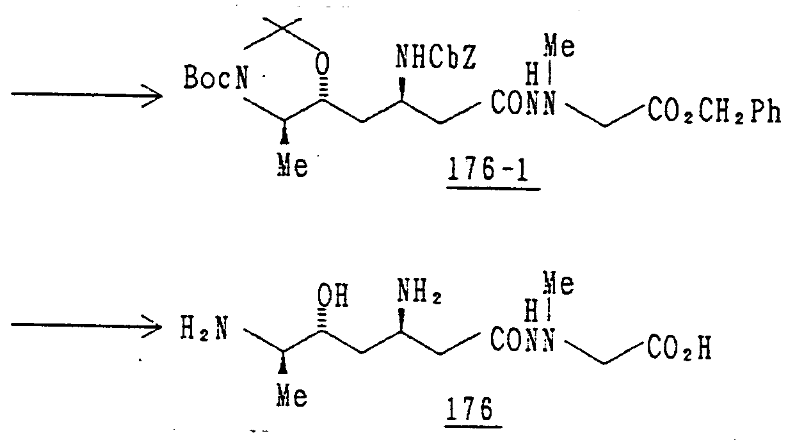

176-1

176

By a process similar to that of Example 175, Compound 176 was obtained from Compound 164a via Compound 176-1. 176-1 : colorless oily product, yield 75%
IR$\nu_{max}$(Neat)$cm^{-1}$: 3260, 2960, 1680(b), 1390
NMR(90MHz,$CDCl_3$)ppm : 1.25(3H,d,J=7Hz), 1.44(9H,s), 1.53, 1.58(3Hx2,sx2), 1.60-2.20(2H,m), 2.41-(2H,d,J=6Hz), 2.75(3H,s), 3.20-3.88(2H,m), 3.70(2H,s), 3.90-4.38(1H,m), 5.10,5.18(2Hx2,sx2), 7.34,7.37-(5Hx2,sx2)
SIMS:m/e 627($M^+$)
176 : white powder, yield 85%
IR$\nu_{max}$(KBr)$cm^{-1}$: 3400-2900, 1655, 1580, 1400
NMR(90MHz,$D_2O$)ppm : 1.33(3H,d,J=7Hz), 1.60-1.95(2H,m), 2.47(2H,d,J=7Hz), 2.72(3H,s), 2.95-40(3H,m), 2.49(2H,s)
$[\alpha]_D^{22}$ +14.0° (c=0.25, $H_2O$

| Elemental Analysis for $C_{10}H_{22}N_4O_4 \cdot 1.2H_2O$ | | | |
|---|---|---|---|
| Calcd. | C, 42.30; | H, 8.66; | N, 19.73 |
| Found | C, 42.43; | H, 8.38; | N, 19.83 |

Example 177

167a

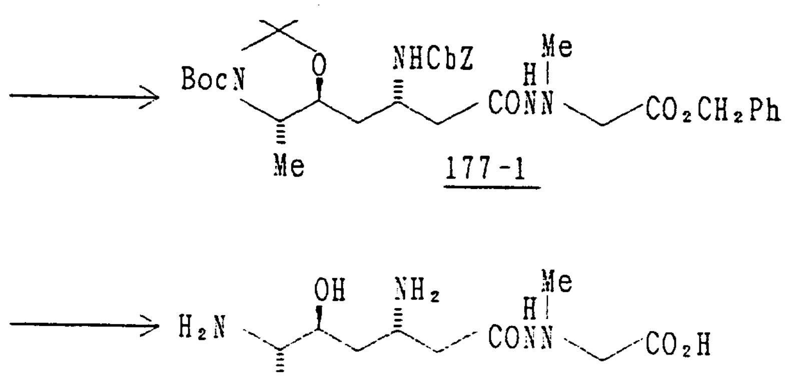

177-1

177

By a process similar to that of Example 175, Compound 177 was obtained from Compound 167a via 177-1.
177-1 : colorless oily product, yield 64%

NMR(90MHz,CDCℓ$_3$)ppm : 1.24(3H,d,J=7Hz), 1.43(9H,s), 1.53,1.56(3Hx2,sx2), 1.61-2.20(2H,m), 2.42-(2H,d,J=6Hz), 2.75(3H,s), 3.24-3.90(2H,m), 3.70(2H,s), 3.92-4.34(1H,m), 5.09,5.15(2Hx2,sx2), 7.31,7.33-(5Hx2,sx2)

177 : white powder, yield 81%

IR$\nu_{max}$(KBr)cm$^{-1}$: 3450-2950, 1660-1630, 1580, 1395

NMR(90MHz,$D_2O$)ppm : 1.35(3H,d,J=7Hz), 1.60-2.05(2H,m), 2.46(2H,d,J=7Hz), 2.71(3H,s), 2.85-4.00-(3H,m), 3.48(2H,s)

$[\alpha]_D^{22}$ -14.6° (c=0.185, water)

| Elemental Analysis : $C_{10}H_{22}N_4O_4 \cdot 1.4H_2O$ | | | |
|---|---|---|---|
| Calcd. | C,41.77; | H,8.69; | N,19.49 |
| Found | C,42.03; | H,8.48; | N,19.17 |

Example 178

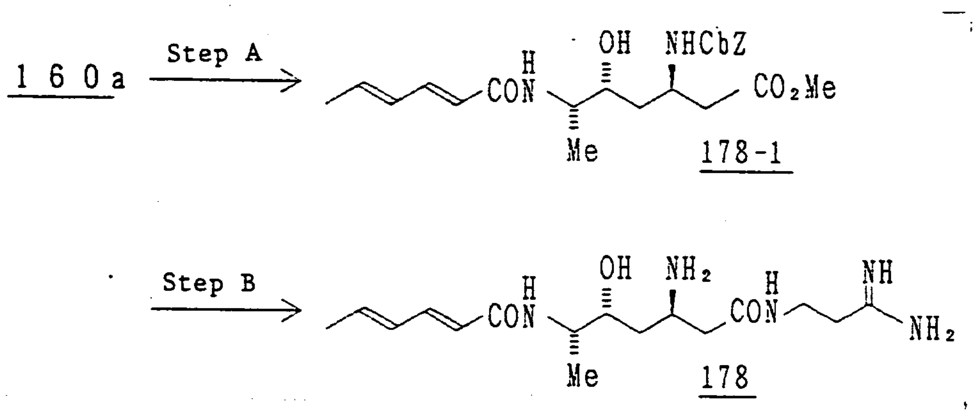

178-1

178

Step A

To a solution of Compound 160a(200 mg) in methanol(10 mℓ) was added diisopropyl ethylamine (one drop), and the mixture was stirred at room temperature for 10 hours, then the solvent was distilled off. To the residue was added, under ice-cooling and stirring, methanol(8 mℓ) saturated with hydrogen chloride, and the mixture was stirred for 2 hours, followed by distilling off the solvent. To the residue was added $CH_2Cℓ_2$(20 mℓ), and to the mixture were added, under ice-cooling and stirring, pyridine(0.3 mℓ) and sorbyl chloride (80 mg). This mixture was stirred for 30 minutes under ice-cooling, and the solvent was distilled off. The residue was dissolved in ethyl acetate. The solution was washed with water, a saturated aqueous solution of potassium hydrogensulfate, a saturated aqueous solution of sodium hydrogencarbonate and an aqueous saline solution, successively, which was dried ($Na_2SO_4$), followed by distilling off the solvent. To the residue was ether, whereupon Compound 178-1(110 mg) was obtained as white powder, m.p.148-150°C.

IR$\nu_{max}$(KBr)cm$^{-1}$: 3300, 1725, 1695, 1665, 1650, 1605

NMR(90MHz,CDCℓ$_3$)ppm : 1.10(3H,d,J=7Hz), 1.40-1.73(2H,m), 1.86(3H,d,J=5Hz), 2.58(2H,d,J=5Hz), 3.63-(3H,s), 3.50-4.45(3H,m), 5.10(2H,s), 5.60-6.21, 7.00-7.60(4H,m), 7.32(5H,s)

Step B

To a solution of Compound 178-1(150 mg) in $CH_2Cℓ_2$ (20 mℓ) were added 2-methoxypropene(1 mℓ) and p-toluenesulfonic acid. anhydride(catalytic amount), and the mixture was stirred at room temperature for 2 hours, then the solvent was distilled off. To the residue were added 5N-NaOH(1 mℓ), methanol(10 mℓ) and water(5 mℓ), and the mixture was stirred at room temperature for 2 hours, followed by distilling off the solvent. To the residue was added water, and the mixture was washed with ether. To the mixture was added a saturated aqueous solution of potassium hydrogensulfate to acidify, which was extracted with ethyl

acetate. The extract solution was dried($MgSO_4$) and concentrated. To the concentrate were added anhydrous THF(10 mℓ), HOBT(48 mg) and DCC(81 mg), successively, then the mixture was stirred at room temperature for 1.5 hours. Precipitating crystals were filtered off, and, to the filtrate was added a solution of 3-aminopropioamidine•hydrobromide (89 mg) and a solution of sodium hydrogencarbonate(60 mg) in water-(10 mℓ), and the mixture was stirred at room temperature for 18 hours. The solvent was distilled off, and the residue was dissolved in chloroform-ethanol(5:1), then the solution was dried($MgSO_4$). The solvent was distilled off, and the residue was dissolved in acetonitrile(20 mℓ). To the solution were added sodium iodide(3 g) and trimethylchlorosilane(2.17 g), and the mixture was stirred at room temperature for 1.5 hours. To the reaction mixture was added an aqueous solution of sodium phosphate to suspend the reaction, and the reaction solution was washed with ether. The aqueous layer was concentrated and purified by means of an Amberlite XAD-2(50 mℓ) column chromatography(water→20% ethanol) and of an Amberlite IRA-401-(Cℓ$^-$-type, 30 mℓ), followed by lyophilization to afford dihydrochloride of Compound 178(145 mg) as white powder.

IR$\nu_{max}$(KBr)$cm^{-1}$: 3420, 1690, 1650, 1340

NMR(90MHz,$D_2O$)ppm : 1.25(3H,d,J = 7Hz), 1.75-2.20(5H,m), 2.81(2H,d,J = 7Hz), 2.68-3.02(2H,m), 3.65-(2H,t,J = 7Hz), 3.50-4.43(3H,m), 5.93-6.64, 7.10-7.45(4H,m)

$[\alpha]_D^{26}$ -33.3° (c = 0.15, water)

SIMS : m/e 340($M^+$)

Example 179-183 (Table 17)

By processes similar to Step A and B of Example 178, the compounds of Examples 179 -183 were obtained. The respective yields and some of the physico-chemical properties of those compounds are shown in the table 17.

Table 17

| Ex. No. | Starting Compound No. | Isolated Compound No. | Yield (%) | Melting Point (℃) | $[\alpha]_D$ solvent (℃, C %) |
|---|---|---|---|---|---|
| 179 | 167a | 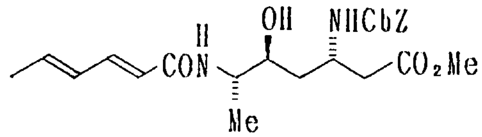 179-1 | 58 | 136 – 140 | -10.4° (chloroform, 23, 0.135) |
|  | 179-1 | 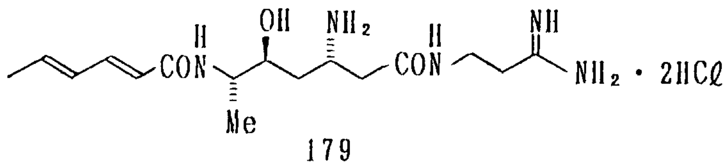 179 | 17 | lyophilized product 1) (not determined) | 1) (not determined) |
| 180 | 169 | 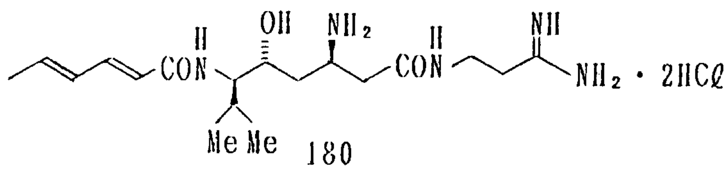 180 | 25 | lyophilized product (not determined) | +40.0° (water, 25, 0.15) |

1) 179-1; IR $\nu_{max}$ (KBr)$cm^{-1}$: 3260, 1685, 1650, 1630

NMR(90MHz, $D_2O$)ppm: 1.28(3H, d, J=7Hz), 1.68-2.12(2H, m), 1.90(3H, d, J=5Hz), 2.77(2H, t, J=9Hz), 2.80(2H, d, J=7Hz), 3.53-4.35(5H, m), 5.96-6.48, 7.10-7.40(4H, m)

| Ex. No. | Starting Compound No. | Isolated Compound No. | Yield (%) | Melting Point (℃) | [α]D (solvent ℃ C %) |
|---|---|---|---|---|---|
| 181 | 170a | 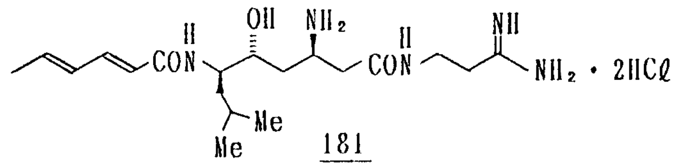 181 | 21 | lyophilized product (not determined) | + 24.8° (water 24 0.125) |
| 182 | 171a | 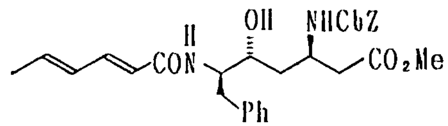 182-1 | 70 | 152 – 155 | + 21.5° (chloroform 24 0.2) |
|  | 182-1 | 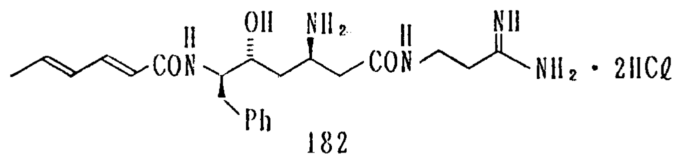 182 | 50 | lyophilized product (not determined) | + 46.9° (water 24 0.30) |

| Ex. No. | Starting Compound No. | Isolate Compound No. | Yield (%) | Melting Point (℃) | $[\alpha]_D$ (solvent ℃ C %) |
|---|---|---|---|---|---|
| 183 | 171b | 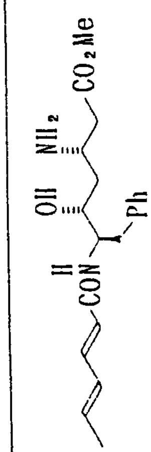 183-1 | 66 | 138 – 141 | + 59.5° (chloroform 24 0.185) |
| | 183-1 | 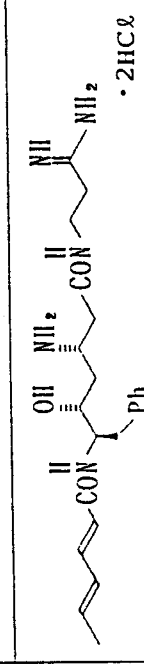 183 | 38 | lyophilized product (not determined) | + 4.6° (water 26 1.375) |

## Claims

**Claims for the following Contracting States : AT, BE, CH, DE, FR, GB, IT, LI, LU, NL, SE**

**1.** A compound of the formula

```
R1-CH-CH-CH2-CH-CH2-CO-R5
   |  |      |
   R2 OR3    R4
```

wherein

- $R^1$ is 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,
- $R^2$ and $R^3$ are hydrogen,
- $R^4$ is amino, methylamino, ethylamino, glycylamino, serylamino, alanylamino or 2-amino-O-methylpimelylamino,
- $R^5$ is 2-amidinoethylamino, 2-amidinopropylamino or 2-(N-methyl)amidinoethylamino or a salt thereof.

**2.** A method of preparing a compound of the formula

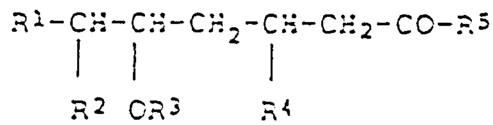

wherein

- $R^1$ is 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,
- $R^2$ and $R^3$ are hydrogen,
- $R^4$ is amino, methylamino, ethylamino, glycylamino, serylamino, alanylamino or 2-amino-O-methylpimelylamino,
- $R^5$ is 2-amidinoethylamino, 2-amidinopropylamino or 2-(N-methyl)amidinoethylamino, or a salt thereof,

which comprises allowing a compound of the formula

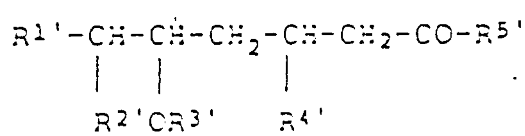

wherein $R^{2'}$ and $R^{3'}$ are hydrogen, $R^{1'}$ is amino or of the same meaning as $R^1$, and $R^{4'}$ is of the same meaning as $R^4$,

(a) to react with a compound capable of introducing

- for $R^1$ 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,
- for $R^4$ amino, methylamino, ethylamino, glycylamino, serylamino, alanylamino or 2-amino-O-methylpimelylamino

into a compound wherein at least one of $R^{1'}$ and $R^{4'}$ is amino or

(b) to react with a compound capable of introducing

- for $R^5$ 2-amidinoethylamino, 2-amidinopropylamino or 2-(N-methyl)amidinoethylamino

into a compound wherein $R^{5'}$ is hydroxyl or a reactive derivative thereof at the carboxyl group.

**Claim for the following Contracting States : ES, GR**

**1.** A method of preparing a compound of the formula

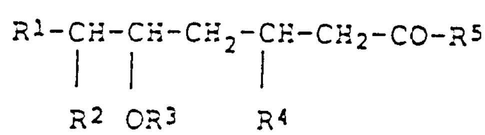

wherein

- $R^1$ is 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,
- $R^2$ and $R^3$ are hydrogen,
- $R^4$ is amino, methylamino, ethylamino, glycylamino, serylamino, alanylamino or 2-amino-O-methylpimelylamino,
- $R^5$ is 2-amidinoethylamino, 2-amidinopropylamino or 2-(N-methyl)amidinoethylamino, or a salt thereof,

which comprises allowing a compound of the formula

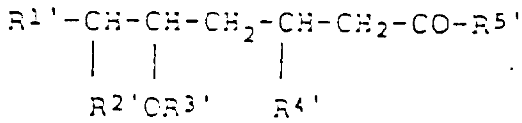

wherein $R^{2'}$ and $R^{3'}$ are hydrogen, $R^{1'}$ is amino or of the same meaning as $R^1$, and $R^{4'}$ is of the same meaning as $R^4$,

(a) to react with a compound capable of introducing

- for $R^1$ 2-methyl-2,4-pentadienoylamino, 2,4-pentadienoylamino, 2,4-hexadienoylamino, 2-methyl-2,4-hexadienoylamino, 6-fluoro-2,4-hexadienoylamino or 5-chloro-2,4-pentadienoylamino,
- for $R^4$ amino, methylamino, ethylamino, glycylamino, serylamino, alanylamino or 2-amino-O-methylpimelylamino

into a compound wherein at least one of $R^{1'}$ and $R^{4'}$ is amino or

(b) to react with a compound capable of introducing

- for $R^5$ 2-amidinoethylamino, 2-amidinopropylamino or 2-(N-methyl)amidinoethylamino

into a compound wherein $R^{5'}$ is hydroxyl or a reactive derivative thereof at the carboxyl group.

## Patentansprüche

**Patentansprüche für folgende Vertragsstaaten : AT, BE, CH, DE, FR, GB IT, LI, LU, NL, SE**

**1.** Verbindung der Formel

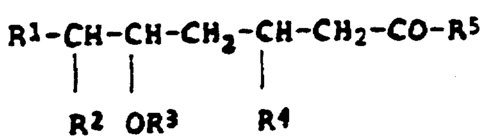

worin

- $R^1$ 2-Methyl-2,4-pentadienoylamino, 2,4-Pentadienoylamino, 2,4-Hexadienoylamino, 2-Methyl-2,4-hexadienoylamino, 6-Fluor-2,4-hexadienoylamino oder 5-Chlor-2,4-pentadienoylamino ist,
- $R^2$ und $R^3$ Wasserstoff sind,
- $R^4$ Amino, Methylamino, Äthylamino, Glycylamino, Serylamino, Alanylamino oder 2-Amino-O-methylpimelylamino ist,
- $R^5$ 2-Amidinoäthylamino, 2-Amidinopropylamino oder 2-(N-Methyl)amidinoäthylamino oder ein Salz davon ist.

**2.** Verfahren zur Herstellung einer Verbindung der Formel

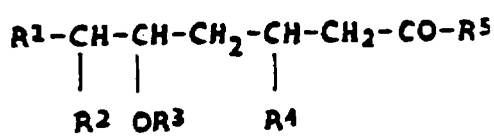

worin

- $R^1$ 2-Methyl-2,4-pentadienoylamino, 2,4-Pentadienoylamino, 2,4-Hexadienoylamino, 2-Methyl-2,4-hexadienoylamino, 6-Fluor-2,4-hexadienoylamino oder 5-Chlor-2,4-pentadienoylamino ist,
- $R^2$ und $R^3$ Wasserstoff sind,
- $R^4$ Amino, Methylamino, Äthylamino, Glycylamino, Serylamino, Alanylamino oder 2-Amino-O-methylpimelylamino ist,
- $R^5$ 2-Amidinoäthylamino, 2-Amidinopropylamino oder 2-(N-Methyl)amidinoäthylamino oder ein Salz davon ist,

umfassend das Umsetzenlassen einer Verbindung der Formel

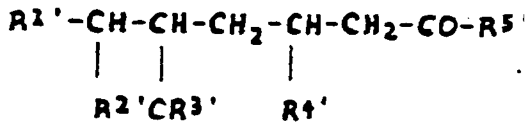

worin $R^{2'}$ und $R^{3'}$ Wasserstoff sind, $R^{1'}$ Amino ist oder die gleiche Bedeutung wie $R^1$ hat, und $R^{4'}$ die gleiche Bedeutung wie $R^4$ hat,

(a) mit einer Verbindung, die fähig ist,

für $R^1$ 2-Methyl-2,4-pentadienoylamino, 2,4-Pentadienoylamino, 2,4-Hexadienoylamino, 2-Methyl-2,4-hexadienoylamino, 6-Fluor-2,4-hexadienoylamino oder 5-Chlor-2,4-pentadienoylamino,

für $R^4$ Amino, Methylamino, Äthylamino, Glycylamino, Serylamino, Alanylamino oder 2-Amino-O-methylpimelylamino

in eine Verbindung einzubringen, worin zumindest eines aus $R^{1'}$ und $R^{4'}$ Amino ist, oder

(b) mit einer Verbindung, die fähig ist,

für $R^5$ 2-Amidinoäthylamino, 2-Amidinopropylamino oder 2-(N-Methyl)amidinoäthylamino

in eine Verbindung einzubringen, worin $R^{5'}$ Hydroxyl oder ein reaktionsfähiges Derivat davon an der Carboxylgruppe ist.

**Patentanspruch für folgende Vertragsstaaten : ES, GR**

**1.** Verfahren zur Herstellung einer Verbindung der Formel

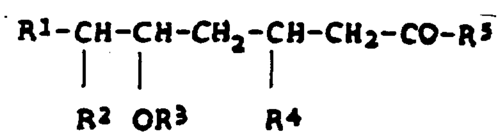

worin

- $R^1$ 2-Methyl-2,4-pentadienoylamino, 2,4-Pentadienoylamino, 2,4-Hexadienoylamino, 2-Methyl-2,4-hexadienoylamino, 6-Fluor-2,4-hexadienoylamino oder 5-Chlor-2,4-pentadienoylamino ist,
- $R^2$ und $R^3$ Wasserstoff sind,
- $R^4$ Amino, Methylamino, Äthylamino, Glycylamino, Serylamino, Alanylamino oder 2-Amino-O-methylpimelylamino ist,
- $R^5$ 2-Amidinoäthylamino, 2-Amidinopropylamino oder 2-(N-Methyl)amidinoäthylamino ist, oder eines Salzes davon,

umfassend das Umsetzenlassen einer Verbindung der Formel

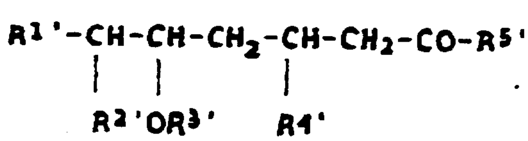

worin $R^{2'}$ und $R^{3'}$ Wasserstoff sind, $R^{1'}$ Amino ist oder die gleiche Bedeutung wie $R^1$ hat, und $R^{4'}$ die

gleiche Bedeutung wie $R^4$ hat,

(a) mit einer Verbindung, die fähig ist,

für $R^1$ 2-Methyl-2,4-pentadienoylamino, 2,4-Pentadienoylamino, 2,4-Hexadienoylamino, 2-Methyl-2,4-hexadienoylamino, 6-Fluor-2,4-hexadienoylamino oder 5-Chlor-2,4-pentadienoylamino,

für $R^4$ Amino, Methylamino, Äthylamino, Glycylamino, Serylamino, Alanylamino oder 2-Amino-O-methylpimelylamino

in eine Verbindung einzubringen, worin zumindest eines aus $R^{1'}$ und $R^{4'}$ Amino ist, oder

(b) mit einer Verbindung, die fähig ist,

für $R^5$ 2-Amidinoäthylamino, 2-Amidinopropylamino oder 2-(N-Methyl)amidinoäthylamino

in eine Verbindung einzubringen, worin $R^{5'}$ Hydroxyl oder ein reaktionsfähiges Derivat davon an der Carboxylgruppe ist.

## Revendications

## Revendications pour les Etats contractants suivants : AT, BE, CH, DE, FR, GB, IT, LI, LU, NL, SE

**1.** Composé de formule :

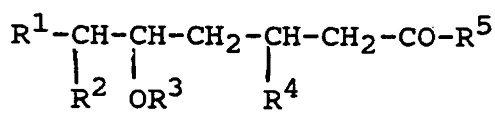

dans laquelle :

$R^1$ représente un groupe 2-méthyl-2,4-pentadiénoylamino, 2,4-pentadiénoylamino, 2,4-hexadiénoylamino, 2-méthyl-2,4-hexadiénoylamino, 6-fluoro-2,4-hexadiénoylamino ou 5-chloro-2,4-pentadiénoylamino,

$R^2$ et $R^3$ représentent des atomes d'hydrogène,

$R^4$ représente un groupe amino, méthylamino, éthylamino, glycylamino, sérylamino, alanylamino ou 2-amino-O-méthylpimélylamino,

$R^5$ représente un groupe 2-amidinoéthylamino, 2-amidinopropylamino ou 2-(N-méthyl)-amidinoéthylamino,

ou un sel d'un tel composé.

**2.** Procédé de préparation d'un composé de formule :

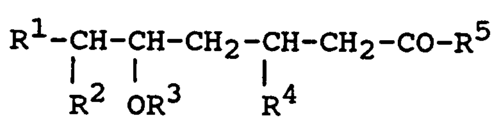

dans laquelle

$R^1$ représente un groupe 2-méthyl-2,4-pentadiénoylamino, 2,4-pentadiénoylamino, 2,4-hexadiénoylamino, 2-méthyl-2,4-hexadiénoylamino, 6-fluoro-2,4-hexadiénoylamino ou 5-chloro-2,4-pentadiénoylamino,

$R^2$ et $R^3$ représentent des atomes d'hydrogène,

$R^4$ représente un groupe amino, méthylamino, éthylamino, glycylamino, sérylamino, alanylamino ou 2-amino-O-méthylpimélylamino,

$R^5$ représente un groupe 2-amidinoéthylamino, 2-amidinopropylamino ou 2-(N-méthyl)-amidinoéthylamino,

ou d'un sel d'un tel composé,

qui comprend l'étape consistant à faire réagir un composé de formule :

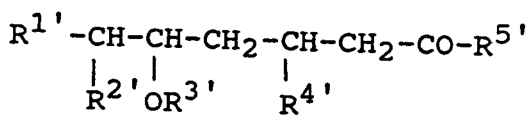

dans laquelle $R^{2'}$ et $R^{3'}$ représentent des atomes d'hydrogène, $R^{1'}$ représente un groupe amino ou a la même signification que $R^1$, et $R^{4'}$ a la même signification que $R^4$,
avec (a) un composé capable d'introduire :
pour $R^1$, un groupe 2-méthyl-2,4-pentadiénoylamino, 2,4-pentadiénoylamino, 2,4-hexadiénoylamino, 2-méthyl-2,4-hexadiénoylamino, 6-fluoro-2,4-hexadiénoylamino ou 5-chloro-2,4-pentadiénoylamino,
pour $R^4$, un groupe amino, méthylamino, éthylamino, glycylamino, sérylamino, alanylamino ou 2-amino-O-méthylpimélylamino,
dans un composé dans lequel au moins l'un de $R^{1'}$ et $R^{4'}$ est un groupe amino,
ou avec (b) un composé capable d'introduire, pour $R^5$, un groupe 2-amidinoéthylamino, 2-amidinopropylamino ou 2-(N-méthyl)amidinoéthylamino dans un composé dans lequel $R^{5'}$ représente un groupe hydroxyle ou dans un composé réactif, dérivant du précédent par transformation du groupe carboxyle.

**Revendication pour les Etats contractants suivants : ES, GR**

**1.** Procédé de préparation d'un composé de formule :

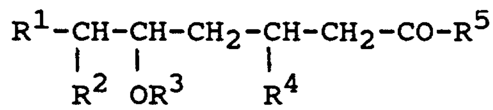

dans laquelle
$R^1$ représente un groupe 2-méthyl-2,4-pentadiénoylamino, 2,4-pentadiénoylamino, 2,4-hexadiénoylamino, 2-méthyl-2,4-hexadiénoylamino, 6-fluoro-2,4-hexadiénoylamino ou 5-chloro-2,4-pentadiénoylamino,
$R^2$ et $R^3$ représentent des atomes d'hydrogène,
$R^4$ représente un groupe amino, méthylamino, éthylamino, glycylamino, sérylamino, alanylamino ou 2-amino-O-méthylpimélylamino,
$R^5$ représente un groupe 2-amidinoéthylamino, 2-amidinopropylamino ou 2-(N-méthyl)-amidinoéthylamino,
ou d'un sel d'un tel composé,
qui comprend l'étape consistant à faire réagir un composé de formule :

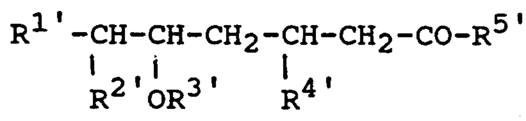

dans laquelle $R^{2'}$ et $R^{3'}$ représentent des atomes d'hydrogène, $R^{1'}$ représente un groupe amino ou a la même signification que $R^1$, et $R^{4'}$ a la même signification que $R^4$,
avec (a) un composé capable d'introduire :
pour $R^1$, un groupe 2-méthyl-2,4-pentadiénoylamino, 2,4-pentadiénoylamino, 2,4-hexadiénoylamino, 2-méthyl-2,4-hexadiénoylamino, 6-fluoro-2,4-hexadiénoylamino ou 5-chloro-2,4-pentadiénoylamino,
pour $R^4$, un groupe amino, méthylamino, éthylamino, glycylamino, sérylamino, alanylamino ou 2-amino-O-méthylpimélylamino,
dans un composé dans lequel au moins l'un de $R^{1'}$ et $R^{4'}$ est un groupe amino,
ou avec (b) un composé capable d'introduire, pour $R^5$, un groupe 2-amidinoéthylamino, 2-amidinopropylamino ou 2-(N-méthyl)amidinoéthylamino dans un composé dans lequel $R^{5'}$ représente un groupe hydroxyle ou dans un composé réactif, dérivant du précédent par transformation du groupe carboxyle.